# EUROPEAN PATENT APPLICATION

(11) **EP 3 804 759 A1**
(43) Date of publication of application: **14.04.2021**
(21) Application number: 19814696.1
(22) Date of filing: 06.06.2019
(51) Int. Cl.: A61K 45/00, A61P 9/00, A61P 11/00, A61P 11/06, A61P 13/12, A61P 17/00, A61P 17/06, A61P 19/02, A61P 25/00, A61P 29/00, A61P 31/14, A61P 37/06, A61P 37/08, A61P 43/00, C07K 16/40, C12Q 1/48, C12N 15/52

(54) **METHOD FOR TREATING AND/OR PREVENTING REGNASE-1-RELATED DISEASE**

(30) Priority: 06.06.2018 JP 2018108400; 06.09.2018 JP 2018167326; 07.12.2018 JP 2018229845; 09.05.2019 JP 2019089270
(71) Applicant: Osaka University, Suita-shi, Osaka 565-0871 (JP); CHUGAI SEIYAKU KABUSHIKI KAISHA, Tokyo 115-8543 (JP)
(72) Inventor: AKIRA, Shizuo, Suita-shi, Osaka 565-0871 (JP); SATOH Takashi, Suita-shi, Osaka 565-0871 (JP); TANAKA, Hiroki, Suita-shi, Osaka 565-0871 (JP); SAITO, Keiko, Kamakura-shi, Kanagawa 247-8530 (JP); YAMAGISHI, Yusuke, Synapse, 138623 (SG)
(74) Representative: Vossius & Partner Patentanwälte Rechtsanwälte mbB
(86) International application number: PCT/JP2019/022582
(87) International publication number: WO 2019/235581

(57) **Abstract**

The present invention found that, for example, inhibiting phosphorylation of a Ser residue in Regnase-1 is effective in treating and/or preventing diseases. The invention also found that, for example, inhibiting the binding of Regnase-1 with at least one factor selected from the group consisting of TBK1, IKKi, Act-1, IKK, and IRAK is effective in treating and/or preventing diseases.

## Description

### [Technical Field]

The present invention relates to methods for treating and/or preventing diseases associated with Regnase-1.

### [Background Art]

Regnase-1 (also known as "Zc3h12a" or "MCPIP-1" and sometimes referred to as such herein) is a nuclease that is a member of the Regnase family with a CCCH type zinc finger domain and a PIN-like domain and that recognizes and degrades mRNAs (NPL 1). Regnase-1 destabilizes the mRNAs of interleukin (IL)-6 and IL-12p40 and is involved in the post-transcriptional regulation thereof (NPL 2). It was reported that Ser(serine)435 and Ser439 in mouse Regnase-1 were phosphorylation sites by IκB kinase (IKK) induced by IL-1β stimulation, that Regnase-1 where these amino acid residues were replaced with Ala (alanine) had resistance to degradation by IL-1β stimulation, and that the expression of IL-6 mRNA after IL-1β stimulation was suppressed in cells expressing Regnase-1 with the alanine replacements compared to cells expressing wild-type Regnase-1 (NPL 3). In the model experiments of experimental autoimmune encephalomyelitis and psoriasis, Regnase-1 heterozygous KO mice were reported to have deteriorated conditions (NPLs 4 and 5).

### [Citation List]

### [Non-Patent Literature]

[NPL 1] Akira, S. Regnase-1, a ribonuclease involved in the regulation of immune responses. Cold Spring Harbor symposia on quantitative biology 78, 51-60 (2013).
[NPL 2] Matsushita, K. et al. Zc3h12a is an RNase essential for controlling immune responses by regulating mRNA decay. Nature 458, 1185-1190 (2009).
[NPL 3] Iwasaki, H. et al. The IkappaB kinase complex regulates the stability of cytokine-encoding mRNA induced by TLR-IL-1R by controlling degradation of regnase-1. Nat Immunol 12, 1167-1175 (2011).
[NPL 4] Garg, A.V. et al. MCPIP1 Endoribonuclease Activity Negatively Regulates Interleukin-17-Mediated Signaling and Inflammation. Immunity 43, 475-487 (2015).
[NPL 5] L. Monin et al. MCPIP1/Regnase-1 Restricts IL-17A- and IL-17C-Dependent Skin Inflammation. J. Immunol. 198, 767-775 (2017).

### [Summary of Invention]

### [Technical Problem]

However, methods of treating or preventing inflammatory diseases, autoimmune diseases, allergic diseases, fibrotic diseases, and such by inhibiting phosphorylation of Regnase-1 were not known. In one aspect, an objective of the present invention is to provide treatment methods and such of diseases by inhibiting phosphorylation of Regnase-1.

### [Solution to Problem]

To solve the above problem, the present inventors searched for kinases capable of phosphorylating Regnase-1 and discovered the amino acid residues of Regnase-1 that were phosphorylated by these kinases. The present inventors further discovered that inhibition of phosphorylation of specific residues among these amino acid residues was effective in the treatment and/or prevention of inflammatory diseases, autoimmune diseases, allergic diseases, fibrotic diseases, and such, which led to the completion of the present invention.

In a non-limiting specific embodiment, the present invention includes the following:
[a1] A method for treating and/or preventing a disease associated with Regnase-1 by inhibiting phosphorylation of a Ser residue in a Regnase-1 selective manner.
[a2] A method for suppressing inflammation by inhibiting phosphorylation of a Ser residue in a Regnase-1 selective manner.
[a3] A method for suppressing fibrosis of a cell, tissue, or organ; or epithelial hyperplasia by inhibiting phosphorylation of a Ser residue in a Regnase-1 selective manner.
[a4] A method for suppressing destabilization and/or intracellular degradation of Regnase-1 by inhibiting phosphorylation of a Ser residue in a Regnase-1 selective manner.
[a5] The method of [a4], wherein the destabilization and/or intracellular degradation of Regnase-1 is destabilization and/or intracellular degradation of Regnase-1 downstream of signaling associated with at least one molecule selected from the group consisting of IL-17, IL-1, IL-36, and a TLR ligand.
[a6] A method for suppressing production of inflammatory factors by inhibiting phosphorylation of a Ser residue in a Regnase-1 selective manner.
[a7] A method for suppressing expression of at least one mRNA selected from the group consisting of IL6, IL1a, CXCL1, CXCL2, HBEGF, CTGF, DDR1, and PDGFB by inhibiting phosphorylation of a Ser residue in a Regnase-1 selective manner.
[a8] The method of any one of [a1] to [a7], wherein the Ser residue is a Ser residue at at least one position selected from the group consisting of positions corresponding respectively to positions 513, 494, 439, and 435 of SEQ ID NO: 1 in Regnase-1.
[a9] The method of any one of [a1] to [a8], wherein the positions corresponding respectively to positions 513, 494, 439, and 435 of SEQ ID NO: 1 are (i) positions 513, 494, 439, and 435 of SEQ ID NO: 1; or (ii) positions 516, 497, 442, and 438 of SEQ ID NO: 2.
[a10] The method of any one of [a1] to [a9], wherein the Ser residue is a Ser residue comprised in at least one amino acid sequence selected from the group consisting of YWSEP (SEQ ID NO: 3), HFSVP (SEQ ID NO: 4), and DSGIGS (SEQ ID NO: 5) comprised in an amino acid sequence of Regnase-1.
[a11] The method of any one of [a1] to [a10], wherein the Regnase-1 is a mammalian Regnase-1.
[a12] The method of any one of [a1] to [a11], wherein the Ser residue is a Ser residue in (i) and (ii) below:
   (i) a Ser residue at either or both positions corresponding respectively to positions 513 and 494 of SEQ ID NO: 1 in Regnase-1; and
   (ii) a Ser residue at either or both positions corresponding respectively to positions 439 and 435 of SEQ ID NO: 1 in Regnase-1.

In a non-limiting specific embodiment, the present invention also includes the following:
[A1] A method for treating and/or preventing a disease associated with Regnase-1 by inhibiting the binding between Regnase-1 and at least one binding molecule selected from the group consisting of TBK1, IKKi, Act-1, IKK, and IRAK
[A2] A method for suppressing inflammation by inhibiting the binding between Regnase-1 and at least one binding molecule selected from the group consisting of TBK1, MKi, Act-1, IKK, and IRAK
[A3] A method for suppressing fibrosis of a cell, tissue, or organ; or epithelial hyperplasia by inhibiting the binding between Regnase-1 and at least one binding molecule selected from the group consisting of TBK1, MKi, Act-1, IKK, and IRAK
[A4] A method for suppressing destabilization and/or intracellular degradation of Regnase-1 by inhibiting the binding between Regnase-1 and at least one binding molecule selected from the group consisting of TBK1, IKKi, Act-1, IKK, and IRAK
[A5] The method of [A4], wherein the destabilization and/or intracellular degradation of Regnase-1 is destabilization and/or intracellular degradation of Regnase-1 downstream of signaling associated with at least one molecule selected from the group consisting of IL-17, IL-1, IL-36, and a TLR ligand.
[A6] A method for suppressing production of inflammatory factors by inhibiting the binding between Regnase-1 and at least one binding molecule selected from the group consisting of TBK1, IKKi, Act-1, IKK, and IRAK
[A7] A method for suppressing expression of at least one mRNA selected from the group consisting of IL6, IL1a, CXCL1, CXCL2, HBEGF, CTGF, DDR1, and PDGFB by inhibiting the binding between Regnase-1 and at least one binding molecule selected from the group consisting of TBK1, IKKi, Act-1, IKK, and IRAK
[A8] The method of any one of [A1] to [A7], wherein phosphorylation of a Ser residue at at least one position selected from the group consisting of positions corresponding respectively to positions 513, 494, 439, and 435 of SEQ ID NO: 1 in Regnase-1 is inhibited.
[A9] The method of any one of [A1] to [A8], wherein the at least one binding molecule selected from the group consisting of TBK1, IKKi, Act-1, IKK, and IRAK is at least one binding molecule selected from the group consisting of TBK1, IKKi, and Act-1.
[A10] The method of any one of [A1] to [A9], wherein the Regnase-1 is a mammalian Regnase-1.
[A11] The method of any one of [A1] to [A10], wherein the at least one binding molecule selected from the group consisting of TBK1, IKKi, Act-1, IKK, and IRAK is TBK1 and IKK.

In a non-limiting specific embodiment, the present invention also includes the following:
[B1] A composition for treating and/or preventing a disease associated with Regnase-1, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.
[B2] A composition for treating and/or preventing a disease associated with Regnase-1, comprising a Regnase-1-binding molecule that inhibits phosphorylation of a Ser residue comprised in at least one amino acid sequence selected from the group consisting of YWSEP (SEQ ID NO: 3), HFSVP (SEQ ID NO: 4), and DSGIGS (SEQ ID NO: 5) comprised in an amino acid sequence of Regnase-1.
[B3] A composition for treating and/or preventing a disease associated with Regnase-1, comprising a Regnase-1-binding molecule that inhibits binding between Regnase-1 and at least one binding molecule selected from the group consisting of TBK1, IKKi, Act-1, IKK, and IRAK.
[B4] The composition of [B3], wherein phosphorylation of a Ser residue at at least one position selected from the group consisting of positions corresponding respectively to positions 513, 494, 439, and 435 of SEQ ID NO: 1 in Regnase-1 is inhibited.
[B5] The composition of any one of [B1] to [B4], wherein the disease associated with Regnase-1 is at least one disease selected from the group consisting of an inflammatory disease, autoimmune disease, allergic disease, fibrotic disease, and RNA virus infection.
[B6] The composition of any one of [B1] to [B5], wherein the disease associated with Regnase-1 is a disease the formation, exacerbation, and/or continuation of which are associated with at least one factor selected from the group consisting of IL6, IL1a, CXCL1, CXCL2, HBEGF, CTGF, DDR1, and PDGFB.
[B7] The composition of any one of [B1] to [B6], wherein the disease associated with Regnase-1 is a disease the formation, exacerbation, and/or continuation of which are associated with at least one factor selected from the group consisting of IL-17, IL-1, IL-36, and a TLR ligand.
[B8] The composition of any one of [B1] to [B7], wherein the disease associated with Regnase-1 is a disease in at least one tissue or organ selected from the group consisting of kidney, lung, skin, blood vessel, eye, brain, and nerve.
[B9] The composition of any one of [B1] to [B8], wherein the disease associated with Regnase-1 is a TH17 cell-related disease.
[B10] The composition of any one of [B1] to [B9], wherein the disease associated with Regnase-1 is at least one disease selected from the group consisting of multiple sclerosis, psoriasis, scleroderma, nephritis, uveitis, pulmonary fibrosis, renal fibrosis, vascular fibrosis, keloid, rheumatoid arthritis, systemic lupus erythematosus, Sjögren's syndrome, pneumonia, dermatitis, vasculitis, neuritis, arthritis, eye inflammation, encephalomyelitis, and asthma.
[B11] The composition of any one of [B1] to [B10], wherein the disease associated with Regnase-1 is a disease accompanied by fibrosis of a cell, tissue, or organ.
[B12] The composition of any one of [B1] to [B10], wherein the disease associated with Regnase-1 is a disease accompanied by epithelial hyperplasia.
[B13] The composition of any one of [B1] to [B12], wherein the composition is for suppressing expression of at least one mRNA selected from the group consisting of IL6, IL1a, CXCL1, CXCL2, HBEGF, CTGF, DDR1, and PDGFB.
[B14] The composition of any one of [B1] to [B13], wherein the composition is for suppressing production of inflammatory factors.
[B15] A composition for suppressing inflammation, comprising a Regnase-1-binding molecule that inhibits phosphorylation of a Ser residue at at least one position selected from the group consisting of positions corresponding respectively of positions 513, 494, 439, and 435 of SEQ ID NO: 1 in Regnase-1.
[B16] A composition for suppressing fibrosis of a cell, tissue, or organ, or epithelial hyperplasia, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.
[B17] A composition for suppressing expression of at least one mRNA selected from the group consisting of IL6, IL1a, CXCL1, CXCL2, HBEGF, CTGF, DDR1, and PDGFB, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.
[B18] A composition for suppressing production of inflammatory factors, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.
[B19] The composition of any one of [B1] to [B18], wherein the positions corresponding respectively to positions 513, 494, 439, and 435 of SEQ ID NO: 1 are (i) positions 513, 494, 439, and 435 of SEQ ID NO: 1; or (ii) positions 516, 497, 442, and 438 of SEQ ID NO: 2.
[B20] The composition of any one of [B1] to [B19], comprising a Regnase-1-binding molecule that inhibits phosphorylation of a Ser residue at at least one position selected from the group consisting of positions corresponding respectively to positions 513 and 494 of SEQ ID NO: 1.
[B21] The composition of [B20], wherein the positions corresponding respectively to positions 513 and 494 of SEQ ID NO: 1 are (i) positions 513 and 494 of SEQ ID NO: 1; or (ii) positions 516 and 497 of SEQ ID NO: 2.
[B22] The composition of any one of [B3] to [B21], wherein the at least one binding molecule selected from the group consisting of TBK1, IKKi, Act-1, IKK, and IRAK is at least one binding molecule selected from the group consisting of TBK1, IKKi, and Act-1.
[B23] The composition of any one of [B1] to [B22], wherein the Regnase-1 is a mammalian Regnase-1.
[B24] The composition of any one of [B1] to [B23], wherein the Ser residue at at least one position selected from the group consisting of positions corresponding respectively to positions 513, 494, 439, and 435 of SEQ ID NO: 1 in Regnase-1 is a Ser residue in (i) and (ii) below:
   (i) a Ser residue at either or both positions corresponding respectively to positions 513 and 494 of SEQ ID NO: 1 in Regnase-1; and
   (ii) a Ser residue at either or both positions corresponding respectively to positions 439 and 435 of SEQ ID NO: 1 in Regnase-1.
[B25] The composition of any one of [B1] to [B24], wherein the at least one binding molecule selected from the group consisting of TBK1, IKKi, Act-1, IKK, and IRAK is TBK1 and IKK.
[B26] The composition of any one of [B1] to [B25], wherein the Regnase-1-binding molecule is the Regnase-1-binding molecule of any one of [H1] to [H24].

In a non-limiting specific embodiment, the present invention also includes the following:
[D1] A method for identifying a substance that inhibits phosphorylation of Regnase-1, wherein phosphorylation of a Ser residue at at least one position selected from the group consisting of positions corresponding respectively to positions 513, 494, 439, and 435 of SEQ ID NO: 1 is used as an index.
[D2] The method of [D1], comprising the steps of (a) and (b) below:
   (a) mixing a kinase capable of phosphorylating Regnase-1 with Regnase-1 in the presence of a test substance and detecting phosphorylation of Regnase-1 by the kinase;
   (b) identifying a substance that inhibits the phosphorylation of Regnase-1 by the kinase as compared to the absence of the test substance.
[D3] The method of [D1] or [D2], wherein the kinase is a kinase capable of phosphorylating a Ser residue at at least one position selected from the group consisting of positions corresponding respectively to positions 513, 494, 439, and 435 of SEQ ID NO: 1.
[D4] The method of any one of [D1] to [D3], wherein the kinase is at least one kinase selected from the group consisting of TBK1, IKKi, IKK, and IRAK
[D5] The method of any one of [D1] to [D4], wherein the detecting of phosphorylation of Regnase-1 in step (a) is performed by using an antibody capable of detecting phosphorylation of a Ser residue at at least one position selected from the group consisting of positions corresponding respectively to positions 513, 494, 439, and 435 of SEQ ID NO: 1.
[D6] The method of any one of [D1] to [D5], wherein the Regnase-1 is human Regnase-1 and the positions corresponding respectively to positions 513, 494, 439, and 435 of SEQ ID NO: 1 are positions 516, 497, 442, and 438 of SEQ ID NO: 2.
[D7] The method of any one of [D1] to [D6], wherein phosphorylation of a Ser residue at at least one position selected from the group consisting of positions corresponding respectively to positions 513 and 494 of SEQ ID NO: 1 is used as an index.
[D8] The method of any one of [D2] to [D7], wherein the test substance is a Regnase-1-binding molecule.

In a non-limiting specific embodiment, the present invention also includes the following:
[E1] A composition for identifying a substance that inhibits phosphorylation of Regnase-1, wherein the composition comprises a predetermined amount of a kinase and Regnase-1.
[E2] The composition of [E1], wherein the kinase is at least one kinase selected from the group consisting of TBK1, IKKi, IKK, and IRAK
[E3] The composition of [E1] or [E2], wherein the phosphorylation of Regnase-1 is phosphorylation of a Ser residue at at least one position selected from the group consisting of positions corresponding respectively to positions 513, 494, 439, and 435 of SEQ ID NO: 1.
[E4] The composition of any one of [E1] to [E3], wherein the Regnase-1 is human Regnase-1 and the positions corresponding respectively to positions 513, 494, 439, and 435 of SEQ ID NO: 1 are positions 516, 497, 442, and 438 of SEQ ID NO: 2.
[E5] The composition of any one of [E1] to [E4], wherein the phosphorylation of Regnase-1 is phosphorylation of a Ser residue at at least one position selected from the group consisting of positions corresponding respectively to positions 513 and 494 of SEQ ID NO: 1.
[E6] The composition of any one of [E1] to [E5], further comprising a Regnase-1-binding molecule.

In a non-limiting specific embodiment, the present invention also includes the following:
[F1] A method for identifying a substance that inhibits binding between Regnase-1 and at least one binding molecule selected from the group consisting of TBK1, IKKi, Act-1, IKK, and IRAK, comprising the steps of (a) and (b) below:
   (a) mixing at least one binding molecule selected from the group consisting of TBK1, IKKi, Act-1, IKK, and IRAK with Regnase-1 in the presence of a test substance and measuring the binding activity between the binding molecule and Regnase-1;
   (b) identifying a substance capable of reducing the binding activity between the binding molecule and Regnase-1 as compared to the absence of the test substance.
[F2] The method of [F1], wherein the at least one binding molecule selected from the group consisting of TBK1, IKKi, Act-1, IKK, and IRAK is at least one binding molecule selected from the group consisting of TBK1, IKKi, and Act-1.
[F3] A composition for identifying a substance that inhibits binding between Regnase-1 and at least one binding molecule selected from the group consisting of TBK1, IKKi, Act-1, IKK, and IRAK, wherein the composition comprises a predetermined amount of Regnase-1 and the binding molecule.
[F4] A method for identifying a substance that inhibits binding between Regnase-1 and at least one binding molecule selected from the group consisting of TBK1, IKKi, Act-1, IKK, and IRAK and that inhibits phosphorylation of Regnase-1, comprising the method of any one of [D1] to [D8] and the method of [F1] or [F2].
[F5] The method of any one of [F1], [F2], and [F4], wherein the test substance is a Regnase-1-binding molecule.

In a non-limiting specific embodiment, the present invention also comprises the following:
[G1] An antibody that specifically recognizes Regnase-1 with a phosphorylated Ser residue at at least one position selected from the group consisting of positions corresponding respectively to positions 513, 494, 439, and 435 of SEQ ID NO: 1.
[G2] An antibody that specifically recognizes Regnase-1 with a phosphorylated Ser residue at at least one position selected from the group consisting of positions corresponding respectively to positions 513 and 494 of SEQ ID NO: 1.
[G3] An antibody that specifically recognizes Regnase-1 with a phosphorylated Ser residue corresponding to position 513 of SEQ ID NO: 1.
[G4] An antibody that specifically recognizes Regnase-1 with a phosphorylated Ser residue at at least one position selected from the group consisting of positions corresponding respectively to positions 439 and 435 of SEQ ID NO: 1.
[G5] An antibody that specifically recognizes Regnase-1 with phosphorylated Ser residues at positions corresponding respectively to positions 439 and 435 of SEQ ID NO: 1.
[G6] An antibody that specifically recognizes human Regnase-1 with a phosphorylated Ser residue at at least one position selected from positions 516, 497, 442, and 438 of SEQ ID NO: 2.
[G7] An antibody that specifically recognizes human Regnase-1 with a phosphorylated Ser residue at at least one position selected from positions 516 and 497 of SEQ ID NO: 2.
[G8] An antibody that specifically recognizes human Regnase-1 with a phosphorylated Ser residue at position 516 of SEQ ID NO: 2.
[G9] An antibody that specifically recognizes human Regnase-1 with a phosphorylated Ser residue at at least one position selected from positions 442 and 438 of SEQ ID NO: 2.
[G10] An antibody that specifically recognizes human Regnase-1 with phosphorylated Ser residues at positions 442 and 438 of SEQ ID NO: 2.
[G11] The antibody of any one of [G1] to [G10], wherein the antibody can bind to both human Regnase-1 and mouse Regnase-1.

In a non-limiting specific embodiment, the present invention also comprises the following:
[H1] A Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.
[H2] The Regnase-1-binding molecule of [H1], wherein the molecule competes for binding to Regnase-1 with at least one compound selected from PP1 to PP25, PP7+tag, PP10+tag, and PP23+tag shown below: 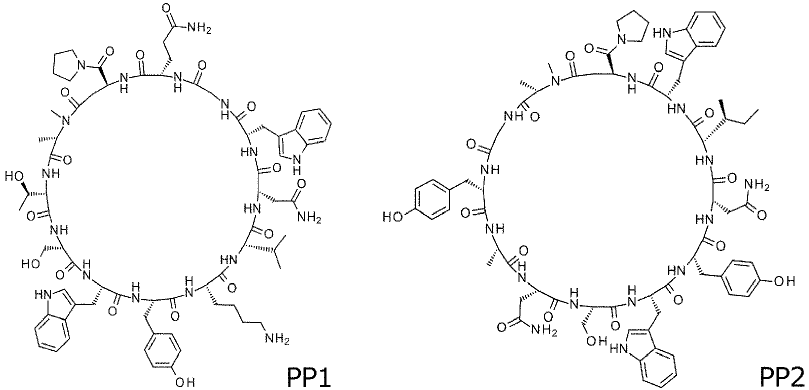 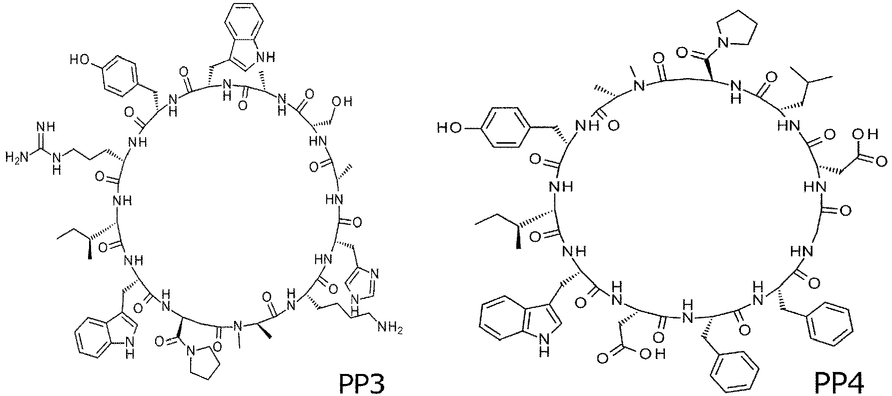 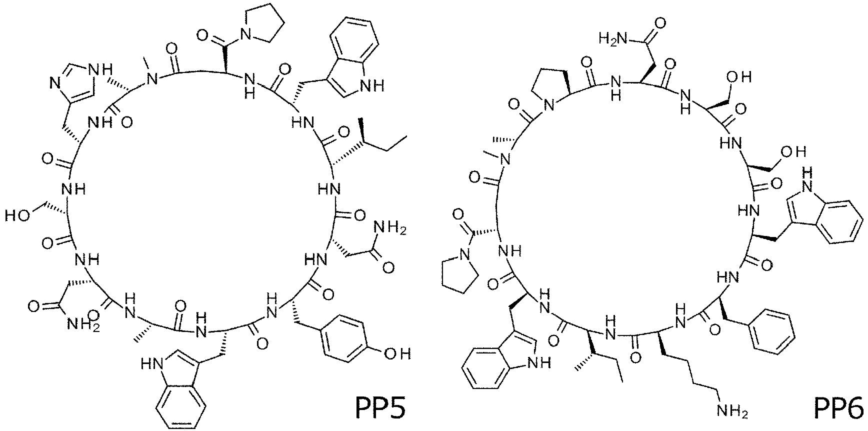 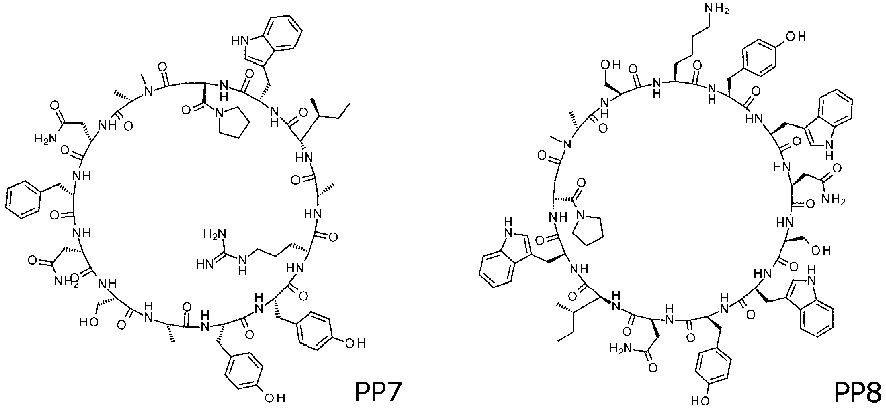 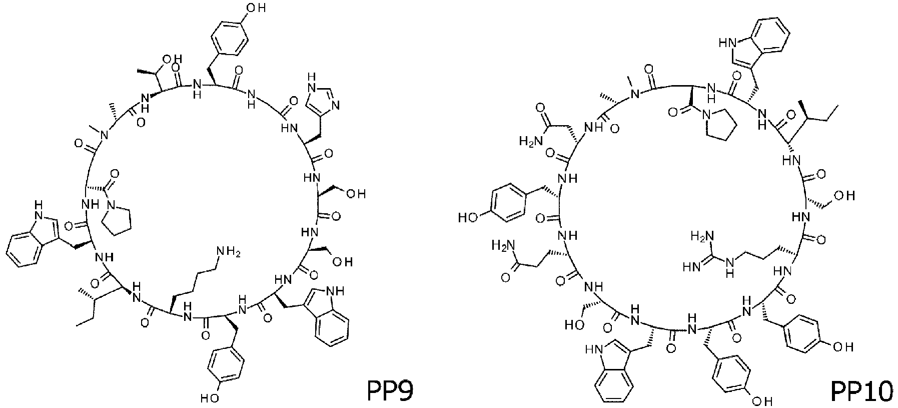 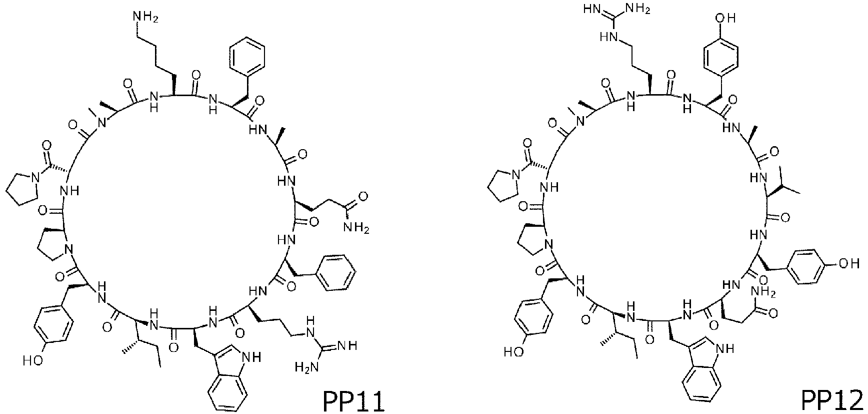 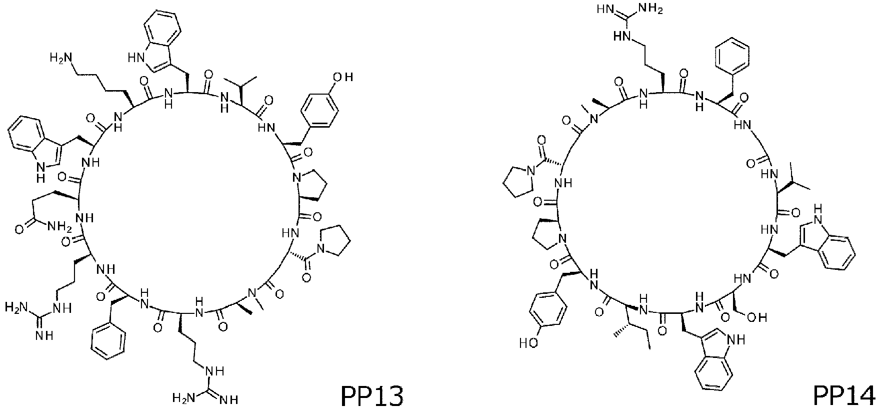 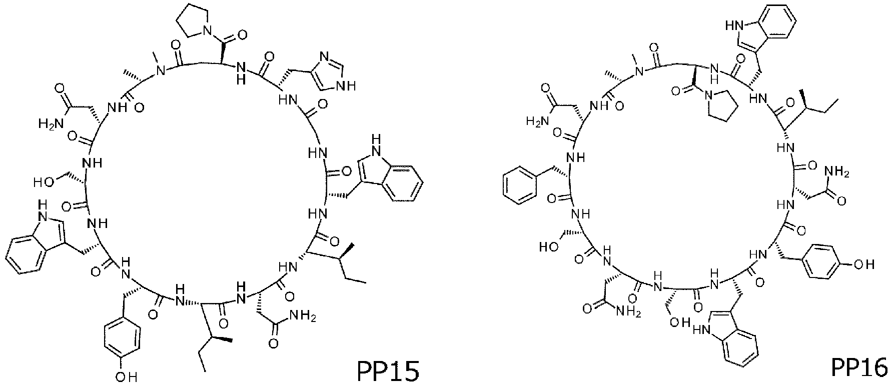 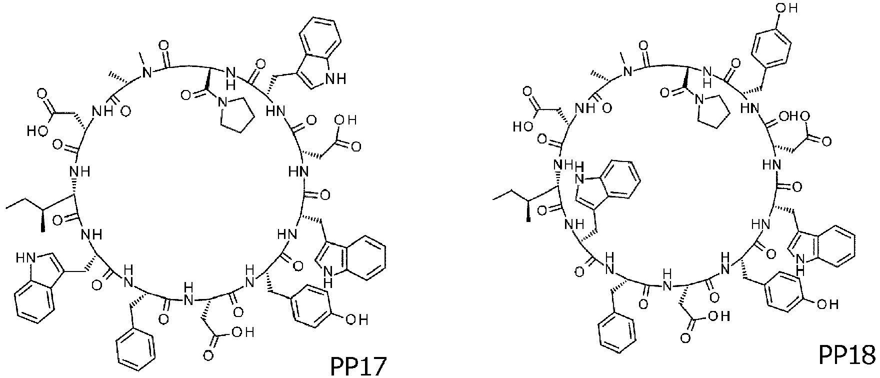 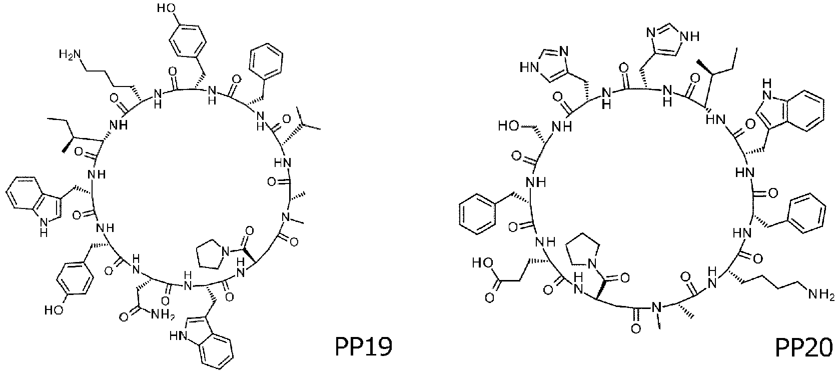 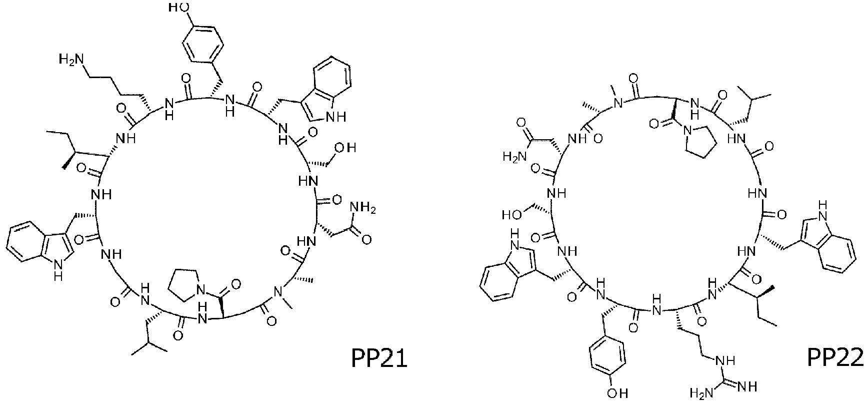 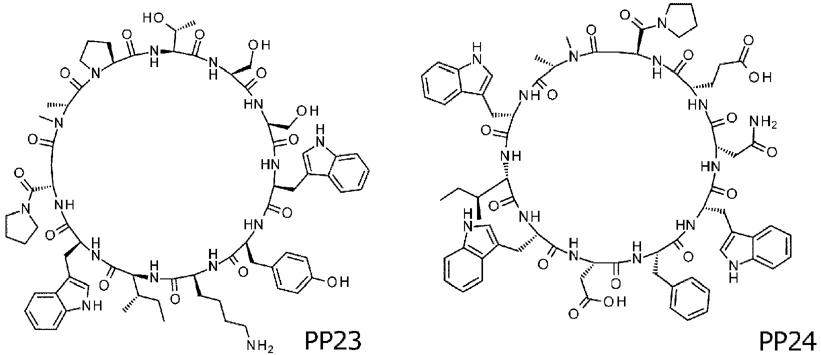 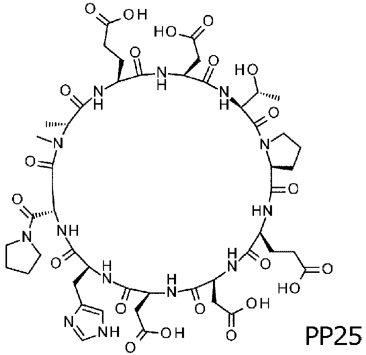 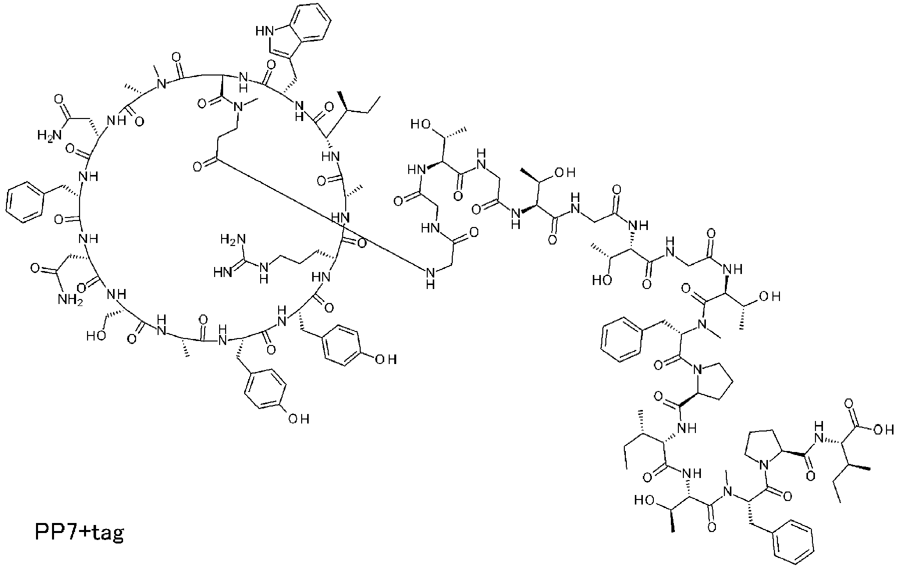 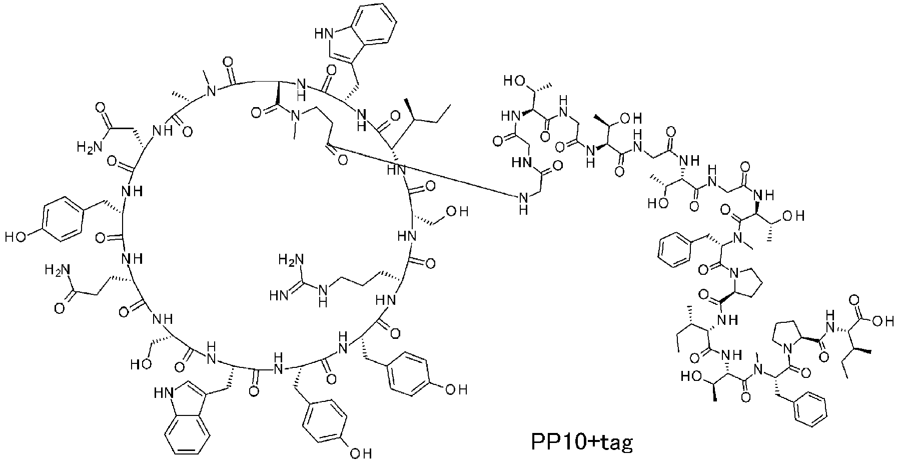 , and 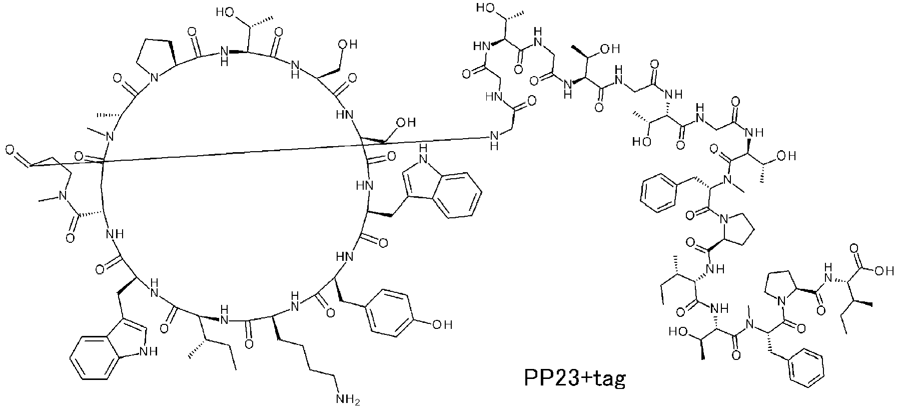 or competes for binding to Regnase-1 with at least one antibody selected from the following antibodies:
   (i) an antibody comprising a heavy chain comprising the amino acid sequence of SEQ ID NO: 20 and a light chain comprising the amino acid sequence of SEQ ID NO: 21 (REA0023);
   (ii) an antibody comprising a heavy chain comprising the amino acid sequence of SEQ ID NO: 22 and a light chain comprising the amino acid sequence of SEQ ID NO: 23 (REA0027);
   (iii) an antibody comprising a heavy chain comprising the amino acid sequence of SEQ ID NO: 24 and a light chain comprising the amino acid sequence of SEQ ID NO: 25 (REB0007);
   (iv) an antibody comprising a heavy chain comprising the amino acid sequence of SEQ ID NO: 26 and a light chain comprising the amino acid sequence of SEQ ID NO: 27 (REB0014); and
   (v) an antibody comprising a heavy chain comprising the amino acid sequence of SEQ ID NO: 28 and a light chain comprising the amino acid sequence of SEQ ID NO: 29 (REB0022).
[H3] The Regnase-1-binding molecule of [H1] or [H2], wherein the molecule competes for binding to Regnase-1 with compound PP7.
[H4] The Regnase-1-binding molecule of any one of [H1] to [H3], wherein the molecule competes for binding to Regnase-1 with compound PP23.
[H5] The Regnase-1-binding molecule of any one of [H1] to [H4], wherein the molecule competes for binding to Regnase-1 with compound PP10.
[H6] The Regnase-1-binding molecule of any one of [H1], [H2], [H4], or [H5], wherein the molecule does not compete for binding to Regnase-1 with compound PP7.
[H7] The Regnase-1-binding molecule of any one of [H1] to [H3], [H5], or [H6], wherein the molecule does not compete for binding to Regnase-1 with compound PP23.
[H8] The Regnase-1-binding molecule of any one of [H1] to [H4], [H6], or [H7], wherein the molecule does not compete for binding to Regnase-1 with compound PP10.
[H9] The Regnase-1-binding molecule of any one of [H1] to [H8], wherein the molecule specifically binds to Regnase-1.
[H10] The Regnase-1-binding molecule of any one of [H1] to [H9], wherein the positions corresponding respectively to positions 513, 494, 439, and 435 of SEQ ID NO: 1 are (i) positions 513, 494, 439, and 435 of SEQ ID NO: 1; or (ii) positions 516, 497, 442, and 438 of SEQ ID NO: 2.
[H11] The Regnase-1-binding molecule of any one of [H1] to [H10], wherein the molecule inhibits phosphorylation of a Ser residue in (i) and (ii) below:
   (i) a Ser residue at either or both positions corresponding respectively to positions 513 and 494 of SEQ ID NO: 1 in Regnase-1; and
   (ii) a Ser residue at either or both positions corresponding respectively to positions 439 and 435 of SEQ ID NO: 1 in Regnase-1.
[H12] The Regnase-1-binding molecule of any one of [H1] to [H11], wherein the molecule inhibits phosphorylation of a Ser residue at at least one position selected from the group consisting of positions corresponding respectively to positions 513 and 494 of SEQ ID NO: 1 in Regnase-1.
[H13] The Regnase-1-binding molecule of any one of [H1] to [H12], wherein the molecule inhibits phosphorylation of a Ser residue at at least one position selected from the group consisting of positions corresponding respectively to positions 439 and 435 of SEQ ID NO: 1.
[H14] The Regnase-1-binding molecule of any one of [H1] to [H13], wherein the molecule binds to Regnase-1 at the same site as the binding site on Regnase-1 to which a compound selected from compounds PP1 to PP25, PP7+tag, PP10+tag, and PP23+tag or an antibody selected from antibodies REA0023, REA0027, REB0007, REB0014, and REB0022 binds.
[H15] The Regnase-1-binding molecule of any one of [H1] to [H14], wherein the molecule binds to Regnase-1 at the same site as the binding site on Regnase-1 to which compound PP7 binds.
[H16] The Regnase-1-binding molecule of any one of [H1] to [H15], wherein the molecule binds to Regnase-1 at the same site as the binding site on Regnase-1 to which compound PP23 binds.
[H17] The Regnase-1-binding molecule of any one of [H1] to [H16], wherein the molecule binds to Regnase-1 at the same site as the binding site on Regnase-1 to which compound PP10 binds.
[H18] The Regnase-1-binding molecule of any one of [H1] to [H17], wherein the Ser residue is a Ser residue comprised in at least one amino acid sequence selected from the group consisting of YWSEP (SEQ ID NO: 3), HFSVP (SEQ ID NO: 4), and DSGIGS (SEQ ID NO: 5) comprised in an amino acid sequence of Regnase-1.
[H19] The Regnase-1-binding molecule of any one of [H1] to [H18], wherein the molecule inhibits binding between Regnase-1 and at least one molecule selected from the group consisting of TBK1, IKKi, Act-1, IKK, and IRAK
[H20] The Regnase-1-binding molecule of any one of [H1] to [H19], wherein the phosphorylation is a phosphorylation that can be induced by at least one molecule selected from the group consisting of IL-17 and IL-1.
[H21] A Regnase-1-binding molecule that inhibits binding between Regnase-1 and at least one molecule selected from the group consisting of TBK1, IKKi, Act-1, IKK, and IRAK
[H22] The Regnase-1-binding molecule of any one of [H1] to [H21], wherein the molecule binds to an amino acid residue comprised in the amino acid sequence of the 544th to 596th set forth in SEQ ID NO: 1 or the amino acid sequence of the 547th to 599th set forth in SEQ ID NO: 2.
[H23] The Regnase-1-binding molecule of any one of [H1] to [H22], wherein the molecule is a cyclic polypeptide.
[H24] The Regnase-1-binding molecule of any one of [H1] to [H22], wherein the molecule is an antibody.

In a non-limiting specific embodiment, the present invention also includes the following:
[I1] A method for treating and/or preventing a disease associated with Regnase-1, comprising administering the composition of any one of [B1] to [B26] or the Regnase-1-binding molecule of any one of [H1] to [H24] to a subject in need thereof (wherein the subject in need thereof may be a subject having or at risk of having the disease associated with Regnase-1).
[I2] Use of the composition of any one of [B1] to [B26] or the Regnase-1-binding molecule of any one of [H1] to [H24] in the manufacture of a medicament for treating and/or preventing a disease associated with Regnase-1.
[I3] The composition of any one of [B1] to [B26] or the Regnase-1-binding molecule of any one of [H1] to [H24] for use in the treatment and/or prevention of a disease associated with Regnase-1.

### [Brief Description of Drawings]

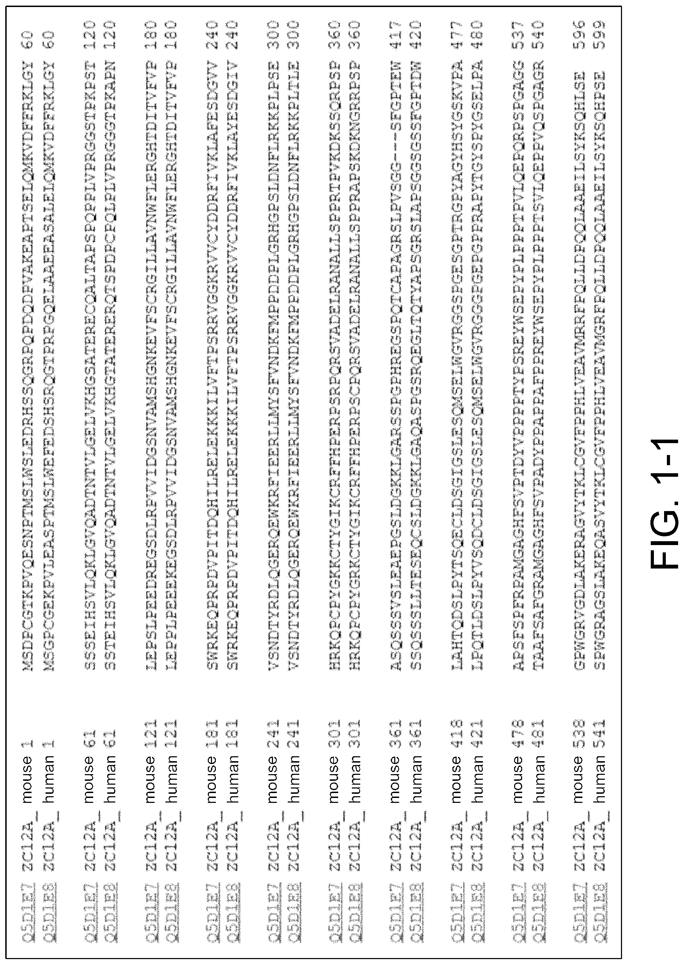

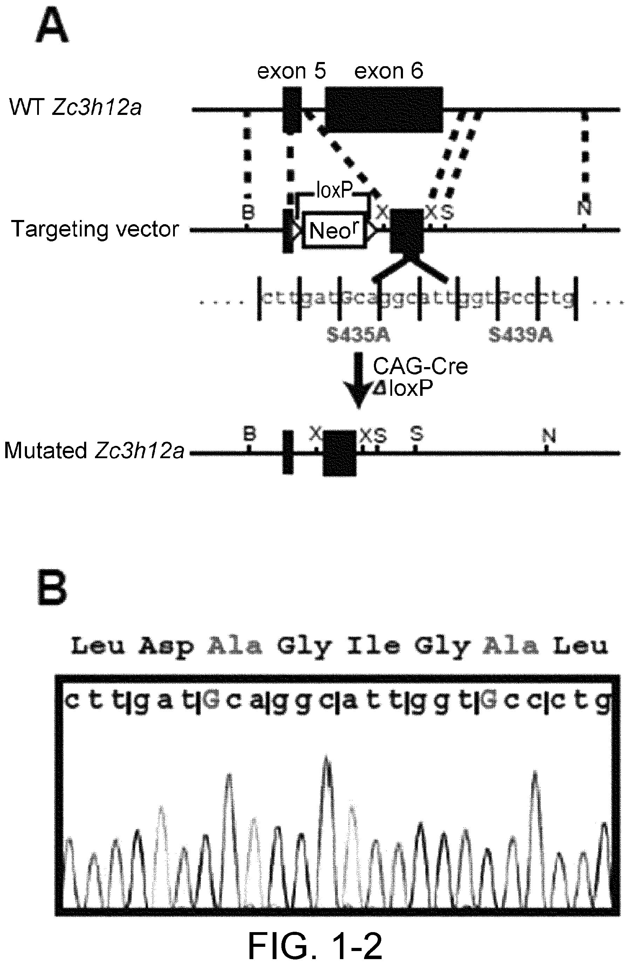

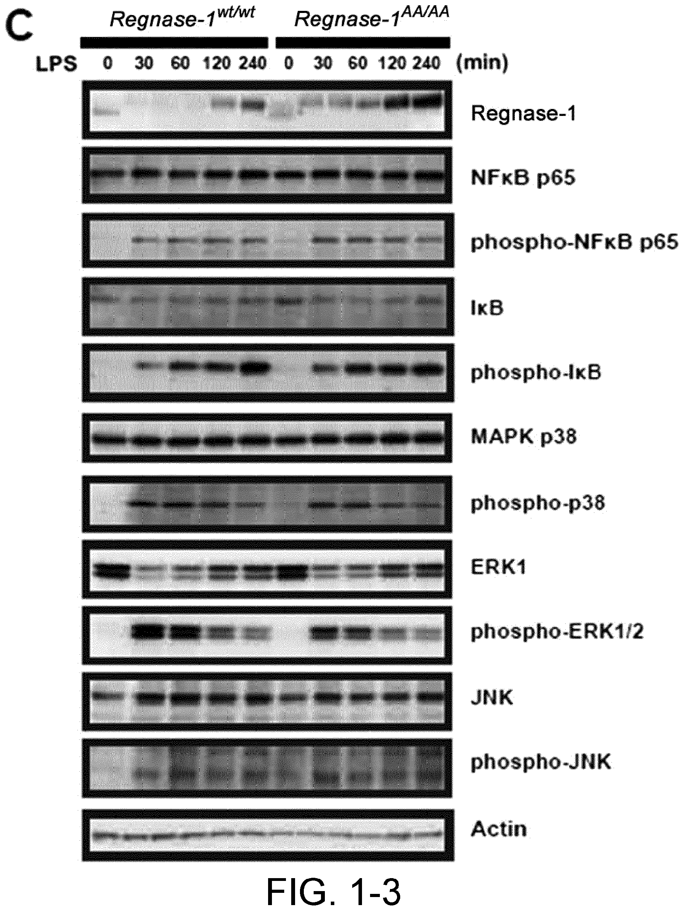

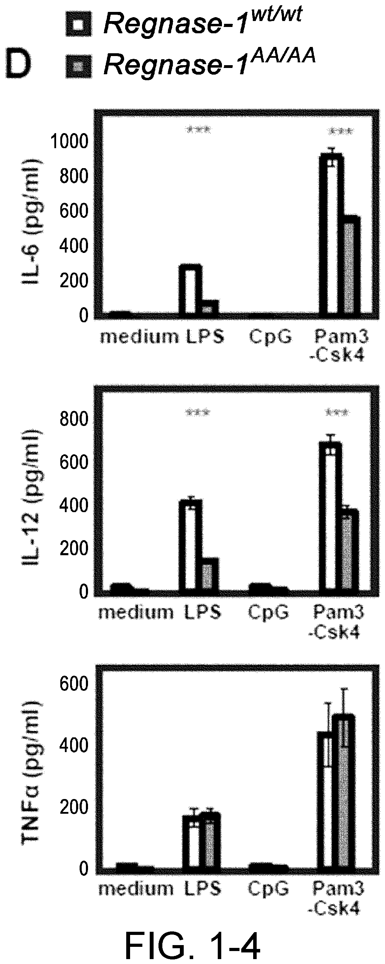

[Figure 1-1] Fig. 1-1 shows the comparison between the amino acid sequences of mouse Regnase-1 (SEQ ID NO: 1) and human Regnase-1 (SEQ ID NO: 2). Q5D1E7 and Q5D1E8 represent Uniprot accession numbers.
[Figure 1-2] (A) The figure depicts a method of producing Regnase-1 S435A/S439A amino acid substitution mutant (Regnase-1AA/AA) mice. Schematic diagram of wild-type Regnase-1 gene (top), targeting vector (middle), and putative mutant allele (bottom) is shown. The targeting vector comprises the S435A and S439A mutations in exon 6. (B) The figure shows the sequencing result of Regnase-1 exon 6 in the Regnase-1AA/AA mouse genome. The sequence chromatogram shows that TCA and TCC in Ser435 and Ser439 are replaced with GCA and GCC, respectively.
[Figure 1-3] (C) The figure shows the immunoblot analysis results of NFκB, phospho-NFκB, IκB, phospho-IκB, MAPK p38, phospho-MAPK p38, ERK1, phospho-ERK1, JNK, and phospho-JNK in wild-type and Regnase-1AA/AA macrophages stimulated with LPS (100 ng/ml) for 0 to 240 minutes.
[Figure 1-4] (D) The figure shows the results of IL-6, IL-12, and TNF-α production by wild-type and Regnase-1AA/AA macrophages stimulated with a low concentration of LPS (10 ng/ml), CpG (0.1 µM), or Pam3Csk4 (10 ng/ml) for 24 hours. The results of cytokine production in cell supernatants evaluated by ELISA. The error bars represent mean ± SEM. ***P < 0.005.

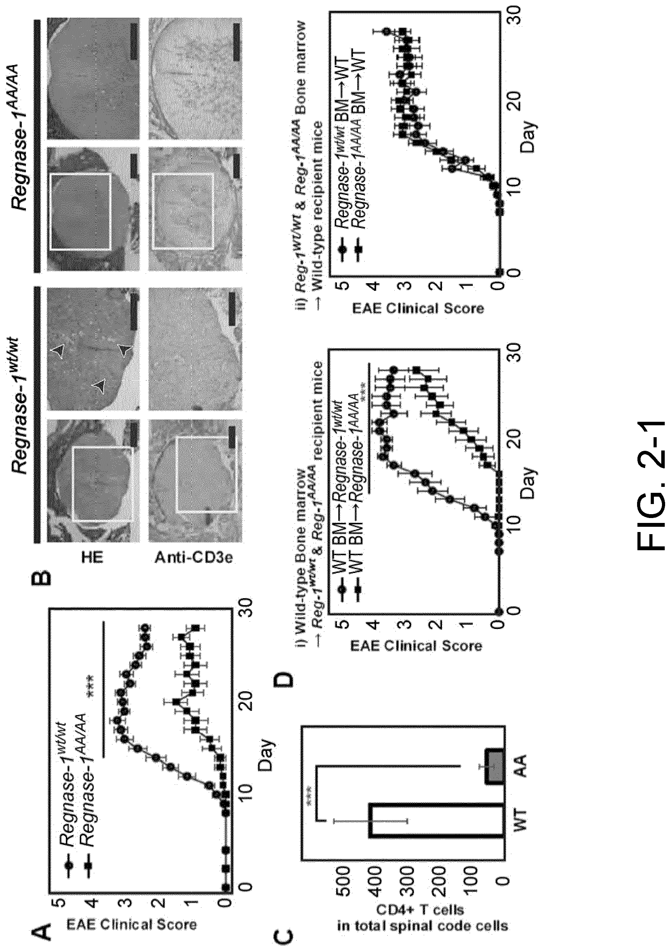

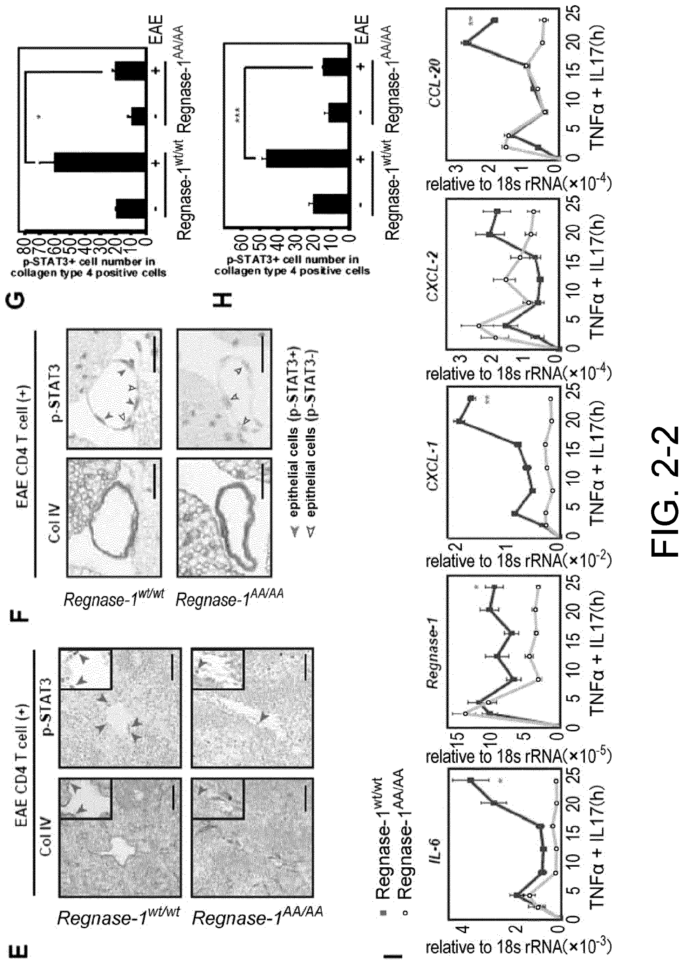

[Figure 2-1] (A) The figure shows the EAE clinical scores of wild-type (black circle; n = 15) and Regnase-1AA/AA mice (black square; n = 12). (B) The figure shows the histological analysis results of CD4+ T cell infiltration into the spinal cord. The results of staining frozen sections with hematoxylin-eosin (upper row) and anti-CD3ε (lower row). The arrows indicate inflammatory cell infiltration. Scale bars, 200 µm. (C) The figure shows the number of CD4+ T cells in spinal cord cells (1.0 x 10⁵ cells) 15 days after immunization. The analysis results using flow cytometry. (D) The figure shows the EAE clinical scores of chimeric mice produced by (i) intravenous injection of wild-type bone marrow cells to wild-type or Regnase-1AA/AA mice (n = 8 for each group) and (ii) intravenous injection of wild-type or Regnase-1AA/AA bone marrow cells to wild-type mice (n = 12 for each group).
[Figure 2-2] (E and F) The figures show the histological analysis of endothelial cell inflammation in wild-type and Regnase-1AA/AA mice. The results of staining sections of spleen (E) and the fifth lumbar spinal cord (F), prepared 12 hours after intravenous injection of pathogenic CD4+ T cells (1.5 x 10⁷ cells/mouse), with an anti-type IV collagen antibody and anti-phospho-STAT3 antibody. The black and white arrows indicate phospho-STAT3 positive and negative endothelial cells, respectively. Scale bars, 50 µm. (G and H) The figures show the results of the relative number of anti-phospho-STAT3 positive cells in vascular endothelial cells (type IV collagen positive), measured in spleen (H) and the fifth lumbar spinal cord (I). (I) The figure shows the qPCR analysis of IL-6, Regnase-1, CXCL-1, CXCL2, and CCL-20 mRNA in wild-type and Regnase-1AA/AA MEFs. The results after stimulating cells with TNF-α (20 ng/ml) and IL-17A (50 ng/ml) for 0 to 24 hours. The error bars represent mean ± SEM. *P < 0.05, **P < 0.01, and ***P < 0.005.

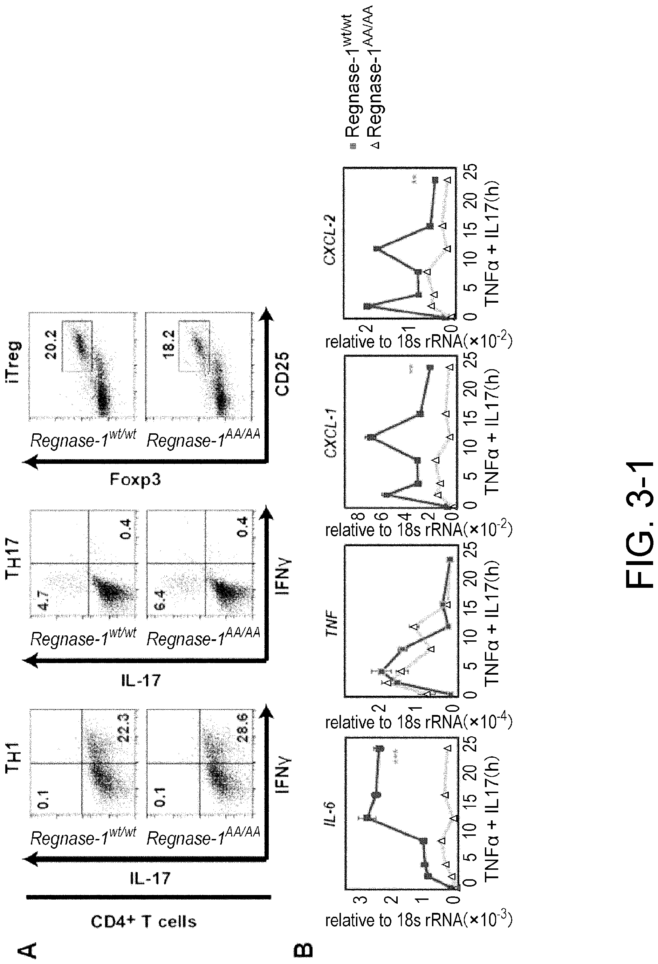

[Figure 3-1] (A) The figure shows the flow cytometry analysis results of CD4+ T cell subsets (TH1, TH17, and iTreg) differentiated from naive CD4+ T cells under in vitro conditions. (B) The figure shows the qPCR analysis of IL-6, TNF-α, CXCL-1, and CXCL2 mRNA in wild-type and Regnase-1AA/AA liver sinusoidal endothelial cells (LSEC). The results after stimulating cells with TNF-α (20 ng/ml) and IL-17A (50 ng/ml) for 0 to 24 hours.

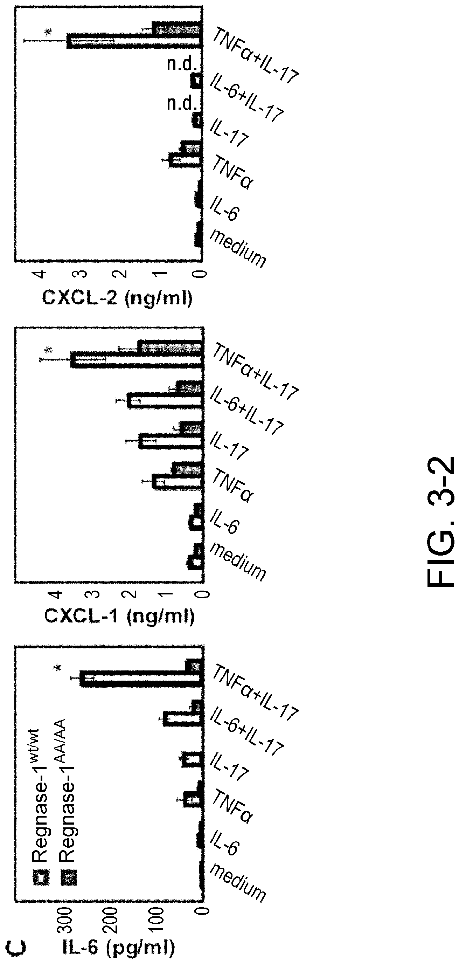

[Figure 3-2] (C) The figure shows IL-6, CXCL-1, and CXCL-2 production by mouse LSECs in response to 24-hour exposure to IL-6 (20 ng/ml), TNF-α (20 ng/ml), IL-17A (50 ng/ml), IL-6 + IL-17A, or TNF-α + IL-17A. The results of ELISA evaluating the amount of protein production in cell supernatants.

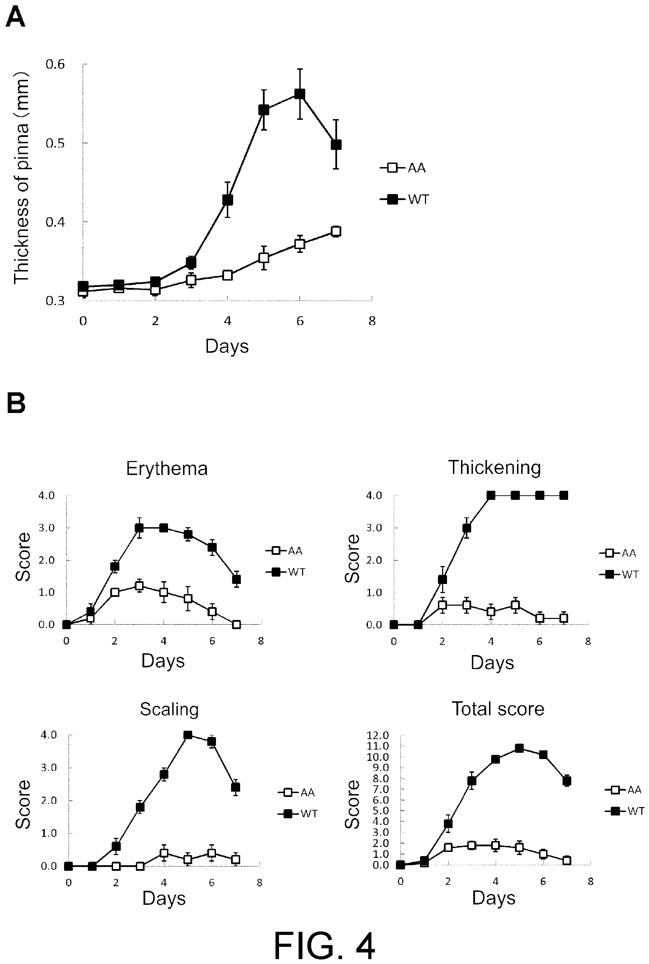

[Figure 4] (A) The figure shows thickness of the pinna at the application site of wild-type mice and Regnase-1AA mutant mice in imiquimod-induced psoriasis model (mean ± standard error; five mice for each group). (B) The figure shows macroscopic findings of the dorsocervical skin of wild-type mice and Regnase-1AA mutant mice in imiquimod-induced psoriasis model (mean ± standard error; five mice for each group).

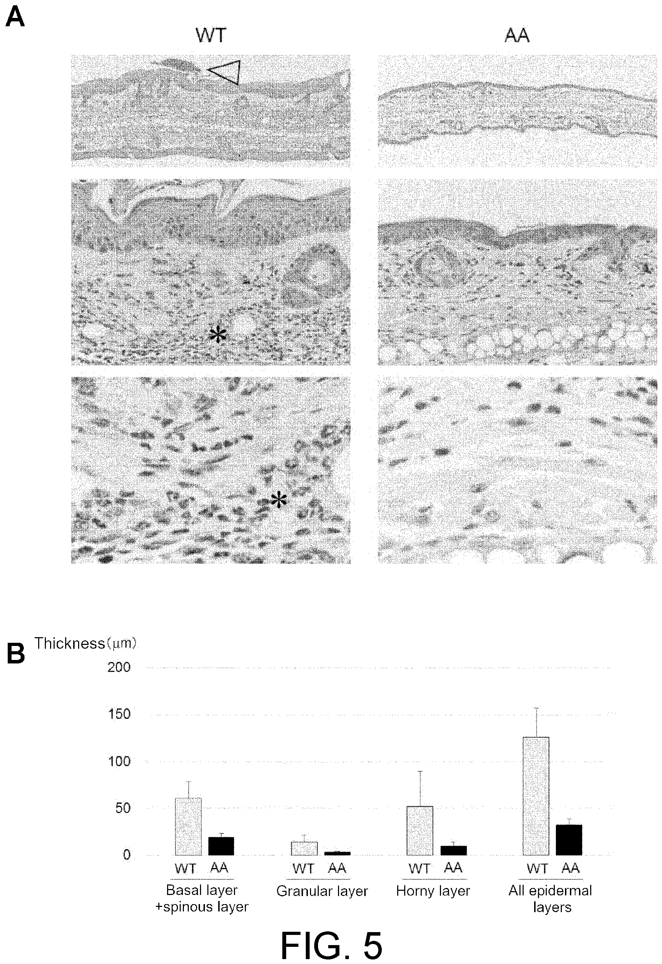

[Figure 5] (A) The figure shows histopathological images of the skin of the pinna at the application site of wild-type mice and Regnase-1AA mutant mice in imiquimod-induced psoriasis model (hematoxylin-eosin stained specimens) (neutrophil infiltration (*); microabscess (arrowhead)). (B) The figure shows the thickness of the epidermis of the pinna at the application site of wild-type mice and Regnase-1AA mutant mice in imiquimod-induced psoriasis model (mean ± standard deviation; five mice for each group).

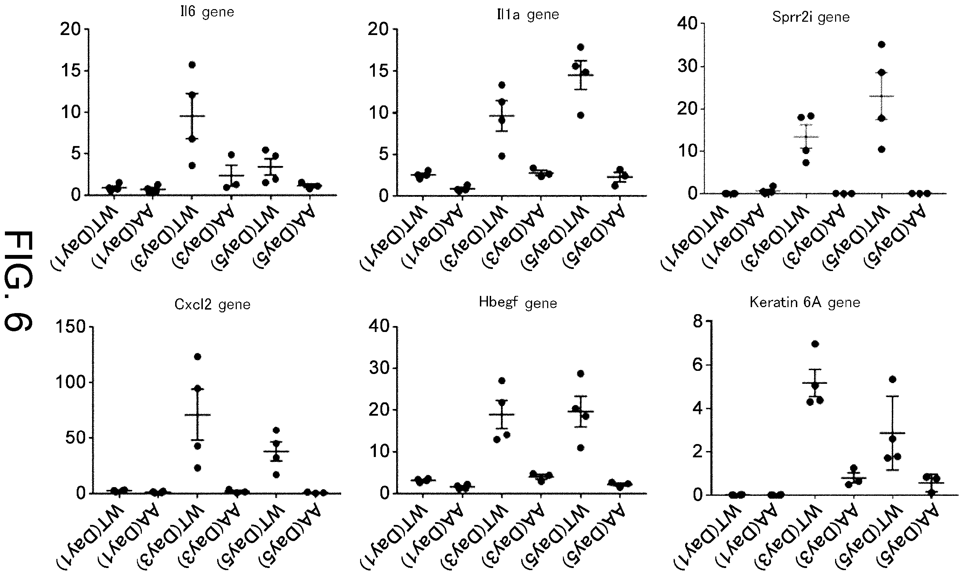

[Figure 6] The figure shows the change in the expression levels of various genes in the skin of the pinna at the application site of wild-type mice and Regnase-1AA mutant mice in imiquimod-induced psoriasis model (normalized using B2m expression level).

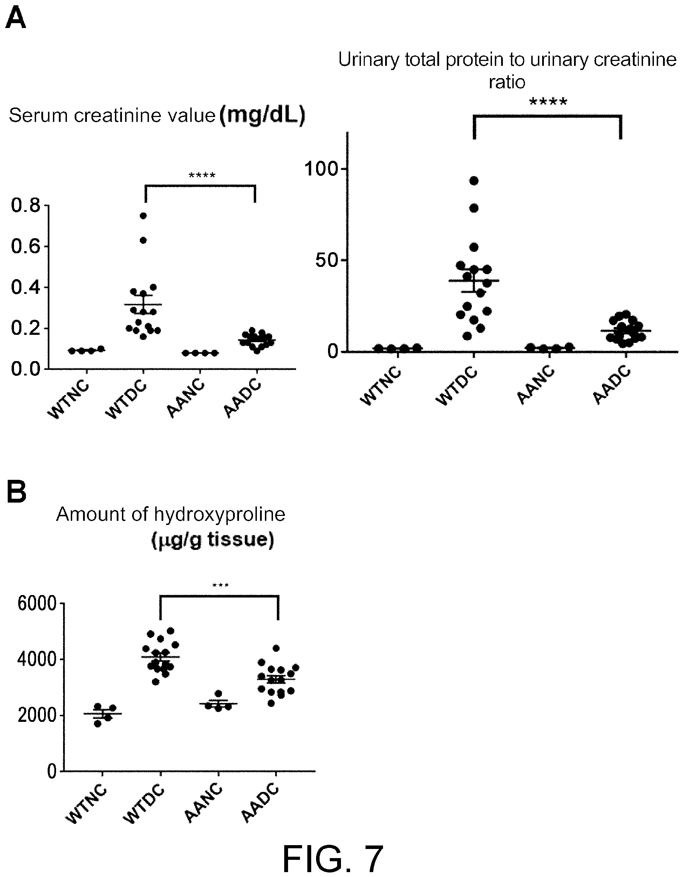

[Figure 7] (A) The figure shows serum creatinine values and urine total protein to creatinine value ratios of wild-type mice and Regnase-1AA mutant mice in anti-glomerular basement membrane antibody-induced nephritis model (Mann-Whitney U test; ****: p < 0.0001). (B) The figure shows hydroxyproline contents per kidney weight of wild-type mice and Regnase-1AA mutant mice in anti-glomerular basement membrane antibody-induced nephritis model (Mann-Whitney U test; ***: p < 0.001).

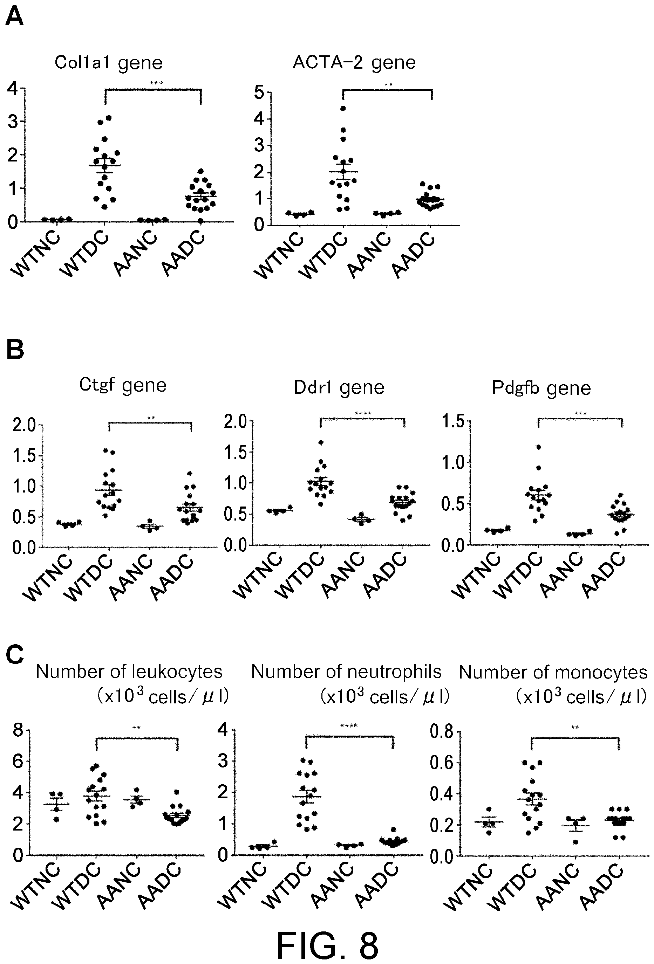

[Figure 8] (A) The figure shows the change in Colla1 and Acta-2 expression levels in the kidneys of wild-type mice and Regnase-1AA mutant mice in anti-glomerular basement membrane antibody-induced nephritis model (normalized using GAPDH expression level, Mann-Whitney U test; **: p < 0.01, ***: p < 0.001). (B) The figure shows the change in the expression levels of various genes in the kidneys of wild-type mice and Regnase-1AA mutant mice in anti-glomerular basement membrane antibody-induced nephritis model (normalized using the GAPDH expression level, Mann-Whitney U test; **: p < 0.01, ***: p < 0.001, ****: p < 0.0001). (C) The figure shows the change in the number of blood cells in the blood of wild-type mice and Regnase-1AA mutant mice in anti-glomerular basement membrane antibody-induced nephritis model (The number of leukocytes, neutrophils, and monocytes per 1 µL blood is shown.) (Mann-Whitney U test; **: p < 0.01, ****: p < 0.0001).

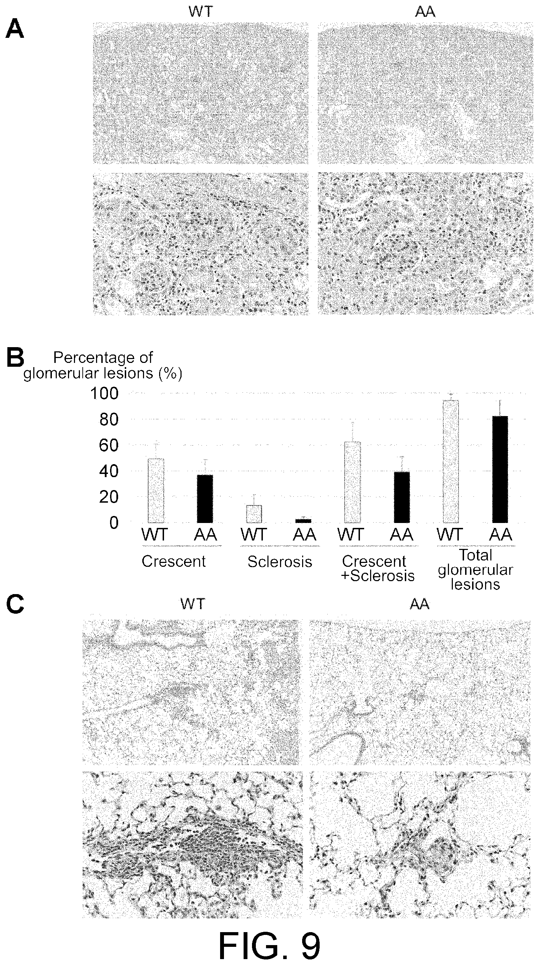

[Figure 9] (A) The figure shows histopathological images of the kidneys of wild-type mice and Regnase-1AA mutant mice in anti-glomerular basement membrane antibody-induced nephritis model (hematoxylin-eosin stained specimens). (B) The figure shows the proportion of glomerular lesions of wild-type mice and Regnase-1AA mutant mice in anti-glomerular basement membrane antibody-induced nephritis model (mean and standard deviation; 15 mice for each group). (C) The figure shows histopathological images of the lungs of wild-type mice and Regnase-1AA mutant mice in anti-glomerular basement membrane antibody-induced nephritis model (hematoxylin-eosin stained specimens).

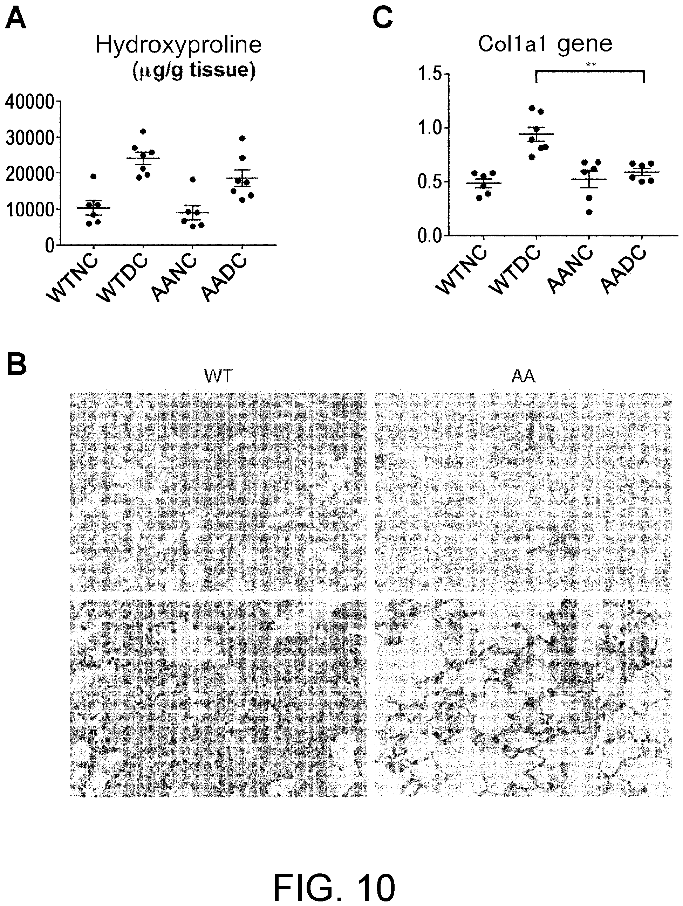

[Figure 10] (A) The figure shows hydroxyproline contents per skin weight of wild-type mice and Regnase-1AA mutant mice in bleomycin-induced scleroderma model. (B) The figure shows histopathological images of the lungs of wild-type mice and Regnase-1AA mutant mice in bleomycin-induced scleroderma model (hematoxylin-eosin stained specimens). (C) The figure shows the change in Colla1 expression levels in the lungs of wild-type mice and Regnase-1AA mutant mice in bleomycin-induced scleroderma model (normalized using the GAPDH expression level, Mann-Whitney U test; **: p < 0.01).

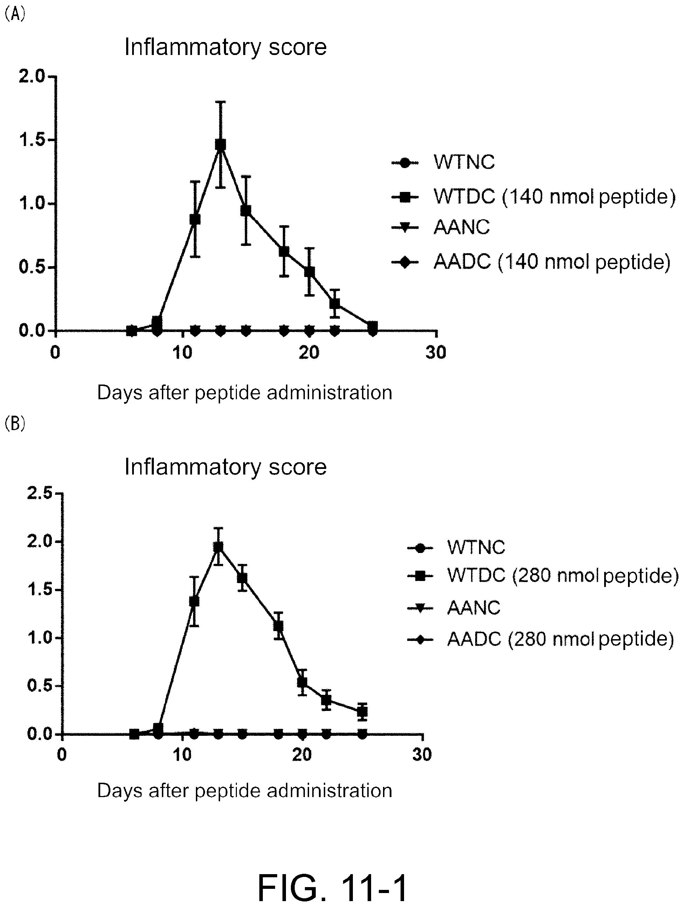

[Figure 11-1] The figures show the pathological analysis results of experimental autoimmune uveitis model mice. In the figures, WTNC indicates the results of non-disease-induced wild-type mice, WTDC indicates that of disease-induced wild-type mice, AANC indicates that of non-disease-induced Regnase-1 AA mutant mice, and AADC indicates that of disease-induced Regnase-1 AA mutant mice, respectively. (A) The figure shows the inflammatory scores of both eyes of mice under the condition that the peptide dose was 140 nmol. The horizontal axis of the graph represents the days after peptide administration and the vertical axis represents the inflammatory scores (mean ± standard error), respectively. (B) The figure shows the inflammatory scores of both eyes of mice under the condition that the peptide dose was 280 nmol. The horizontal axis of the graph represents the days after peptide administration and the vertical axis represents the inflammatory scores (mean ± standard error), respectively.

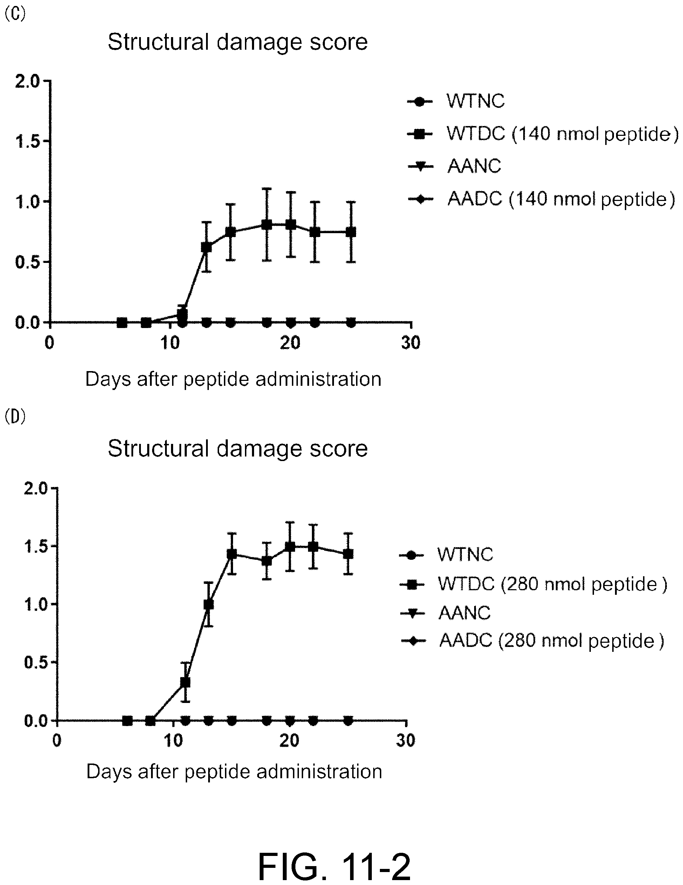

[Figure 11-2] (C) The figure shows the structural damage scores of both eyes of mice under the condition that the peptide dose was 140 nmol. The horizontal axis of the graph represents the days after peptide administration and the vertical axis represents the structural damage scores (mean ± standard error), respectively. (D) The figure shows the structural damage scores of both eyes of mice under the condition that the peptide dose was 280 nmol. The horizontal axis of the graph represents the days after peptide administration and the vertical axis represents the structural damage scores (mean ± standard error), respectively.

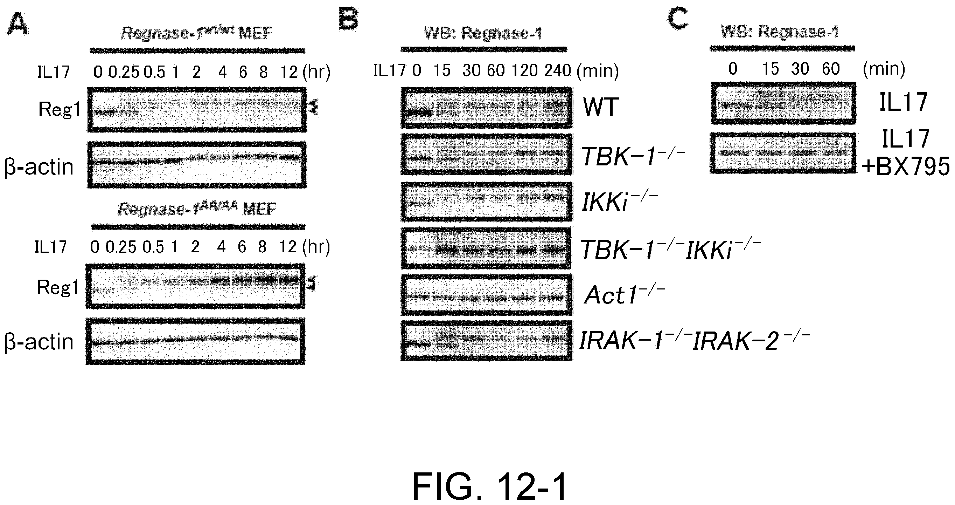

[Figure 12-1] (A) The figure shows the immunoblot analysis results of Regnase-1 in wild-type and Regnase-1AA/AA MEFs stimulated with IL-17. The two arrows indicate the phosphorylated form (upper) and unphosphorylated form (lower) of Regnase-1. (B) The figure shows the immunoblot analysis results of Regnase-1 in wild-type MEFs and MEFs deficient in each molecule stimulated with IL-17. (C) The figure shows the immunoblot analysis results of Regnase-1 in wild-type MEFs stimulated with IL-17 in the presence of BX795 (50 µM).

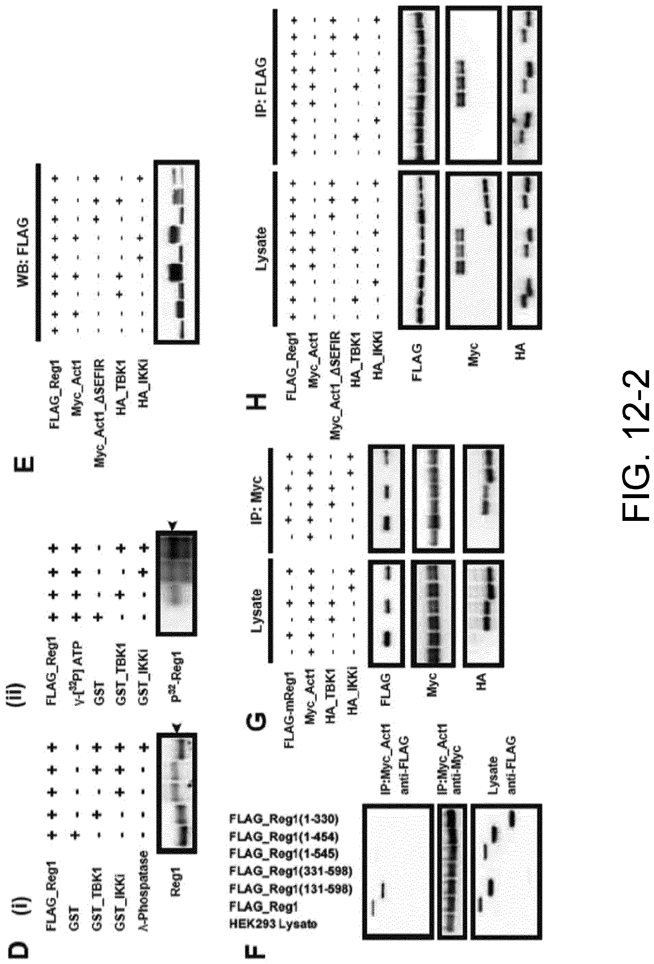

[Figure 12-2] (D) The figure shows the phosphorylation of Regnase-1 by TBK1 and IKKi under in vitro conditions. Purified Regnase-1 obtained from Regnase-1-deficient MEFs expressing FLAG-tagged Regnase-1 AA mutant was incubated with recombinant TBK1 and/or IKKi in the presence/absence of γ-phosphatase for three hours. Regnase-1 phosphorylation was analyzed by western blotting (i) and [32P]-autoradiography (ii). The arrows indicate phosphorylated Regnase-1. (E) The figure shows the immunoblot analysis results of FLAG-tagged Regnase-1 in HEK293 cells transfected with FLAG Regnase-1, Myc-tagged Act-1, Myc-tagged Act-1 mutant (Myc-Act1 ASEFIR), HA-tagged TBK1, and HA-tagged IKKi. (F) The figure shows the results of co-immunoprecipitation between full-length, or N-terminally or C-terminally truncated Regnase-1 and Act1. FLAG-tagged Regnase-1 variants were co-immunoprecipitated with Myc-tagged Act1. Eluted proteins were subjected to immunoblot analysis using an anti-FLAG antibody and anti-Myc antibody. (G and H) The figures show the results of co-immunoprecipitation of FLAG-tagged Regnase-1, Myc-tagged Act-1 variants (full-length and C-terminally truncated form), HA-tagged TBK1, and HA-tagged IKKi. Cell lysates from HEK293 transfectants were mixed as described and co-immunoprecipitated with anti-Myc covered beads (G) or anti-FLAG M2 agarose beads (H). Eluted proteins were subjected to immunoblot analysis using an anti-FLAG antibody, anti-Myc antibody, anti-HA antibody, and anti-actin antibody.

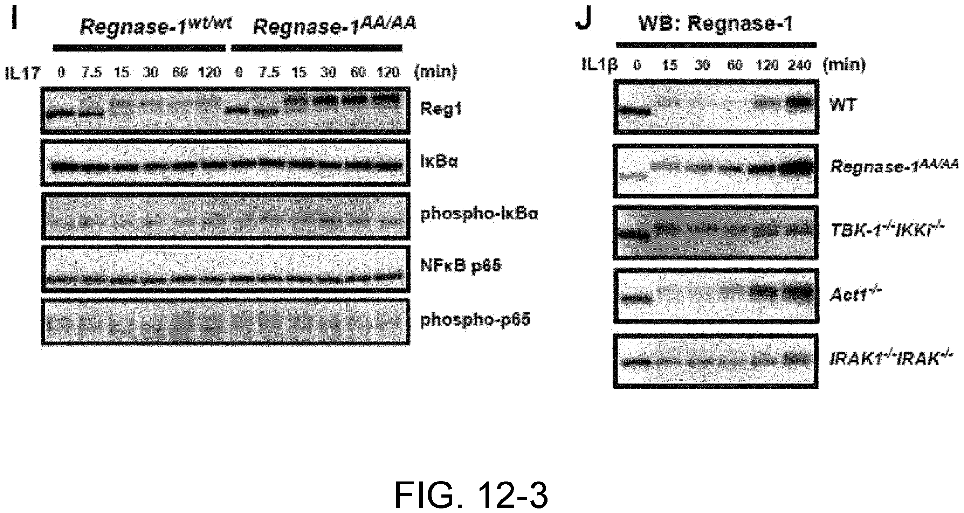

[Figure 12-3] (I) The figure shows the immunoblot analysis results of Regnase-1, IκB, phospho-IκB, NEκB, and phospho-NFκB expression in wild-type and Regnase-1AA/AA MEFs stimulated with IL-17A. (J) The figure shows the immunoblot analysis results of Regnase-1 expression in wild-type, Regnase-1AA/AA, TBK1/IKKi double-deficient, Act1-deficient, and IRAK1/IRAK2 double-deficient MEFs stimulated with IL-1β.

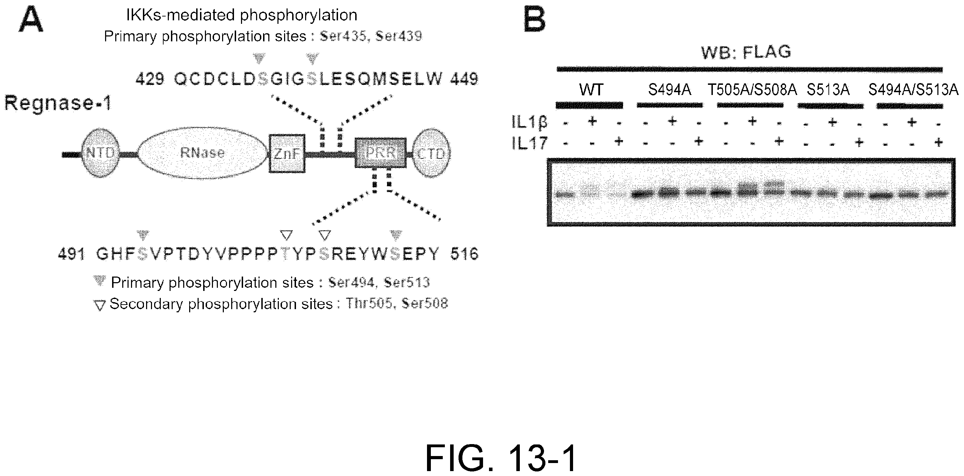

[Figure 13-1] (A) The figure shows the domain schematic of Regnase-1 and the mapping of sites phosphorylated by IKK (IKKα and IKKβ) and TBK1/IKKi. (B) The figure shows the immunoblot analysis results of Regnase-1 in HeLa cells transfected with Regnase-1 mutants (wild-type, S494A, T505A/S508A, S513A, and S494A/S513A). Cells were stimulated with IL-1β (10 ng/ml) and IL-17A (50 ng/ml) for one hour.

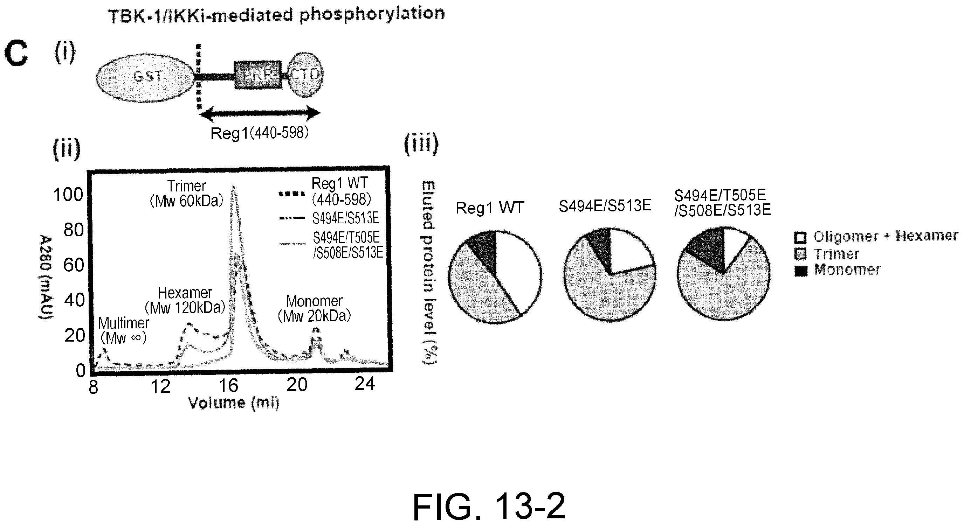

[Figure 13-2] (C) (i) The figure shows the diagram of the construct of GST-fused Regnase-1 (440-598). (ii) The figure shows the gel filtration results of wild-type and mutant (S494E/S513E or S494E/T505E/S508E/S513E) Regnase-1 (440-598). The molecular weight of each of the elution peaks was estimated using a molecular weight standard marker, and they were defined as multimers (Mw: ∞), hexamer (Mw: 120 kDa), trimer (Mw: 60 kDa), or monomer (Mw: 20 kDa). (iii) The figure shows the results of quantitating the eluted fractions (multimers + hexamer, trimer, and monomer) as a percentage relative to the total eluted proteins.

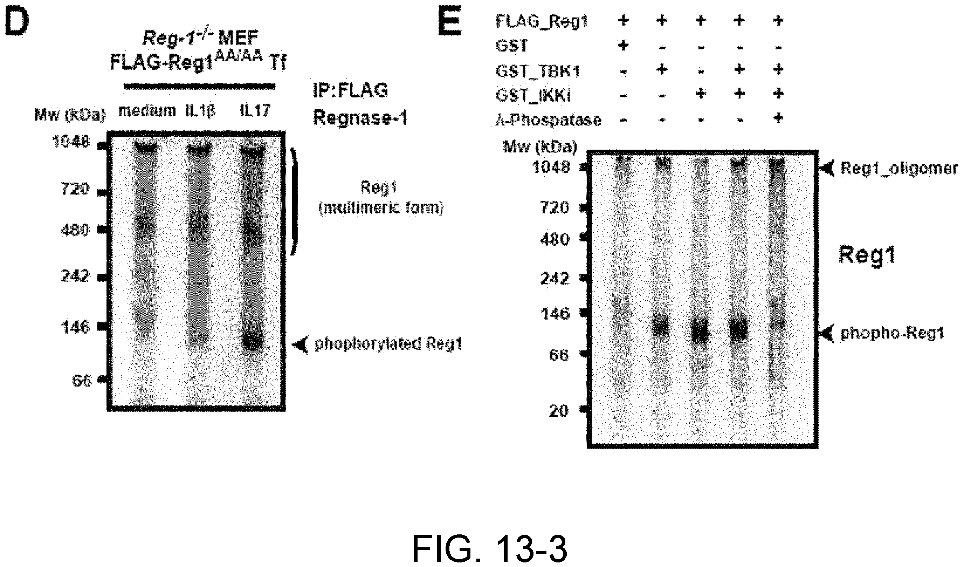

[Figure 13-3] (D) The figure shows the immunoblotting results of Regnase-1. Regnase-1 was obtained from Regnase-1-deficient MEFs expressing FLAG-tagged Regnase-1 AA mutant that were stimulated with IL-1β (10 ng/ml) and IL-17A (50 mg/ml) for one hour. Purified Regnase-1 was analyzed by native PAGE and western blotting. (E) The figure shows the immunoblot analysis results of Regnase-1 phosphorylated by TBK1 and IKKi. Purified Regnase-1 was incubated with GST-fused TBK1 and/or IKKi in the presence/absence of γ-phosphatase for three hours. Proteins were separated by native PAGE and SDS-PAGE, and analyzed by western blotting of Regnase-1.

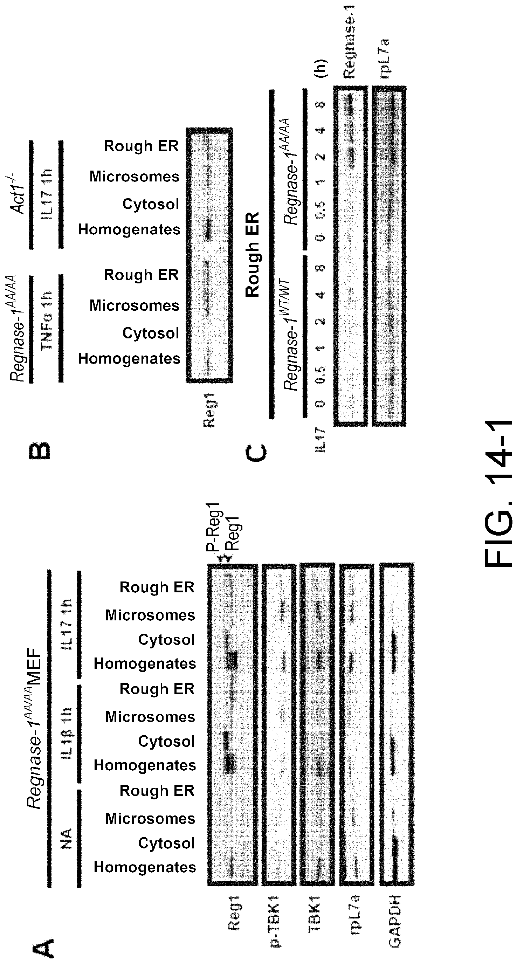

[Figure 14-1] (A and B) The figures show the immunoblot analysis results of intracellular organelle fractions. Regnase-1, ribosomal protein L7a (rpL7a; an ER marker), and GAPDH (a cytoplasmic marker) in cell homogenates, soluble cytosolic fractions, microsomes, and rough ER membranes. The fractions were prepared from Regnase-1AA/AA MEFs stimulated with IL-1β (10 ng/ml) and IL-17A (50 ng/ml) for one hour each (A) and Regnase-1AA/AA and Act1-deficient MEFs stimulated with TNF-α (20 ng/ml) and IL-17A (50 ng/ml) for one hour each (B). The arrows indicate phosphorylated form (upper) and unphosphorylated form (lower) of Regnase-1. (C) The figure shows the immunoblot analysis results of Regnase-1 and rpL7a in rough ER membranes prepared from wild-type and Regnase-1AA/AA MEFs stimulated with IL-17A (50 ng/ml) for 0 to 8 hours.

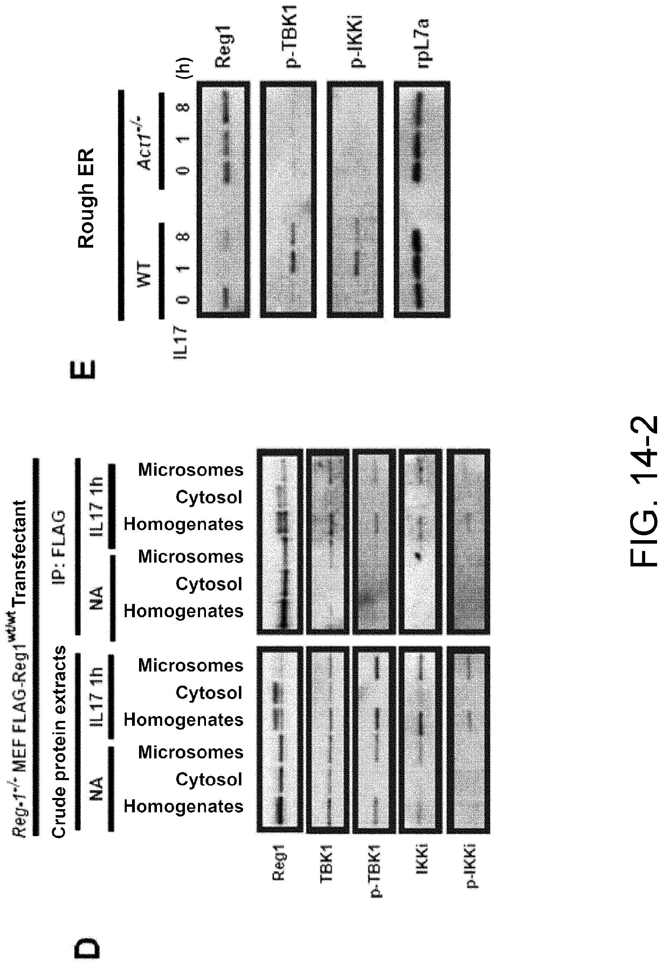

[Figure 14-2] (D) FLAG-tagged Regnase-1 was immunoprecipitated from intracellular organelle fractions of Regnase-1-deficient MEFs expressing FLAG-tagged Regnase-1 AA mutant that were stimulated with or without IL-17A (50 ng/ml) for one hour. The immunoprecipitate was subjected to immunoblot analysis of Regnase-1, TBK1, phospho-TBK1, IKKi, and phospho-IKKi. (E) The figure shows the immunoblot analysis results of Regnase-1, phospho-TBK1, phospho-IKKi, and rpL7a in ER membrane fractions isolated from wild-type and Act1-deficient MEFs. Cells were stimulated with IL-17A (50 ng/ml) for 0, 1, and 8 hours.

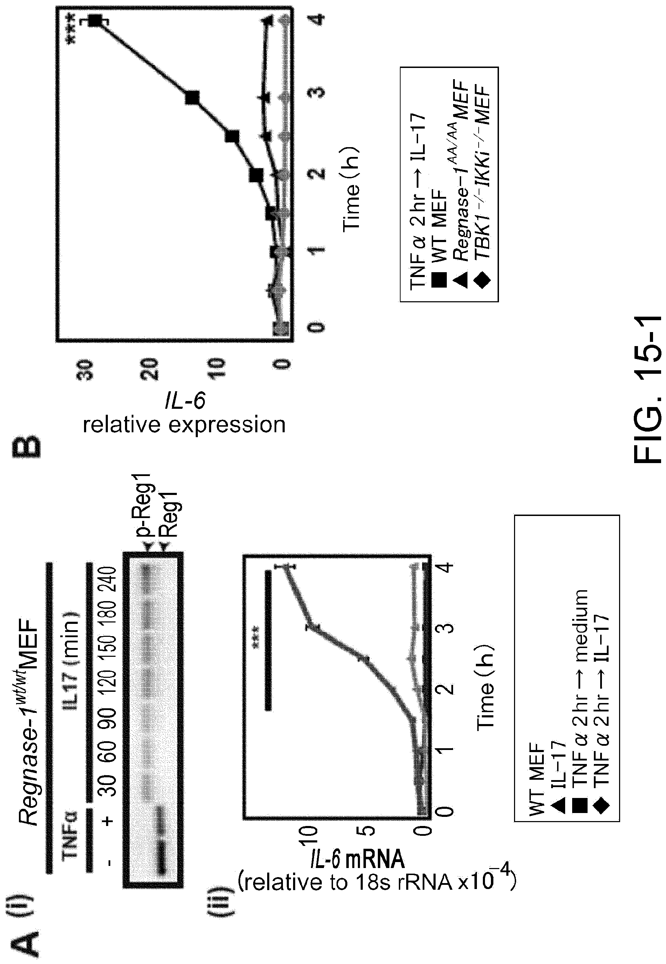

[Figure 15-1] (A) The figure shows the analysis results of immunoblotting and quantitative PCR (qPCR) of wild-type MEFs stimulated with TNF-α for two hours, subsequently with IL-17A for 0 to 4 hours in a time-dependent manner. (i) Cell lysates were analyzed by immunoblotting of Regnase-1. (ii) The figure shows IL-6 mRNA expression in wild-type cells stimulated by the combination of TNF-α and IL-17A as described above. (B) The figure shows IL-6 mRNA expression in wild-type MEFs and Regnase-1AA/AA and TBK1/IKKi double-deficient MEFs stimulated with TNF-α for two hours, subsequently with IL-17A for 0 to 4 hours.

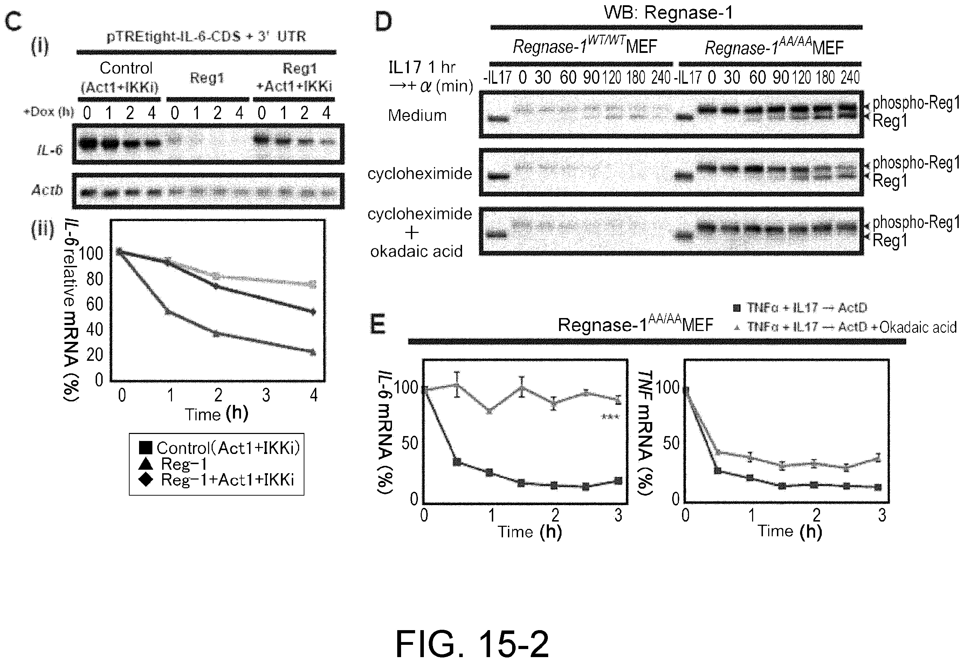

[Figure 15-2] (C) (i) The figure shows the autoradiography results of IL-6 (upper) and Actb (lower) mRNA levels in Tet-off HEK293 cells co-transfected with the expression plasmids of control (Act1 + IKKi), Regnase-1, or Regnase-1 + Act1 + IKKi and the pTRE-tight-IL6-CDS+3'UTR vectors. Total mRNA was prepared from cells treated with doxycycline for 0 to 4 hours, and then subjected to Northern blotting using [32P] labelled probes. (ii) The figure shows the relative IL-6 mRNA levels in Tet-off HEK293 cells during doxycycline treatment. (D) The figure shows the immunoblot analysis results of Regnase-1 in Regnase-1AA/AA MEFs. Cells were treated with IL-17A (50 ng/ml) for one hour, and subsequently incubated in a medium with no additives (control), a medium containing cycloheximide (100 µM), or a medium containing both cycloheximide and okadaic acid (0.5 µM) for 0 to 240 minutes. (E) The figure shows the qPCR analysis results of IL-6 and TNF mRNA levels in Regnase-1AA/AA MEFs treated with TNF-α (20 ng/ml) and IL-17A (50 ng/ml) for two hours, subsequently incubated in a medium containing ActD (5 µg/ml) or a medium containing both ActD and okadaic acid (0.5 µM) for 0 to 180 minutes. The qPCR data (A, B, and E) were collected from four independent experiments. The error bars represent mean ± SEM. ***P < 0.005.

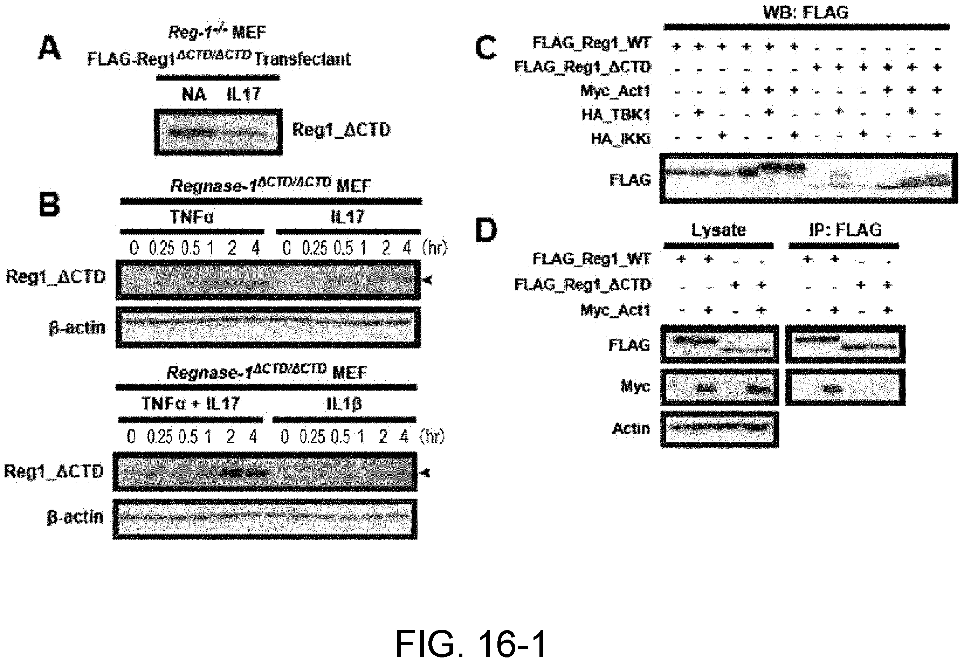

[Figure 16-1] (A) The figure shows the immunoblot analysis results of lysates of Regnase-1-deficient MEFs that stably express FLAG-tagged Regnase-1ΔCTD/ΔCTD. Cells were stimulated with or without IL-17A for one hour and probed with an anti-FLAG antibody. (B) The figure shows the immunoblot analysis results of Regnase-1 and β-actin in Regnase-1ΔCTD/ΔCTD MEFs stimulated with TNF-α, IL-17A, TNF-α + IL-17A, and IL-1β for 0 to 4 hours. Regnase-1 is indicated by arrows. (C) The figure shows the co-expression results of Act-1, TBK-1, and IKKi with FLAG-tagged wild-type Regnase-1 or Regnase-1ΔCTD in HEK293 cells. Cell lysates were subjected to immunoblot analysis using an anti-FLAG antibody. (D) The figure shows the co-immunoprecipitation results of FLAG-tagged wild-type Regnase-1, Regnase-1ΔCTD, and Myc-tagged Act-1. Cell lysates from HEK293 transfectants were mixed as described and co-immunoprecipitated with anti-FLAG M2 agarose beads. Eluted proteins were subjected to immunoblot analysis using an anti-FLAG antibody, anti-Myc antibody, and anti-actin antibody.

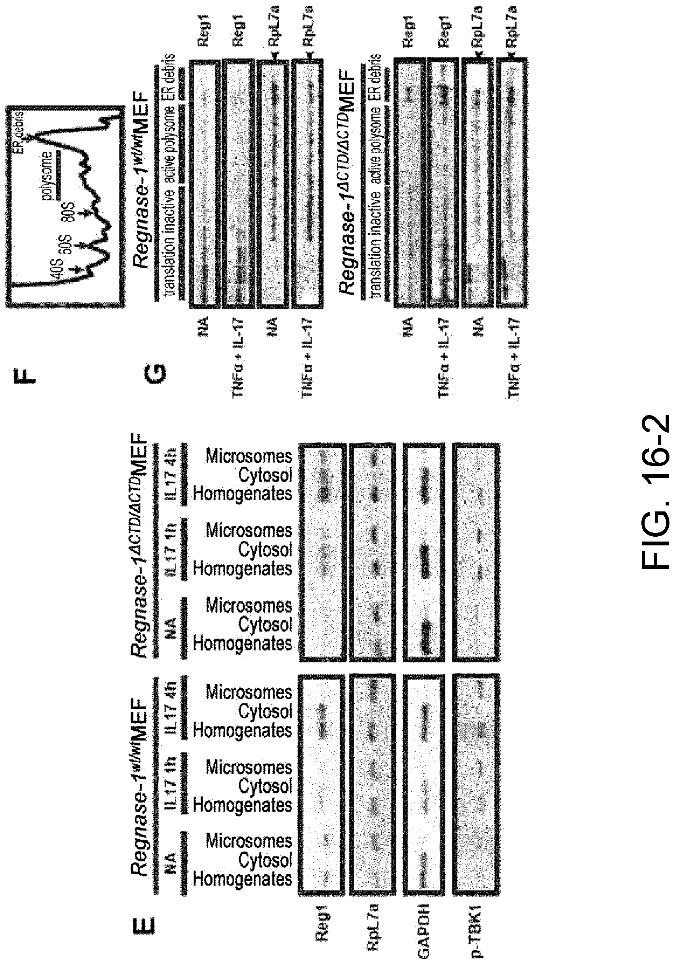

[Figure 16-2] (E) The figure shows the immunoblot analysis results of Regnase-1, Rpl7a, GAPDH, and phospho-TBK-1 in intracellular organelles (homogenates, cytosols, and microsomes) isolated from wild-type and Regnase-1ΔCTD/ΔCTD MEFs after stimulated with 50 ng/ml IL-17A for 0, 1, and 4 hours. (F and G) The figures show the immunoblot analysis results of polysome fractions. (F) The figure shows the UV absorbance profile (at 260 nm) of sucrose gradient fractions from cell lysates of MEFs. (G) The figure shows the immunoblot analysis results of Regnase-1 and RpL7a in sucrose gradient fractions isolated from wild-type and Regnase-1ΔCTD/ΔCTD cell lysates. RpL7a is indicated by arrows.

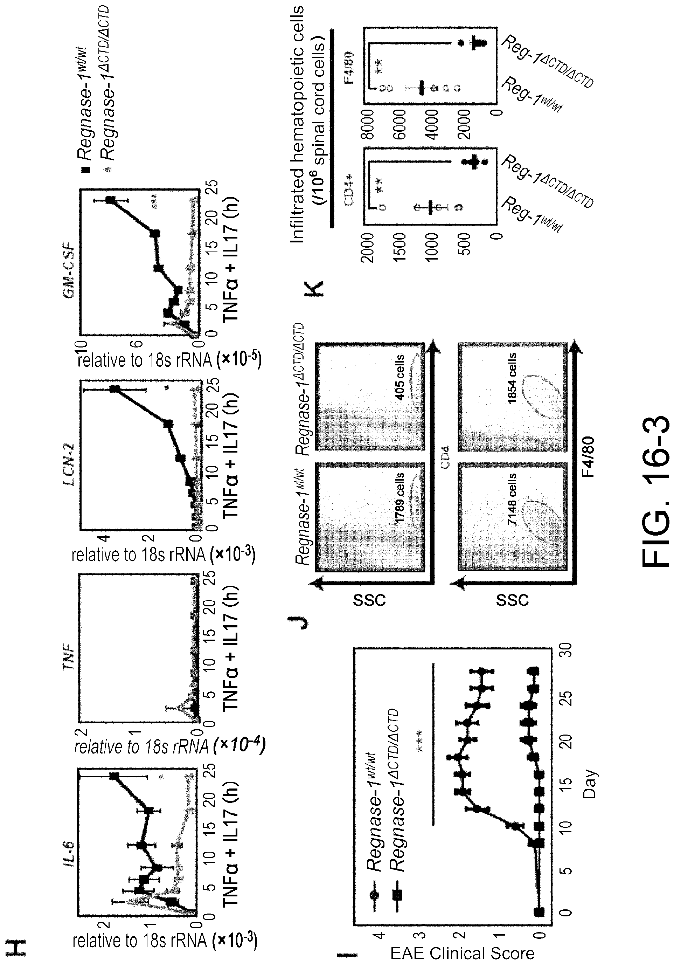

[Figure 16-3] (H) The figure shows the qPCR analysis results of IL-6, TNF, LCN-2, and GM-CSF mRNA in wild-type, Regnase-1ΔCTD/+, and Regnase-1ΔCTD/ΔCTD MEFs. Cells were stimulated with TNF-α (20 ng/ml) and IL-17A (50 ng/ml) for 0 to 24 hours. (I) The figure shows the EAE clinical scores of wild-type (black circle; n = 8) and Regnase-1ΔCTD/ΔCTD mice (black square; n = 8) over 28 days. (J and K) The figures show the flow cytometry analysis results of spinal cord cells (1.0 x 10⁶ cells) 28 days after immunization. (J) The figure shows the populations of CD4+ T cells (upper) and F4/80+ macrophages (lower) in the spinal cord from wild-type and Regnase-1ΔCTD/ΔCTD mice. (K) The figure shows the number of CD4+ T cells and F4/80 macrophages in (I) (n = 5 for each group). The error bars represent mean ± SEM. *P < 0.05, **P < 0.01, and ***P < 0.005.

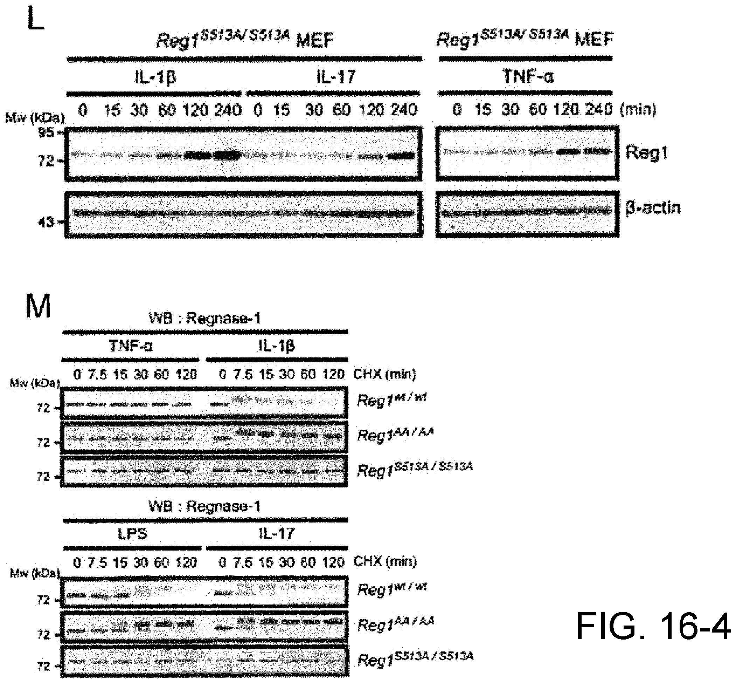

[Figure 16-4] (L) The figure shows the immunoblot analysis results of Regnase-1 and β-actin in Regnase-1 S513A MEFs stimulated with IL-1β, IL-17A, or TNF-α for 0 to 4 hours. (M) The figure shows the immunoblot analysis results of Regnase-1 in wild-type, Regnase-1AA/AA, and Regnase-1 S513A MEFs. Cells were stimulated with TNF-α, IL-1β, LPS, or IL-17A in the presence of cycloheximide, a transcriptional repression agent, for 0 to 120 minutes.

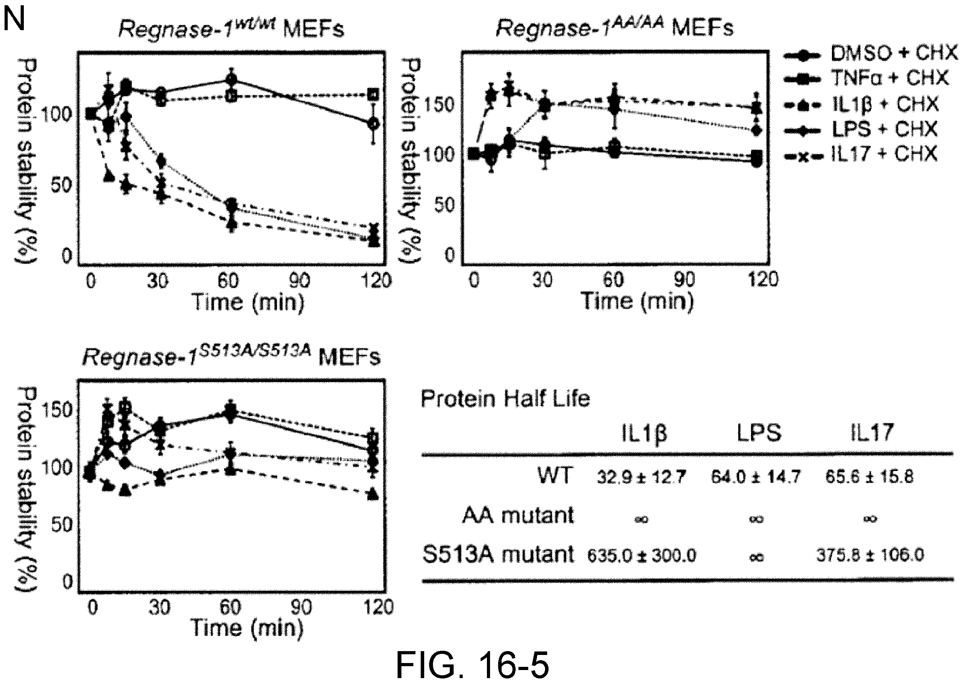

[Figure 16-5] (N) The figure shows the quantitative results of the Regnase-1 protein measured by immunoblot and normalized using β-actin (control). The figure also shows the half-life of the Regnase-1 protein calculated therefrom.

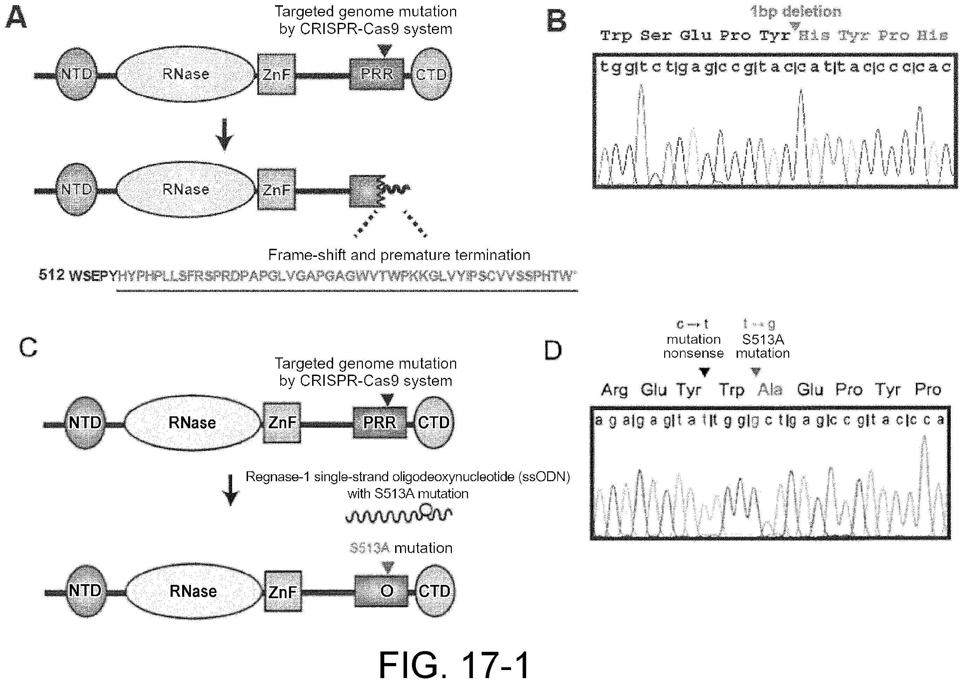

[Figure 17-1] (A) The figure shows the schematic diagram of wild-type Regnase-1 (upper) and Regnase-1 with 1 bp deletion (lower). The CRISPR-Cas9 targeting site is located in the proline-rich region of Regnase-1. The amino acid sequence introduced by the frameshift mutation and the premature stop codon 146 base pairs downstream of the mutation is underlined. (B) Sequencing of Regnase-1 exon 6 in the mouse genome mutated using the CRISPR-Cas9 system. The sequence chromatogram shows the deletion of a cytosine base at Pro517 for initiating the frameshift mutation. (C) The figure shows the schematic diagram of wild-type Regnase-1 (upper) and Regnase-1 with S513A mutation (lower). The CRISPR-Cas9 targeting site is located at Ser513 of Regnase-1. (D) Sequencing of Regnase-1 exon 6 in the mouse genome mutated using the CRISPR-Cas9 system. The sequence chromatogram shows the replacement of TAC with TAT (nonsense mutation) at Tyr511 and the replacement of TCT with GCT (S513A mutation) at Ser513, respectively.

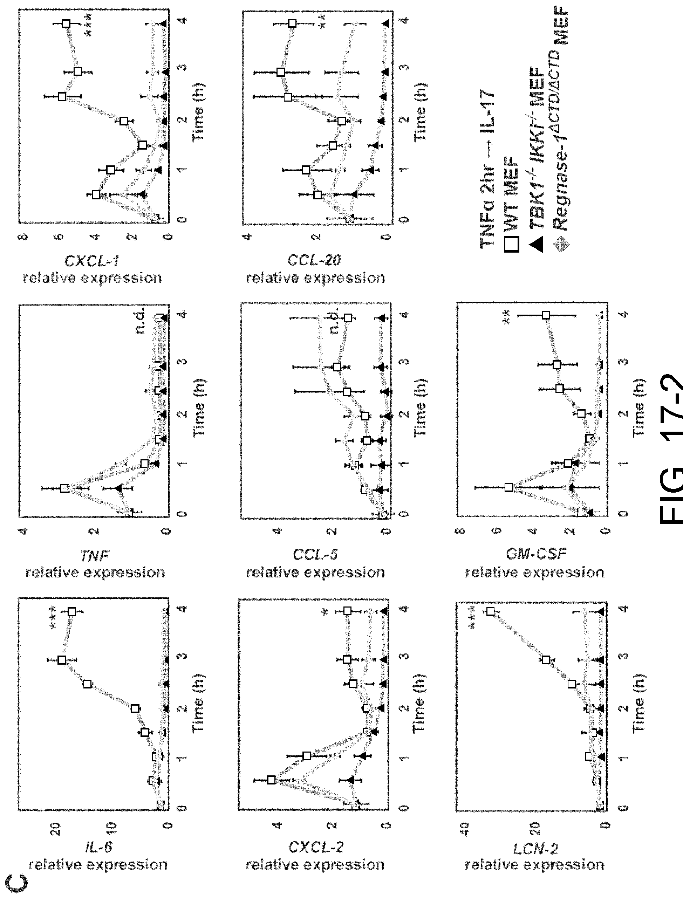

[Figure 17-2] (C) The figure shows the qPCR analysis results of IL-6, TNF, CXCL-1, CXCL-2, CCL-5, CCL20, LCN-2, and GM-CSF expression in wild-type, Regnase-1ΔCTD mutant, and TBK1/IKKi double-deficient MEFs. The error bars represent mean ± SEM. *P < 0.05, **P < 0.01, and ***P < 0.005.

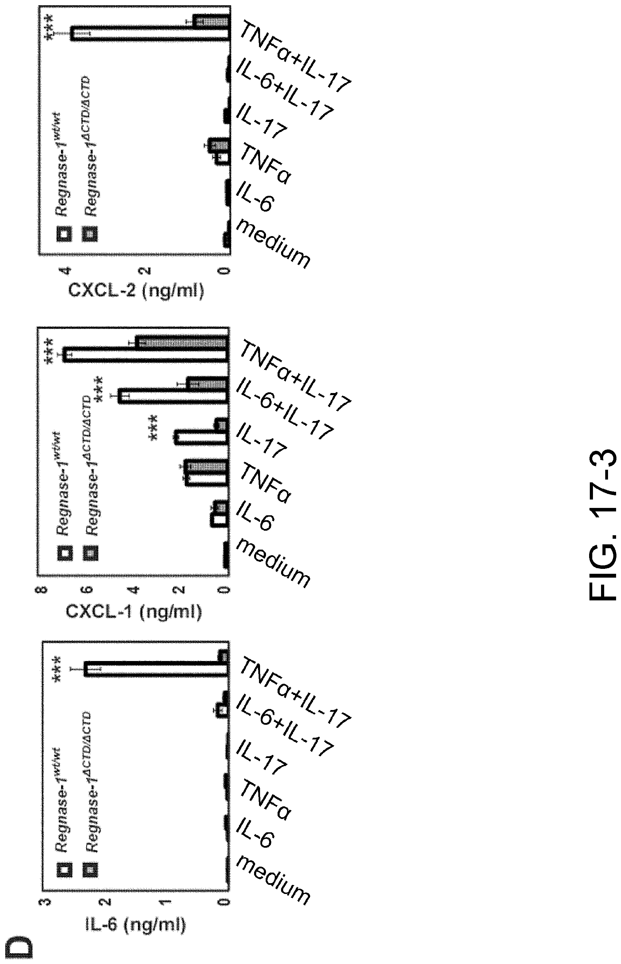

[Figure 17-3] (D) Production of IL-6, CXCL-1, and CXCL-2 by wild-type and Regnase-1ΔCTD/ΔCTD MEFs in response to 24 hours exposure to IL-6 (20 ng/ml), TNF-α (20 ng/ml), IL-17A (50 ng/ml), IL-6 + IL-17A, or TNF-α + IL-17A. Protein production in cell supernatants was evaluated by ELISA. The error bars represent mean ± SEM. *P < 0.05, **P < 0.01, and ***P < 0.005.

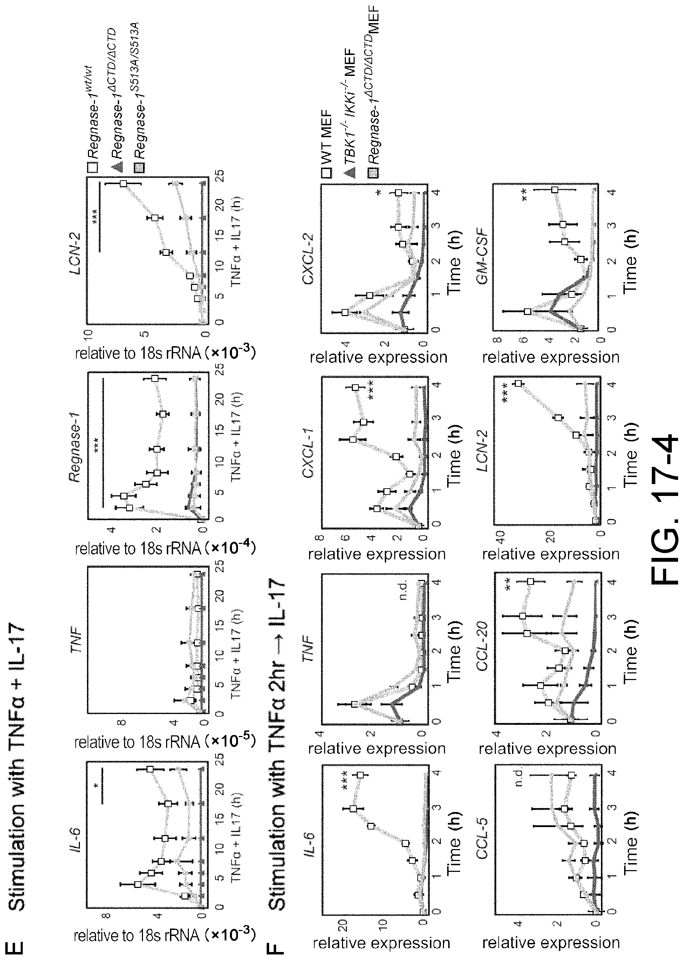

[Figure 17-4] (E) The figure shows the qPCR analysis results of IL-6, TNF, Regnase-1, and LCN-2 mRNA in wild-type, Regnase-1ΔCTD/ΔCTD, and Regnase-1 S513A MEFs. Cells were stimulated by TNF-α and IL-17A for 0 to 24 hours. (F) The figure shows the qPCR analysis results of IL-6, TNF, CXCL-1, CXCL-2, CCL-5, CCL-20, LCN-2, and GM-CSF mRNA in wild-type, Regnase-1ΔCTD/ΔCTD, and TBK1/IKKi double-deletion MEFs. Cells were stimulated with TNF-α (20 ng/ml) for two hours, and subsequently with IL-17A (50 ng/ml) for 0 to 4 hours.

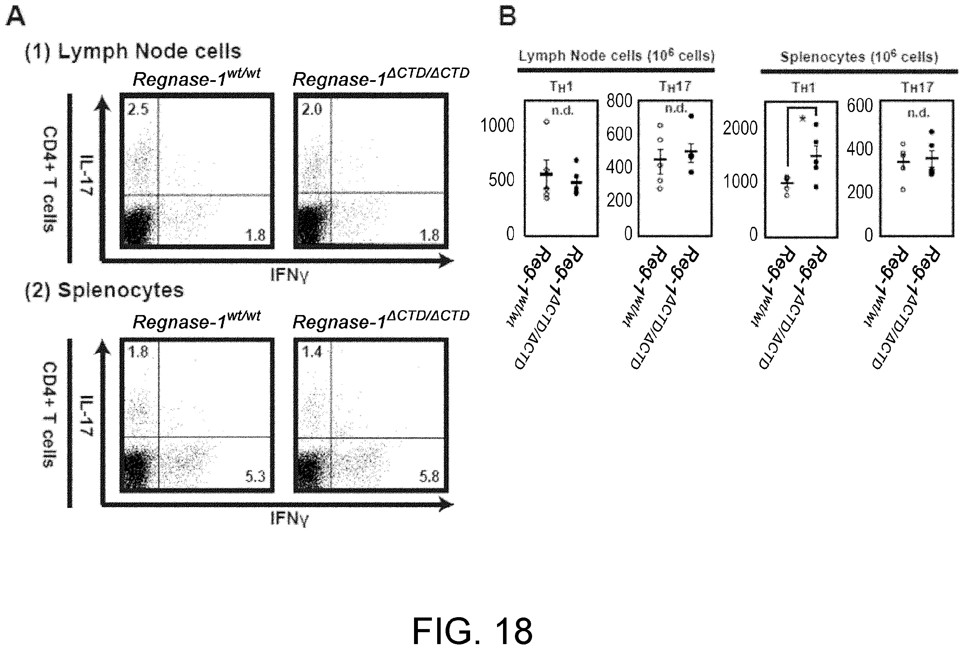

[Figure 18] (A) The figure shows the flow cytometry analysis results of CD4+ T cell subsets (TH1 and TH17) in (1) lymph node cells (1.0 x 10⁶ cells) and (2) splenocytes (1.0 x 10⁶ cells) from wild-type and Regnase-1ΔCTD/ΔCTD mice 28 days after EAE immunization. (B) The figure shows the number of TH1 cells and TH17 cells in (A) (n = 5 for each group). The error bars represent mean ± SEM. *P < 0.05.

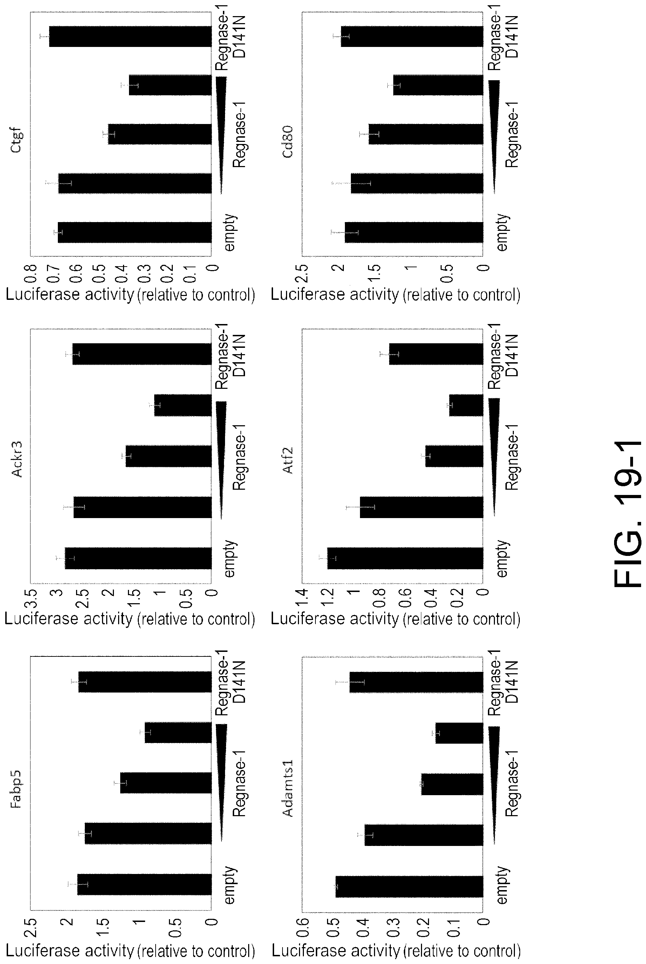

[Figure 19-1] The figure shows the luciferase assay results of each of the target genes. The values of the measured firefly luciferase activities that are normalized using internal standards, Renilla luciferase activities and pGL3-empty plasmids, are shown. The error bars represent the standard deviation (S.D.) of two sets.

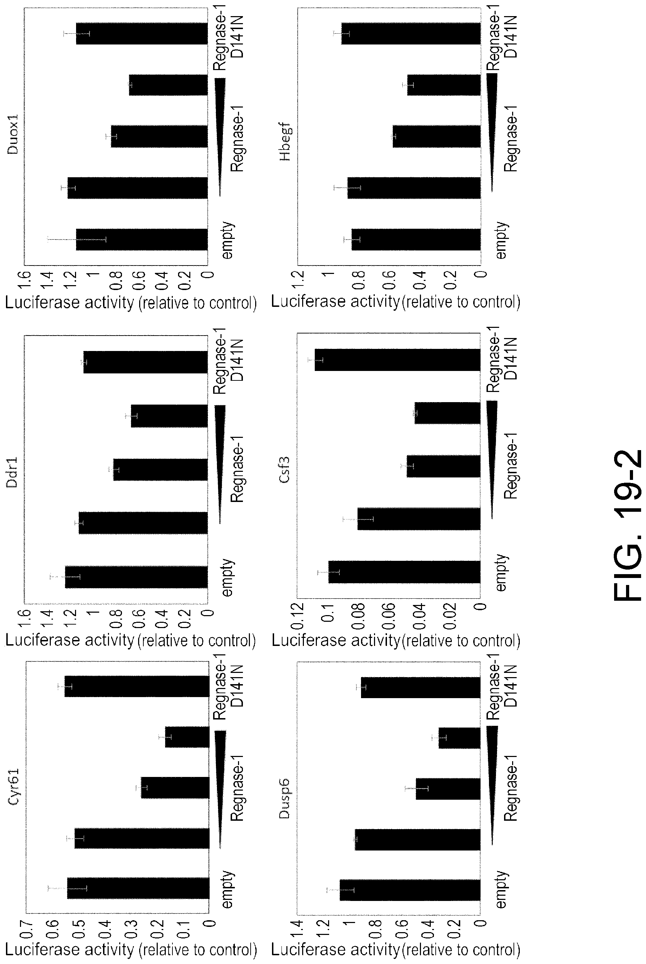

[Figure 19-2] The figure shows the continuation of Figure 19-1.

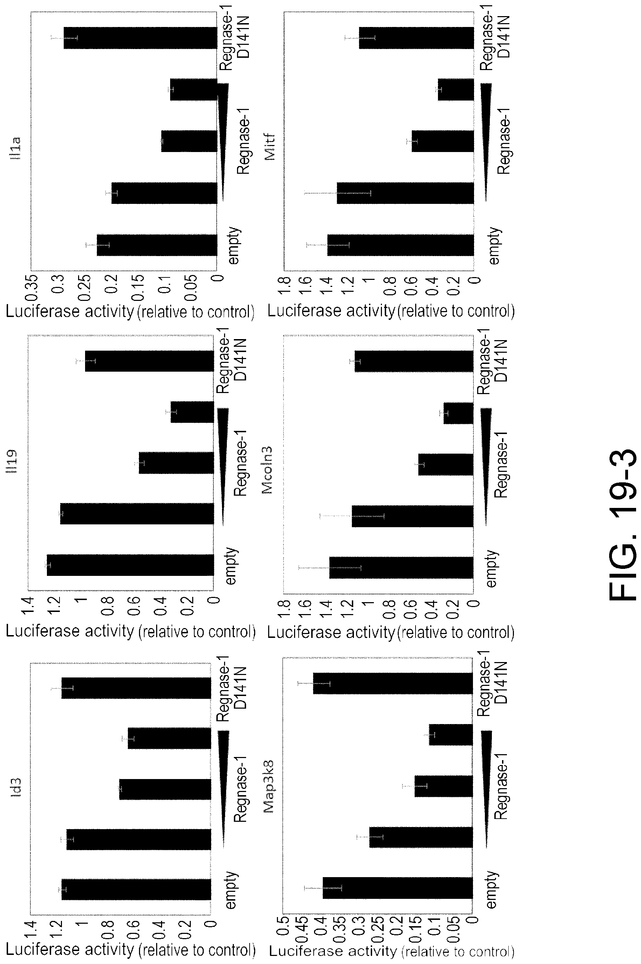

[Figure 19-3] The figure shows the continuation of Figure 19-2.

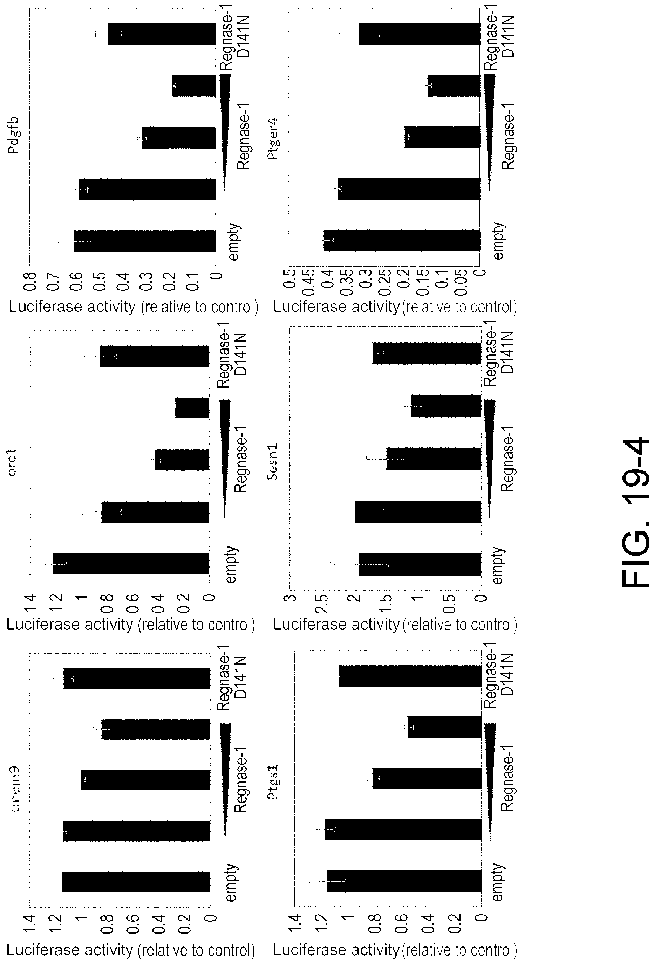

[Figure 19-4] The figure shows the continuation of Figure 19-3.

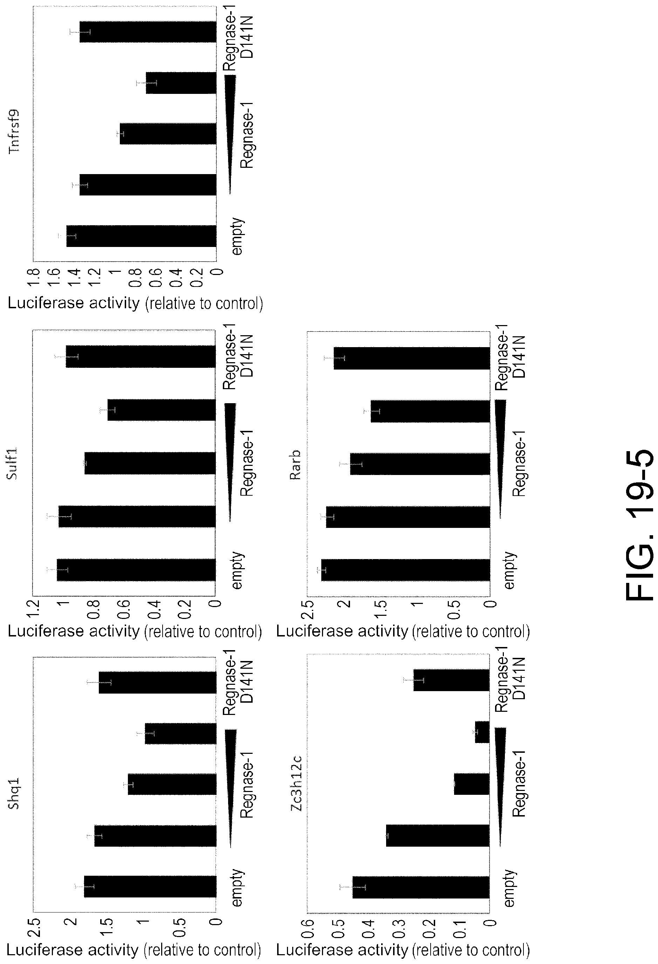

[Figure 19-5] The figure shows the continuation of Figure 19-4.

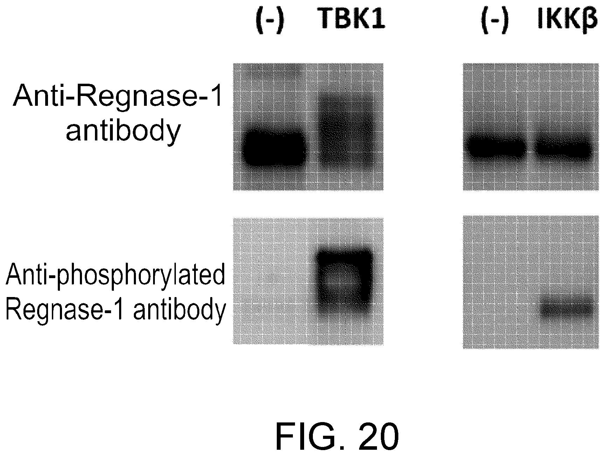

[Figure 20] The figure shows the results of detecting phosphorylated Regnase-1 by western blotting.

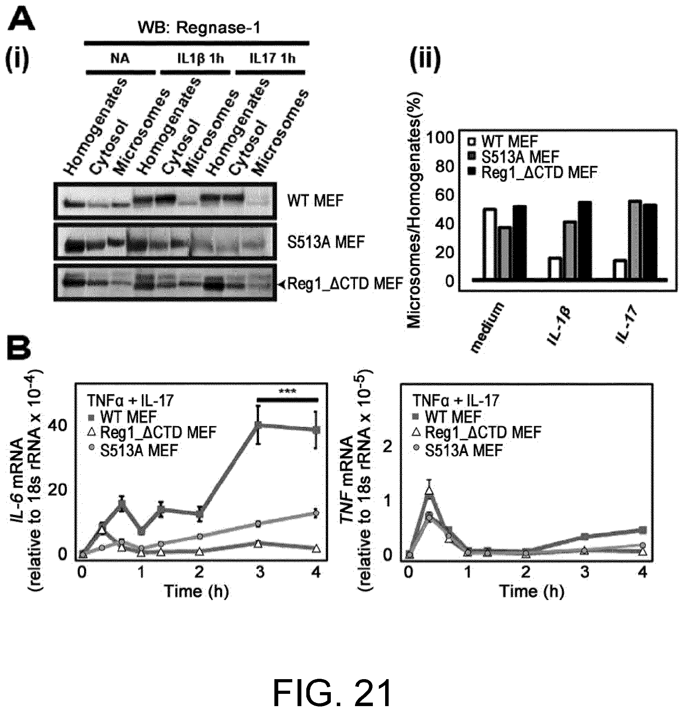

[Figure 21] (A) (i) The immunoblot analysis of Regnase-1 in intracellular organelle fractions (cell homogenates, cytosolic fractions, and microsomes) prepared from the following mutant MEF cell lines: wild-type, Regnase-1 S513A (Ser513 is replaced with Ala), and Regnase-1 ΔCTD (lacking a C-terminal domain) stimulated with IL-1β (10 ng/ml) or IL-17A (50 mg/ml) for one hour. (ii) The protein levels of Regnase-1 bound to microsomes were evaluated as a percentage relative to the total cell homogenates. (B) The qPCR analysis of IL-6 and TNF mRNA in wild-type, Regnase-1 S513A, and Regnase-1 ΔCTD MEFs co-stimulated with TNF-α (20 ng/ml) and IL-17A (50 ng/ml) for 0 to 4 hours.

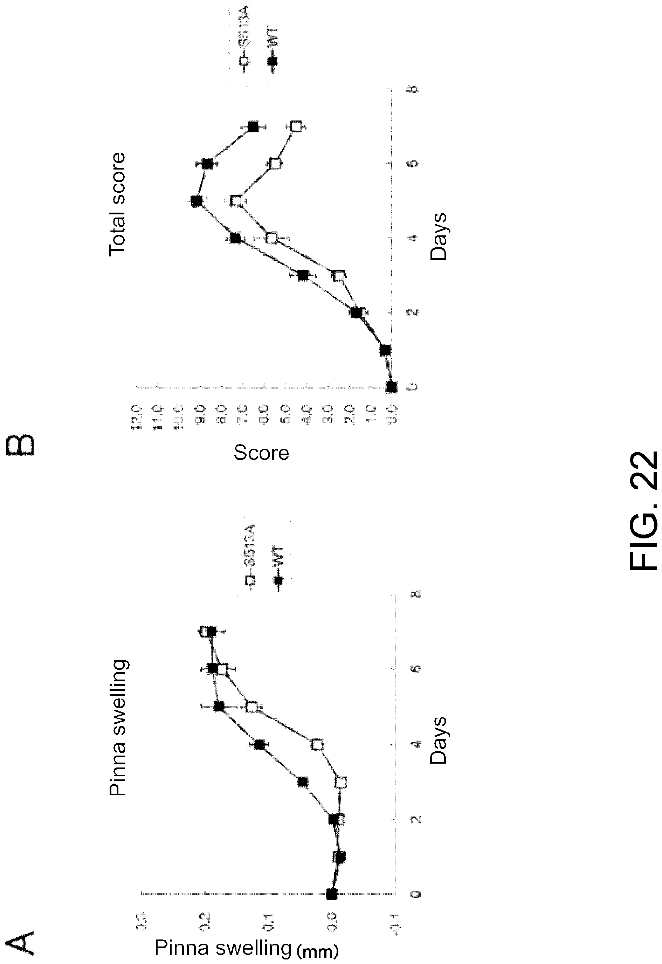

[Figure 22] (A) The figure shows the thickness of the pinna at the application site of wild-type mice and Regnase-1 S513A mutant mice in imiquimod-induced psoriasis model (mean ± standard error; six mice for each group). (B) The figure shows the total scores of macroscopic findings in the dorsocervical skin of wild-type mice and Regnase-1 S513A mutant mice in imiquimod-induced psoriasis model (mean ± standard error; six mice for each group).

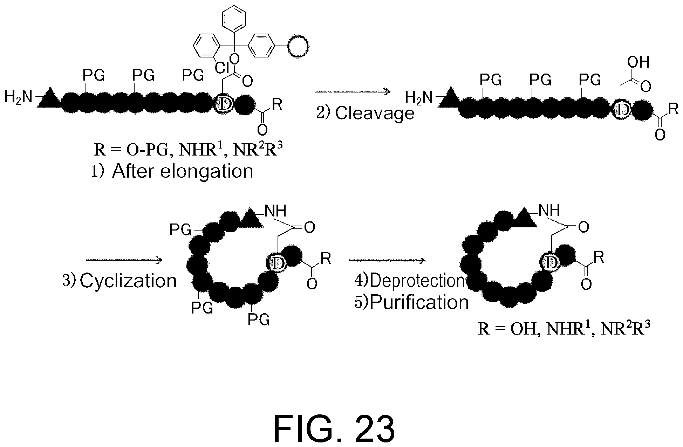

[Figure 23] The figure shows the synthetic scheme of a peptide compound. The scheme mainly includes the five steps: (1) the elongation reaction of a peptide on a resin; (2) cleavage of the peptide from the resin; (3) the cyclization reaction of the peptide; (4) deprotection of functional groups on side chains of the peptide; and (5) purification of the peptide.

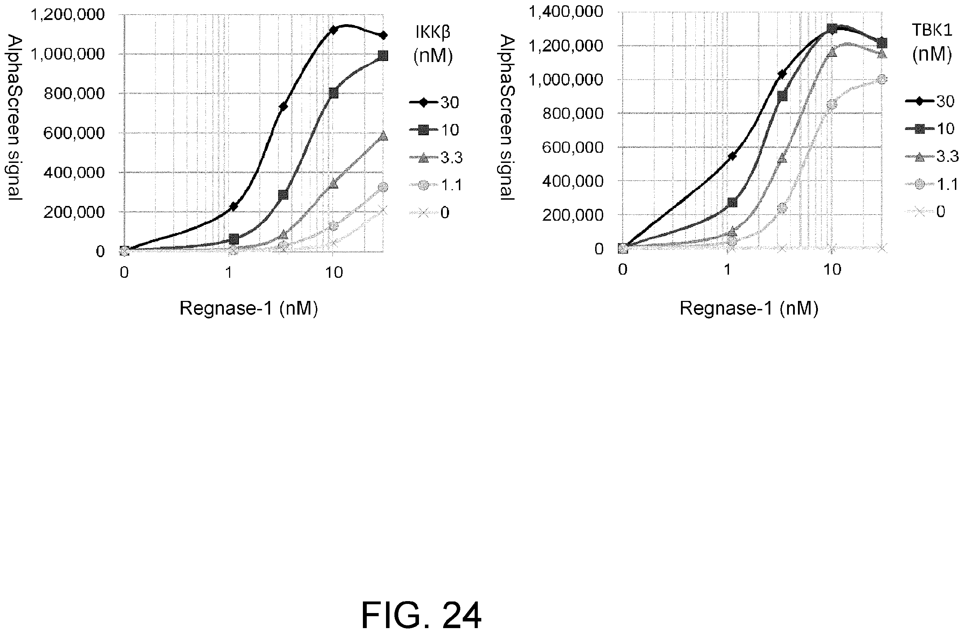

[Figure 24] The figure shows the results of detecting phosphorylated Regnase-1 by AlphaScreen. Regnase-1 was phosphorylated by reacting Regnase-1 with kinase (IKKβ or TBK1) in the presence of ATP. After the above reaction, Regnase-1 was reacted with an antibody against phosphorylated Regnase-1, and the bonding amount was measured by AlphaScreen. The left of the figure and the right of the figure show the result using IKKβ and TBK1, respectively.

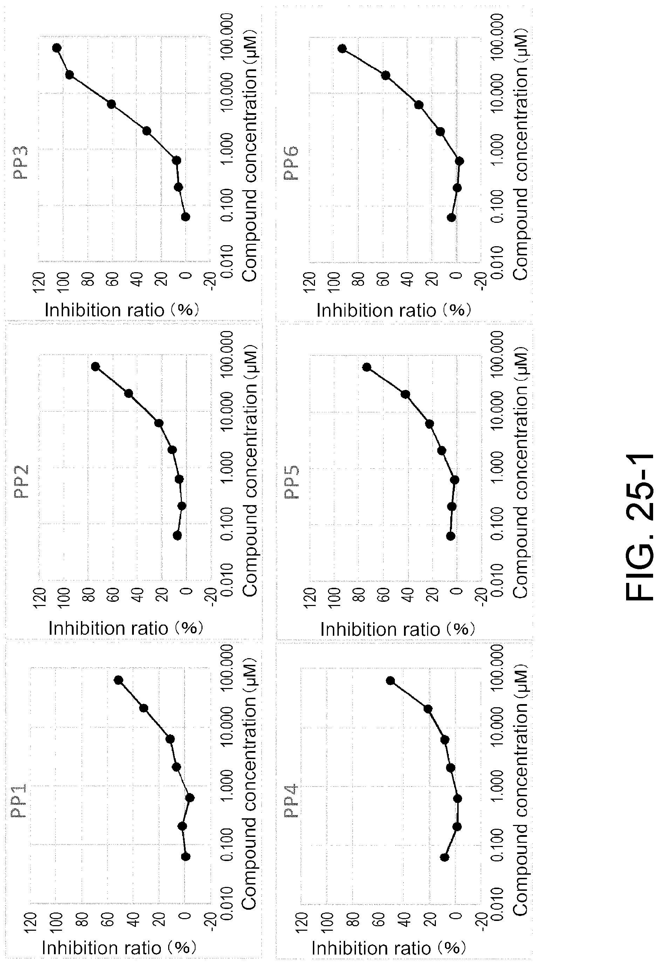

[Figure 25-1] The figure shows the results of inhibiting phosphorylation of full-length Regnase-1 (FL_Reg1) by a compound. FL_Reg1 was mixed with the compound (PP1, PP2, PP3, PP4, PP5, or PP6), and then phosphorylated with TBK1. The phosphorylated Regnase-1 thus generated was measured by AlphaScreen.

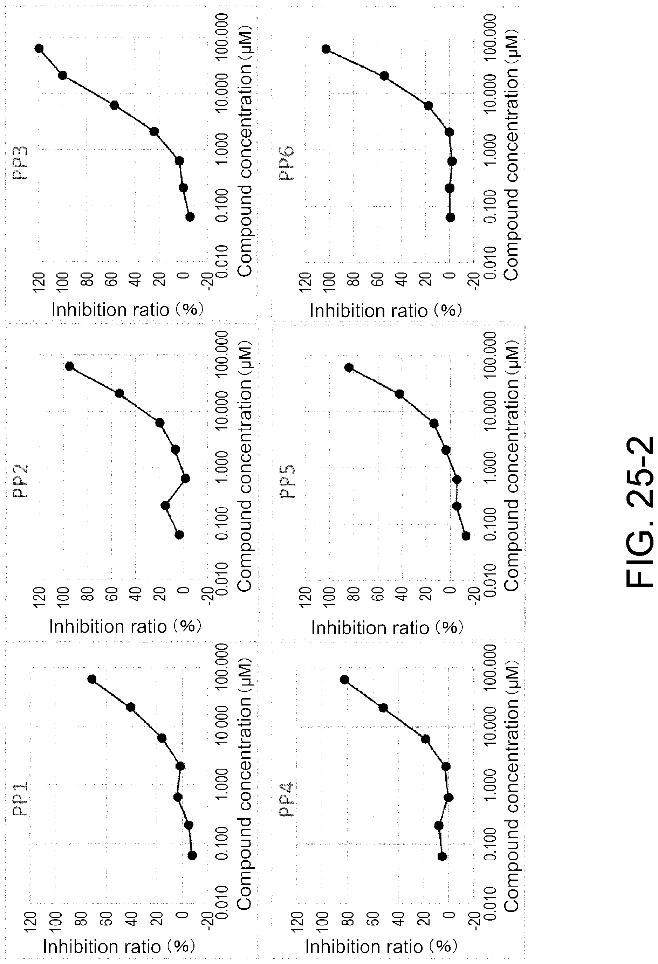

[Figure 25-2] The figure shows the results of inhibiting phosphorylation of full-length Regnase-1 (FL_Reg1) by a compound. FL_Reg1 was mixed with the compound (PP1, PP2, PP3, PP4, PP5, or PP6), and then phosphorylated with IKKβ. The phosphorylated Regnase-1 thus generated was measured by AlphaScreen.

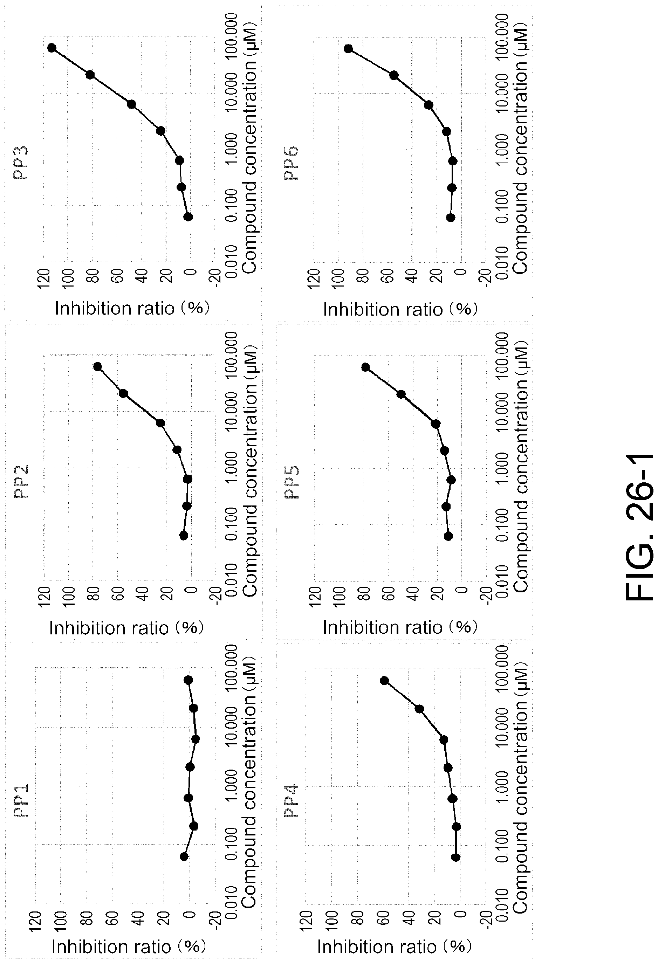

[Figure 26-1] The figure shows the results of inhibiting phosphorylation of C-terminal domain-deleted Regnase-1 (ΔCTD_Reg1) by a compound. ΔCTD_Reg1 was mixed with the compound (PP1, PP2, PP3, PP4, PP5, or PP6), and then phosphorylated with TBK1. The phosphorylated Regnase-1 thus generated was measured by AlphaScreen.

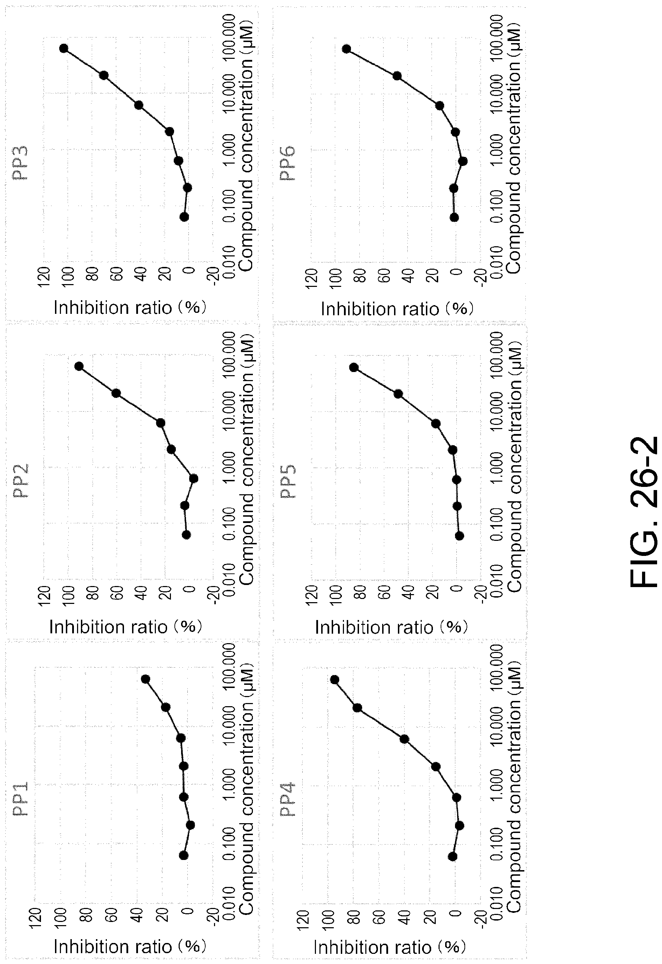

[Figure 26-2] The figure shows the results of inhibiting phosphorylation of C-terminal domain-deleted Regnase-1 (ΔCTD_Reg1) by a compound. ΔCTD_Reg1 was mixed with the compound (PP1, PP2, PP3, PP4, PP5, or PP6), and then phosphorylated with IKKβ. The phosphorylated Regnase-1 thus generated was measured by AlphaScreen.

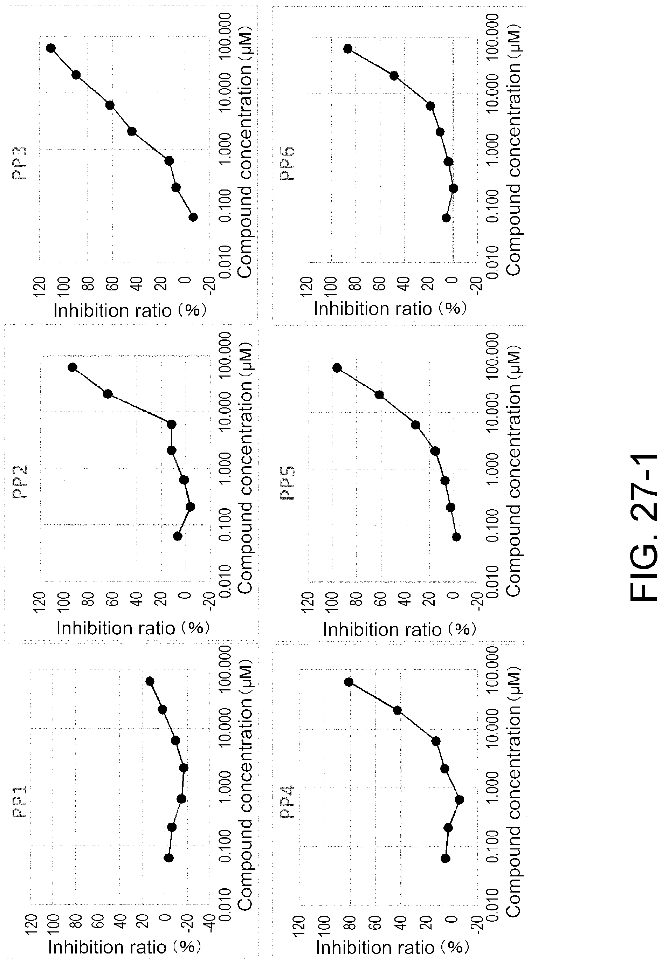

[Figure 27-1] The figure shows the results of inhibiting the binding of kinase and Regnase-1 by a compound. Full-length Regnase-1 (FL_Reg1) was mixed with the compound (PP1, PP2, PP3, PP4, PP5, or PP6), then TBK1 was added thereto, and the binding between TBK1 and Regnase-1 was measured by AlphaScreen.

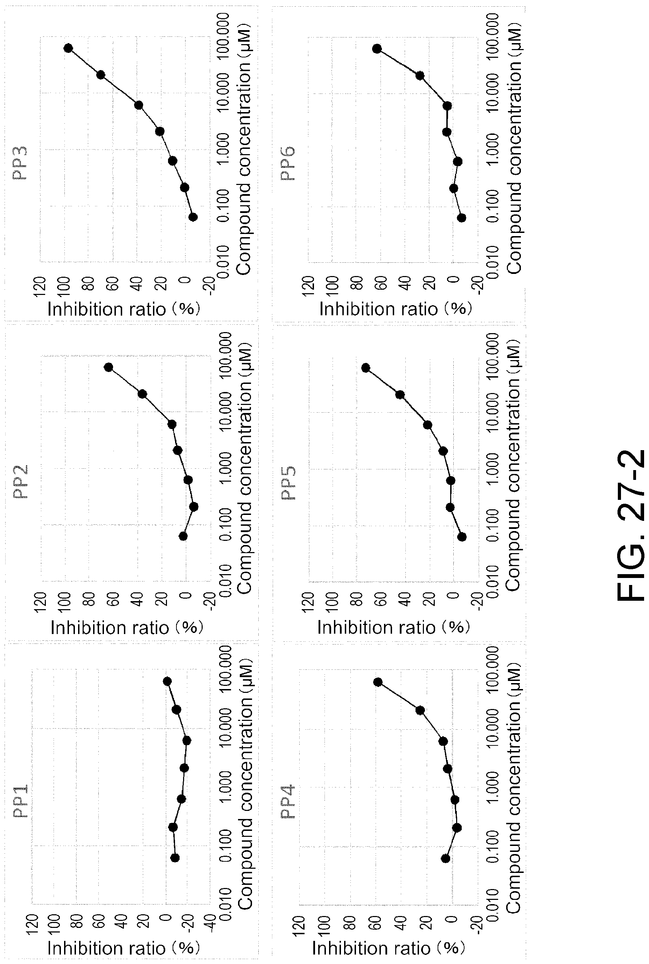

[Figure 27-2] The figure shows the results of inhibiting the binding of kinase and Regnase-1 by a compound. Full-length Regnase-1 (FL_Reg1) was mixed with the compound (PP1, PP2, PP3, PP4, PP5, or PP6), then IKKβ was added thereto, and the binding between IKKβ and Regnase-1 was measured by AlphaScreen.

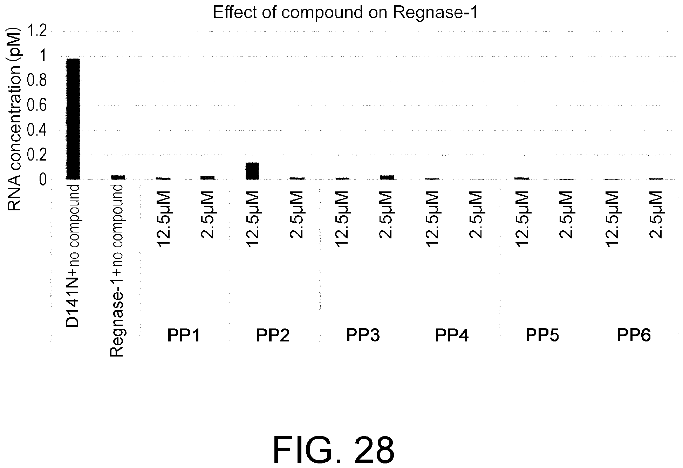

[Figure 28] The figure shows the effect of a compound on the RNA degrading activity of wild-type Regnase-1. To the mixture solution of RNA and wild-type Regnase-1, the compound (PP1, PP2, PP3, PP4, PP5, or PP6) was added and reacted, and then the RNA concentration in the reaction solution was measured.

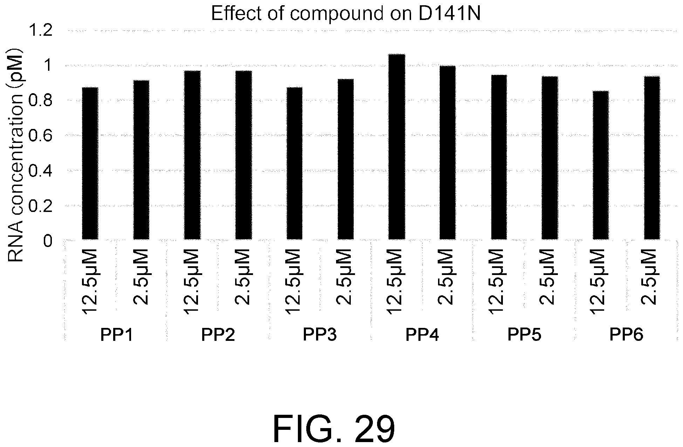

[Figure 29] The figure shows the effect of a compound on the RNA degrading activity of mutant Regnase-1 (D141N). To the mixture solution of RNA and mutant Regnase-1 (D141N), the compound (PP1, PP2, PP3, PP4, PP5, or PP6) was added and reacted, and then the RNA concentration in the reaction solution was measured.

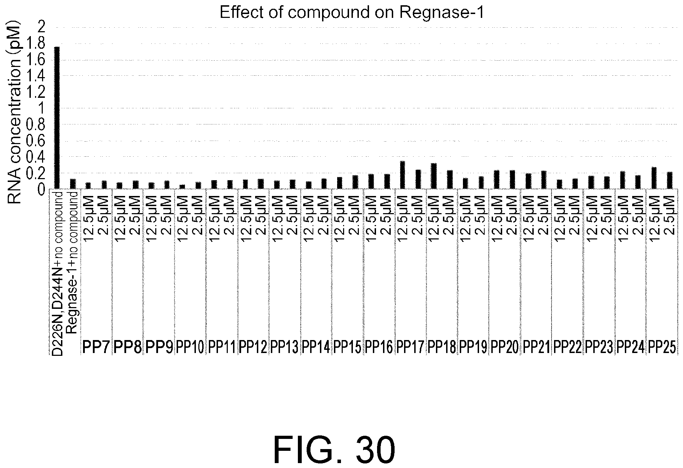

[Figure 30] The figure shows the effect of a compound on the RNA degrading activity of wild-type Regnase-1. To the mixture solution of RNA and wild-type Regnase-1, the compound (each of compounds PP7 to PP25) was added and reacted, and then the RNA concentration in the reaction solution was measured.

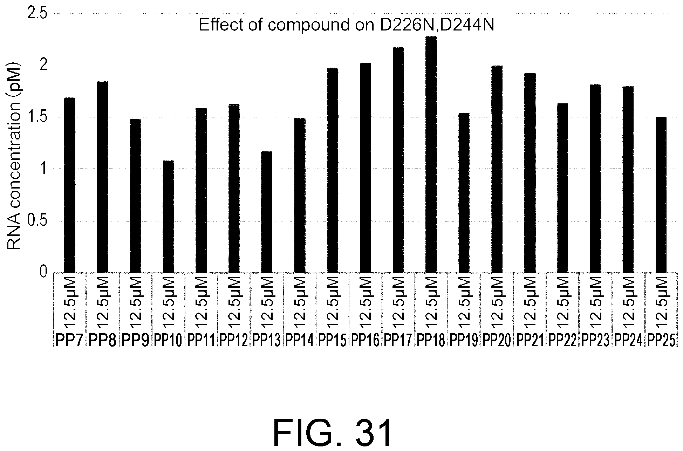

[Figure 31] The figure shows the effect of a compound on the RNA degrading activity of mutant Regnase-1 (D226N,D244N). To the mixture solution of RNA and mutant Regnase-1 (D226N,D244N), the compound (each of compounds PP7 to PP25) was added and reacted, and then the RNA concentration in the reaction solution was measured.

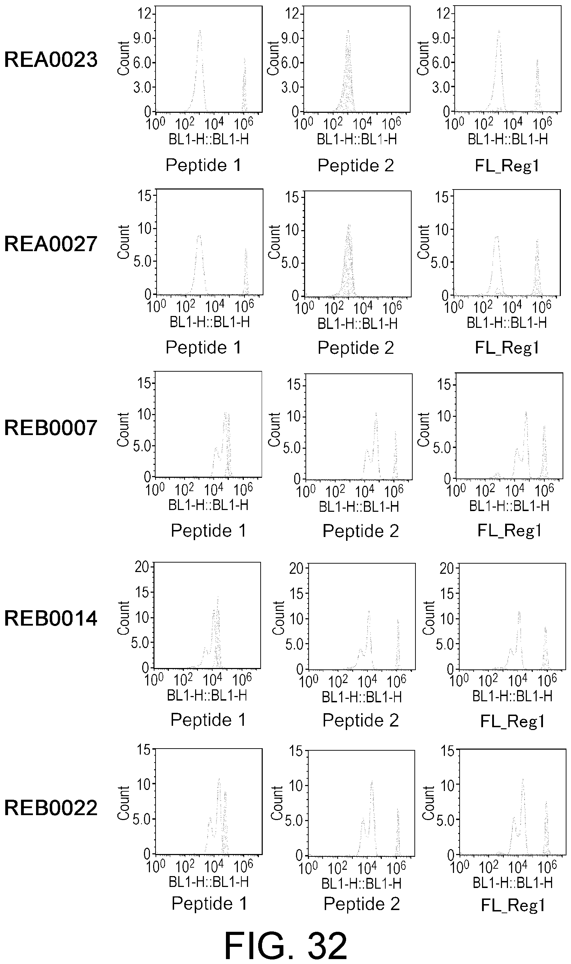

[Figure 32] The figure shows the binding of an anti-Regnase-1 antibody to a human Regnase-1 peptide and human full-length Regnase-1. REA0023, REA0027, REB0007, REB0014, and REB0022 were used as the anti-Regnase-1 antibody. Both of peptide 1 (CLDSGIGSLESQMSELWGVRGG) and peptide 2 (AFPPREYWSEPYPLPPPTC-NH2) in the figure are partial peptides of human Regnase-1. FL_Reg1 represents human full-length Regnase-1.

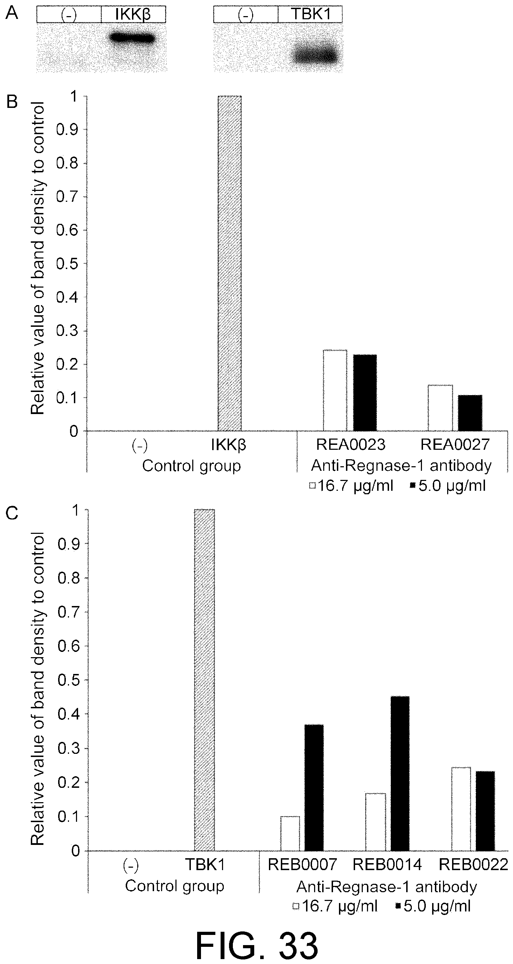

[Figure 33] The figure shows the results of evaluating the activity of an anti-Regnase-1 antibody to inhibit Regnase-1 phosphorylation. (A) The figure shows the results of detecting Regnase-1 phosphorylation by each kinase (IKKβ or TBK1) by western blotting. (B) The figure shows the inhibitory activity of the anti-Regnase-1 antibody (REA0023 and REA0027) against Regnase-1 phosphorylation by IKKβ. The anti-Regnase-1 antibody both at the final concentration of 16.7 µg/ml and 5.0 µg/ml was evaluated. (C) The figure shows the inhibitory activity of the anti-Regnase-1 antibody (REB0007, REB0014, and REB0022) against Regnase-1 phosphorylation by TBK1. The anti-Regnase-1 antibody both at the final concentration of 16.7 µg/ml and 5.0 µg/ml was evaluated.

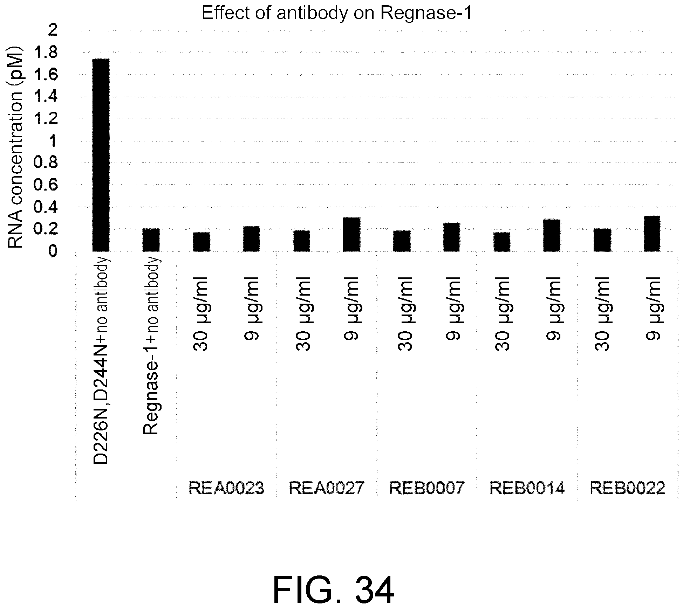

[Figure 34] The figure shows the effect of an anti-Regnase-1 antibody on the RNA degrading activity of wild-type Regnase-1. To the mixture solution of RNA and wild-type Regnase-1, the anti-Regnase-1 antibody (REA0023, REA0027, REB0007, REB0014, and REB0022) was added and reacted, and then the RNA concentration in the reaction solution was measured.

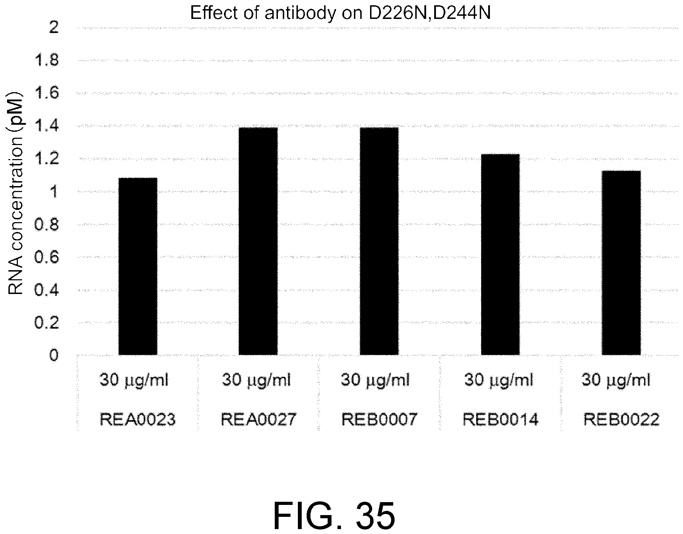

[Figure 35] The figure shows the effect of an anti-Regnase-1 antibody on the RNA degrading activity of mutant Regnase-1 (D226N,D244N). To the mixture solution of RNA and mutant Regnase-1 (D226N,D244N), the anti-Regnase-1 antibody (REA0023, REA0027, REB0007, REB0014, and REB0022) was added and reacted, and then the RNA concentration in the reaction solution was measured.

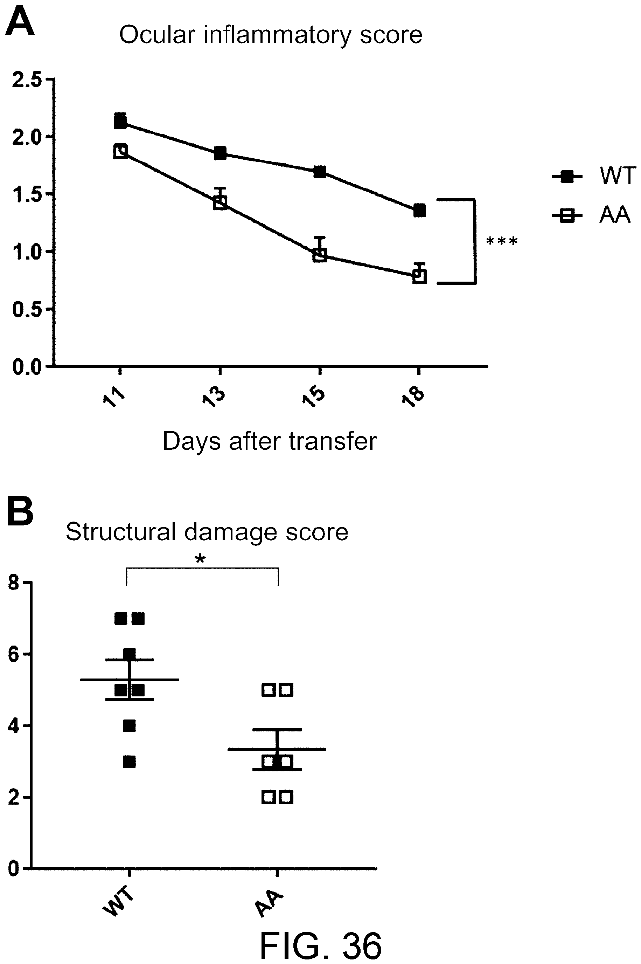

[Figure 36] The figures show the pathological analysis results of wild-type mice and Regnase-1 AA mutant mice in experimental autoimmune uveitis T cell transfer model. (A) The figure shows the inflammatory scores in the funduscopic examination (mean ± standard error; seven mice for each group). Statistical analysis was performed by calculating the AUC of each individual and using Mann-Whitney U test (***: P < 0.001). (B) The figure shows the structural damage scores in the histopathological analysis. Statistical analysis was performed using Mann-Whitney U test (*: P < 0.05).

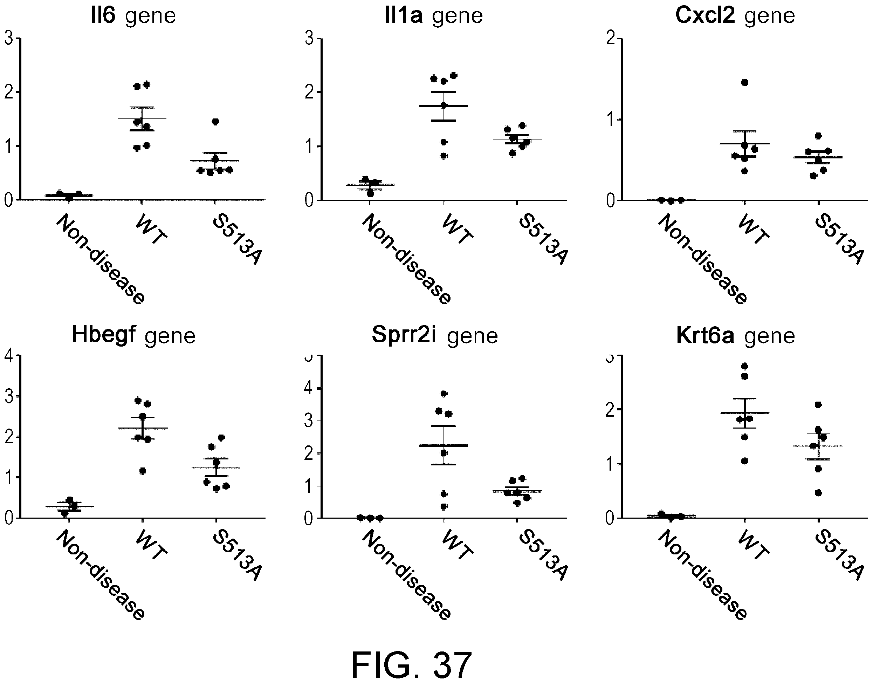

[Figure 37] The figure shows the change in the expression of various genes in the skin of the pinna at the application site of wild-type mice and S513A mutant mice in imiquimod-induced psoriasis model (normalized using the B2m expression level, mean ± standard error). "Non-disease" in the figure indicates mice where disease was not induced.

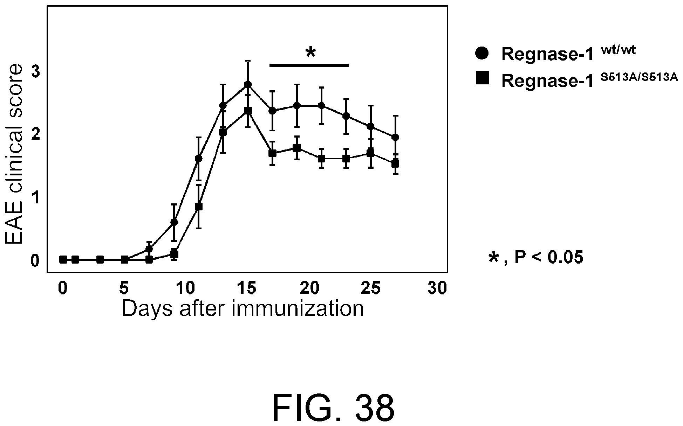

[Figure 38] The figure shows the EAE clinical scores of wild-type (black circle; n = 10) and Regnase-1 S513A mutant mice (black square; n = 10).

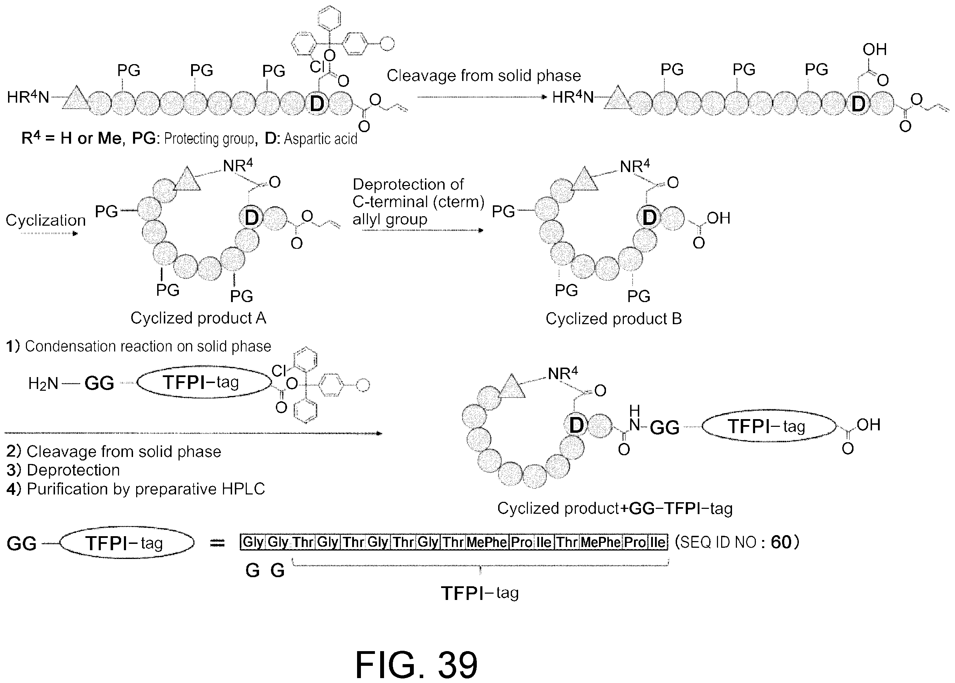

[Figure 39] The figure shows the synthetic scheme of a compound where GG-TFPI-tag is linked to the C-terminus of a cyclic polypeptide.

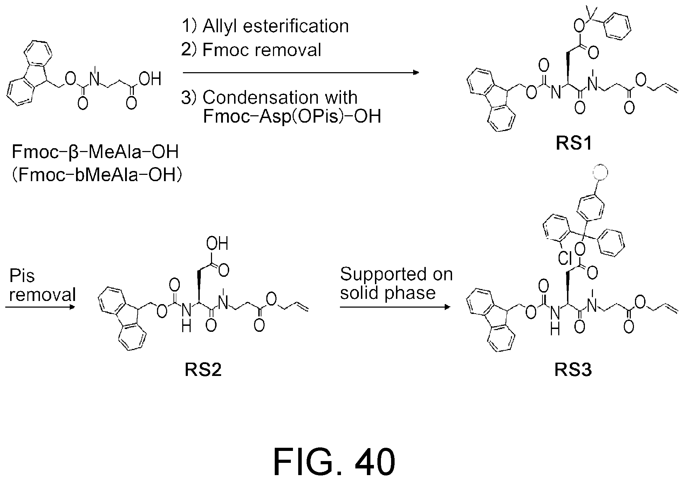

[Figure 40] The figure shows the synthetic scheme of Fmoc-Asp(O-Trt(2-Cl)-resin)-bMeAla-OAllyl (compound RS3).

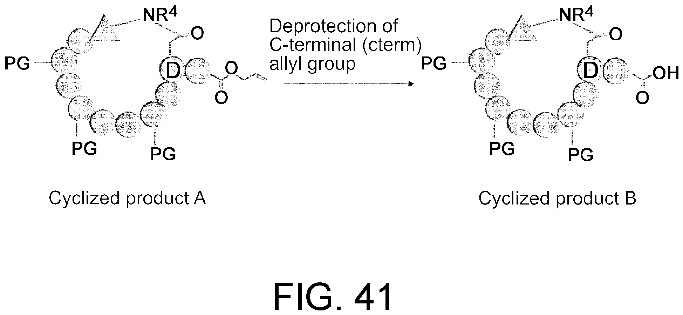

[Figure 41] The figure shows the process of synthesizing cyclized product B from cyclized product A.

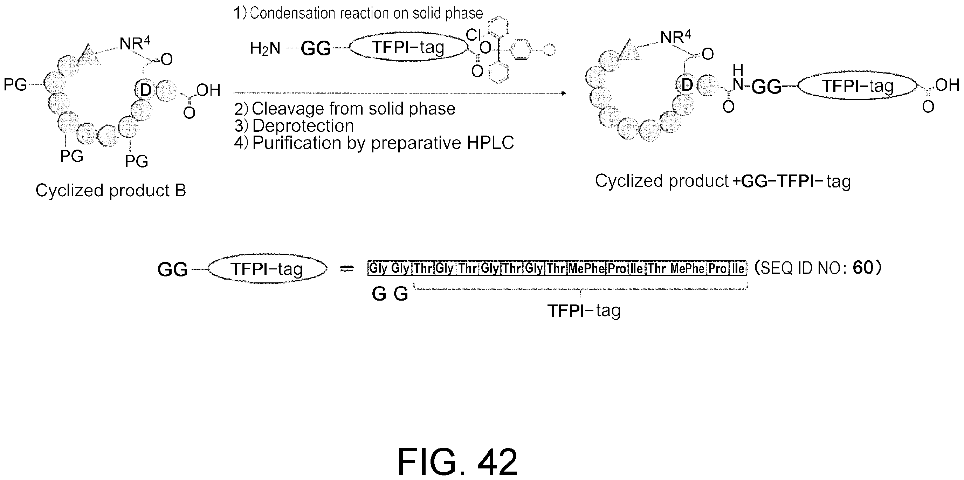

[Figure 42] The figure shows the process of synthesizing a cyclized product+GG-TFPI-tag compound from cyclized product B.

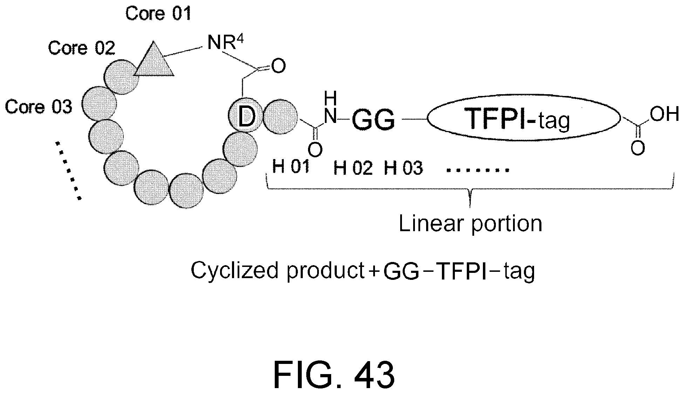

[Figure 43] The figure shows the structural information of a cyclized product+GG-TFPI-tag compound.

### [Description of Embodiments]

### 1. Definition

The term "Regnase-1" (also known as Zc3h12a or MCPIP-1) as described herein refers to any natural form Regnase-1 from any vertebrate sources including mammals such as primates (for example, humans) and rodents (for example, mice and rats) unless otherwise indicated. The term includes "full-length" unprocessed Regnase-1 and Regnase-1 resulting from processing in cells. An exemplary mouse Regnase-1 amino acid sequence is published as Uniprot Accession No: Q5D1E7 (SEQ ID NO: 1) and an exemplary human Regnase-1 amino acid sequence is published as Uniprot Accession No: Q5D1E8 (SEQ ID NO: 2). References describing Regnase-1 include, for example, WO 2010/098429; Nature immunology, Vol. 12, NUMBER 12, DECEMBER 2011, p. 1167-1175; Nature 458, 2009, p. 1185-1190; Cold Spring Harbor Symposia on Quantitative Biology, Volume LXXVIII, 2013, p. 51-60; and Biochimica et Biophysica Acta 1823, 2012, p. 1905-1913. Regnase-1 herein is preferably mammalian Regnase-1.

A "disease associated with Regnase-1" as used herein means a disease the formation, exacerbation, and/or continuation of which are associated with Regnase-1. A "disease the formation, exacerbation, and/or continuation of which are associated with ..." includes not only diseases the formation, exacerbation, and/or continuation of which are directly associated with ... but also diseases the formation, exacerbation, and/or continuation of which are indirectly associated with .... A "disease associated with Regnase-1" may mean such as, but is not limited to, a disease the formation, exacerbation, and/or continuation of which are associated with destabilization and/or intracellular degradation of Regnase-1. A "disease associated with Regnase-1" includes inflammatory diseases, autoimmune diseases, allergic diseases, fibrotic diseases, and RNA virus infection. A "disease associated with Regnase-1" may be a TH17 cell-related disease.

An "inflammatory disease" as used herein is a disease or illness arising from hyperactivation of an individual's immune system. Inflammatory diseases can arise from the pathological stage that causes inflammation, typically flux of leukocytes and/or neutrophil chemotaxis, but are not limited to the ones resulting from the above. Examples of such diseases include inflammatory skin diseases (including psoriasis and atopic dermatitis); systemic sclerosis; nephritis; responses related to inflammatory bowel diseases (Crohn's disease and ulcerative colitis); ischemic perfusion diseases including surgical tissue reperfusion injury, myocardial ischemic symptoms such as myocardial failure, heart failure, perfusion after cardiac surgery and perfusion after percutaneous transluminal coronary angioplasty, stroke, and abdominal aortic aneurysm; postictal cerebral edema; cranial trauma; hypovolemic shock; respiratory arrest; adult respiratory distress syndrome; acute lung injury; Behçet's disease; dermatomyositis; polymyositis; multiple sclerosis; dermatitis; meningitis; encephalitis; uveitis; ocular inflammation; diabetic retinopathy; diabetic macular edema; osteoarthritis; lupus nephritis; diabetic nephropathy; autoimmune diseases such as rheumatoid arthritis, Sjögren's syndrome, and vasculitis; spondyloarthritis (including ankylosing spondylitis and psoriatic arthritis); diseases including leukocyte leakage; multiple organ injury after central nervous system (CNS) inflammatory diseases, sepsis, or trauma; alcoholic hepatitis; bacterial pneumonia; antigen-antibody complex mediated diseases including glomerulonephritis; sepsis; sarcoidosis; graft rejection after tissue/organ transplantation; graft-versus-host disease; age-related macular degeneration; Kawasaki disease; eosinophilic esophagitis; neuritis; pulmonary inflammation including pleurisy, alveolitis, pneumonia, chronic bronchitis, bronchiectasis, diffuse panbronchiolitis, hypersensitivity pneumonia, idiopathic pulmonary fibrosis (IPF), and cystic fibrosis. Preferred symptoms include acute lung injury, adult respiratory distress syndrome, ischemic perfusion (including surgical tissue perfusion injury, myocardial ischemia, and acute myocardial failure), hypovolemic shock, asthma, bacterial pneumonia, and inflammatory bowel diseases such as ulcerative colitis. Inflammatory diseases overlap in part with diseases in other categories such as autoimmune diseases, allergic diseases, and fibrotic diseases, and vice versa.

An "autoimmune disease" as used herein refers to a disease or disorder that arises from tissues of an individual itself and is directed to tissues of the individual itself. Herein, autoimmune diseases explicitly exclude malignant or cancerous diseases or symptoms, and specifically exclude B-cell lymphoma, acute lymphoblastic leukemia (ALL), chronic lymphocytic leukemia (CLL), hairy cell leukemia, and chronic myeloblastic leukemia. Examples of autoimmune diseases or disorders include, but are not limited to, the following: inflammatory responses such as inflammatory skin diseases including psoriasis and dermatitis (*e.g.,* atopic dermatitis); systemic scleroderma and sclerosis; responses associated with inflammatory bowel diseases (*e.g*., Crohn's disease and ulcerative colitis); respiratory distress syndrome (including adult respiratory distress syndrome (ARDS)); skin inflammation; meningitis; encephalitis; uveitis; ocular inflammation; colitis; glomerulonephritis; allergic conditions such as eczema and asthma, and other conditions accompanied by T cell infiltration and chronic inflammatory responses; atherosclerosis; leukocyte adhesion failure; rheumatoid arthritis; systemic lupus erythematosus (SLE) (including, but not limited to, lupus nephritis and cutaneous lupus); diabetes (*e.g*., type I diabetes mellitus or insulin-dependent diabetes mellitus); multiple sclerosis; Raynaud syndrome; autoimmune thyroiditis; Hashimoto thyroiditis; allergic encephalomyelitis; autoimmune encephalomyelitis; Sjögren's syndrome; juvenile-onset diabetes; as well as immune responses associated with acute and delayed hypersensitivity mediated by cytokines and T lymphocytes typically seen in tuberculosis, sarcoidosis, polymyositis, granulomatosis, and vasculitis; pernicious anemia (Addison's disease); diseases accompanied by leukocyte leakage; central nervous system (CNS) inflammatory disorders; multiple organ injury syndrome; hemolytic anemia (including, but not limited to, cryoglobulinemia or Coombs-positive anemia); myasthenia gravis; antigen-antibody complex-mediated diseases; anti-glomerular basement membrane disease; antiphospholipid syndrome; allergic neuritis; Graves' disease; Lambert-Eaton myasthenic syndrome; bullous pemphigoid; pemphigus; autoimmune polyglandular endocrine disorder; Reiter's disease; Stiffman's syndrome; Behçet's disease; giant cell arteritis; immune complex nephritis; IgA nephropathy; IgM polyneuropathy; and immune thrombocytopenic purpura (ITP) or autoimmune thrombocytopenia. Autoimmune diseases overlap in part with diseases in other categories such as inflammatory diseases, allergic diseases, and fibrotic diseases, and vice versa.

An "allergic disease" as used herein means any symptom, tissue damage, or loss of tissue function that arises from allergy. Allergic diseases include hypersensitivity categorized into immediate type and delayed type or allergic diseases categorized into allergy types I to IV. Examples of such diseases include, but are not limited to, type I allergy (including, for example, systemic anaphylaxis, bronchial asthma, and pollinosis), type II allergy (including, for example, hemolysis in blood group mismatch transfusion and autoimmune hemolytic anemia), type III allergy (including, for example, serum disease, glomerulonephritis, and rheumatoid arthritis), and type IV allergy (including, for example, contact dermatitis, granuloma, and rejection in transplantation). Examples of allergic diseases include asthma; allergic encephalomyelitis; autoimmune encephalomyelitis; allergic neuritis; contact hypersensitivity; delayed hypersensitivity; respiratory tract hypersensitivity; atopic dermatitis; antigen-specific allergy including pollinosis; allergic rhinitis; and hives. Allergic diseases overlap in part with diseases in other categories such as autoimmune diseases, inflammatory diseases, and fibrotic diseases, and vice versa.

A "TH17 cell-related disease" as used herein is a disease where TH17 cells play a certain role in formation, exacerbation, and/or continuation of the disease. Such diseases include, for example, inflammatory diseases, autoimmune diseases, and allergic diseases the formation, exacerbation, and/or continuation of which are associated with TH17 cells, and in particular include, for example, multiple sclerosis, rheumatoid arthritis, scleroderma, psoriasis, nephritis (*e.g*., glomerulonephritis), asthma, contact hypersensitivity, delayed hypersensitivity, and respiratory tract hypersensitivity.

A "fibrotic disease" as used herein is a condition accompanied by abnormal or excessive formation of fibrous connective tissues in cells, organs, or tissues. Fibrotic diseases can develop as a part of recovery or a response process in cells, tissues, or organs due to, for example, somatic injury, inflammation, and infection. The term "fibrotic disease" can be used herein interchangeably with the terms "fibrosis," "fibrotic disorder," and "fibrotic symptom."

Examples of fibrotic disease include, but are not limited to, the following: vascular fibrosis, pulmonary fibrosis (*e.g.,* idiopathic pulmonary fibrosis), skin fibrosis (*e.g.,* scarring of the skin due to scleroderma, after trauma, and due to surgery, keloid, and keloid formation of the skin), scleroderma, systemic sclerosis, liver fibrosis, (*e.g*., after hepatitis C virus infection or after liver transplantation), renal fibrosis (*e.g.*, interstitial fibrosis in focal segmental glomerulosclerosis and renal systemic fibrosis), musculoskeletal fibrosis, cardiac fibrosis (*e.g*., endocardial myocardial fibrosis and idiopathic cardiomyopathy), splenic fibrosis, ocular fibrosis (*e.g.,* ocular sclerosis, glaucoma, conjunctival and corneal scar, and pterygium), progressive systemic sclerosis (PSS), chronic graft versus host disease, Peyronie's disease, connective tissue disease, urethral stricture after cystoscope, mediastinal fibrosis, idiopathic and pharmacologically induced retroperitoneal fibrosis, progressive severe fibrosis, proliferative fibrosis, neoplastic fibrosis, and fibrosis arising from surgical implantation of artificial organ. Other diseases, disorders, and symptoms associated with fibrosis include, for example, cirrhosis that can lead to liver fibrosis, diffuse lung disease, pain syndrome after vasectomy, tuberculosis that can lead to pulmonary fibrosis, sickle cell anemia that can lead to spleen hypertrophy and eventually fibrosis, rheumatoid arthritis, and Crohn's disease that can give rise to recurrent inflammation and healing of intestinal tissues leading to intestinal wall fibrosis. Fibrotic diseases can also develop as viral hepatitis, alcoholism, complications of hemochromatosis, Wilson's disease, schistosomiasis, bile duct disorder, exposure to toxin, and metabolic disorders. Fibrotic diseases overlap in part with diseases in other categories such as autoimmune diseases, allergic diseases, and inflammatory diseases, and vice versa.

An "RNA virus" as used herein means a virus having an RNA genome. RNA viruses include single-stranded RNA viruses (plus-stranded RNA viruses and minus-stranded RNA viruses) and double-stranded RNA viruses. The term "RNA virus infection" means any disorders resulting from invasion of an RNA virus to the surface, spot, or whole body of a host. The host may be an individual as used herein.

A "treatment" (and its grammatically derived words such as "treat" and "treating") as used herein means clinical intervention intended to alter the natural course of an individual to be treated and can be performed for prevention as well as during the course of clinical conditions. Desirable effects of a treatment include, but are not limited to, prevention of onset or recurrence of a disease, mitigated symptoms, any attenuated pathological effects directly or indirectly due to a disease, prevention of metastasis, reduced progression rate of a disease, recovery or alleviation of diseased state, and remission or improved prognosis. In some embodiments, the Regnase-1-binding molecule of the present invention is used to delay development of a disease or slow progress of a disease.

Herein, "inhibit phosphorylation" of a molecule means reducing the degree of phosphorylation of the molecule or preventing the phosphorylation of the molecule.

Herein, "inhibit phosphorylation in a Regnase-1-selective manner" means inhibiting the phosphorylation of Regnase-1 while not inhibiting the phosphorylation of other molecules, or means that the degree of inhibiting phosphorylation of molecules other than Regnase-1 is smaller than the degree of inhibiting phosphorylation of Regnase-1 (for example, it may be 50% or less, 40% or less, 30% or less, 20% or less, or 10% or less). In one embodiment, without limitation, phosphorylation may be inhibited in a Regnase-1-selective manner by inhibiting phosphorylation of Regnase-1 using a Regnase-1-binding molecule. "Inhibiting phosphorylation in a Regnase-1-selective manner" does not include embodiments where the activity of a kinase that phosphorylates Regnase-1 is inhibited so that phosphorylation of substrates of the kinase (including molecules other than Regnase-1) is inhibited in a non-substrate-selective manner.

Herein, "positions corresponding to" can be used to characterize amino acid residues in an amino acid sequence of Regnase-1 from different origins (sources) or processed Regnase-1 by reference to mouse Regnase-1 (SEQ ID NO: 1). Alignments for determining the corresponding positions can be achieved by using various methods within the technology in the art, such as BLAST, BLAST-2, ALIGN, and Megalign (DNASTAR) software or publicly available computer software such as GENETYX (registered trademark) (GENETYX CORPORATION). One of ordinary skill in the art can determine appropriate parameters for obtaining sequence alignments, including any necessary algorithms for achieving the maximum alignment along the entire length of the sequences to be compared.

Fig. 1-1 shows the alignment between the amino acid sequences of mouse and human Regnase-1, produced using GENETYX (registered trademark). Table 1 shows the amino acid residues in human Regnase-1 at positions corresponding to some amino acid residues in mouse Regnase-1.

**[Table 1]**

| | |
|---|---|
| The representative amino acid residues and positions of mouse Regnase-1 (SEQ ID NO: 1) and the amino acid residues and positions of human Regnase-1 (SEQ ID NO: 2) corresponding to them. | |

| mouse Regnase-1 | human Regnase-1 |
|---|---|
| Ser435 | Ser438 |
| Ser439 | Ser442 |
| Ser494 | Ser497 |
| Ser513 | Ser516 |

Herein, "suppressing inflammation" may mean that inflammation does not occur, inflammation progresses more slowly compared to a control group not being treated, inflammation that has already occurred is alleviated, or the area of inflammation is decreased. An index of inflammation suppression may be, but is not limited thereto, suppression of inflammatory factor production.

An "inflammatory factor" herein includes inflammatory cytokines and leukocyte migration factors. Examples of inflammatory factors are disclosed herein.

Herein, "becoming a target of' or "being targeted by" Regnase-1 means that a certain molecule can be degraded by the RNase activity of Regnase-1. Whether or not a certain mRNA can become a target of Regnase-1 can be determined, for example, by the methods described in the "Activity assay" section in the present specification.

Herein, "suppressing fibrosis" means that fibrotic lesions in the tissues where fibrosis occurred are decreased or eliminated, or further progression of fibrosis is delayed or blocked (expansion of fibrotic lesions is suppressed).

"Epithelial hyperplasia" herein refers to the state where the number of normal cells that are normally arranged in epithelial tissues is abnormally increased. Epithelial hyperplasia is known as a feature of many disorders including psoriasis. Herein, "suppressing epithelial hyperplasia" means that the number of normal cells increased in epithelial tissues is decreased or their further growth is delayed or blocked.

Herein, "suppressing the growth of keratinocytes" means that the number of keratinocytes is decreased or their further growth is delayed or blocked. Whether or not a certain substance suppresses the growth of keratinocytes can be tested, for example, using histological examination.

Herein, "intracellular degradation of Regnase-1" means that the intracellular protein amount of Regnase-1 is decreased or Regnase-1 is eliminated from cells, and includes degradation via the ubiquitin-proteasome system. For example, when the protein amount of Regnase-1 is larger in cells treated with a test substance compared to that in cells not treated with the test substance, then it can be considered that intracellular degradation of Regnase-1 is suppressed by the treatment with the test substance.

Herein, "destabilization of Regnase-1" means that the RNase activity of Regnase-1 is diminished compared to a control (for example, unphosphorylated Regnase-1 can be employed). For example, when Regnase-1 exists but has lost the ability to degrade a target mRNA, the Regnase-1 is described as being destabilized. Without limitation, destabilization of Regnase-1 can be determined by the methods described in the present specification (for example, see the "Activity assay" section). For example, IL-6 mRNA may be used as a target. Such Regnase-1 with diminished RNase activity is sometimes called "an inactive form."

Herein, "suppressing destabilization of Regnase-1" may mean that destabilization of Regnase-1 is suppressed or emergence of an inactive form of Regnase-1 is suppressed.

Herein, "inhibition of dissociation of a Regnase-1 oligomer" refers to suppression or inhibition of the dissociation of a Regnase-1 oligomer into an associate formed of fewer molecules or a monomer in vitro or in vivo. Examples include suppression or inhibition of the dissociation of a Regnase-1 hexamer or larger associate into a trimer or a monomer.

Herein, "inhibition of release of Regnase-1 from an endoplasmic reticulum" refers to suppression or inhibition of the release of Regnase-1 from an endoplasmic reticulum in vitro or in vivo. "Endoplasmic reticulum" is sometimes denoted as "ER." An "endoplasmic reticulum" herein is preferably a rough endoplasmic reticulum.

Herein, "a method for identifying a substance that inhibits phosphorylation" includes, but is not limited to, methods of screening for a substance that inhibits phosphorylation, methods of confirming that a substance is a substance that inhibits phosphorylation, and such.

A "binding molecule" herein means a molecule that can bind to a certain molecule. For example, when A can bind to B, A is described as a binding molecule of B.

A "Regnase-1-binding molecule" herein means a molecule that can bind to Regnase-1. Examples of Regnase-1-binding molecules include synthetic low-molecular compounds, peptides, polypeptides, proteins, antibodies, carbohydrates, nucleic acids, and derivatives thereof, but are not limited thereto. A "Regnase-1-binding molecule" may be a molecule that can specifically bind to Regnase-1.

A "polypeptide" herein refers to a substance composed of four or more amino acids and/or amino acid analogs linked by amide bonds and/or ester bonds. Polypeptides may be any of natural polypeptides, synthetic polypeptides, recombinant polypeptides, and so on. Polypeptides include antibodies and cyclic polypeptides.

The term "antibody" is used in the broadest sense and includes a variety of antibody structures, including monoclonal antibodies, polyclonal antibodies, multispecific antibodies (for example, bispecific antibodies), modified antibodies, and antibody fragments, but is not limited thereto as long as the antibody shows the desired antigen binding activity.

The term "modified antibody" refers to an antibody in which an amino acid, glycosylation state, or such is modified relative to an unmodified parent antibody. For example, modification to enhance affinity to an antigen, modification to prolong the half-time in blood, modification to change binding to C1q or complement-dependent cytotoxicity (CDC), modification to enhance the ability of an antibody to transfer into cells, are included. Herein, modified antibodies include, for example, antibody derivatives to which a non-protein moiety (*e.g.,* an agent, polyethylene glycol (PEG), or nucleic acid) is added.

The term "antibody fragment" refers to a molecule that includes a part of a complete antibody binding to an antigen to which the complete antibody binds and that is other than the complete antibody. Examples of antibody fragments include, but are not limited to, Fv, Fab, Fab', Fab'-SH, F(ab')₂; diabodies; linear antibodies; single-chain antibody molecules (*e.g*., scFv); and multispecific antibodies formed of antibody fragments.

The terms "full-length antibody," "complete antibody," and "entire antibody" are used interchangeably herein and refer to an antibody that has a structure substantially similar to a natural antibody structure or that has a heavy chain containing an Fc region.

The term "cyclic polypeptide" means a polypeptide containing a cyclic structure formed of four or more amino acids and/or amino acid analogs. Cyclic polypeptides may have a linear portion in addition to a cyclic portion. The binding mode of a cyclization site is not specifically limited and may be a bond other than an amide bond or an ester bond. The binding mode of a cyclization site is preferably exemplified by, for example, covalent bonds such as amide bonds, carbon-carbon bonds, disulfide bonds, ester bonds, thioester bonds, thioether bonds, lactam bonds, bonds via azoline skeleton, bonds via triazole structure, and bonds via fluorophore structure. The position of functional groups used for cyclization, such as carboxy groups and amino groups, may be on the main chain or on the side chain, and is not specifically limited as long as it is located at a cyclizable position. Herein, "the binding mode of a cyclization site" is the binding mode of the site where cyclization is formed by cyclization reaction.

An "amino acid" herein includes natural amino acids and non-natural amino acids. A "natural amino acid" herein refers to Gly (glycine), Ala (alanine), Ser (serine), Thr (threonine), Val (valine), Leu (leucine), Ile (isoleucine), Phe (phenylalanine), Tyr (tyrosine), Trp (tryptophan), His (histidine), Glu (glutamic acid), Asp (aspartic acid), Gln (glutamine), Asn (asparagine), Cys (cysteine), Met (methionine), Lys (lysine), Arg (arginine), and Pro (proline). Examples of non-natural amino acids include, but are not specifically limited to, β-amino acids, γ-amino acids, D-type amino acids, N-substituted amino acids, α,α-disubstituted amino acids, and amino acids with a side chain different from that of natural amino acids. Any steric configuration is permitted for amino acids herein. There is no specific restriction on the selection of the side chain of amino acids. Herein, amino acids where the main chain amino group is substituted are referred to as "N-substituted amino acids." Although limitation is not intended, N-substituted amino acids include N-alkyl amino acids, and in particular are preferably exemplified by N-methyl amino acids. An "amino acid analog" herein preferably means hydroxycarboxylic acid, more preferably α-hydroxycarboxylic acid. The side chain of α-hydroxycarboxylic acid is not specifically limited as is the case with amino acids.

Herein, amino acids that constitute proteins, polypeptides, and peptides are sometimes referred to as amino acid residues. A serine residue may be referred to as "Ser residue" and a threonine residue may be referred to as a "Thr residue." For example, the 513th serine residue in an amino acid sequence is sometimes represented as S513 or Ser513, and substituting this serine residue with alanine is sometimes denoted as S513A or Ser513Ala.

The term "affinity" refers to the total strength of non-covalent interactions between one binding site of a molecule (*e.g.,* an antibody) and a binding partner of the molecule (*e.g.,* an antigen). A "binding affinity" as used herein refers to an inherent binding affinity that reflects 1:1 interactions between members of a binding pair (*e.g.,* an antibody and an antigen) unless otherwise indicated. The affinity of molecule X to its partner Y can generally be represented by dissociation constant (KD). Affinities can be measured by usual methods known in the art. Specific examples and exemplary embodiments for measuring binding affinities will be described below.

The terms "a molecule that can specifically bind to Regnase-1" and "a molecule that can specifically recognize Regnase-1" are used interchangeably and refer to a molecule that can specifically bind to Regnase-1 with sufficient affinity, and as a result is useful as a diagnosis agent and/or a therapeutic agent when Regnase-1 is targeted. In one embodiment, the degree of binding of "a molecule that can specifically bind to Regnase-1" to irrelevant non-Regnase-1 proteins is less than about 10% of the binding to Regnase-1 when measured, for example, by surface plasmon resonance assay, radioimmunoassay (RIA), and enzyme-linked immunosorbent assay. In a specific embodiment, "a molecule that can specifically bind to Regnase-1" has a dissociation constant (KD) of 1 µM or less, 100 nM or less, 10 nM or less, 1 nM or less, 0.1 nM or less, 0.01 nM or less, or 0.001 nM or less (for example, 10⁻⁸ M or less, for example, 10⁻⁸ M to 10⁻¹³ M, and for example, 10⁻⁹ M to 10⁻¹³ M). In one embodiment, the degree of binding of "a molecule that can specifically bind to Regnase-1" to irrelevant non-Regnase-1 proteins is less than about 10% of the binding to Regnase-1 when measured, for example, by the method described herein using surface plasmon resonance assay. In a specific embodiment, "a molecule that can specifically bind to Regnase-1" binds to a Regnase-1 epitope that is conserved among Regnase-1 from different species but is not limited thereto. In a specific embodiment, "a molecule that can specifically bind to Regnase-1" binds to mouse and human Regnase-1 but is not limited thereto.

Herein, the terms "a molecule that can specifically bind to Regnase-1 phosphorylated at a specific site" and "a molecule that can specifically recognize Regnase-1 phosphorylated at a specific site" are used interchangeably. In one embodiment, the degree of binding to Regnase-1 not phosphorylated at a specific site may be less than about 10% of the binding to Regnase-1 phosphorylated at the specific site, when measured, for example, by surface plasmon resonance assay, radioimmunoassay (RIA), and western blotting.

Herein, "a molecule that can specifically bind to Regnase-1" includes, but is not limited to, polypeptides such as antibodies and cyclic polypeptides.

Herein, "a Toll-like receptor (TLR) ligand" includes, but is not limited to, TLR1 ligands, TLR2 ligands, TLR7 ligands, or TLR4 ligands (lipopolysaccharides (LPS)).

An "effective amount" of an agent (*e.g.,* a pharmaceutical formulation) refers to the amount at a required dose over a required period of time that is effective in achieving a desired therapeutic or preventive result.

The terms "host cell," "host cell line," and "host cell culture" are used interchangeably and refer to a cell to which a foreign nucleic acid is introduced (including offspring of such a cell). Host cells include "transformants" and "transformed cells," which include primary transformed cells and offspring derived from the cells regardless of the number of passages. Offspring may not have nucleic acid contents completely identical to those of parent cells and may include mutation. Mutant offspring having the same function or biological activity as that used in the screening or selection of the original transformed cell are also included herein.

The term "individual" or "subject" is a mammal. Mammals include, but are not limited to, domestic animals (for example, cows, sheep, cats, dogs, and horses), primates (for example, humans and non-human primates such as monkeys), rabbits, and rodents (for example, mice and rats). In a specific embodiment, an individual or a subject is a human.

The term "monoclonal antibody" refers to an antibody obtained from a substantially homogeneous antibody population. That is, individual antibodies that constitute the population are the same and/or bind to the same epitope except for possible mutant antibodies (for example, mutant antibodies containing naturally occurring mutation or mutant antibodies generated in the process of manufacturing a monoclonal antibody preparation. There is usually a small amount of such mutants.). In contrast to polyclonal antibody preparations, which typically contain different antibodies against different determinants (epitopes), each monoclonal antibody in monoclonal antibody preparations is against a single determinant on an antigen. Accordingly, the modifier "monoclonal" indicates the characteristic of antibodies, *i.e.,* obtained from a substantially homogeneous antibody population, and should not be construed as requiring the manufacture of antibodies by some specific method. For example, monoclonal antibodies used according to the present invention may be produced by various means that include, but are not limited to, hybridoma method, recombinant DNA method, phage display method, and methods using transgenic animals that include all or a part of human immunoglobulin locus.

The term "polyclonal antibody" refers to a population that typically contains different antibodies against different determinants (epitopes). The modifier "polyclonal" indicates the characteristic of antibodies and should not be construed as requiring the manufacture of antibodies by some specific method.

The term "TBK1" is a serine/threonine kinase also known as TANK binding kinase 1. An example of human TBK1 and mouse TBK1 amino acid sequences can be obtained from Uniprot accession Nos. Q9UHD2 and Q9WUN2, respectively.

The term "IKKi" is a kinase also called inducible IκB kinase or IKK-E. An example of human IKKi and mouse IKKi amino acid sequences can be obtained from Uniprot accession Nos. Q14164 and Q9R0T8, respectively.

The term "Act-1" is an adaptor molecule also called TRAF3IP2, CIKS, or Nuclear factor NF-kappa-B activator 1. An example of human Act-1 amino acid sequence can be obtained from Uniprot accession No. 043734.

The term "IKK" is used synonymously with IκB kinase. IKK includes IKKα and/or IKKβ. Herein, IKK is preferably exemplified by IKKβ.

The term "IRAK" is used synonymously with IL-1 receptor-associated kinase (IL-1R-associated kinase). IRAK includes IRAK1 and IRAK2. Herein, IRAK is preferably exemplified by IRAK1 and IRAK2.

The term "pharmaceutical formulation" refers to a preparation that contains an active ingredient in such a form that its biological activity can exert effect and does not contain additional elements that are unacceptably toxic to a subject to which the formulation is administered.

A "pharmaceutically acceptable carrier" refers to an ingredient that is non-toxic to a subject and that is other than an active ingredient in a pharmaceutical formulation. Pharmaceutically acceptable carriers include, but are not limited to, buffer solution, excipients, stabilizing agents, or preserving agents.

The term "vector" as used herein refers to a nucleic acid molecule that can increase another nucleic acid to which it is linked. The term includes vectors as self-replicating nucleic acid structures and vectors that are incorporated into the genome of a host cell to which they are introduced. Some vectors can allow for expression of a nucleic acid to which they are operably linked. Such vectors are also referred to as "expression vectors" herein.

### 2. Therapeutic method, therapeutic composition, and such

In this section, the "method for treating and/or preventing" may be simply referred to as "method of treating". In addition, "composition for treating and/or preventing" may be simply referred to as "composition for treating".

In one aspect, the present invention is based on the finding that inhibiting phosphorylation of specific sites in Regnase-1 is effective for treatment and/or prevention of inflammatory diseases, autoimmune diseases, allergic diseases, fibrotic diseases, RNA virus infections, TH17 cell-related diseases, and such. In one aspect, the present invention is based on the finding that inhibiting phosphorylation of a specific site in Regnase-1 is effective for at least one selected from the group consisting of: (i) treatment and/or prevention of diseases associated with Regnase-1; (ii) suppression of inflammation; (iii) suppression of fibrosis of cells, tissues, or organs; (iv) suppression of epithelial hyperplasia; (v) suppression of expression of at least one mRNA selected from the group consisting of IL6, IL1a, CXCL1, CXCL2, HBEGF, CTGF, DDR1, and PDGFB; (vi) suppression of destabilization of Regnase-1; (vii) suppression of inflammatory factor production; (viii) suppression of intracellular degradation of Regnase-1; (ix) inhibition of release of Regnase-1 from the endoplasmic reticulum; (x) inhibition of dissociation of Regnase-1 oligomers; and xi) suppression of keratinocyte proliferation. In one aspect, the present invention is based on the finding that (a) TBK1 or IKKi and (b) Act-1 are involved in phosphorylation of Regnase-1 by IL-17 stimulation.

Regnase-1 is present in various cells, such as macrophages and fibroblasts, even in an unstimulated state despite its rapid induction of mRNA, and it may be suppressing unwanted inflammatory responses (Nature immunology, Vol.12, NUMBER 12, DECEMBER 2011, p.1167-1175). Meanwhile, Regnase-1 is considered to be phosphorylated by IκB kinase (IKK) in response to external MyD88-mediated stimuli of Toll-like receptor (TLR) ligands and the IL-1 family and such, and undergo ubiquitin-dependent degradation (Nature immunology, Vol.12, NUMBER 12, DECEMBER 2011, p. 1167-1175). In parallel, IκB phosphorylated by IKK is similarly degraded, and NF-κB is released. As a result, NF-κB moves into the nucleus and various inflammation-related genes including Regnase-1 are induced. It is considered that Regnase-1 thus induced degrades the target mRNA and controls the biological reaction by a negative feedback mechanism that suppresses the persistence of excessive inflammation.

Although not intending to be bound by a specific theory, the present inventors thought as follows. That is, according to the results disclosed herein, while mouse Regnase-1 having S435A and S439A mutations is phosphorylated and converted into an inactive form, its degradation is suppressed. It was considered that subsequently Regnase-1 was dephosphorylated in part to generate an active form of Regnase-1 having RNase activity. As a result of experiments in which various diseases were induced in animals expressing such Regnase-1 variants (EAE, which is a model of multiple sclerosis, and models of psoriasis, glomerulonephritis, and scleroderma), the therapeutic utility of inhibiting destabilization and/or intracellular degradation of Regnase-1 was demonstrated. On the other hand, since Regnase-1 having S435A and S439A mutations is also phosphorylated and changed to an inactive form, if this can be suppressed, a stronger therapeutic effect can be expected. In addition, while the results of studies of IL-17 and IL-1 stimulation are shown below, as described later, stimulation with LPS (known as a TLR4 ligand) also shows phosphorylation pattern similar to IL-17 and IL-1 stimulation, stimulation of Regnase-1AA/AA cells with Pam3-Csk4 (known as TLR1 and TLR2 ligands) results in a decrease in production of IL-6 and IL-12, which are targets of Regnase-1 , and it is known that imiquimod, which was used to induce the psoriasis model, is an agonist of TLR7. Therefore, in diseases associated with TLR ligands, suppressing destabilization and/or intracellular degradation of Regnase-1 was considered to be also effective.

From these findings, in one aspect, the present inventors discovered that inhibiting phosphorylation of sites where Regnase-1 is phosphorylated by IKK (Ser residues corresponding to positions 435 and 439 of SEQ ID NO: 1) is effective in at least one selected from the group consisting of: (i) treating and/or preventing diseases associated with Regnase-1; (ii) suppressing inflammation; (iii) suppressing fibrosis of cells, tissues, or organs; (iv) suppressing epithelial hyperplasia; (v) suppressing expression of at least one mRNA selected from the group consisting of IL6, IL1a, CXCL1, CXCL2, HBEGF, CTGF, DDR1, and PDGFB; (vi) suppressing inflammatory factor production; (vii) suppressing intracellular degradation of Regnase-1; and (viii) suppressing keratinocyte proliferation.

Although not intending to be bound by a specific theory, the present inventors considered the following (1) to (4).
(1) Among the amino acid residues of mouse Regnase-1 that were found to be phosphorylated by IL-17 and IL-1 stimulation, the present inventors focused on Ser494, Thr505, Ser508, and Ser513 of SEQ ID NO: 1. With an objective of verifying the effect of phosphorylation of each amino acid residue on the destabilization of Regnase-1, Regnase-1 variants in which each of these residues was substituted with alanine (Ala) were constructed to inhibit phosphorylation of these four residues. Using these Regnase-1 variants, the phosphorylation of Regnase-1 after stimulation with IL-17 and IL-1 was verified by Western blotting. As a result, a band shift indicating phosphorylation was detected for Regnase-1 with T505A/S508A mutation; however, the band shift was not detected in Regnase-1 having S494A, S513A, and S494A and S513A mutations. This suggested that phosphorylation of Ser residues at positions corresponding to 494 and/or 513 of SEQ ID NO: 1 in Regnase-1 contributed to the band shift.
(2) The present inventors showed by an experiment targeting IL-6 mRNA that phosphorylated Regnase-1 corresponding to the same shifted band as described above generated by IL-17 and IL-1 stimulation is an inactive form with reduced ability to degrade target mRNA. Therefore, inhibiting the phosphorylation of Ser494 and Ser513 of Regnase-1 was considered to be able to suppress the production of inactive phosphorylated Regnase-1 (able to suppress Regnase-1 destabilization), and able to maintain the activity of Regnase-1.
(3) Based on the research results disclosed for the first time in this specification, the present inventors considered the role of the aforementioned Ser494 and Ser513 in cells as follows. Regnase-1 exists in an oligomeric form in the endoplasmic reticulum, a ribosome-containing organelle. The phosphorylation of Regnase-1 caused by cell stimulation allows Regnase-1 oligomers to dissociate and promotes their release from the endoplasmic reticulum and subsequent translocation to the cytoplasm. Phosphorylated Regnase-1 loses RNase activity. Phosphorylated Regnase-1 is degraded in the cytoplasm by proteasomes. By inhibiting phosphorylation of the Ser residue at a position corresponding to positions 494 and/or 513 in SEQ ID NO: 1, which are amino acid residues that play a central role in phosphorylation of Regnase-1 by IL-17 and/or IL-1 stimulation, dissociation of Regnase-1 oligomers caused by cell stimulation can be suppressed, release from the endoplasmic reticulum can be inhibited, and degradation of Regnase-1 can be suppressed. Regnase-1 degrades the target mRNA in the endoplasmic reticulum. Therefore, by inhibiting phosphorylation of Ser residues at positions corresponding to positions 494 and/or 513 in SEQ ID NO: 1, and by inhibiting release of Regnase-1 from the endoplasmic reticulum (and subsequent transfer to the cytoplasm), the target mRNA degradation activity may continue to be exhibited by Regnase-1 (the destabilization of Regnase-1 can be suppressed) even after stimulation.
(4) In addition, based on the findings disclosed herein that TBK1 and IKKi are kinases that phosphorylate the aforementioned Ser494 and Ser513, the present inventors proceeded with studies using animals expressing Regnase-1ΔCTD which was made to avoid phosphorylation by TBK1 and IKKi. As a result, it was shown that inhibiting phosphorylation of the aforementioned Ser513 and Ser494 exerts a strong effect on the treatment and/or prevention of diseases associated with Regnase-1. This therapeutic and/or prophylactic effect was greater than the effect of inhibiting phosphorylation of the aforementioned Ser435 and Ser439 by replacing these residues with Ala. In addition, it was shown in this process that Act-1 contributes to phosphorylation of Regnase-1 through TBK1 and IKKi. Furthermore, IRAK was assumed to be the kinase that phosphorylates the aforementioned Ser494 and Ser513 upon IL-1 stimulation.

From these findings, in one aspect, the present inventors discovered that inhibiting phosphorylation of a Ser residue at at least one position selected from the group consisting of positions corresponding respectively to positions 513 and 494 of SEQ ID NO: 1 in Regnase-1, and/or inhibiting the interaction of Regnase-1 with at least one selected from the group consisting of TBK1, IKKi, Act-1, IKK, and IRAK, are effective for at least one selected from the group consisting of: (i) treatment and/or prevention of diseases associated with Regnase-1; (ii) suppression of inflammation; (iii) suppression of fibrosis of cells, tissues, or organs; (iv) suppression of epithelial hyperplasia; (v) suppression of expression of at least one mRNA selected from the group consisting of IL6, IL1a, CXCL1, CXCL2, HBEGF, CTGF, DDR1, and PDGFB; (vi) suppression of inflammatory factor production; (vii) suppression of destabilization of Regnase-1; (viii) suppression of intracellular degradation of Regnase-1; and (ix) suppression of keratinocyte proliferation.

Although not intending to be bound by a specific theory, the present inventors also consider as follows. The signal by IL-36 stimulation uses the pathway *via* MyD88 as in stimulation by IL-1 or TLR ligands. Regnase-1 destabilization and degradation by IL-1 and TLR ligand stimulation is considered to proceed by activation of IKK and IRAK *via* MyD88 followed by phosphorylation of Regnase-1 by these kinases. For this reason, it is considered that in IL-36 stimulation as well, Regnase-1 phosphorylation *via* IKK and IRAK activation takes place, and causes destabilization and/or degradation of Regnase-1. In fact, it has been reported that Regnase-1 is degraded by IL-36 stimulation, and the relationship between IL-36-associated diseases and Regnase-1 has also been shown (Journal of Investigative Dermatology (2018) 138, 1439-1442). This suggests that suppression of the destabilization and/or intracellular degradation of Regnase-1 is also effective for diseases associated with IL-36, and therefore, inhibiting phosphorylation of Regnase-1 may become a powerful approach.

Although not intending to be bound by a specific theory, the present inventors consider as follows. Regnase-1 has been reported to show anti-RNA virus activity (J Immunol 2014; 193: 4159-4168; Proc Natl Acad Sci USA 2013; 110: 19083-19088; Nucleic Acids Res 2013; 41: 3314-3326; Nature 2009; 461: 399-401). In one embodiment, since the present invention can suppress destabilization and/or intracellular degradation of Regnase-1, the RNase activity of Regnase-1 is maintained, and the invention may be effective against RNA virus infections.

In some embodiments, the method of the present invention may be a method that inhibits phosphorylation of Ser residues in a Regnase-1-selective manner. Alternatively, the method of the present invention may include the step of inhibiting phosphorylation of Ser residues in a Regnase-1-selective manner.

In some embodiments, the composition of the invention may inhibit phosphorylation of a Ser residue in Regnase-1, and in one embodiment, it may inhibit phosphorylation in a Regnase-1-selective manner. In some embodiments, the composition of the present invention may comprise a Regnase-1-binding molecule that inhibits phosphorylation of a Ser residue in Regnase-1.

In some embodiments, the method or composition of the present invention may inhibit binding between Regnase-1 and at least one binding molecule selected from the group consisting of TBK1, IKKi, Act-1, IKK and IRAK (herein these molecules are also referred to as "Regnase-1-acting molecules"), and in an embodiment, the method or composition of the present invention may inhibit binding between Regnase-1 and any of the following binding molecules: (i) at least one binding molecule selected from the group consisting of TBK1, IKKi, Act-1, and IKK; (ii) at least one binding molecule selected from the group consisting of TBK1, IKKi, Act-1, and IRAK; (iii) at least one binding molecule selected from the group consisting of TBK1, IKKi, and Act-1; (iv) TBK1 and IKKi; (v) Act-1; (vi) TBK1, IKKi, and Act-1; (vii) TBK1; (viii) IKKi; (ix) IRAK; (x) IKK; and (xi) TBK1 and IKK. Without limitation, IKKβ is an example of IKK in the aforementioned (i) to (xi).

In some embodiments, the composition of the present invention may contain a Regnase-1-binding molecule that inhibits binding between Regnase-1 and at least one binding molecule selected from the group consisting of TBK1, IKKi, Act-1, IKK, and IRAK

In some embodiments, the method or composition of the present invention may be a method or composition for at least one selected from the group consisting of: (i) treating and/or preventing diseases associated with Regnase-1; (ii) suppressing inflammation; (iii) suppressing fibrosis of cells, tissues, or organs; (iv) suppressing epithelial hyperplasia; (v) suppressing expression of at least one mRNA selected from the group consisting of IL6, IL1a, CXCL1, CXCL2, HBEGF, CTGF, DDR1, and PDGFB; (vi) suppressing destabilization of Regnase-1; (vii) suppressing inflammatory factor production; (viii) suppressing intracellular degradation of Regnase-1; (ix) inhibiting release of Regnase-1 from the endoplasmic reticulum; (x) inhibiting dissociation of Regnase-1 oligomers; and xi) suppressing keratinocyte proliferation.

Without limitation, in some embodiments, the method or composition of the present invention may be a method or composition for two or more, three or more, four or more, or five or more selected from the aforementioned (i) to (xi).

Whether a substance or composition inhibits the release of Regnase-1 from the endoplasmic reticulum can be confirmed, for example, by the method described in the Examples (methods in which intracellular compartments are isolated and their protein distribution is analyzed using Western blotting). Whether or not a certain substance inhibits dissociation of Regnase-1 oligomer can be confirmed, for example, using nondenaturing PAGE analysis described in the Examples.

In some embodiments, the Ser residue in the present invention may be a Ser residue at at least one position or Ser residues at two or more positions selected from the group consisting of positions corresponding respectively to positions 513, 494, 439, and 435 of SEQ ID NO: 1 in Regnase-1, or it may be a Ser residue at at least one position or Ser residues at two or more positions selected from the group consisting of (i) positions 513, 494, 439, and 435 of SEQ ID NO: 1; or (ii) positions 516, 497, 442, and 438 of SEQ ID NO: 2.

In some embodiments, the Ser residue in the present invention may be a Ser residue at at least one position selected from the group consisting of positions corresponding respectively to positions 513 and 494 of SEQ ID NO: 1 in Regnase-1, or Ser residues at both positions, or it may be a Ser residue at at least one position selected from the group consisting of (i) positions 513 and 494 of SEQ ID NO: 1; or (ii) positions 516 and 497 of SEQ ID NO: 2, or Ser residues at both positions.

In some embodiments, the Ser residue in the present invention may be a Ser residue corresponding to position 513 of SEQ ID NO: 1 in Regnase-1, or a Ser residue at (i) position 513 of SEQ ID NO: 1; or (ii) position 516 of SEQ ID NO: 2.

In some embodiments, the Ser residue in the present invention may be a Ser residue at at least one position selected from the group consisting of positions corresponding respectively to positions 439 and 435 of SEQ ID NO: 1 in Regnase-1, or Ser residues at both positions, or it may be a Ser residue at at least one position selected from the group consisting of (i) positions 439 and 435 of SEQ ID NO: 1; or (ii) positions 442 and 438 of SEQ ID NO: 2, or Ser residues at both positions.

In some embodiments, the Ser residue in the present invention can be both of the following:
(i) a Ser residue(s) at either or both positions corresponding respectively to 513 and 494 of SEQ ID NO: 1 in Regnase-1; and
(ii) a Ser residue(s) at either or both positions corresponding respectively to 439 and 435 of SEQ ID NO: 1 in Regnase-1.

While not intending to be bound by a particular theory, in Regnase-1, inhibiting the phosphorylation of Ser residues in both the aforementioned (i), phosphorylated by TBK1 and IKKi, and the aforementioned (ii), phosphorylated by IKK, may enable the effects of the present invention to be exerted more strongly. For example, higher effects can be obtained in the treatment and/or prevention of inflammatory diseases, autoimmune diseases, allergic diseases, fibrotic diseases, RNA virus infections, TH17 cell-related diseases, and the like.

In some embodiments, the Ser residue in the present invention may be a Ser residue included in at least one, or two or more amino acid sequences selected from the group consisting of YWSEP (SEQ ID NO: 3), HFSVP (SEQ ID NO: 4), and DSGIGS (SEQ ID NO: 5) included in the amino acid sequence of Regnase-1.

In some embodiments, the substance capable of inhibiting phosphorylation of Ser residues in the present invention may be at least one compound (cyclic polypeptide) selected from PP1 to PP25 described herein. PP1 to PP25 have the amino acid sequences set forth in SEQ ID NOs: 11 to 16 and 30 to 48, respectively.

In some embodiments, the substance capable of inhibiting phosphorylation of Ser residues in the present invention may be at least one compound (a cyclic polypeptide having a linear portion) selected from PP7+tag, PP10+tag, and PP23+tag described herein. PP7+tag, PP10+tag, and PP23+tag have the amino acid sequences set forth in SEQ ID NOs: 57 to 59, respectively.

In some embodiments, the substance capable of inhibiting phosphorylation of Ser residues in the present invention is an antibody. The antibody can be selected from, for example, anti-Regnase-1 antibodies comprising the amino acid sequences described below:
REA0023 which comprises a heavy chain comprising the amino acid sequence of SEQ ID NO: 20 and a light chain comprising the amino acid sequence of SEQ ID NO: 21;
REA0027 which comprises a heavy chain comprising the amino acid sequence of SEQ ID NO: 22 and a light chain comprising the amino acid sequence of SEQ ID NO: 23;
REB0007 which comprises a heavy chain comprising the amino acid sequence of SEQ ID NO: 24 and a light chain comprising the amino acid sequence of SEQ ID NO: 25;
REB0014 which comprises a heavy chain comprising the amino acid sequence of SEQ ID NO: 26 and a light chain comprising the amino acid sequence of SEQ ID NO: 27; and
REB0022 which comprises a heavy chain comprising the amino acid sequence of SEQ ID NO: 28 and a light chain comprising the amino acid sequence of SEQ ID NO: 29.

In some embodiments, the phosphorylation in the present invention may be phosphorylation that can be induced by at least one molecule selected from the group consisting of IL-17, IL-1, IL-36, and TLR ligands; or the group consisting of IL-17, IL-1, and TLR ligands, or it may be phosphorylation that can be induced preferably by at least one molecule selected from the group consisting of IL-17 and IL-1, or more preferably by IL-17. In one embodiment, without limitation, the aforementioned IL-17; IL-1; IL-36; and TLR ligands are each independently IL-17A; IL-1β; IL-36α; and a ligand for TLR1, a ligand for TLR2, a ligand for TLR4, a ligand for TLR7, or LPS. The cells stimulated by the molecules here are not particularly limited, but may be non-hematopoietic cells, which are exemplified by macrophages, fibroblasts (for example, mouse fetal fibroblasts (MEF) can be used experimentally), and endothelial cells (for example, liver sinusoidal endothelial cells (LSEC) can be used experimentally).

In another embodiment, the phosphorylation in the present invention may be phosphorylation by at least one kinase selected from the group consisting of TBK1 (TANK-binding kinase 1), IKKi (inducible IκB kinase), IRAK (IL-1R-associated kinase) 1, IRAK2, and IKK (IκB kinase), phosphorylation by at least one kinase selected from the group consisting of TBK1, IKKi, and IKK, phosphorylation by IKK, phosphorylation by IRAK, or phosphorylation by TBK1 and/or IKKi.

The degree of inhibition of Regnase-1 phosphorylation in the present invention is not particularly limited. If the degree of phosphorylation of Regnase-1 when a test substance is added is reduced compared to the negative control without the test substance, the phosphorylation may be regarded as being inhibited. Illustratively, the aforementioned degree of phosphorylation may be reduced to 50% or less, 40% or less, 30% or less, 20% or less, or 10% or less.

In the present invention, the degree of inhibition of binding between Regnase-1 and at least one binding molecule selected from the group consisting of TBK1, IKKi, Act-1, IKK, and IRAK is not particularly limited. If the degree of binding between the binding molecule and Regnase-1 when a test substance is added is reduced compared to the negative control without the test substance, the binding between the binding molecule and Regnase-1 may be regarded as being inhibited. Illustratively, the binding may be reduced to 50% or less, 40% or less, 30% or less, 20% or less, or 10% or less.

The test substance in the present invention is not particularly limited, and examples thereof include peptides, proteins, non-peptidic compounds, synthetic compounds, fermentation products, and cell extracts, and preferably antibodies and cyclic polypeptides.

In some embodiments, the methods and/or compositions of the invention may be for treating and/or preventing diseases associated with Regnase-1.

"Diseases associated with Regnase-1" may be at least one disease selected from the group consisting of inflammatory diseases, autoimmune diseases, allergic diseases, fibrotic diseases, RNA virus infections, and TH17 cell-related diseases. In one embodiment, the "diseases associated with Regnase-1" may be at least one disease selected from a group consisting of inflammatory diseases accompanied by fibrosis and/or epithelial hyperplasia; autoimmune diseases; allergic diseases; RNA virus infections; and TH17 cell-related diseases. By applying the method and/or composition of the present invention against such diseases, fibrosis-suppressing effect and epithelial hyperplasia-suppressing effect may be exhibited along with anti-inflammatory effect. Examples of "diseases associated with Regnase-1" include, but are not limited to, multiple sclerosis, psoriasis, scleroderma, nephritis (including but not limited to glomerulonephritis), uveitis, pulmonary fibrosis, kidney fibrosis, vascular fibrosis, keloid, rheumatoid arthritis, systemic lupus erythematosus, Sjogren's syndrome, pneumonia, dermatitis, vasculitis, neuritis, arthritis, ocular inflammation, encephalomyelitis, and asthma.

In one embodiment, without limitation, a "disease associated with Regnase-1" may be a disease in the following tissues or organs: kidney, lung, skin, liver, heart, pancreas, bone marrow, blood vessel (including vascular endothelium cells), nerves, eyes, uterus, brain, and prostate. Examples include, but are not limited to, at least one tissue or organ selected from the group consisting of kidney, skin, lung, blood vessel, eye, brain, and nerve.

Without limitation, in one embodiment, the "disease associated with Regnase-1" may be at least one disease selected from the group consisting of the following: (i) a disease the formation, exacerbation and/or continuation of which are associated with expression of mRNA that may be targeted by Regnase-1; (ii) a disease the formation, exacerbation and/or continuation of which are associated with TH17 cells; (iii) a disease the formation, exacerbation and/or continuation of which are associated with at least one selected from the group consisting of IL-17, IL-1, and TLR ligands; (iv) a disease the formation, exacerbation and/or continuation of which are associated with at least one selected from the group consisting of IL-17 and IL-1; (v) a disease accompanied by fibrosis of cells, tissues or organs; (vi) a disease accompanied by epithelial hyperplasia; (vii) a disease the formation, exacerbation and/or continuation of which are associated with at least one molecule selected from the group consisting of IL6, IL1a, CXCL1, CXCL2, HBEGF, CTGF, DDR1 and PDGFB; and (viii) a disease the formation, exacerbation and/or continuation of which are associated with at least one selected from the group consisting of IL-17, IL-1, IL-36, and TLR ligands. Without limitation, in one embodiment, the IL-17, IL-1, IL-36 and TLR ligands may be each independently IL-17A; IL-1β; IL-36α; and a ligand for TLR1, a ligand for TLR2, a ligand for TLR4, a ligand for TLR7, or LPS.

The "mRNA that may be targeted by Regnase-1" in the aforementioned (i) is not limited as long as it may be degraded by Regnase-1. Examples of mRNA that may be degraded by Regnase-1 include: FABP5, ACKR3, CTGF, ADAMTS1, ATF2, CD80, CYR61, DDR1, DUOX, DUSP6, CSF3, HBEGF, ID3, IL19, MAP3K8, IL1a, MCOLN3 , MITF, ORC1, PDGFB, PTGS1, SESN1, PTGER4, SHQ1, SULF1, TNFRSF9, ZC3H12C, RARB and TMEM9 (see the Examples); CXCL1, CXCL2, CXCL3, NFKIBZ, NFKBID, PTGS2, ID1, MAFK, ZC3H12A, TM2D3, and IL6 (Cell. 2015 May 21; 161(5): 1058-1073); REL, TNFRSF4, IL2, ICOS, CD44, TNFRSF1B, IL1b, and NFATC1 (Cell. 2013 May 23; 153(5): 1036-49); GATA3 (J Allergy Clin Immunol. 2017 Oct 27. pii: S0091-6749 (17)31654-8); CEBPB (PLoS One. 2017 Mar 22; 12(3): e0174381); FURIN, IL12RB1, RC3H1, RC3H2, and IL18R1 (J Immunol. 2017 Dec 15; 199(12): 4066-4077); BCL2L1, RELB, BIRC3, and BCL3 (Cancer Res. 2016 Mar 15; 76(6): 1429-40); IL12b and CALCR (Nature. 2009 Apr 30; 458(7242): 1185-90); and TFRC and EGLN3 (Cell Rep. 2017 May 23; 19(8): 1614-1630). The "mRNA that may be targeted by Regnase-1" may be at least one selected from the group consisting of IL6, IL12b, IL1a, CXCL1, CXCL2, HBEGF, CTGF, DDR1, and PDGFB. Protein information corresponding to these mRNAs can be obtained from the Uniprot database. Whether or not an mRNA may be targeted by Regnase-1 can be confirmed using a known method described in the literature cited above or a method described herein. In one embodiment, the "mRNA that may be targeted by Regnase-1" may be an mRNA of a molecule produced from a non-hematopoietic cell. Herein, the name of each mRNA may be written using uppercase and lowercase letters without distinguishing between them (for example, "HBEFG" and "Hbefg" represent the same mRNA).

Although limitation is not intended, the aforementioned mRNAs which may be degraded by Regnase-1 can be IL6, IL1a, IL1b, IL12b, CXCL1, CXCL2, and CXCL3 (inflammatory factors); CTGF, DDR1, and PDGFB (organ fibrosis-related factors); and IL2 and HBEGF (cell growth factor), and preferred examples include IL6 and IL1a; CXCL1 and CXCL2; HBEGF; and CTGF, DDR1, and PDGFB. The correspondence between the aforementioned mRNA and the protein produced therefrom is as follows: IL6 (IL-6), IL1a (IL-1α), IL1b (IL-1β), IL12b (IL-12 subunit β), CXCL1 (CXCL-1), CXCL2 (CXCL-2), CXCL3 (CXCL-3); CTGF (Connective tissue growth factor), DDR1 (Epithelial discoidin domain-containing receptor 1), PDGFB (Platelet-derived growth factor subunit B (PDGF-2)); IL2 (IL-2), and HBEGF (Proheparin-binding EGF-like growth factor).

In some embodiments, the present invention may degrade the target mRNA described in any of (i) to (iii) below. In one embodiment, the target mRNA described in any of the following (i) to (iii) may be degraded by inhibiting phosphorylation of Regnase-1; and/or inhibiting binding between Regnase-1 and at least one binding molecule selected from the group consisting of TBK1, IKKi, Act-1, IKK, and IRAK

### (i) Fibrosis-related factors

Examples include CTGF, DDR1, and PDGFB. The present inventors have found for the first time that CTGF, DDR1, and PDGFB, which are indicators of organ fibrosis, may be targeted by Regnase-1. In addition, the inventors found that inhibition of phosphorylation of Regnase-1 suppresses the expression of these factors and suppresses fibrosis of organs in animal models. In one aspect, the present invention is based on these findings, and provides a method or composition for suppressing the expression of at least one, two or more, or all mRNAs selected from the group consisting of CTGF, DDR1, and PDGFB. In another aspect, a method or composition for suppressing fibrosis of a cell, tissue, or organ by suppressing the expression of at least one, two or more, or all mRNAs selected from the group consisting of CTGF, DDR1, and PDGFB is provided.

### (ii) Inflammatory factors

Examples include IL2, IL6, IL12b, IL12p40, IL1a, IL1b, CXCL1, CXCL2, CXCL3, CCL5 CCL30, GMCSF, PTGS2, NFKBIZ, NFKBID, ICOS, OX40, c-Rel, NFATC1, Gata3, C/EBPb, IL-18R, CXL2L1, RELB, Ackr3, Adamts1, Atf2, CD80, Cyr61, Duox, Dusp6, Csf3, ID3, IL19, Map3k8, Ptgs1, Ptger4, Tnfrsf9, and zc3h12c. Among these, IL6, IL1a, IL1b, IL2, IL12b, CXCL1, CXCL2, and CXCL3 are preferred examples. The inventors have found for the first time that Ackr3, Adamts1, Atf2, CD80, Cyr61, Duox, Dusp6, Csf3, ID3, IL19, Map3k8, Ptgs1, Ptger4, Tnfrsf9, and zc3h12c may be targeted by Regnase-1. The inventors also found that in animal models, inhibition of Regnase-1 phosphorylation suppresses the expression of IL6, IL1a, CXCL1, and CXCL2, and suppresses inflammation. In one aspect, the present invention is based on these findings and provides a method or composition for suppressing the expression of at least one, or two or more mRNAs selected from the group consisting of IL6, IL1a, IL1b, IL2, IL12b, CXCL1, CXCL2, and CXCL3; or the group consisting of IL6, IL1a, CXCL1, and CXCL2. In another aspect, a method or composition for suppressing inflammation by inhibiting the expression of at least one, or two or more mRNAs selected from the group consisting of IL6, IL1a, IL1b, IL2, IL12b, CXCL1, CXCL2, and CXCL3; or the group consisting of IL6, IL1a, CXCL1, and CXCL2, is provided.

### (iii) Cell growth factors

Examples include ID1, TM2D3, CD44, BIRC3, BCL3, Fabp5, Hbefg, mcoln3, Mitf, Orc1, Sesn1, Sulf1, Rarb, and Tmem9. The present inventors discovered for the first time that cell growth factors ID1, TM2D3, CD44, BIRC3, BCL3, Fabp5, Hbefg, mcoln3, Mitf, Orc1, Sesn1, Sulf1, Rarb, and Tmem9 may be targeted by Regnase-1. In addition, the inventors found that inhibition of phosphorylation of Regnase-1 suppresses the expression of HBEFG and suppresses the proliferation of keratinocytes in animal models. In one aspect, the present invention is based on these findings, and provides a method or composition for suppressing the expression of HBEGF mRNA. In another aspect, a method or composition for suppressing epithelial hyperplasia by suppressing the expression of HBEGF mRNA is provided. The protein corresponding to HBEGF mRNA is known as proheparin-binding EGF-like growth factor (HBEGF).

Without limitation, in some embodiments, the methods and/or compositions of the invention may suppress the expression of mRNA that may be targeted by Regnase-1. In one embodiment, the expression of at least one mRNA selected from the group consisting of the molecules listed as the above-mentioned "mRNAs that may be targeted by Regnase-1" may be suppressed. In particular, the expression of at least one mRNA selected from the group consisting of inflammatory factors; cell growth factors; and fibrosis-related factors may be suppressed. In one embodiment, such molecules may be molecules produced from non-hematopoietic cells.

Without limitation, in some embodiments, the methods and/or compositions of the present invention may suppress the expression of at least one mRNA selected from the group consisting of IL6, IL12b, IL1a, CXCL1, CXCL2, CCL5, CCL20, LCN2, GMCSF, HBEGF, SPRR2I, KERATIN 6A, COL1A1, ACTA2, CTGF, DDR1, and PDGFB, or may suppress the expression of at least one mRNA selected from the group consisting of IL6, IL12b, IL1a, CXCL1, CXCL2, HBEGF, CTGF, DDR1, and PDGFB.

Without limitation, in some embodiments, the methods and/or compositions of the present invention may have at least one feature selected from the group consisting of the following (i) to (iv): (i) able to suppress the production of inflammatory factors; (ii) able to suppress the production of cell growth factors; (iii) able to suppress the production of fibrosis-related factors; and (iv) able to inhibit the activation of STAT-3.

"Inflammatory cells" herein include, but are not limited to, T cells and neutrophils.

In some embodiments, the methods and/or compositions of the present invention may be used to treat and/or prevent symptoms in the following tissues or organs: kidney, lung, skin, liver, heart, pancreas, bone marrow, blood vessel (including vascular endothelial cells), nerve, eye, uterus, brain, and prostate. Illustrative examples include, but are not limited to, at least one tissue or organ selected from the group consisting of kidney, skin, lung, blood vessel, eye, brain, and nerve. Examples of the symptoms include, but are not limited to, inflammation, autoimmune reaction, fibrosis, and epithelial hyperplasia.

In some embodiments, destabilization and/or intracellular degradation of Regnase-1 in the present invention may be destabilization and/or intracellular degradation of Regnase-1 downstream of at least one signal selected from any of the following groups: the group consisting of IL-17, IL-1, IL-36 and a TLR ligand; the group consisting of IL-17, IL- 1, and a TLR ligand; and the group consisting of IL-17 and IL-1. Without limitation, in one embodiment, the aforementioned IL-17; IL-1; IL-36; and TLR ligand are each independently IL-17A; IL-1β; IL-36α; and a ligand for TLR1, a ligand for TLR2, a ligand for TLR4, a ligand for TLR7, or LPS.

In some embodiments, an effective amount of the composition of the present invention may be administered to a mammal, and the preferred mammal is a human.

Any of the Regnase-1-binding molecules of the present invention may be used in the therapeutic and/or prophylactic methods. In one aspect, Regnase-1-binding molecules for use as pharmaceuticals are provided. In one aspect, the Regnase-1-binding molecules of the present invention may inhibit phosphorylation of a Ser residue in Regnase-1. In one aspect, the Regnase-1-binding molecules of the present invention may inhibit the binding between Regnase-1 and at least one binding molecule selected from the group consisting of TBK1, IKKi, Act-1, IKK, and IRAK. In a further aspect, Regnase-1-binding molecules for use in the treatment and/or prevention of diseases associated with Regnase-1 are provided. In certain embodiments, Regnase-1-binding molecules of the present invention for use in treatment and/or prevention methods are provided. In particular embodiments, the present invention provides Regnase-1-binding molecules of the present invention for use in a method for treating an individual having a disease associated with Regnase-1 and/or a method of prevention for an individual who may develop a disease associated with Regnase-1, which method comprises the step of administering an effective amount of a Regnase-1-binding molecule of the present invention to the individual. In one such embodiment, the method further comprises the step of administering to the individual an effective amount of at least one additional therapeutic agent (such as those described below). In a further embodiment, the present invention provides a Regnase-1-binding molecule of the present invention for use in (i) suppression of fibrosis, (ii) suppression of epithelial hyperplasia, and/or (iii) suppression of inflammation. In a particular embodiment, the present invention provides a Regnase-1-binding molecule for use in a method for the aforementioned (i), (ii) and/or (iii) in an individual, which method comprises the step of administering an effective amount of a Regnase-1-binding molecule in the present invention to the individual for the aforementioned (i), (ii) and/or (iii). An "individual" according to any of the above embodiments is preferably a human.

In a further aspect, the present invention provides use of a Regnase-1-binding molecule in the present invention in the manufacture or preparation of a pharmaceutical. In one embodiment, the pharmaceutical is for the treatment and/or prevention of diseases associated with Regnase-1. In a further embodiment, the pharmaceutical is for use in a method for treating a disease associated with Regnase-1, which comprises the step of administering an effective amount of the pharmaceutical to an individual having the disease. In one such embodiment, the method further comprises the step of administering to the individual an effective amount of at least one additional therapeutic agent (such as those described below). In a further embodiment, the pharmaceutical is for (i) suppression of fibrosis, (ii) suppression of epithelial hyperplasia, and/or (iii) suppression of inflammation. In a further embodiment, the pharmaceutical is for use in a method for the aforementioned (i), (ii) and/or (iii) in an individual, which method comprises the step of administering an effective amount of the pharmaceutical to the individual for the aforementioned (i), (ii) and/or (iii). An "individual" according to any of the above embodiments may be a human.

In a further aspect, the present invention provides a method for treating and/or preventing a disease associated with Regnase-1. In one embodiment, the method comprises the step of administering an effective amount of a Regnase-1-binding molecule in the present invention to an individual who has, or may have in the future, such a disease associated with Regnase-1. In one such embodiment, the method further comprises the step of administering to the individual an effective amount of at least one additional therapeutic agent (such as those described below). An "individual" according to any of the above embodiments may be a mammal, preferably a human.

In a further aspect, the present invention provides a method for (i) suppressing fibrosis, (ii) suppressing epithelial hyperplasia, and/or (iii) suppressing inflammation in an individual. In one embodiment, the method comprises the step of administering an effective amount of a Regnase-1-binding molecule of the present invention to an individual for the aforementioned (i), (ii) and/or (iii). In one embodiment, an "individual" is a mammal, preferably a human.

In a further aspect, the present invention provides a pharmaceutical composition comprising any Regnase-1-binding molecule in the present invention (for example, for use in any of the therapeutic and/or prophylactic methods described above). In one embodiment, the Regnase-1-binding molecule of the present invention can inhibit phosphorylation of a Ser residue in Regnase-1. In another embodiment, the Regnase-1-binding molecule of the present invention can inhibit the binding of Regnase-1 with at least one binding molecule selected from the group consisting of TBK1, MKi, Act-1, IKK, and IRAK. In one embodiment, the pharmaceutical composition comprises any of the Regnase-1-binding molecules in the present invention and a pharmaceutically acceptable carrier. In another embodiment, the pharmaceutical composition comprises any of the Regnase-1-binding molecules in the present invention and at least one additional therapeutic agent (such as those described below).

The Regnase-1-binding molecule of the present invention may be used in therapy either alone or in combination with other agents. For example, the Regnase-1-binding molecule of the present invention may be co-administered with at least one additional therapeutic agent.

The Regnase-1-binding molecule (and any additional therapeutic agent) of the present invention may be administered by any suitable means including oral administration, parenteral administration, intrapulmonary administration, and nasal administration, and intralesional administration if desired for local treatment. Parenteral injection includes intramuscular, intravenous, intraarterial, intraperitoneal, or subcutaneous administration. Pharmaceutical administration may be by any suitable route, such as by injection, for example, intravenous or subcutaneous injection, depending in part on whether administration is short or long term. Various dosing schedules are contemplated herein, including, but not limited to, single administration or repeated administration over various time points, bolus administration, and pulse infusions.

### (Pharmaceutical composition)

The present invention provides a pharmaceutical composition comprising the Regnase-1-binding molecule of the present invention. The pharmaceutical composition of the present invention can be formulated by a known method by introducing a pharmaceutically acceptable carrier in addition to the Regnase-1-binding molecule of the present invention. For the formulation, commonly used excipients, binders, lubricants, colorants, flavoring agents, and, if necessary, stabilizers, emulsifiers, absorption promoters, surfactants, pH adjusters, preservatives, antioxidants, or the like can be used, and the formulation is carried out through an ordinary method by combining components that are generally used as raw materials for pharmaceutical formulations. For example, to produce an oral preparation, the compound according to the present invention or its pharmaceutically acceptable salt and excipient, and if necessary, a binder, a disintegrant, a lubricant, a coloring agent, a flavoring agent, and such are added, and then made into powders, fine granules, granules, tablets, coated tablets, capsules, and such by ordinary methods.

Examples of these components include animal and vegetable oils such as soybean oil, beef tallow, and synthetic glycerides; hydrocarbons such as liquid paraffin, squalene, and solid paraffin; ester oils such as octyldodecyl myristate and isopropyl myristate; higher alcohols such as cetostearyl alcohol and behenyl alcohol; silicone resins; silicone oils; surfactants such as polyoxyethylene fatty acid esters, sorbitan fatty acid esters, glycerin fatty acid esters, polyoxyethylene sorbitan fatty acid esters, polyoxyethylene hardened castor oil, and polyoxyethylene polyoxypropylene block copolymers; water-soluble macromolecules such as hydroxyethylcellulose, polyacrylic acid, carboxyvinyl polymer, polyethylene glycol, polyvinylpyrrolidone, and methylcellulose; lower alcohols such as ethanol and isopropanol; polyhydric alcohols such as glycerin, propylene glycol, dipropylene glycol and sorbitol; sugars such as glucose and sucrose; inorganic powders such as silicic anhydride, magnesium aluminum silicate, and aluminum silicate; and purified water.

Examples of the excipient include lactose, corn starch, sucrose, glucose, mannitol, sorbitol, crystalline cellulose, and silicon dioxide.

Examples of the binder include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, polypropylene glycol-polyoxyethylene block polymer, and meglumine.

Examples of the disintegrant include starch, agar, gelatin powder, crystalline cellulose, calcium carbonate, sodium bicarbonate, calcium citrate, dextrin, pectin, and carboxymethylcellulose calcium.

Examples of the lubricant include magnesium stearate, talc, polyethylene glycol, silica, and hardened vegetable oil.

As coloring agents, those permitted to be added to pharmaceuticals are used, and as flavoring agents, cocoa powder, menthol, aromatic powder, mentha oil, borneol, cinnamon powder, and such are used.

Of course, these tablets and granules may be suitably coated with sugar coating and other coatings as necessary. Furthermore, when a liquid agent such as a syrup agent or a formulation for injection is produced, a pH adjuster, a solubilizer, an isotonizing agent, and such, and if necessary, a solubilizing agent, stabilizer, and such are added to a compound according to the present invention or a pharmacologically acceptable salt thereof, and formulated by a common method.

For example, it can be used parenterally in the form of an injection of a suspension or a sterile solution with water or other pharmaceutically acceptable liquid. For example, formulation may be carried out by appropriately combining with a pharmacologically acceptable carrier or medium, specifically, sterile water or physiological saline, vegetable oil, emulsifier, suspending agent, surfactant, stabilizer, flavoring agent, excipient, vehicle, preservative, binder, or such, and mixing in a unit dosage form required for generally accepted pharmaceutical practice. Specific examples of carriers include light anhydrous silicic acid, lactose, crystalline cellulose, mannitol, starch, carmellose calcium, carmellose sodium, hydroxypropylcellulose, hydroxypropylmethylcellulose, polyvinylacetal diethylaminoacetate, polyvinylpyrrolidone, gelatin, medium chain fatty acid triglyceride, polyoxyethylene hardened castor oil 60, sucrose, carboxymethylcellulose, corn starch, and inorganic salts. The amount of active ingredient in these preparations is such that an appropriate volume within the indicated range can be obtained. Sterile compositions for injection can be formulated according to normal formulation practice using a vehicle such as distilled water for injection.

Aqueous solutions for injection or bases for eye drops include, for example, physiological saline, and isotonic solutions containing glucose and other adjuvants, such as D-sorbitol, D-mannose, D-mannitol, and sodium chloride. Appropriate solubilizing agents such as alcohols, specifically ethanol, polyalcohols such as propylene glycol and polyethylene glycol, nonionic surfactants such as polysorbate 80 (registered trademark), and HCO-50 may be used in combination.

Examples of the oily liquid include sesame oil and soybean oil, which may be used in combination with benzyl benzoate or benzyl alcohol as a solubilizing agent. Moreover, it may be blended with a buffer such as phosphate buffer and sodium acetate buffer, an analgesic such as procaine hydrochloride, a stabilizer such as benzyl alcohol, phenol, and an antioxidant. The prepared injection solution is usually filled into a suitable ampule.

Administration is preferably oral administration, but the administration method is not limited to oral administration. Specific examples of parenteral administration include injection, nasal administration, pulmonary administration, transdermal administration, and eye drop forms. Examples of the injection forms are those that allow systemic or local administration by intravenous injection, intramuscular injection, intraperitoneal injection, subcutaneous injection, intravitreal injection, and the like.

Moreover, the administration method can be appropriately selected depending on the age and symptoms of the patient. The dose of the pharmaceutical composition containing a peptide compound produced by the method of the present invention can be selected, for example, in the range of 0.0001 mg to 1000 mg per kg of body weight at a time. Alternatively, for example, the dose can be selected in the range of 0.001 to 100,000 mg/body per patient, but is not necessarily limited to these values. As an eye drop, it can be administered, for example, at a concentration of 0.0001% to 10% (w/v), preferably 0.01% to 5% (w/v), once to several times a day, or at intervals of several days, but without limitation thereto. The dose and administration method vary depending on the weight, age, symptoms, etc. of the patient, but can be appropriately selected by those skilled in the art.

### 3. Regnase-1-binding molecule

In one aspect, the present invention is based in part on the finding that inhibition of phosphorylation of Regnase-1 and/or inhibition of binding between Regnase-1 and TBK1, IKKi, Act-1, IKK, and IRAK is effective for treatment and/or prevention of certain diseases. In some embodiments, Regnase-1-binding molecules that inhibit phosphorylation of Regnase-1 are provided. In one embodiment, a Regnase-1-binding molecule that inhibits binding between Regnase-1 and at least one binding molecule (Regnase-1 acting molecule) selected from the group consisting of TBK1, IKKi, Act-1, IKK, and IRAK is provided. The Regnase-1-binding molecule of the present invention is useful, for example, for the treatment and/or prevention of diseases associated with Regnase-1.

While not intending to be bound by a particular theory, in one aspect, the present invention is based on the fact that the present inventors found for the first time that Regnase-1 interacts with TBK1, IKKi, and Act-1, and further found that inhibition of phosphorylation of Regnase-1 by these interactions plays an important role in exhibiting effects *via* degradation of target mRNA by Regnase-1, such as anti-inflammation, fibrosis suppression, and suppression of epithelial hyperplasia. In addition, experiments using Regnase-1 carrying S513A and/or S494A mutation suggested that phosphorylation sites that lead Regnase-1 to an inactive form (Ser513 and Ser494 of SEQ ID NO: 1) were common in both IL-17 and IL-1 stimuli. Therefore, it can be understood that inhibition of the phosphorylation of Regnase-1 by interaction with IRAK, which functions as a kinase that phosphorylates Regnase-1 downstream of IL-1, will bring about the same effect as described above. Therefore, any method, molecule, and composition capable of inhibiting the interaction between Regnase-1 and at least one molecule selected from the group consisting of TBK1, IKKi, Act-1, IKK, and IRAK may be included in some embodiments of the present invention. Those skilled in the art will be able to identify and produce a substance that can inhibit the interaction between Regnase-1 and at least one molecule selected from the group consisting of TBK1, IKKi, Act-1, IKK, and IRAK based on the disclosure of the present specification. For example, a substance of interest can be identified and manufactured by analyzing the interaction between Regnase-1 and TBK1, IKKi, or Act-1 using a known method such as surface plasmon resonance (SPR) in a system to which a substance capable of binding to Regnase-1 is added.

While not intending to be bound by any particular theory, in one aspect, the present invention is based on the fact that the present inventors found that inhibition of the phosphorylation of a particular Ser residue of Regnase-1 (at least one selected from the group consisting of positions corresponding respectively to positions 513, 494, 439, and 435 of SEQ ID NO: 1; preferably at least one selected from the group consisting of positions corresponding respectively to positions 513 and 494; and preferably both of the following:
(i) either or both positions corresponding respectively to positions 513 and 494 of SEQ ID NO: 1; and
(ii) either or both positions corresponding respectively to positions 439 and 435 of SEQ ID NO: 1) plays an important role in exhibiting effects *via* degradation of target mRNA by Regnase-1, such as anti-inflammation, fibrosis suppression, and suppression of epithelial hyperplasia. As such, any method, molecule, and composition that can inhibit phosphorylation of such Ser residues may be included in some embodiments of the present invention. Those skilled in the art can identify and produce a substance capable of inhibiting phosphorylation of the Ser residue based on the disclosure of the present specification. For example, a substance of interest can be identified and manufactured by analyzing inhibition of the phosphorylation of Regnase-1 by TBK1 or IKKi using a method disclosed in this specification or a known technique in a system to which a substance capable of binding to Regnase-1 is added.

In one aspect, the present invention provides a Regnase-1-binding molecule that inhibits phosphorylation of Regnase-1. In one aspect, the present invention provides a Regnase-1-binding molecule that inhibits binding between Regnase-1 and at least one binding molecule selected from the group consisting of TBK1, IKKi, Act-1, IKK, and IRAK

In some embodiments, the Regnase-1-binding molecule of the present invention may inhibit phosphorylation of a Ser residue or Thr residue contained in Regnase-1, and preferably inhibits phosphorylation of a Ser residue.

In some embodiments, the amino acid residues of Regnase-1 whose phosphorylation can be inhibited by the Regnase-1-binding molecule of the present invention may be amino acid residues at at least one, two or more, or three or more positions selected from the group consisting of positions corresponding respectively to Ser28, Ser124, Thr115, Ser288, Ser494, Ser508, Ser513, Thr109, Ser404, Ser435, Ser454, Ser470, Ser482, Thr498, Thr505, Ser592, Ser21, Ser268, Ser386, Ser439, and Ser474 in SEQ ID NO: 1 (mouse Regnase-1). In some embodiments, when the Regnase-1-binding molecule of the present invention may inhibit the phosphorylation of multiple amino acid residues of Regnase-1, such amino acid residues may be either or both amino acid residues at positions corresponding respectively to Ser513 and Ser494 of SEQ ID NO: 1 (mouse Regnase-1), and either or both amino acid residues at positions corresponding respectively to Ser439 and Ser435 of SEQ ID NO: 1 (mouse Regnase-1).

In some embodiments, the Regnase-1-binding molecule of the present invention may inhibit the phosphorylation of a Ser residue of Regnase-1. In some embodiments, the Ser residue may be the Ser residue in the present invention as already described. In some embodiments, the phosphorylation may be the phosphorylation in the present invention as already described.

In some embodiments, the Ser residue of Regnase-1 whose phosphorylation is inhibited by the Regnase-1-binding molecule of the present invention, may be a Ser residue contained in at least one amino acid sequence selected from the group consisting of YWSEP (SEQ ID NO: 3), HFSVP (SEQ ID NO: 4), and DSGIGS (SEQ ID NO: 5) contained in the amino acid sequence of Regnase-1.

In some embodiments, the Regnase-1-binding molecule of the invention may be a compound that competes with at least one compound selected from PP1 to PP25 described herein, for example, at least one compound selected from the group consisting of PP7, PP23 and PP10, for binding to Regnase-1. In certain embodiments, the Regnase-1-binding molecule of the present invention may be a compound that does not compete with at least one compound selected from the group consisting of PP7, PP23, and PP10. In certain embodiments, the Regnase-1-binding molecule of the present invention may be a compound that competes with PP7 and PP23 and does not compete with PP10.

Such a Regnase-1-binding molecule is preferably a molecule that specifically binds to Regnase-1, and is preferably a molecule that binds to the same site in Regnase-1 as a compound selected from PP1 to PP25, for example, at least one compound selected from the group consisting of PP7, PP23, and PP10, binds to.

Furthermore, the Regnase-1-binding molecule in the present invention includes compounds that compete with at least one compound selected from PP7+tag, PP10+tag, and PP23+tag described herein for binding to Regnase-1.

In some embodiments, the Regnase-1-binding molecule of the present invention may be a compound that competes with at least one antibody selected from REA0023, REA0027, REB0007, REB0014, and REB0022 described herein, for binding to Regnase-1.

Such a Regnase-1-binding molecule is preferably a molecule that specifically binds to Regnase-1, and is preferably a molecule that binds to the same site in Regnase-1 as an antibody selected from REA0023, REA0027, REB0007, REB0014, and REB0022 binds to.

Whether or not a Regnase-1-binding molecule competes with another Regnase-1-binding molecule for binding to Regnase-1 can be confirmed, for example, by the competitive assay described in the section "B. Binding assay and other assays" in "9. Measurement method (Assay)" of this specification.

In some embodiments, the Regnase-1-binding molecule of the present invention binds to amino acid residues included in the sequence from the 544th to the 596th amino acids shown in SEQ ID NO: 1, or the sequence from the 547th to the 599th amino acids shown in SEQ ID NO: 2.

In some embodiments, the Regnase-1-binding molecule of the present invention binds to amino acid residues included in the sequence from the 1st to the 543rd amino acids shown in SEQ ID NO: 1, or the sequence from the 1st to the 546th amino acids shown in SEQ ID NO: 2.

In some embodiments, the Regnase-1-binding molecule of the present invention binds to amino acid residues included in the sequence from the 301st to the 596th amino acids shown in SEQ ID NO: 1, or the sequence from the 301st to the 599th amino acids shown in SEQ ID NO: 2.

In some embodiments, the Regnase-1-binding molecule of the present invention binds to amino acid residues included in the sequence from the 1st to the 300th amino acids shown in SEQ ID NO: 1, or the sequence from the 1st to the 300th amino acids shown in SEQ ID NO: 2.

In some embodiments, the Regnase-1-binding molecules of the present invention do not substantially inhibit or reduce the RNase activity of Regnase-1. In certain embodiments, in the presence of the Regnase-1-binding molecule of the present invention, the remaining RNase activity of Regnase-1 is 50% or more, 60% or more, or 70% or more compared to the activity in the absence of the molecule. In a further embodiment, in the presence of the Regnase-1-binding molecule of the present invention, the remaining RNase activity of Regnase-1 is 80% or more, 85% or more, 90% or more, or 95% or more compared to the activity in the absence of the molecule. The RNase activity can be measured, for example, according to the method described in the section "C. Activity assay" in "9. Measurement method (Assay)" of the present specification.

In some embodiments, the Regnase-1-binding molecule of the present invention may inhibit binding between Regnase-1 and any of the following binding molecules: (i) at least one binding molecule selected from the group consisting of TBK1, IKKi, Act-1, and IKK; (ii) at least one binding molecule selected from the group consisting of TBK1, IKKi, Act-1, and IRAK; (iii) at least one binding molecule selected from the group consisting of TBK1, IKKi, and Act-1; (iv) TBK1 and IKKi; (vii) Act-1; (vi) TBK1, IKKi, and Act-1; (vii) TBK1; (viii) IKKi; (ix) IRAK; (x) IKK; and (xi) TBK1 and IKK. The IKK in (i) to (xi) mentioned above may be IKKβ.

In some embodiments, the Regnase-1-binding molecule in the present invention may have the property of being effective in at least one selected from the group consisting of: (i) treatment and/or prevention of diseases associated with Regnase-1; (ii) suppression of inflammation; (iii) suppression of fibrosis of cell, tissue, or organ; (iv) suppression of epithelial hyperplasia; (v) suppression of expression of at least one mRNA selected from the group consisting of IL6, IL1a, CXCL1, CXCL2, HBEGF, CTGF, DDR1, and PDGFB; (vi) suppression of Regnase-1 destabilization; (vii) suppression of inflammatory factor production; (viii) suppression of intracellular degradation of Regnase-1; (ix) inhibition of release of Regnase-1 from the endoplasmic reticulum; (x) inhibition of dissociation of Regnase-1 oligomer; and (xi) suppression of proliferation of keratinocytes.

Although limitation is not intended, in some embodiments, the Regnase-1-binding molecule in the present invention may be a polypeptide, and the polypeptide may be a cyclic polypeptide. In some embodiments, the molecular weight of the cyclic polypeptide in the present invention may be 500 to 4000, 500 to 3000, or 500 to 2000.

In some embodiments, the cyclic polypeptide of the present invention may contain at least one selected from the group consisting of natural amino acids, unnatural amino acids, and amino acid analogs. The ratio of these amino acids is not particularly limited.

In some embodiments, the total number of amino acids and amino acid analogs contained in the cyclic polypeptide in the present invention is 4 to 20, 4 to 15, 6 to 15, 8 to 15, 9 to 13, or 10 to 13. In certain embodiments, the total number of amino acids and amino acid analogs contained in the cyclic portion of the cyclic polypeptide in the present invention may be 5 to 15, 7 to 12, 10 to 13, or 9 to 11.

In some embodiments, when the cyclic polypeptide of the present invention has a linear portion, the number of amino acids and/or amino acid analogs in the linear portion is preferably 0 to 17, more preferably 0 to 8, even more preferably 0 to 5, and particularly preferably 0 to 3. In one non-limiting embodiment, the "linear portion" herein may include natural amino acids and unnatural amino acids (including chemically modified and skeleton-converted amino acids).

In one embodiment, the linear portion of the cyclic polypeptide of the present invention may be a linear portion composed of a tag and a linker. The tag in the present invention can include at least one, two, three, or four amino acid residues selected from, for example, Thr, MePhe, Pro, and I1e. As such a tag, more preferably, a tag comprising one, two, or three or more sequences consisting of Thr-MePhe-Pro-Ile (SEQ ID NO: 61), more preferably a tag referred to as a "TFIP tag" in the present specification can be presented as an example, but without limitation thereto. In the present invention, various tags known to those skilled in the art can be suitably used in addition to the FLAG tag, GST tag, HA tag, and Myc tag described herein.

As the linker constituting the linear portion of the present invention, a Gly-Gly linker or a linker composed of Gly and Ser (for example, one to three repetitions of Gly-Gly-Gly-Ser (SEQ ID NO: 62)), and a linker composed of Thr and Gly (for example, one to three repetitions of Thr-Gly) can be presented as examples, but without limitation thereto.

The linear portion in the present invention may be one in which the C-terminal amino acid residue is protected with a protecting group.

In some aspects, the polypeptide in the present invention may be modified to enhance the ability to be internalized into cells. Such a modification is not particularly limited because a known method can be used, but a method of binding a cell membrane-penetrating peptide is presented as an example. As the cell membrane-penetrating peptide, known sequences can be used, and examples include a Tat peptide derived from HIV Tat protein (GRKKRRQRRRPPQ [SEQ ID NO: 10]) (Brooks, H. et al. Advanced Drug Delivery Reviews, Vol 57, Issue 4, 2005, p.559-577), or polyarginine consisting of 6-12 arginine residues (Nakase, I. et al. Advanced Drug Delivery Reviews, Vol 60, 2008, p.598-607). It has also been reported that linking a fatty acid or stilbene derivative allows the peptide to access the cytoplasmic side (Covic, L. et al. (2002) Nat Med. 8: 1161; Endres, P. J. et al. (2006) Molecular Imaging 4: 485; and Goubaeva, F. et al., J. Biol. Chem. 278: 19634). By using such a method, the polypeptide can be transferred into the cell.

In some embodiments, the cyclic polypeptide in the present invention can be at least one compound selected from PP1-PP25 described herein.

In some embodiments, the cyclic polypeptide in the present invention may be at least one compound (cyclic polypeptide having a linear portion) selected from PP7+tag, PP10+tag, and PP23+tag described herein.

Although not intended to be limiting, in some embodiments, the polypeptide in the present invention may be an antibody (anti-Regnase-1 antibody). That is, in some embodiments, the antibody of the present invention can be at least one antibody selected from the antibodies REA0023, REA0027, REB0007, REB0014, and REB0022 described herein.

In a further aspect of the present invention, the anti-Regnase-1 antibody is a monoclonal antibody, including a chimeric, humanized, or human antibody. In one embodiment, the anti-Regnase-1 antibody is, for example, an antibody fragment, such as an Fv, Fab, Fab', scFv, diabody, or F(ab')₂ fragment. In another embodiment, the antibody is, for example, an IgG antibody or a full-length antibody of another antibody class or isotype. In another embodiment, the antibody is a multispecific antibody (for example, bispecific antibody).

### (Drug delivery to an intracellular target)

Certain embodiments of the present invention provide antibodies that can be targeted to intracellular Regnase-1 or antigen-binding fragments thereof. An antibody that inhibits phosphorylation of Regnase-1 can be delivered into cells by, for example, altering or modifying the antibody using techniques known to those skilled in the art. In one embodiment, the antibodies of the present invention can be expressed intracellularly as intrabodies (antibodies expressed inside cells). The term "intrabody" as used herein refers to, for example, an antibody or antigen-binding fragment thereof expressed in a cell and capable of selectively binding to a target molecule, as described in Marasco, Gene Therapy 4: 11-15 (1997); Kontermann, Methods 34: 163-170 (2004); US Patent. Nos. 6,004,940 and 6,329,173; US Patent Application Publication No. 2003/0104402, and PCT Publication WO2003/077945. See also, for example, WO96/007321 published on March 14, 1996 concerning the use of gene therapy to generate intracellular antibodies.

Intracellular expression of an intrabody may be accomplished by introducing a nucleic acid encoding the desired antibody or an antigen-binding fragment thereof into a target cell (usually lacking the wild-type leader sequence and secretion signal associated with the gene encoding the antibody or antigen-binding fragment). One or more nucleic acids encoding all or a portion of an antibody of the present invention may be delivered to target cells so as to allow expression of one or more intrabodies capable of binding to the intracellular target polypeptide and adjusting the activity of the target polypeptide. Any standard method for introducing nucleic acids into cells including (but not limited to) the following may be used: microinjection, ballistic injection, electroporation, calcium phosphate precipitation, liposomes, and transfection using retrovirus, adenovirus, adeno-associated virus, and vaccinia vector retaining the nucleic acid of interest.

In certain embodiments, the nucleic acid (optionally contained in a vector) can be introduced into patient's cells by *in vivo* and *ex vivo* methods. In one example *of in vivo* delivery, the nucleic acid is injected directly into the patient, for example, at the site where therapeutic intervention is considered necessary. In further examples *of in vivo* delivery, the nucleic acid is introduced into cells by transfection using a viral vector (for example, adenovirus, type I herpes simplex virus, or adeno-associated virus) and by using a lipid-based system (lipids useful for lipid-mediated gene transfer are, for example, DOTMA, DOPE, and DC-Chol). For a review of specific gene markings and gene therapy protocols, see Anderson et al., Science 256: 808-813 (1992) and WO93/25673 and references cited therein. In the example *of ex vivo* treatment, patient cells are removed, nucleic acids are introduced into those isolated cells, and the modified cells are administered directly to the patient or, for example, encapsulated within a porous membrane which will be implanted into the patient (see, for example, US Patent Nos. 4,892,538 and 5,283,187). A commonly used vector for *ex vivo* delivery of nucleic acids is a retroviral vector.

In another embodiment, an internalization antibody (internalizing antibody) is provided. The antibody can be equipped with certain characteristics that improve delivery of the antibody to the cell, or may be modified to be equipped with such characteristics. Techniques for accomplishing this are known in the art. For example, an antibody having a complete immunoglobulin form (Cytotransmab) is known, which has a humanized light chain variable region (VL) single domain that can penetrate into cells and be distributed in the cytoplasm (see for example, WO2016/013870). Using a heavy chain variable region (VH) library, a heavy chain variable region (VH) having a specific binding ability to Regnase-1 is selected, and by replacing this with the VH of the aforementioned antibody having a complete immunoglobulin form that can penetrate into cells and be distributed in the cytoplasm, anti-Regnase-1 antibody having a complete immunoglobulin form (iMab: internalizing & interfering monoclonal antibody) that can penetrate into cells and specifically bind to Regnase-1 in the cytoplasm can be produced (see for example, WO2016/013870). In addition, it is known that, for example, binding of a phosphorothioate nucleic acid or a phosphorothioate polymer backbone to an antibody enables delivery of the antibody into cells (see, for example, WO2015/031837). An antibody capable of penetrating into cells and specifically binding to Regnase-1 in the cytoplasm can be produced by covalently or non-covalently binding a phosphorothioate nucleic acid or a phosphorothioate polymer backbone to an antibody against Regnase-1. Also, for example, cationization of antibodies is known to promote their cellular uptake (see for example, US Patent No. 6,703,019). Lipofection or liposomes may also be used to deliver antibodies into cells. When antibody fragments are used, the smallest fragment with inhibitory action that specifically binds to the target protein may be used. For example, peptide molecules that retain the ability to bind to a target protein sequence may be designed based on the variable region sequence of the antibody. Such peptides may be synthesized chemically and/or produced by recombinant DNA techniques. For example, see Marasco et al., Proc. Natl. Acad. Sci. USA 90: 7889-7893 (1993). Alternatively, the antibody may be treated with an enzyme such as papain or pepsin to generate antibody fragments, or they may be produced by constructing DNAs encoding these antibody fragments or low molecular weight antibodies and introducing them into an expression vector, and then expressing them in appropriate host cells (see for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

Entry of antibodies into target cells can be enhanced by other methods known in the art. For example, certain sequences, such as those from HIV Tat or Antennapedia homeodomain proteins, can produce efficient uptake of heterologous proteins across the cell membrane. See for example, Chen et al., Proc. Natl. Acad. Sci. USA 96: 4325-4329 (1999).

Without limitation, in some embodiments, the Regnase-1 binding molecule of the present invention may be a dominant negative of at least one molecule selected from the group consisting of TBK1, IKKi, Act-1, IKK, and IRAK. Such a dominant negative is not particularly limited as long as it binds to Regnase-1 but lacks the ability to phosphorylate Regnase-1. Illustratively, the dominant negative form of TBK1 or IKKi may lack kinase activity, and the dominant negative form of Act-1 may lack the ability to bind to TBK1 and/or IKKi.

### 4. Method for identifying a substance that inhibits phosphorylation of Regnase-1

As described above, as a result of dedicated research by the present inventors, inhibition of Regnase-1 phosphorylation was found to be effective in treating and/or preventing certain diseases. In particular, phosphorylation of the Ser residue corresponding to positions 513 and/or 494 in SEQ ID NO: 1 was considered to induce dissociation of the Regnase-1 oligomer (for example, monomerization), release from the endoplasmic reticulum, generation of the inactive form of Regnase-1 (destabilization of Regnase-1), and subsequent degradation of Regnase-1.

In some embodiments, the identification method of the present invention may be a method for identifying a substance that inhibits phosphorylation of Regnase-1, which uses as an indicator, phosphorylation of the Ser residue at at least one position selected from the group consisting of positions corresponding respectively to positions 513, 494, 439, and 435 of SEQ ID NO: 1. In one embodiment, the method for identifying a substance that inhibits phosphorylation according to the present invention may use as an indicator, Ser residue phosphorylation at at least one position selected from the group consisting of positions corresponding respectively to positions 513 and 494 of SEQ ID NO: 1.

In one embodiment, the method for identifying a substance that inhibits phosphorylation according to the present invention may use as an indicator phosphorylation of Ser residues of both (i) and (ii) below:
(i) Ser residues at either or both positions corresponding respectively to positions 513 and 494 of SEQ ID NO: 1 in Regnase-1; and
(ii) Ser residues at either or both positions corresponding respectively to positions 439 and 435 of SEQ ID NO: 1 in Regnase-1.

In some embodiments, the method for identifying a substance that inhibits phosphorylation of the present invention may comprise the following steps:
(a) mixing Regnase-1 and a kinase capable of phosphorylating Regnase-1 in the presence of a test substance, and detecting phosphorylation of Regnase-1 by the kinase; and
(b) specifying a substance that inhibits phosphorylation of Regnase-1 by the kinase as compared to when the test substance is absent.

Alternatively, in some embodiments, the method for identifying a substance that inhibits phosphorylation of the present invention may comprise the following steps:
(a) contacting Regnase-1 with a test substance under conditions allowing phosphorylation of Regnase-1, and detecting phosphorylation of Regnase-1; and
(b) specifying a substance that inhibits phosphorylation of Regnase-1 as compared to when the test substance is absent.

In some embodiments, the method for identifying a substance that inhibits phosphorylation of the present invention may be performed by comparing the degree of phosphorylation of a specific amino acid residue in Regnase-1 in the presence and absence of a test substance, and selecting the test substance that reduces the degree of phosphorylation.

In some embodiments, the test substance in the method for identifying a substance that inhibits phosphorylation of the present invention may be a Regnase-1-binding molecule.

In some embodiments, the method for identifying a substance that inhibits phosphorylation of the present invention may include a method of screening for a substance having a specific binding capacity to Regnase-1.

Whether or not a substance inhibits phosphorylation of a Ser residue corresponding to position 513, 494, 439, or 435 of SEQ ID NO: 1 in Regnase-1 can be confirmed using the methods disclosed herein, for example, by using antibodies that recognize the phosphorylated Ser residue.

In some embodiments, the method for identifying a substance that inhibits phosphorylation of the present invention may be performed using an antibody capable of detecting phosphorylation of the Ser residue at at least one position selected from the group consisting of positions corresponding respectively to positions 513, 494, 439, and 435 of SEQ ID NO: 1.

In some embodiments, the method for identifying a substance that inhibits phosphorylation of the present invention may be a method for identifying a substance that inhibits phosphorylation of human Regnase-1. In one embodiment, the identification method of the present invention may be an identification method for a substance that inhibits phosphorylation of Regnase-1, using phosphorylation of the Ser residue at positions 516, 497, 442, and 438 of SEQ ID NO: 2 as the indicator.

In some embodiments, the above-mentioned "substance that inhibits phosphorylation of Regnase-1" may be a Regnase-1-binding molecule. Therefore, in an embodiment of the identification method of the present invention, the method may further comprise the steps of measuring the binding activity of a test substance to Regnase-1 and/or specifying or selecting a test substance having binding activity to Regnase-1.

In one aspect, the present invention provides an antibody that specifically recognizes phosphorylated Regnase-1. As described above, such an antibody can be used for specifying a substance that inhibits phosphorylation of Regnase-1.

In some embodiments, the antibody of the present invention may be an antibody that recognizes Regnase-1 in which a Ser residue is phosphorylated, and it may be an antibody that specifically recognizes Regnase-1 in which the Ser residue is phosphorylated at at least one position selected from the group consisting of positions corresponding respectively to positions 513, 494, 439, and 435 of SEQ ID NO: 1.

In one embodiment, the antibody of the present invention may be an antibody capable of binding to phosphorylated human Regnase-1, and it may be an antibody that can bind to both phosphorylated mouse Regnase-1 and phosphorylated human Regnase-1.

In one aspect, the present invention provides a composition for specifying a substance that inhibits phosphorylation of Regnase-1. Such a composition may comprise a predetermined amount of kinase and/or a predetermined amount of Regnase-1.

In some embodiments, the kinase used in the identification method of the present invention, or the kinase included in the composition of the present invention, may be a kinase which can phosphorylate the Ser residue at at least one position selected from the group consisting of positions corresponding respectively to positions 513, 494, 439, and 435 of SEQ ID NO: 1 in Regnase-1. Examples of such kinases include at least one kinase selected from the group consisting of TBK1, IKKi, IRAK, and IKK, and they may be at least one kinase selected from the group consisting of TBK1, IKKi, and IKK, the group consisting of TBK1, IKKi, and IRAK, or the group consisting of TBK1 and IKKi.

In some embodiments, phosphorylation in the present invention may be phosphorylation of the Ser residue at at least one position selected from the group consisting of positions corresponding respectively to positions 513, 494, 439, and 435 of SEQ ID NO: 1, it may be phosphorylation of the Ser residue at at least one position selected from the group consisting of positions corresponding respectively to positions 513 and 494 of SEQ ID NO: 1, it may be phosphorylation of the Ser residue at at least one position selected from the group consisting of positions 516, 497, 442, and 438 of SEQ ID NO: 2, it may be phosphorylation of the Ser residue at at least one position selected from the group consisting of positions 516 and 497 of SEQ ID NO: 2, it may be phosphorylation of the Ser residue at position 516 of SEQ ID NO: 2, and it may be phosphorylation of the Ser residue at at least one position selected from the group consisting of positions 442 and 438 of SEQ ID NO: 2.

In some embodiments, the compositions of the present invention may comprise Regnase-1-binding molecules and may comprise a predetermined amount of Regnase-1-binding molecules.

In some embodiments, Regnase-1 in the present invention may be human Regnase-1.

In some embodiments, the compositions of the present invention may comprise a predetermined amount of kinase and a predetermined amount of Regnase-1. In some embodiments, Regnase-1 included in the composition of the present invention may be dephosphorylated Regnase-1, or Regnase-1 subjected to dephosphorylation treatment.

The "predetermined amount" in the present invention is not particularly limited, and may be an amount set before performing the assay.

### 5. Method for identifying a substance that inhibits binding between a binding molecule and

### Regnase-1

As a result of dedicated research by the present inventors, inhibiting the binding between TBK1 and IKKi, or Act-1 and Regnase-1, was found to be effective in the treatment and/or prevention of certain diseases. In one aspect, the present invention provides an identification method for a substance that inhibits binding between Regnase-1 and at least one binding molecule selected from the group consisting of TBK1, IKKi, Act-1, IKK, and IRAK

In some embodiments, a method for identifying a substance that inhibits binding of the present invention may comprise the following steps:
(a) mixing Regnase-1 with at least one binding molecule selected from the group consisting of TBK1, IKKi, Act-1, IKK, and IRAK in the presence of a test substance, and measuring the binding activity between the binding molecule and Regnase-1; and
(b) specifying a substance that may reduce the binding activity between the binding molecule and Regnase-1 as compared to when the test substance is absent.

The binding activity in the present invention can be measured by the method described below.

In the present specification, "inhibit binding" means reducing the binding activity between the first molecule and the second molecule, or preventing both molecules from binding.

In some embodiments, the "substance that inhibits binding between Regnase-1 and at least one binding molecule selected from the group consisting of TBK1, IKKi, Act-1, IKK, and IRAK" may be a Regnase-1-binding molecule. Therefore, in one embodiment, the identification method of the present invention may further include a step of measuring the binding activity of a test substance to Regnase-1 and/or a step of specifying or selecting a test substance having binding activity to Regnase-1.

Alternatively, in some embodiments, the "substance that inhibits binding between Regnase-1 and at least one binding molecule selected from the group consisting of TBK1, IKKi, Act-1, IKK, and IRAK" may be a substance that inhibits the phosphorylation of Regnase-1. Therefore, as an embodiment of the identification method of the present invention, the method may further comprise a step of measuring the activity of the test substance in phosphorylating Regnase-1 and/or the step of specifying or selecting the test substance having the activity.

Alternatively, in one embodiment, the method for identifying a substance that inhibits the binding between Regnase-1 and at least one binding molecule selected from the group consisting of TBK1, IKKi, Act-1, IKK, and IRAK of the present invention may be used in combination with the method for identifying a substance that inhibits phosphorylation of Regnase-1.

In one aspect, the present invention provides a composition for identifying a substance that inhibits binding between Regnase-1 and at least one binding molecule selected from the group consisting of TBK1, IKKi, Act-1, IKK, and IRAK. In one embodiment, the composition of the present invention may comprise a predetermined amount of binding molecule and Regnase-1, and may comprise a predetermined amount of binding molecule and a predetermined amount of Regnase-1.

In some embodiments, the identification method of the present invention may be a method for identifying a substance that inhibits binding between Regnase-1 and any of the following (i) to (x), and the composition of the present invention may comprise Regnase-1 and any of the following binding molecules (i) to (x): (i) at least one binding molecule selected from the group consisting of TBK1, IKKi, Act-1, and IKK; (ii) at least one binding molecule selected from the group consisting of TBK1, IKKi, Act-1, and IRAK; (iii) at least one binding molecule selected from the group consisting of TBK1, IKKi, and Act-1; (iv) TBK1 and IKKi; (v) Act-1; (vi) TBK1, IKKi, and Act-1; (vii) TBK1; (viii) IKKi; (ix) IRAK; (x) IKK; and (xi) TBK1 and IKK. IKK in the above-mentioned (i) to (xi) may be IKKβ.

In some embodiments, Regnase-1 used in the identification method of the present invention, or Regnase-1 contained in the composition of the present invention may be dephosphorylated Regnase-1, or Regnase-1 subjected to dephosphorylation treatment.

### 6. Method for identifying a substance that competes with a reference substance for binding to Regnase-1

### 7. Method for identifying a substance that binds to the same site on Regnase-1 as the reference substance

In one aspect, the present invention relates to a method for identifying a test substance that competes with a reference substance in terms of binding to Regnase-1. In one aspect, the present invention relates to a method for identifying a test substance that binds to the same site on Regnase-1 as a reference substance. By using these methods, a Regnase-1-binding molecule that inhibits phosphorylation of a Ser residue of Regnase-1 can be obtained. Such a molecule can be used in the treatment and/or prevention of diseases associated with Regnase-1.

For example, competition assays may be used to identify such substances. That is, the method for identifying a substance that competes with a reference substance in terms of binding to Regnase-1 in the present invention can illustratively include performing a competition assay as described in the section "B. Binding assay and other assays" in "9. Measurement method (Assay)" of this specification.

The amount of the reference substance that binds to Regnase-1 indirectly correlates with the binding capacity of a candidate competing substance (test substance) that competes with the reference substance for binding to Regnase-1, or more specifically, a candidate competing substance (test substance) that competes for binding to the site on Regnase-1 where the reference substance binds. That is, as the amount and affinity of the test substance that binds to the same site on Regnase-1 as the reference substance increases, the amount of binding of the reference substance to Regnase-1 decreases and the amount of test substance bound to Regnase-1 increases. Specifically, an appropriately labeled reference substance and a test substance to be evaluated are simultaneously added to Regnase-1, and the bound reference substance is detected using the label. The amount of the reference substance bound to Regnase-1 can be easily measured by labeling the substance in advance. This labeling is not particularly limited, but a labeling method corresponding to the technique is selected. Specific examples of the labeling method include fluorescent labeling, radiolabeling, enzyme labeling, and the like.

Alternatively, in the present invention, a substance that binds to the same site on Regnase-1 as the reference substance can also be obtained by a known epitope mapping method (for epitope mapping, see also the section "B. Binding assay and other assays" in "9. Measurement method (Assay)" of the present specification). Specifically, by analyzing the site (epitope) on Regnase-1 to which the reference substance binds by the epitope mapping method using Regnase-1, its partial peptide, or such, and using the peptide containing the identified epitope, a substance that binds to the peptide may be prepared and thereby a substance that binds to the same site on Regnase-1 as the reference substance can be obtained. Therefore, the method for identifying a substance that binds to the same site on Regnase-1 as the reference substance in the present invention can illustratively include performing an epitope mapping method.

The substance thus obtained is expected to exhibit the same inhibitory action as the compounds PP1 to PP25, PP7+tag, PP10+tag, and PP23+tag or the antibodies REA0023, REA0027, REB0007, REB0014, and REB0022 obtained in the Examples, regarding the inhibitory activity on Regnase-1 Ser residue phosphorylation. Thus, substances that compete with the compounds PP1 to PP25, PP7+tag, PP10+tag, and PP23+tag or with the antibodies REA0023, REA0027, REB0007, REB0014, and REB0022 isolated in the Examples in terms of binding to Regnase-1, which are substances having inhibitory activity on Regnase-1 Ser residue phosphorylation, or substances that bind to substantially the same site on Regnase-1 as the compounds PP1 to PP25, PP7+tag, PP10+tag, and PP23+tag or the antibodies REA0023, REA0027, REB0007, REB0014, and REB0022 obtained in the Examples, which are substances having inhibitory activity on Regnase-1 Ser residue phosphorylation, can be used suitably as Regnase-1-binding molecules in the present invention.

The test substance in the present invention is not particularly limited, and examples thereof include peptides, proteins, non-peptidic compounds, synthetic compounds, fermentation products, and cell extracts, and preferably antibodies and cyclic polypeptides.

In one non-limiting embodiment, the site on Regnase-1 to which the Regnase-1-binding molecule and/or the reference substance binds may include the amino acid sequence at positions 544 to 596 of SEQ ID NO: 1, or the amino acid sequence at positions 547 to 599 of SEQ ID NO: 2, or at least one amino acid residue contained in these amino acid sequences.

In one non-limiting embodiment, the site on Regnase-1 to which the Regnase-1-binding molecule and/or the reference substance binds may include the amino acid sequence at positions 1 to 543 of SEQ ID NO: 1, or the amino acid sequence at positions 1 to 546 of SEQ ID NO: 2, or at least one amino acid residue contained in these amino acid sequences.

In one non-limiting embodiment, the site on Regnase-1 to which the Regnase-1-binding molecule and/or the reference substance binds may include the amino acid sequence at positions 301 to 596 of SEQ ID NO: 1, or the amino acid sequence at positions 301 to 599 of SEQ ID NO: 2, or at least one amino acid residue contained in these amino acid sequences.

In one non-limiting embodiment, the site on Regnase-1 to which the Regnase-1-binding molecule and/or the reference substance binds may include the amino acid sequence at positions 1 to 300 of SEQ ID NO: 1, or the amino acid sequence at positions 1 to 300 of SEQ ID NO: 2, or at least one amino acid residue contained in these amino acid sequences.

The reference substance in the present invention is not particularly limited as long as it can bind to Regnase-1 and inhibit phosphorylation. In one non-limiting embodiment, at least one compound selected from the following PP1 to PP25, PP7+tag, PP10+tag, and PP23+tag, or at least one antibody selected from REA0023, REA0027, REB0007, REB0014, and REB0022 described herein, can be utilized as a reference substance.

The present invention may further comprise the step of measuring the phosphorylation activity of at least one binding molecule (Regnase-1 acting molecule) selected from the group consisting of TBK1, IKKi, Act-1, IKK, and IRAK using the test substance selected by the above-described competitive assay, and the step of selecting the test substance that inhibited or decreased the phosphorylation activity. The phosphorylation activity can be measured, for example, according to the method described in the section "A. Method for detecting phosphorylation inhibition" in "9. Measurement Method (Assay)" of this specification.

Alternatively, the present invention may further include the step of measuring the RNase activity of Regnase-1 using the test substance selected by the above-mentioned competition assay, and the step of selecting the test substance that does not inhibit or reduce the RNase activity. The RNase activity can be measured, for example, according to the method described in the section "C. Activity assay" in "9. Measurement method (Assay)" of the present specification.

The test substance in the present invention is not particularly limited, and examples thereof include peptides, proteins, non-peptidic compounds, synthetic compounds, fermentation products, and cell extracts, and preferably antibodies and cyclic polypeptides.

### 8. Method for producing a Regnase-1-binding molecule of the present invention

The methods for producing Regnase-1-binding molecules of the present invention are not particularly limited, but for example, the later-described method for chemically synthesizing polypeptides, or the method for expressing recombinant polypeptides using cells can be used. In one embodiment, the production methods may include the aforementioned method for identifying a substance that inhibits phosphorylation of Regnase-1 according to the present invention, and illustratively, this enables production of Regnase-1-binding molecules that inhibit phosphorylation of Regnase-1. In one embodiment, the production methods may comprise the aforementioned method for identifying a substance that competes with a reference substance (for example, any of the above-mentioned compounds PP1 to PP25, PP7+tag, PP10+tag, and PP23+tag, or the antibodies REA0023, REA0027, REB0007, REB0014, and REB0022) in terms of binding to Regnase-1 according to the present invention.

The method for producing a Regnase-1-binding molecule of the present invention may include a method for identifying a Regnase-1-binding molecule. Methods known to those skilled in the art can be used in the method for identifying a Regnase-1-binding molecule. For example, an animal may be immunized with Regnase-1 or a peptide fragment thereof, and an antibody that binds to Regnase-1 may be identified. These methods are also described herein. In addition, peptides that bind to Regnase-1 may be identified using a peptide library. Such identification methods are known, for example, in WO2013/100132 and WO2012/033154.

### A. Method for chemically synthesizing a polypeptide

Examples of methods for chemically synthesizing polypeptides that bind to Regnase-1 according to the present invention include liquid phase synthesis methods, solid phase synthesis methods using Fmoc, Boc, and such, and combinations thereof. In Fmoc synthesis, an amino acid used as a basic unit has its main chain amino group protected by the Fmoc group, its side chain functional group protected as necessary with a protecting group that is not cleavable by bases such as piperidine, and its main chain carboxylic acid unprotected. Other than that, the basic unit is not particularly limited as long as it is a combination having an Fmoc-protected amino group and a carboxylic acid group. For example, a dipeptide may be used as a basic unit. The basic unit placed at the N-terminus may be a unit other than the Fmoc amino acid. For example, it may be a Boc amino acid or a carboxylic acid analog having no amino group. The main chain carboxylic acid group is supported on a solid phase by a chemical reaction with the functional group of the solid phase carrier. Subsequently, the Fmoc group is deprotected with a base such as piperidine or DBU, and by performing a condensation reaction between this newly generated amino group and a subsequently added protected amino acid having a carboxylic acid, which is the basic unit, a peptide bond is generated. In the condensation reaction, various combinations such as a combination of DIC and HOBt, a combination of DIC and HOAt, and a combination of HATU and DIPEA are possible. The desired peptide sequence can be generated by repeating the Fmoc group deprotection and the subsequent peptide bond generation reaction. After the desired sequence is obtained, cleavage from the solid phase and deprotection of the protecting groups introduced as necessary to the side chain functional groups are performed. It is also possible to perform structural transformation or cyclization of the peptide before cleaving from the solid phase. The cleavage from the solid phase and the deprotection may be performed under the same conditions, for example, 90:10 TFA/H₂O, or deprotection may be carried out under separate conditions as necessary. Cleavage from the solid phase may be possible with a weak acid such as 1% TFA, while Pd or such may be used as a protecting group to utilize the orthogonality of two chemical reactions. Steps such as cyclization can be performed during or at the end of these steps. For example, the side chain carboxylic acid and the N-terminal main chain amino group can be condensed, or the side chain amino group and the C-terminal main chain carboxylic acid can be condensed. In this case, reaction orthogonality is necessary between the C-terminal carboxylic acid and the side-chain carboxylic acid to be cyclized, or between the N-terminal main-chain amino group or hydroxy group and the side-chain amino group to be cyclized, and as described above, the orthogonality of the protecting groups is taken into consideration to select the protecting groups. Furthermore, a chloroacetyl group may be placed at the N-terminus to be cyclized with the side chain thiol group of a cysteine residue. The reaction products thus obtained can be purified by a reverse phase column, a molecular sieve column, or the like. The details are described, for example, in the solid-phase synthesis handbook issued by Merck on May 1, 2002.

### B. Method for expressing a recombinant polypeptide using cells

Polypeptides that bind to Regnase-1 can be produced using recombinant methods and configurations. In one embodiment, when the polypeptide is an antibody, an isolated nucleic acid encoding an anti-Regnase-1 antibody described herein is provided, for example, as described in US Patent No. 4,816,567. Such a nucleic acid may encode an amino acid sequence comprising a VL and/or an amino acid sequence comprising a VH of an antibody (for example, an antibody light and/or heavy chain). In further embodiments, one or more vectors (for example, expression vectors) comprising such nucleic acids are provided. In a further embodiment, host cells comprising such nucleic acids are provided. In one such embodiment, the host cell comprises (1) a vector comprising a nucleic acid encoding an amino acid sequence comprising the antibody VL and an amino acid sequence comprising the antibody VH, or (2) a first vector comprising a nucleic acid encoding an amino acid sequence comprising the antibody VL, and a second vector comprising a nucleic acid encoding an amino acid sequence comprising the antibody VH (such as a transformed host cell). In one embodiment, the host cells are eukaryotic (for example, Chinese hamster ovary (CHO) cells) or lymphoid cells (for example, Y0, NS0, and Sp2/0 cells)). In one embodiment, a method for producing an anti-Regnase-1 antibody is provided, which comprises culturing a host cell comprising a nucleic acid encoding the antibody as described above under conditions suitable for expression of the anti-Regnase-1 antibody, and optionally, recovering the antibody from the host cells (or host cell culture).

For recombinantly producing anti-Regnase-1 antibodies, nucleic acid encoding the antibody (such as that described above) is isolated, and this is inserted into one or more vectors for further cloning and/or expression in a host cell. Such nucleic acids will be readily isolated and sequenced using conventional procedures (for example, by using oligonucleotide probes capable of specifically binding to the genes encoding antibody heavy and light chains).

Suitable host cells for cloning or expression of antibody-encoding vectors include prokaryotic or eukaryotic cells described herein. For example, antibodies may be produced in bacteria, particularly when glycosylation and Fc effector function are not required. See, for example, US Patent Nos. 5,648,237, 5,789,199, and 5,840,523 for expression of antibody fragments and polypeptides in bacteria. (In addition, see also Charlton, Methods in Molecular Biology, Vol. 248 (B.K.C. Lo, ed., Humana Press, Totowa, NJ, 2003), pp.245-254, which describes the expression of antibody fragments in *Escherichia coli.)* After expression, the antibody may be isolated from the bacterial cell paste into a soluble fraction and can be further purified.

In addition to prokaryotes, eukaryotic microorganisms such as filamentous fungi or yeast, including fungal and yeast strains in which the glycosylation pathway is "humanized", which brings about the production of antibodies with partial or complete human glycosylation patterns, are suitable hosts for cloning or expressing the antibody-encoding vectors. See Gerngross, Nat. Biotech. 22: 1409-1414 (2004) and Li et al., Nat. Biotech. 24: 210-215 (2006).

Vertebrate cells can also be used as hosts. For example, mammalian cell lines adapted to grow in a suspended state will be useful. Other examples of useful mammalian host cell lines include monkey kidney CV1 strain (COS-7) transformed with SV40; human embryonic kidney strain (293 or 293 cells as described in Graham et al., J. Gen Virol. 36:59 (1977) etc.); baby hamster kidney cells (BHK); mouse Sertoli cells (TM4 cells as described in Mather, Biol. Reprod. 23: 243-251 (1980) etc.); monkey kidney cells (CV1); African green monkey kidney cells (VERO-76); human cervical cancer cells (HELA); canine kidney cells (MDCK); buffalo rat liver cells (BRL 3A); human lung cells (W138); human liver cells (Hep G2); mouse breast cancer (MMT 060562); TRI cells (for example, described in Mather et al., Annals N.Y. Acad. Sci. 383: 44-68 (1982)); MRC5 cells; and FS4 cells. Other useful mammalian host cell lines include Chinese hamster ovary (CHO) cells, including DHFR-CHO cells (Urlaub et al., Proc. Natl. Acad. Sci. USA 77: 4216 (1980)); and myeloma cell lines such as Y0, NS0, and Sp2/0. For a review of specific mammalian host cell lines suitable for antibody production, see, for example, Yazaki and Wu, Methods in Molecular Biology, Vol. 248 (B.K.C. Lo, ed., Humana Press, Totowa, NJ), pp. 255-268 (2003).

### 9. Measurement method (Assay)

Regnase-1-binding molecules in the present invention may be identified, screened, or unveiled regarding their physical/chemical properties and/or biological activities by various assays known in the art.

In the assay of the present invention, tagged Regnase-1 or tagged kinase may be used as appropriate. As the tag, FLAG, GST, HA, Myc, and the like can be used, but are not limited thereto.

### A. Method for detecting phosphorylation inhibition

The method for detecting whether a substance inhibits phosphorylation of Regnase-1 is not particularly limited, but illustrative examples are a method using radioisotope-labeled ATP and a method using an antibody that specifically recognizes phosphorylated Regnase-1, and a method using a cell, a method using a cell lysate, a cell free assay, and such are applicable. Examples of kinases that can be used for experiments include TBK1, IKKi, IKK, IRAK1, and IRAK2.

Examples of the method using radioisotope-labeled ATP include a method in which phosphorylation reaction is performed on Regnase-1 by a certain kinase using [γ-³²P] ATP, and then phosphorylated Regnase-1 is visualized by autoradiography. A more specific illustration is the later-described method of the Example.

In addition, by using an antibody that specifically recognizes phosphorylated Regnase-1, it is possible to detect whether phosphorylation of a specific amino acid residue of Regnase-1 is inhibited. For example, by using an antibody that specifically recognizes human Regnase-1 in which Ser at position 516 of SEQ ID NO: 2 has been phosphorylated, phosphorylation of Ser516 mentioned above can be detected by Western blotting or the like. A more specific illustration is the later-described method of the Example.

In some embodiments, the antibody that specifically recognizes phosphorylated Regnase-1 provided by the present invention may be an antibody that binds to phosphorylated Regnase-1 and does not bind to unphosphorylated Regnase-1, or an antibody that binds strongly to phosphorylated Regnase-1 as compared to the binding to unphosphorylated Regnase-1. The antibody may be a polyclonal antibody or a monoclonal antibody. In some embodiments, the antibody may be an antibody that specifically recognizes at least one Regnase-1 selected from the group consisting of the following (i) to (viii):
(i) mouse Regnase-1 in which Ser435 and/or Ser439 of SEQ ID NO: 1 is/are phosphorylated;
(ii) mouse Regnase-1 in which Ser494 of SEQ ID NO: 1 is phosphorylated;
(iii) mouse Regnase-1 in which Ser513 of SEQ ID NO: 1 is phosphorylated;
(iv) human Regnase-1 in which Ser438 and/or Ser442 of SEQ ID NO: 2 is/are phosphorylated;
(v) human Regnase-1 in which Ser437 of SEQ ID NO: 2 is phosphorylated;
(vi) human Regnase-1 in which Ser516 of SEQ ID NO: 2 is phosphorylated;
(vii) mouse Regnase-1 in which Ser435, Ser439, Ser494, and Ser513 of SEQ ID NO: 1 are phosphorylated; and
(viii) mouse Regnase-1 in which Ser438, Ser442, Ser437, and Ser516 of SEQ ID NO: 2 are phosphorylated.

In some embodiments, the antibody of the present invention may be an antibody capable of detecting phosphorylation of human Regnase-1, it may be an antibody capable of detecting phosphorylation of both mouse Regnase-1 and human Regnase-1, and it may be an antibody that specifically recognizes Regnase-1 of the aforementioned (i) and (iv); the aforementioned (ii) and (v); the aforementioned (iii) and (iv); or the aforementioned (vii) and (viii).

An antibody specifically recognizing phosphorylated Regnase-1 can be prepared by a known method using Regnase-1, in which a specific amino acid residue is phosphorylated, and preferably using a partial peptide as an antigen. For example, an animal such as a rabbit can be immunized with the antigen and an antibody can be obtained from the serum of the animal using an ordinary method, but is not limited to this method. A more specific illustration is the later-described method of the Example.

Whether the antibody obtained by the above method specifically recognizes phosphorylated Regnase-1 can be confirmed by evaluating the binding activity to phosphorylated Regnase-1 and non-phosphorylated Regnase-1 using techniques such as Western blotting.

In one aspect, the present invention provides an antibody that specifically recognizes phosphorylated Regnase-1 such as that described above.

### B. Binding assay and other assays

In one aspect, the Regnase-1-binding molecule of the present invention is tested for its Regnase-1 binding activity by known methods such as ELISA, Western blotting, and surface plasmon resonance assay.

In another aspect, competition assays may be used to identify Regnase-1-binding molecules that compete with a reference substance concerning binding to Regnase-1. In certain embodiments, such competing molecules bind to the same site on Regnase-1 (epitope, for example, linear or conformational epitope) as the reference substance. A detailed illustrative method for mapping the epitope to which a polypeptide binds is provided by Morris (1996) "Epitope Mapping Protocols," in Methods in Molecular Biology vol. 66 (Humana Press, Totowa, NJ).

As such a reference substance, for example, at least one compound selected from PP1 to PP25, PP7+tag, PP10+tag, and PP23+tag described in the present specification, or at least one antibody selected from REA0023, REA0027, REB0007, REB0014, and REB0022 described herein can be used, but it is not limited thereto. As the reference substance, for example, at least one compound selected from the group consisting of PP7, PP23, and PP10 described in the present specification may be used.

In an exemplary competition assay, immobilized Regnase-1 is incubated in a solution comprising a first labeled substance that binds Regnase-1 and a second unlabeled Regnase-1-binding molecule to be tested for its ability to compete with the first substance in terms of binding to Regnase-1. As a control, immobilized Regnase-1 is incubated in a solution comprising the first labeled substance but not the second unlabeled Regnase-1-binding molecule. After incubation under conditions that allow binding of the first substance to Regnase-1, excess unbound substances are removed and the amount of label bound to immobilized Regnase-1 is measured. If the amount of label bound to immobilized Regnase-1 is substantially reduced in the test sample as compared to the control sample, it indicates that the second molecule competes with the first substance in terms of binding to Regnase-1. See Harlow and Lane (1988) Antibodies: A Laboratory Manual ch.14 (Cold Spring Harbor Laboratory, Cold Spring Harbor, NY).

Another exemplary competitive assay uses BIACORE (registered trademark) analysis to determine the ability of a test substance to compete with a second (reference) substance in terms of binding to Regnase-1. In a further aspect of operating a BIACORE (registered trademark) instrument (for example, BIACORE (registered trademark) 3000) according to the manufacturer's recommendations, Regnase-1 is captured on a CM5 BIACORE (registered trademark) chip using standard techniques known in the art to produce a surface coated with Regnase-1. Typically, 200-800 resonance units of Regnase-1 are bound to the chip (an amount that provides an easily measurable level of binding but is easily saturable at the concentration of the test substance used). Two substances to be evaluated for their ability to compete with each other (i.e., test substance and reference substance) are mixed in a suitable buffer with a molar ratio of binding sites of 1:1 to produce a mixture. When calculating the concentration based on the binding site, the molecular weight of the test substance or reference substance is obtained by dividing the total molecular weight of the corresponding substance by the number of Regnase-1 binding sites on the substance. The concentration of each of the substances (i.e., test substance and reference substance) in the mixture must be sufficiently high to easily saturate the binding sites of the substances on Regnase-1 molecules captured on the BIACORE (registered trademark) chip. The test substance and reference substance in the mixture are at the same molar concentration (based on binding), typically 1.00-1.5 micromolar (based on binding site). Separate solutions containing only the test substance and only the reference substance are also prepared. The test substance and reference substance in these solutions are in the same buffer as the mixture and they may have the same concentration and conditions. The mixture containing the test substance and reference substance is passed over a BIACORE (registered trademark) chip coated with Regnase-1 and the total amount of binding is recorded. The chip is then treated to remove the test substance or reference substance bound without damaging Regnase-1 bound to the chip. Typically, this is done by treating the chip with 30 mM HCl for 60 seconds. Thereafter, a solution containing only the test substance is passed over the surface coated with Regnase-1, and the amount of binding is recorded. Again, the chip is treated to remove all of the bound substances without damaging Regnase-1 bound to the chip. Thereafter, a solution containing only the reference substance is passed over the surface coated with Regnase-1, and the amount of binding is recorded. Next, the theoretical maximum binding value of the mixture of the test substance and reference substance is calculated, which is the sum of the binding of each substance (i.e., test and reference) when it passes alone over the Regnase-1 surface. If the actually recorded binding of the mixture is less than this theoretical maximum, the test substance and reference substance are competing with each other in terms of binding to Regnase-1. Therefore, in general, the competing test substance is a substance that binds to Regnase-1 in the assay so that in the presence of the reference substance during the above-described BIACORE (registered trademark) blocking assay, the recorded binding is between 80% and 0.1% (for example, 80% > to 4%) of the theoretical maximum binding value (as defined above) for the test substance and reference substance in combination, and in particular, between 75% and 0.1% (for example, 75% to 4%) of the theoretical maximum binding value, and more specifically, between 70% and 0.1% (for example, 70% to 4%) of the theoretical maximum binding value.

Another exemplary competitive assay uses BIACORE (registered trademark) analysis to determine the ability of a test substance to compete with a second (reference) substance in terms of binding to Regnase-1. In a further aspect of operating a BIACORE (registered trademark) instrument (for example, BIACORE (registered trademark) 3000) according to the manufacturer's recommendations, the reference substance is captured on a BIACORE (registered trademark) chip using standard techniques known in the art, to create a surface coated with a reference substance. Typically, 200-800 resonance units of reference substance are bound to the chip (an amount that provides an easily measurable level of Regnase-1 binding). Regnase-1 and a test substance to be evaluated for its ability to compete with the reference substance are mixed in an appropriate buffer to produce a mixture. A separate solution containing only Regnase-1 is also prepared. Regnase-1 in these solutions is in the same buffer as the mixture and can be at the same concentration and conditions. A solution containing only Regnase-1 is passed over a BIACORE (registered trademark) chip coated with the reference substance and the total amount of binding is recorded. For chip regeneration, the chip is treated to remove the reference substance coated on the chip surface. For example, when the reference substance is coated *via* an antibody to a BIACORE (registered trademark) chip on which Protein A is immobilized by covalent bonding, it is treated with 10 mM Glycine-HCl to remove the antibody bound to Protein A. The mixture containing the test substance and Regnase-1 is then passed over a BIACORE (registered trademark) chip coated with the reference substance and the total amount of binding is recorded. When the total amount of binding recorded when passing the mixture containing the test substance and Regnase-1 is smaller than the total amount of binding recorded when passing a solution containing only Regnase-1, the test substance and reference substance are competing substances. Although not limited, competition between a test substance and a reference substance may mean that the value obtained by dividing (the total amount of binding when a test mixture containing the test substance and Regnase-1 is passed) by
(the total amount of binding when a solution containing only Regnase-1 is passed) is 0.8 or less, 0.7 or less, 0.6 or less, or 0.5 or less.

In order to capture the reference substance on the BIACORE (registered trademark) chip, a tag may be bound to the reference substance, and examples of such tags include TFPI-tag and FLAG-tag described herein. The reference substance and the tag may be linked *via* a linker, and examples of such a linker include a Gly-Gly linker and a linker composed of Gly and Ser (for example, one to three repetitions of Gly-Gly-Gly-Ser (SEQ ID NO: 62)), and a linker composed of Thr and Gly (for example, one to three repetitions of Thr-Gly). Although not limited, examples of the tagged reference substance include PP7+tag, PP23+tag, and PP10+tag disclosed herein. A tagged reference substance may be captured on the chip *via* an antibody against the tag. A more detailed method for capturing the tagged reference substance onto the chip is described in the Examples.

### C. Activity Assay

In one aspect, an assay for identifying the biological activity of a Regnase-1-binding molecule is provided. The biological activity may include, for example, an activity of degrading the target mRNA (RNase activity) or an activity of suppressing the expression of the target mRNA. Also provided are Regnase-1-binding molecules having such biological activity under *in vivo* and/or *in vitro* conditions.

In the present specification, "suppressing the expression of mRNA" means reducing the amount of mRNA, and includes reducing the amount of mRNA by degrading mRNA.

In certain embodiments, Regnase-1-binding molecules of the present invention are tested for such biological activity. The activity of degrading the target mRNA can be measured by using the method described herein. Illustratively, measurements can be taken by overexpressing IL-6 mRNA and 3'UTR, which are the target mRNAs, in HEK293 cells in the presence of Regnase-1, and evaluating the difference in IL-6 mRNA levels in the presence and absence of the test substance by Northern blotting. The target is not limited to IL-6 mRNA. Alternatively, a test substance may be administered to a disease model animal, and the target mRNA expression level in a tissue collected from the animal may be measured by quantitative PCR analysis.

### D. Method for measuring affinity

In one embodiment, the dissociation constant (KD) is measured by a radiolabeled antigen binding assay (RIA). In one embodiment, RIA is performed using the Fab version of the antibody of interest and its antigen. For example, the in-solution binding affinity of Fab to an antigen is measured by equilibrating Fab with a minimal concentration of the (¹²⁵I)-labeled antigen in the presence of a gradually increasing series of unlabeled antigen, and then capturing the bound antigen onto a plate coated with an anti-Fab antibody. (See, for example, Chen et al., J. Mol. Biol. 293: 865-881 (1999)). To establish the measurement conditions, MICROTITER (registered trademark) multiwell plates (Thermo Scientific) are coated overnight with 5 µg/mL capture anti-Fab antibody (Cappel Labs) in 50 mM sodium carbonate (pH 9.6), then blocked with 2% (w/v) bovine serum albumin in PBS for 2-5 hours at room temperature (approximately 23°C). In a non-adsorption plate (Nunc #269620), 100 pM or 26 pM of the [¹²⁵I]-antigen is mixed with serial dilutions of the desired Fab (for example, as in the assessment of the anti-VEGF antibody, Fab-12 in Presta et al., Cancer Res. 57: 4593-4599 (1997)). Next, the Fab of interest is incubated overnight, which may be continued for a longer time (for example, approximately 65 hours) to ensure that equilibrium is reached. The mixture is then transferred to a capture plate for incubation at room temperature (for example, for one hour). Subsequently, the solution is removed and the plate is washed eight times with 0.1% polysorbate 20 (TWEEN-20 (registered trademark)) in PBS. When the plate is dry, 150 µL/well scintillant (MICROSCINT-20 (trademark), Packard) is added and the plate is counted for ten minutes in a TOPCOUNT (trademark) gamma counter (Packard). The concentration of each Fab that gives 20% or less of maximum binding is selected for use in the competitive binding assay.

According to another embodiment, KD is measured using a BIACORE (registered trademark) surface plasmon resonance assay. For example, an assay using BIACORE (registered trademark)-2000 or BIACORE (registered trademark)-3000 (BIAcore, Inc., Piscataway, NJ) is performed at 25°C using a CM5 chip on which an antigen is immobilized at approximately 10 response units (RU). In one embodiment, a carboxymethylated dextran biosensor chip (CM5, BIACORE, Inc.) is activated using N-ethyl-N'-(3-dimethylaminopropyl)-carbodiimide hydrochloride (EDC) and N-hydroxysuccinimide (NHS) according to the supplier's instructions. The antigen is diluted to 5 µg/mL (approximately 0.2 µM) using 10 mM sodium acetate at pH 4.8 before being injected at a flow rate of 5 µL/min to achieve binding of the protein at approximately 10 response units (RU). After the injection of antigen, 1 M ethanolamine is injected to block unreacted groups. For kinetic measurements, a two-fold serial dilution of Fab (0.78 nM to 500 nM) in PBS containing 0.05% polysorbate 20 (TWEEN-20 (trademark)) surfactant (PBST) is injected at 25°C and a flow rate of approximately 25 µL/min. The association rate (kₒₙ) and dissociation rate (k_{off}) are calculated by simultaneously fitting the association and dissociation sensorgrams using a simple one-to-one Langmuir association model (BIACORE (registered trademark) evaluation software version 3.2). The equilibrium dissociation constant (Kd) is calculated as the ratio k_{off}/kₒₙ. See, for example, Chen et al., J. Mol. Biol. 293: 865-881 (1999). When the on-rate exceeds 10⁶ M⁻¹s⁻¹ by the surface plasmon resonance assay described above, the on-rate may be determined by using a fluorescence quenching technique that measures the increase or decrease of fluorescence emission intensity at 25°C (excitation = 295 nm; emission = 340 nm, bandpass 16 nm) of 20 nM anti-antigen antibody (Fab form) in PBS at pH 7.2 in the presence of gradually increasing concentrations of antigen measured using a spectrometer (for example, a stopped-flow spectrophotometer (Aviv Instruments) or a 8000 series SLM-AMINCO (trademark) spectrophotometer (ThermoSpectronic) which uses a stirring cuvette).

### 10. Regnase-1 variant

In one aspect, the present invention provides Regnase-1 variants in which Ser residue at at least one position selected from the group consisting of positions corresponding respectively to positions 513 and 494 of SEQ ID NO: 1 in Regnase-1 is substituted with another amino acid residue.

In some embodiments, a Regnase-1 variant of the present invention may be a Regnase-1 variant in which the Ser residue(s) at positions corresponding respectively to positions 513 and 494; position 513; or position 494 of SEQ ID NO: 1 is/are substituted with other amino acids. In one embodiment, such a Regnase-1 variant may be a Regnase-1 variant in which the Ser residue(s) at positions 513 and 494 of SEQ ID NO: 1; at position 513 of SEQ ID NO: 1; at position 494 of SEQ ID NO: 1; at positions 516 and 497 of SEQ ID NO: 2; at position 516 of SEQ ID NO: 2; or at position 497 of SEQ ID NO: 2 is/are substituted with other amino acid(s). Amino acid substitutions can be carried out using methods well known to those skilled in the art.

In some embodiments, the Regnase-1 variant of the present invention may be mammalian Regnase-1, and may be mouse or human Regnase-1.

Herein, "substituted" means that an amino acid residue at a certain position in the reference amino acid sequence is occupied by other amino acid residues, and does not require an actual substitution step as an essential requirement.

In some embodiments, examples of the other amino acids by which the Ser residue in the present invention is substituted include, but are not limited to, Ala or Glu. By substituting with Ala or such, a Regnase-1 variant in which the amino acid at the position of interest is not phosphorylated can be obtained, and this is useful as a control substance for non-phosphorylated Regnase-1. Moreover, since substitution with Glu can simulate phosphorylation of an amino acid at a position of interest, such a variant is useful as a substance simulating phosphorylated Regnase-1.

The present invention also relates to a non-human animal having a mutation in Regnase-1 and its progeny. Such a non-human animal can be obtained by using a method known to those skilled in the art, for example, by producing a transgenic non-human animal into which a gene encoding the Regnase-1 variant described herein has been introduced. Examples of non-human animals include monkeys, pigs, dogs, rats, mice, rabbits, hamsters, bovine, sheep, cats, and horses.

### [Examples]

The following are examples of the present invention. It will be understood that various other embodiments may be implemented in light of the above general description.

### <Materials and methods>

Unless otherwise specified in each Example, experiments were performed using the method described in this section.

### (Production of mouse Regnase-1 S435A/S439A (AA) knock-in mouse)

Genomic DNA containing the Regnase-1 gene was isolated from embryonic stem (ES) cells (GSI-1). An approximately 12 kbp genomic fragment encompassing exon 5, exon 6, and the area downstream of the Regnase-1 gene end was subcloned into the pCR-TOPO vector (Thermo Fisher Scientific). A targeting vector was designed to substitute the Ser435 and Ser439 residues of exon 6 with Ala by site-directed mutagenesis. A neomycin resistance gene flanked by two loxPs was inserted into the intron between exon 5 and exon 6. The linearized vector was introduced into GSI-1 ES cells by electroporation. Target ES cells were screened and identified by genomic PCR and Southern blotting. These cells were microinjected into blastocysts derived from C57BL/6 mice. Chimeric male mice were mated with female C57BL/6 mice to produce F1 heterozygous mice. To remove the neomycin gene cassette, F1 mice were further mated with CAG-Cre transgenic mice. After removing the CAG-Cre allele by crossing with C57BL/6 mice, Regnase-1 AA heterozygous (Regnase-1 AA/+) mice were backcrossed for at least ten generations to C57BL/6 mice, and crossbred, to obtain Regnase-1 AA/AA homozygous mice (herein, this mouse may be referred to as "Regnase-1 AA/AA mouse" or "Regnase-1 AA mutant mouse").

### (Production of mouse Regnase-1 mutant allele having frameshift mutation at the C-terminal amino acid)

Regnase-1 frameshift mutant mice were designed and constructed by the NPO for Biotechnology Research and Development (Dr. M. Ikawa and M. Okabe, Osaka University, Osaka, Japan) using CRISPR/Cas9 genome editing technology. The gRNA sequences used in this study were: 5'-GTGGGTGGGGGTAATGGGTA-3' (SEQ ID NO: 52) and 5'-CCTACCCATCCAGAGTAC-3' (SEQ ID NO: 53). Each gRNA sequence was cloned in-frame into the CRISPR/Cas9 vector pSpCas9(BB)-2A-Puro PX459 (Addgene; # 62988) (Nat Protoc 8, 2281-2308 (2013)). The PX459 vector was introduced into fertilized eggs derived from C57BL/6 x C57BL/6 mice using a previously-described method (Dev Growth Differ 56, 122-129 (2014)). These mouse embryos were transferred to the fallopian tubes of pseudopregnant ICR females. Genetic mutations in Regnase-1 allele were identified by DNA sequencing. Mutant mice containing a frameshift mutation that leads to expression of C-terminal truncated Regnase-1 protein (Regnase-1ΔCTD) were crossbread to obtain Regnase-1ΔCTD homozygous mice (also referred to as "Regnase-1 ΔCTD/ΔCTD").

### (Production of mouse Regnase-1 S513A/S513A mutant allele)

Mouse Regnase-1 S513A mutant mice were constructed by the NPO for Biotechnology Research and Development (Dr. M. Ikawa and M. Okabe, Osaka University, Osaka, Japan) using CRISPR/Cas9 genome editing technology. The gRNA sequences used in this study were: 5'-GTGGGTGGGGGTAATGGGTA-3' and 5'-CCTACCCATCCAGAGTAC-3'. Each gRNA sequence was cloned in-frame into the CRISPR/Cas9 vector pX330-U6-Chimeric_BB-CBhhSpCas9 (Addgene; # 42230) (Science 339, 819-23 (2013)). In addition, a single-stranded oligodeoxynucleotide sequence (103 bases, 5'-CCACCGACTATGTGCCCCCGCCACCCACCTACCCATCCAGAGAGTAtTGGgCTGAGCC GTAtCCATTACCCCCACCCACTCCTGTCCTTCAGGAGCCCCAGAG-3' (SEQ ID NO: 54)) was synthesized for the S513A mutation. According to an existing report (Dev Growth Differ 56, 122-129 (2014)), the above-mentioned PX459 vector and single-stranded oligodeoxynucleotide sequence were introduced into fertilized eggs derived from C57BL/6 x C57BL/6 mice. These mouse embryos were transplanted into the fallopian tubes of pseudopregnant ICR females. Genetic mutations in Regnase-1 allele were identified by DNA sequencing.

### (Plasmid)

Regnase-1 expression vectors including pFLAG-CMV2 (Sigma) and pcDNA3.1-Myc as well as viral expression vectors such as pMRX-FLAG-Regnase-1-ires-puro have been previously described (Nature 458, 1185-1190 (2009); and Nat Immunol 12, 1167-1175 (2011)). A truncated form of Regnase-1 lacking the N-terminus or C-terminus was constructed by PCR amplification of Regnase-1 cDNA and inserted into the pFLAG-CMV2 vector. Point mutations in Regnase-1 expression constructs were prepared using the Quickchange II site-directed mutagenesis kit (Agilent Technologies). For overexpression using *E. coli,* a portion of Regnase-1 containing a proline-rich domain and a C-terminal domain (441 to 598) is amplified from Regnase-1 cDNA and inserted into the pGEX 6P vector (GE Healthcare). Myc-Act1, HA-TBK1, and HA-IKKi were PCR amplified from each cDNA and inserted in-frame into the pcDNA3.1 vector. The pTREtight-IL-6 CDS + 3'UTR vector has been previously described (Nature 458, 1185-1190 (2009)).

### (Mouse Regnase-1 (45-339))

An expression construct was formed by adding a GST tag to the N-terminus of the fragment of amino acid numbers 45-339 of mouse Regnase-1 (ZC3h12a) (Uniprot ID: Q5D1E7) (SEQ ID NO: 1). Mouse Regnase-1 (45-339) was expressed in *E. coli* using the above-mentioned construct and purified with Glutathione-Sepharose 4B (GE Healthcare), the GST portion was cleaved with PreScission Protease (GE Healthcare), and gel filtration chromatography was performed for isolation.

### (Anti-Regnase-1 antibody)

Mouse Regnase-1 (45-339) protein was administered to rabbits for immunization. RNA was prepared from cells of immunized rabbits, RT-PCR was performed, the antibody gene was amplified, and the antibody gene was incorporated into a plasmid. The plasmid containing the antibody gene was introduced into *E. coli,* this was cultured, and the plasmid was purified from the cultured *E. coli.* The plasmid into which the antibody gene was introduced was transfected into HEK293 cells, and the antibody was expressed in the culture supernatant. The antibody in the culture supernatant was purified by Protein A.

### (Anti-phosphorylated Regnase-1 antibody)

The peptide LD(pS)GIG(pS)LESQMSEC (SEQ ID NO: 6) in which a cysteine residue is connected to the end of the sequence containing the 435th phosphorylated serine residue and the 439th phosphorylated serine residue of mouse Regnase-1, and the peptide CTYPSREYW(pS)EPY (SEQ ID NO: 7) in which a cysteine residue is connected to the end of the sequence containing the 513th phosphorylated serine residue of mouse Regnase-1 were synthesized, and the proteins formed by conjugating KLH to the respective cysteine residues were administered to rabbits for immunization. Antibodies were purified from the immunized rabbit's antiserum by performing affinity purification with phosphorylated peptides and absorption with non-phosphorylated peptides. The original amino acid sequence LDSGIGSLESQMSE (SEQ ID NO: 8) and a part of the original sequence REYWSEPY (SEQ ID NO: 9) used here were conserved between mice and humans.

### (Anti-TFPI-tag peptide antibody)

TFPI-tag peptide sequence (Thr-Gly-Thr-Gly-Thr-Gly-Thr-MeF-Pro-Ile-Thr-MeF-Pro-Ile (SEQ ID NO: 56), MeF represents N-methylphenylalanine), which has a PEG5 spacer and a T cell epitope peptide (Phe-Asn-Asn-Phe-Thr-Val-Ser-Phe-Trp-Leu-Arg-Val-Pro-Lys-Val-Ser-Ala-Ser-His-Leu-Glu-Gly (SEQ ID NO: 55), derived from tetanus toxin TT p30) on the N terminus, was synthesized and administered to rabbits for immunization. RNA was prepared from cells of immunized rabbits, RT-PCR was carried out to amplify the antibody gene, and the antibody gene was incorporated into a plasmid. The plasmid containing the antibody gene was introduced into *E. coli,* this was cultured, and the plasmid was purified from the cultured *E. coli.* The plasmid into which the antibody gene was introduced was transfected into HEK293 cells, and the antibody was expressed in the culture supernatant. The antibody in the culture supernatant was purified by Protein A.

### (Reagents and cells)

Recombinant mouse IL-17A was purchased from R&D systems. Recombinant mouse IL-1β, mouse TNF-α, mouse IL-6, and human IL-17A were purchased from Biolegend. Anti-Act1 (H-300), IκB-α (C-21), NFκB p65 (C-20), MAPK p38 (C-20), and ERK1 (K23) were purchased from Santa Cruz Biotechnology. Anti-RPL7A (15340-1-AP) was obtained from Proteintech. Anti-FLAG M2, Myc (9E10), and HA (12CA5) antibodies, FLAG M2 affinity gel, and FLAG x 3 peptide were purchased from Sigma. Anti-phospho-IκBα (Ser32/36), phospho-NFκB p65 (Ser468), phospho-MAPK p38 (Thr180/Tyr182), phospho-ERK 1/2 (Thr202/Tyr204), JNK, phospho-JNK (Thr183/Tyr185), phospho-STAT3 (Tyr705), phospho-TBK1 (Ser172), and phospho-IKKε (Ser172) were obtained from Cell Signaling Technology. Anti-CD3ε and type IV collagen were obtained from Abcam. LPS (S. Minnesota R595) and BX795 were purchased from InvivoGen. Bone marrow-derived macrophages were produced by culturing bone marrow cells in RPMI medium containing 20 ng/mL macrophage colony stimulating factor (M-CSF) (Peprotech). HeLa cells were purchased from the American Type Culture Collection. Wild type and Regnase-1AA/AA MEFs were prepared from 13.5 day gestation mouse embryos. Act1-deficient MEFs were produced from Traf3ip2ADJM mice provided by Dr. Y. Matsushima (J Immunol 185, 2340-2349 (2010)). Regnase-1-/-, TBK1-/-, IKKi-/-, and TBK1-/-/IKKi-/- MEFs were prepared as previously described (Nature 458, 1185-1190 (2009); J Exp Med 199, 1641-1650 (2004); and Nat Immunol 9, 684-691 (2008)). IKKα-/-/IKKβ-/- MEF was provided by Dr. I. Verma (Genes & development 14, 1729-1733 (2000)). Effector CD4+ T cells induced by differentiation under *in vitro* conditions were produced according to a previously reported method (Annu Rev Immunol 28, 445-489 (2010)). For the induction of TH1, TH17, or iTreg cells, naive CD4+ T cells (1.0 x 10⁶ cells) were seeded into 96-well plates coated with anti-CD3ε (BD Bioscience, 10 µg/mL), anti-CD3ε (1 µg/mL) antibody and anti-CD28 antibody (1 µg/mL) were used for activation, and cells were cultured for three days under TH1, TH17, or iTreg differentiation conditions: 10 µg/mL anti-IL-4 (BD Bioscience) and 10 ng/mL IL-12 (Peprotech) for TH1; 10 µg/mL anti-IL-4, 10 µg/mL anti-IFN-y (BD Bioscience), 10 ng/mL TGF-β (Peprotech), 30 ng/mL IL-6, and 50 ng/mL IL-23 (Peprotech) for TH17; or 10 µg/mL anti-IL-4, 10 µg/mL anti-IFN-y, and 10 ng/mL TGF-β for iTreg. LSEC was prepared as previously described (Journal of leukocyte biology 38, 213-230 (1985)). A cell line expressing Regnase-1 with N-terminal FLAG epitope tag was constructed by retroviral infection of Regnase-1-/- immortalized MEFs using virus-containing culture supernatant from Plat-E retrovirus packaging cells transfected with pMRX-FLAG-Regnase-1-ires-puro (Gene therapy 7, 1063-1066 (2000)). Cells were cultured and maintained in DMEM containing 2 µg/mL puromycin.

### (EAE model)

Normal EAE was induced by immunization with myelin oligodendrocyte glycoprotein (MOG) (35-55) peptide (AnaSpec). An emulsion of MOG (35-55) peptide (300 ng/mouse) and complete Freund's adjuvant (CFA, InvivoGen) were mixed at a ratio of 1:1 and injected subcutaneously into mice. After intraperitoneal injection of pertussis toxin on day 0 and day 2, mice were monitored daily and assessed by clinical scoring between days 7 to 28 after immunization. Clinical scores were measured using a previously defined scale (Immunity 14, 471-481 (2001)). For the construction of bone marrow chimeric mice, 4 to 5-week old γ-irradiated mice (10 Gy) were intravenously injected with bone marrow cells (3.0-5.0 x 10⁷ cells/mL). At least four weeks later, chimeric mice were challenged with EAE. EAE challenge induced by passive transplantation of pathogenic CD4+ T cells was performed according to a previously described method (Cell 148, 447-457 (2012)). Briefly, wild type mice were sacrificed ten days after the injection of MOG (35-55) peptide/CFA and pertussis toxin. CD4+ T cells were isolated from splenocytes (4 x 10⁶ cells) and co-cultured with irradiated splenocytes pulsed with MOG peptide. CD4+ T cells isolated from co-cultured cells using CD4 (L3T4) microbeads and autoMACS separator (Miltenyi), were intravenously injected to wild type, Regnase-1AA/AA, and Regnase-1ΔCTD mice (1.5 x 10⁷ cells/mouse). Frozen sections were prepared from the spinal cord, lymph nodes, and spleen using a Leica CM 1850 cryostat (Leica) and immunostained with the indicated antibodies. To prepare spinal cord sections, the present inventors used a previously described method (Archives of histology and cytology 66, 123-143 (2003)).

### (Flow cytometry)

The following antibodies were prepared for flow cytometry: PerCP-Cy5.5 conjugated anti-mouse CD4, PE conjugated anti-mouse IL-17A, FITC conjugated anti-mouse IFN-γ, PE conjugated anti-mouse CD25 (BD bioscience), and Alexa-647 conjugated anti-mouse Foxp3 (Biolegend). Spinal cord cells were stained with anti-CD4 antibody and F4/80 antibody (Biolegend). CD4+ T cells were cultured with 100 nM phorbol 12-myristate 13-acetate (PMA) (Sigma), 1 µM ionomycin (Sigma), and GolgiPlug (BD bioscience) for two hours at 37°C. After cell permeabilization and fixation, intracellular staining (IPNγ, IL-17A, and Foxp3) was carried out using either one of these kits: Cytofix/Cytoperm intracellular staining kit (BD bioscience, in the case of IFNγ and IL-17A); or Foxp3/transcription factor staining buffer kit (Affymetrix, in the case of Foxp3).

### (Immunoprecipitation)

Techniques for transient expression of proteins using HEK293 cells have been previously described (Nat Immunol 12, 1167-1175 (2011)). Cells were disrupted by sonication 24 hours after transfection. After removing cell debris by centrifugation at 20,000 x g for five minutes, the cell lysate was incubated at 4°C for one hour with either anti-FLAG M2 antibody or anti-Myc antibody (Sigma) bound to Dynabeads Protein G (Thermo Fisher Scientific). Thereafter, the beads were washed twice with Tris buffer. The immunoprecipitated protein was eluted with 3x SDS sample buffer and applied to a 10% SDS-PAGE gel. Phosphorylated mouse Regnase-1 was purified from MEFs stably expressing FLAG-Regnase-1. Following stimulation with IL-1β or IL-17A, the cells were disrupted by sonication. Cell lysates were incubated with anti-FLAG M2 affinity gel for one hour at 4°C. Bound protein was eluted with 0.15 mg/mL FLAG x 3 peptide (Sigma) in Tris buffer and subjected to 7.5% native PAGE gel.

### (Gel filtration analysis)

The C-terminal segment (441 to 598) of mouse Regnase-1 was produced as a GST fusion protein in *E. coli* Rosetta2 (DE3) cells (Merck Millipore). Cells were lysed in Bugbuster protein extraction reagent (Merck Millipore) supplemented with protease inhibitors. The fusion protein was purified from the cell lysate by affinity chromatography using Glutathione Sepharose 4B (GE Healthcare), and cleaved in Tris buffer [20 mM Tris-HCl (pH 7.4) and 150 mM NaCl] using PreScission Protease (GE Healthcare) to release the mouse Regnase-1 segment. After addition of 1% CHAPS to increase protein solubility, GST protein was removed using a Glutathione Sepharose 4B column. Purified mouse Regnase-1 segment was loaded onto a Superdex 200 gel filtration column (GE Healthcare). The apparent molecular weight of the elution peak was estimated from the elution pattern of the molecular weight marker for gel filtration chromatography (Sigma).

### (In vitro phosphorylation assay)

Mouse Regnase-1 protein was obtained from MEFs that stably express FLAG-Regnase-1. Cells were suspended in 20 mM Tris-HCl, pH 7.4, and 150 mM NaCl supplemented with Complete mini protease inhibitor and PhosStop phosphatase inhibitor cocktail (Roche) and disrupted using an ultrasonic water bath (Bioruptor Plus, Diagenode). Regnase-1 was purified on a FLAG M2 affinity gel (Sigma). The following recombinant proteins were prepared for *in vitro* phosphorylation assays: GST protein (Sigma), GST-tagged TBK1 (Sigma), GST-tagged IKKi (Thermo Fisher Scientific), and Lambda protein phosphatase (New England BioLabs). FLAG-Regnase-1 (50 µg/mL) was incubated in 0.2 M HEPES at pH 7.0, 20 mM MgCl2, 2 mM ATP (or 50 µCi [γ-³²P] ATP), and 1.0 M mannitol for four hours at 30°C. Samples were mixed with either 3x SDS sample buffer 14 or 4x native PAGE sample buffer (0.2 M Tris-HCl, pH 6.8, 40% glycerol, and 0.4% bromophenol blue) and were loaded onto 10% SDS-PAGE gels or 7.5% native PAGE gels. Regnase-1 was detected by immunoblotting. The phosphorylated protein was visualized by autoradiography in the presence of [γ-³²P] ATP.

### (Isolation of intracellular fraction)

Separation and isolation of the ER membrane fraction was performed according to previously described methods (Rna 9, 1123-1137 (2003)). MEF cells (5 x 10⁷ cells) were suspended in a homogenization buffer [10 mM HEPES-KOH, pH 7.5, 10 mM KoAc, 1.5 mM Mg(OAc)2, 2 mM DTT, 1 mM PMSF, and 200 U/mL RNaseOUT ribonuclease inhibitor (Thermo Fisher Scientific)] and then disrupted with a Dounce homogenizer. To separate the microsomal and cytosolic fractions, the homogenate was subjected to low speed centrifugation (1,500 x g) for five minutes. The supernatant was further subjected to high speed centrifugation (Beckman TLA 45 rotor) at 65,000 x g for 20 minutes at 4°C. The microsomal pellet was resuspended in Tris buffer. To isolate the ER membrane fraction, the homogenate was mixed with 2.5 M sucrose in HKM buffer [50 mM HEPES-KOH, 150 mM KoAc, and 5 mM Mg(OAc)2] at a 1:4 ratio. 0.5 ml of 0.25 M sucrose in HKM buffer and 0.75 ml of 1.3 M sucrose in HKM buffer were layered on top of 2 mL of the mixture. After centrifugation (Beckmann TLA100.3 rotor) at 500,000 x g for 45 minutes at 4°C, the ER membrane within the 1.3 M/2.0 M sucrose interface was extracted and diluted with HKM buffer. After centrifugation at 500,000 x g for 20 minutes at 4°C, the membrane was resuspended in Tris buffer.

### (Polysome profiling)

WT and mouse Regnase-1ΔCTD MEF cells (1-2 x 10⁸ cells) were pretreated with 5 µg/mL cycloheximide for ten minutes prior to cell harvest. The collected cells were suspended in 1 mL homogenization buffer supplemented with 100 µg/mL cycloheximide, and lysed under low osmotic pressure by several strokes of a Dounce homogenizer. To solubilize the membrane containing the cell fraction, 10% digitonin was added to a final concentration of 2%. After five minutes of low speed centrifugation (1500 x g), the resulting lysate supernatant was loaded to the top of a linear gradient 10-60% sucrose polysome buffer [50 mM HEPES-KOH at pH 7.5, 150 mM KCl, 10 mM MgSO4, 2 mM DTT, 1 mM PMSF, 100 µg/mL cycloheximide, and 100 U/ml RNaseOUT ribonuclease inhibitor]. After ultracentrifugation at 36,000 rpm for two hours at 4°C in a Beckman SW41 Ti rotor, fractions were collected from the top of the sucrose gradient using a Piston Gradient Fractionator (Biocomp Instruments), they were transferred to UV-Star (registered trademark) 96-well plate (Greiner Bio-one) and monitored by UV absorbance at 260 nm.

### (Quantitative PCR analysis)

Total RNA was purified by High Pure (trademark) RNA isolation kit (Roche) and reverse transcribed using ReverTra Ace (registered trademark) kit (TOYOBO). For quantitative PCR, cDNA was PCR amplified using a Thunderbird (registered trademark) Probe qPCR mix (TOYOBO). TaqMan probes for mouse IL-6, TNF, LCN-2, GM-CSF, CXCL-1, CXCL2, CCL5, and CCL-20 were purchased from Applied Biosystems. Fluorescence was detected by Viia7 (trademark) real-time PCR system (Applied Biosystems). Expression of 18S rRNA was used to normalize mRNA expression levels.

### (Tet-off system)

The previously described 8pTREtight-IL6-CDS + 3'UTR was simultaneously transfected along with a series of expression vectors encoding mouse Regnase-1, Act1, and IKKi, into HEK293 Tet-off cells (3.0 x 10⁶ cells). After three hours, the cells were divided into three 60-mm culture dishes and cultured overnight. Suppression of IL6-CDS + 3'UTR expression was induced by culturing cells in the presence of 1 µg/mL doxycycline.

### (Statistical analysis)

Unless otherwise stated, statistical analysis of differences between the two groups was performed by Student's t test (two-sided). P < 0.05 was considered statistically significant.

### <Results>

### Example 1

In this example, the S435A/S439A (AA) mutations were shown to prevent IKK-mediated phosphorylation and degradation of Regnase-1.

Regnase-1 is phosphorylated by the IKK complex and then degraded in LPS-activated macrophages *via* the ubiquitin-proteasome system. Two serine residues Ser435 and Ser439 in Regnase-1 have been determined as putative IKK phosphorylation sites. To elucidate how IKK-mediated phosphorylation and degradation of Regnase-1 regulates cytokine expression in the immune response, the inventors produced a knock-in mouse in which both Ser435 and Ser439 in the Regnase-1 protein have undergone amino acid substitution with Ala (Fig. 1-2A). The mutated Regnase-1 gene was confirmed by genomic PCR and direct sequencing (Fig. 1-2B). Homozygous Regnase-1 knock-in mice (Regnase-1 AA/AA) were born at the expected Mendelian ratio, developed normally, and did not have any symptoms of autoimmune disease as previously described for Regnase-1-deficient mice.

To detect the non-phosphorylated and phosphorylated forms of Regnase-1, the present inventors produced monoclonal antibodies with excellent sensitivity. As expected, LPS-stimulated Regnase-1AA/AA macrophages did not show any Regnase-1 proteolysis, suggesting that this mutant protein is resistant to IKK-mediated degradation (Fig. 1-3C). Compared to wild-type macrophages, Regnase-1AA/AA macrophages showed a significant increase in protein production over time. This indicates that IKK-mediated phosphorylation of Regnase-1 is essential for its degradation. The phosphorylation of Regnase-1 by IRAK was detected as a delayed band in electrophoresis (Fig. 1-3C), and this process was typically observed in Regnase-1AA/AA macrophages because it occurs *via* an IKK-independent pathway. Other phosphorylations induced by LPS, for example, IκB and NF-κB p65 in the NF-κB signaling pathway, and mitogen-activated protein kinase (MAPK) p38 and extracellular signal-regulated kinase (ERK) 1/2 in the MAPK pathway, were the same in both wild type and Regnase-1AA/AA macrophages (Fig. 1-3C), indicating that this mutation does not affect the LPS signaling pathway in Regnase-1AA/AA macrophages. Nevertheless, Regnase-1AA/AA macrophages show reduced production of IL-6 and IL-12 compared to the wild type in the presence of very low concentrations of LPS or TLR ligands, but such is not shown regarding TNF-α (Fig. 1-4D), indicating that cytokine production was suppressed by this mutation upon exposure to various TLR ligands under *in vitro* conditions.

### Example 2

In this example, Regnase-1AA/AA mice were shown to be resistant to experimental autoimmune encephalomyelitis (EAE) through a weakened response to IL-17.

To further investigate the immunosuppressive effect of the Regnase-1 AA mutant, the present inventors used the EAE model. Regnase-1AA/AA mice showed delayed onset and slow progression of EAE compared to the control (Fig. 2-1A). Histological analysis of the spinal cord revealed significant reduction in inflammation, demyelination, axonal degeneration, and T cell infiltration into neuronal tissue in Regnase-1AA/AA mice (Fig. 2-1B). The number of infiltrating CD4+ T cells was significantly lower in Regnase-1AA/AA mice than in control mice (Fig. 2-1C). Immunofluorescence analysis of lymph nodes revealed impaired germinal center formation and plasma cell accumulation in lymph nodes of Regnase-1AA/AA mice (data not shown).

The present inventors made a comparison between wild type mice and Regnase-1AA/AA mice, regarding the ability of their naive CD4+ T cells to differentiate into TH1, TH17, or Treg cells under *in vitro* conditions to clarify which cell types expressing Regnase-1AA/AA proteins are responsible for resistance to EAE. These showed similar differentiation patterns (Fig. 3-1A). Next, the inventors investigated, using bone marrow chimeras, which of immune cells produced from bone marrow and non-hematopoietic cells are required for the suppression of EAE pathogenesis in Regnase-1AA/AA mice. Transplantation of wild type bone marrow cells into Regnase-1AA/AA mice resulted in more effective disease suppression than the controls, whereas wild type mice with Regnase-1AA/AA bone marrow cells showed no significant differences (Fig. 2-1D). These results suggest that the improvement of EAE disease in Regnase-1AA/AA mice was caused by non-hematopoietic cells and not by immune cells.

To further confirm this, the present inventors used a transplanted EAE model in which MOG-specific autoreactive CD4+ T cells were transplanted intravenously into wild type and Regnase-1AA/AA mice (Immunity 29, 628-636 (2008); and Cell 148, 447-457 (2012)). In this model, activated TH17 cells can induce activation of signal transducer and activator of transcription 3 (STAT3) and inflammation in the endothelial cells of the dorsal blood vessels of the fifth lumbar spinal cord in a manner dependent on IL-6 and IL-17. Immunohistological analysis of phosphorylated STAT3, an indicator of inflammation, in splenic endothelial cells (type IV collagen positive) revealed low levels of phospho-STAT3 in Regnase-1AA/AA mice. This suggested that Regnase-1 AA mutation suppresses inflammation of endothelial cells induced mainly by pathogenic TH17 cells (Figs. 2-2E and 2-2F). STAT3 phosphorylation was also reduced in endothelial cells of the dorsal blood vessels of the 5th lumbar spinal cord of Regnase-1AA/AA mice (Figs. 2-2G and 2-2H). These findings indicate that this mutated protein suppresses STAT3 activation in endothelial cells by reducing IL-6 production, and that the degradation of Regnase-1 by IKK has an important role in inducing EAE pathogenesis.

IL-17 activates both NF-κB and MAPK signaling pathways, but is a weak activator of NF-κB (Nature reviews. Immunology 9, 556-567 (2009)). In nonhematopoietic cells, cooperative stimulation of IL-6 and IL-17 leads to overexpression of IL-6 in the feedback amplification loop of NF-κB and STAT3 activation (Immunity 29, 628-636 (2008); and J Immunol 189, 1928-1936 (2012)). The present inventors measured cytokine and chemokine production in primary cells derived from wild-type and Regnase-1AA/AA mice following stimulation with IL-17A or IL-17A in combination with TNF-α or IL-6. Mouse embryonic fibroblasts (MEF) and liver sinusoidal endothelial cells (LSEC) derived from Regnase-1AA/AA mice were found to show decreased expression of IL-6, Regnase-1, CXCL-1, CXCL-2, and CCL-20 mRNA than in the wild type, 24 hours after stimulation with TNF-α + IL-17A (Fig. 2-2I [MEF] and Fig. 3-1B [LSEC]). The difference in target mRNA expression in MEF between wild-type and Regnase-1AA/AA cells was more apparent in the latter half of the stimulation period (Fig. 2-2I). Similarly, the levels of IL-6, CXCL-1, and CXCL-2 mRNA after stimulation with IL-17A in combination with IL-6 or TNF-α were also lower in LSEC of Regnase-1AA/AA mice than in wild-type cells (Fig. 3-2C). These results indicated that IKK-mediated phosphorylation and degradation of Regnase-1 takes place *via* NF-κB activation upon stimulation of nonhematopoietic cells with IL-17A, and through this, the expression of inflammatory factor mRNA may be regulated.

### Example 3. Effect of alleviating pathological condition of Regnase-1 AA mutant mice in imiquimod-induced psoriasis model

To the right pinna and the shaved dorsocervical skin of C57BL/6 mice (wild-type mice; WT) or Regnase-1 AA mutant mice (AA), 62.5 mg of imiquimod cream (Beselna cream 5%; Mochida Pharmaceutical Co., Ltd.) was applied for five consecutive days. Evaluation of the pinna lesion was performed by measuring the thickness of the right pinna using a dial thickness gauge (OZAKI MFG. Co., Ltd.) over time from before applying imiquimod. In addition, the dorsocervical skin was evaluated by macroscopically evaluating the extent of erythema, thickening, and scaling, individually in four stages, and the combined total score (12 points) was presented.

As a result, marked thickening of the pinna was observed in the wild-type mice, accompanying the application of imiquimod (Fig. 4A). In addition, psoriasis-like skin lesions such as erythema, thickening, and scaling were induced in the dorsocervical skin to which imiquimod was applied (Fig. 4B). On the other hand, in Regnase-1 AA mutant mice, thickening of the pinna and psoriasis-like skin lesions (erythema, thickening, and scaling) in the back of the neck were reduced compared to those in wild-type mice (Figs. 4A and 4B).

### (Pathological examination)

Three days after the final application of imiquimod, wild-type mice or Regnase-1 AA mutant mice were necropsied after being euthanized by exsanguination under deep anesthesia. The skin of the pinna at the application site was sampled, immersed and fixed in 10% neutral buffered formalin fixative solution, embedded in paraffin by a conventional method, sliced at approximately 3 micrometers to produce hematoxylin-eosin (HE) stained specimens. The HE-stained specimen of the skin of the pinna at the application site was observed histopathologically under a light microscope. In addition, the thickness of the epidermis was measured using a micrometer and classified into the following categories: basal layer and spinous layer, granular layer, horny layer, and all epidermis layers.

As a result, psoriasis-like skin lesions such as microabscess and epidermal hyperplasia with neutrophil infiltration in the dermis were notably observed in wild-type mice (Fig. 5A, Table 2). On the other hand, Regnase-1 AA mutant mice showed alleviation of such psoriasis-like lesions than wild-type mice. In addition, as a result of morphometry of epidermis thickness, Regnase-1 AA mutant mice showed lower values than wild-type mice in all categories (Fig. 5B).

**[Table 2]**

| Histopathological findings in the skin of the pinna at the application site of wild-type mice and Regnase-1 AA mutant mice in imiquimod-induced psoriasis model | | | | | | | | | | |
|---|---|---|---|---|---|---|---|---|---|---|
| Findings | WT | | | | | AA | | | | |
| | #6 | #7 | #8 | #9 | #10 | #1 | #2 | #3 | #4 | #5 |
| Epidermal hyperplasia | + | + | + | + | + | ± | ± | ± | ± | ± |
| Microabscess in the epidermis | + | - | - | + | ++ | - | ± | - | - | - |

| Inflammatory cell infiltration in the dermis | | | | | | | | | | |
|---|---|---|---|---|---|---|---|---|---|---|
| Neutrophils | ++ | ++ | ++ | ++ | ++ | ± | ± | ± | ± | ± |
| Mononuclear cells | + | + | + | + | + | ± | ± | ± | ± | ± |

| | | | | | | | | | | |
|---|---|---|---|---|---|---|---|---|---|---|
| #Animal number Lesion severity: ±, slight; +, mild; ++, moderate; +++, severe; -, no significant change | | | | | | | | | | |

### (Quantification of various gene expressions)

Imiquimod was applied every other day on Days 0, 2, and 4, the mice were euthanized in a stepwise manner the day after application, and the pinnas which were the application site were collected. RNA was extracted according to a conventional method using QIAZOL Lysis Reagent (QIAGEN, No. 79306) and RNeasy 96 kit (QIAGEN, No. 74182). After obtaining complementary DNA by reverse transcription, expression of the following target genes was measured by Taqman PCR method. Taqman PCR was analyzed with LightCycler 480 II (manufactured by Roche) using QuantiTect Probe RT-PCR Kit (QIAGEN, No. 204445). The Taqman probes used are shown below.
B2m (manufactured by Applied biosystems, Mm00437762_ml): endogenous control
116 (manufactured by Applied biosystems, Mm00446190_ml): inflammatory cytokine
Il1a (manufactured by Applied biosystems, Mm00439620_ml): inflammatory cytokine
Cxcl2 (manufactured by Applied biosystems, Mm00436450_ml): leukocyte migration factor
Hbegf (manufactured by Applied biosystems, Mm00439306_ml): cell growth factor
Sprr2i (manufactured by Applied biosystems, Mm00726832_s1): keratinocyte marker
Keratin 6A (manufactured by Applied biosystems, Mm00833464_gl): keratinocyte marker

As a result, in wild-type mice, the genes (116, Il1a, Cxcl2, and Hbegf) which are targets of Regnase-1 (see Figs. 19-1 to 19-5) increased with disease induction (Fig. 6). On the other hand, in Regnase-1 AA mutant mice, the increase in these genes induced by disease induction was suppressed. Inhibitory actions was suggested for cell proliferation activity and inflammatory cytokine production associated with pathological conditions in Regnase-1 AA mutant mice. In wild-type mice, Sprr2i and Keratin 6A which are factors expressed in keratinocytes increased with disease induction (Fig. 6). On the other hand, increase in these genes was suppressed in Regnase-1 AA mutant mice, which reflects the alleviation effect on psoriasis-like lesions. This suggests that proliferation of keratinocytes was suppressed in Regnase-1 AA mutant mice.

### Example 4. Effect of alleviating pathological condition of Regnase-1 AA mutant mice in anti-glomerular basement membrane antibody-induced nephritis model

Nephritis elicited by an anti-glomerular basement membrane antibody was induced in wild-type mice and Regnase-1 AA mutant mice. Mice were immunized with sheep-derived IgG antibodies mixed with an adjuvant, and then sheep antiserum (nephrotoxic serum) obtained by immunizing sheep with rat glomeruli was administered once a day for four consecutive days. Blood was collected at two weeks after nephrotoxic serum administration, and serum creatinine, urea nitrogen, and cystatin C, which are indicators of renal damage, were measured. Similarly, pooled urine collection was performed at 2 weeks, and the urinary total protein level and urinary creatinine level were measured. Serum parameters and urine parameters were measured using TBA-120-FR (manufactured by Toshiba Medical Systems Corporation). Abbreviations used in each figure are as follows: non-disease-induced wild-type mouse (WTNC); non-disease-induced Regnase-1 AA mutant mouse (AANC); disease-induced wild-type mouse (WTDC); and disease-induced Regnase-1 AA mutant mouse (AADC).

As a result, serum creatinine level and urinary total protein-to-urinary creatinine ratio, which are indicators of glomerular damage, were lower in disease-induced Regnase-1 AA mutant mice (AADC) than in disease-induced wild-type mice (WTDC) (Fig. 7A). Regnase-1 AA mutant mice were suggested to have protective actions against renal damage.

### (Quantification of hydroxyproline)

Two weeks after nephrotoxic serum administration, mice were euthanized and kidneys were collected. The collected kidneys were lyophilized and then weighed. 6N Hydrochloric acid was added and hydrolysis treatment was performed overnight at 95°C, and the amount of hydroxyproline (Hyp) per kidney weight was measured by mass spectrometry.

As a result, the amount of hydroxyproline, which is an index of tissue fibrosis, was lower in disease-induced Regnase-1 AA mutant mice (AADC) than in disease-induced wild-type mice (WTDC) (Fig. 7B). Regnase-1 AA mutant mice were suggested to have protective actions against renal fibrosis.

### (Quantification of various gene expressions)

Two weeks after nephrotoxic serum administration, mice were euthanized and kidneys were collected. RNA was extracted from the collected kidney using QIAZOL Lysis Reagent (QIAGEN, No. 79306) and RNeasy 96 kit (QIAGEN, No. 74182) according to a conventional method. After obtaining complementary DNA by reverse transcription, expression of the following target genes was measured by Taqman PCR method. Taqman PCR was analyzed with LightCycler 480 II (manufactured by Roche) using QuantiTect Probe RT-PCR Kit (QIAGEN, No. 204445). The Taqman probes used are shown below.
Gapdh (manufactured by Applied biosystems, Mm99999915_gl): endogenous control
Col1a1 (manufactured by Applied biosystems, Mm00801666_gl): organ fibrosis index
Acta2 (manufactured by Applied biosystems, Mm00725412_s1): organ fibrosis index
Ctgf (manufactured by Applied biosystems, MmOl 192933_gl): organ fibrosis index
Ddr1 (manufactured by Applied biosystems, Mm01273496_ml): organ fibrosis index
Pdgfb (manufactured by Applied biosystems, Mm00440677_ml): organ fibrosis index

As a result, the amount of Col1a1 gene and the amount of Acta2 gene, which are indicators of tissue fibrosis, were lower in disease-induced Regnase-1 AA mutant mice (AADC) than in disease-induced wild-type mice (WTDC) (Fig. 8A). Regnase-1 AA mutant mice were suggested to have protective actions against renal fibrosis.

Further, in wild-type mice, the genes (Ctgf, Ddr1, and Pdgfb) that are targets of Regnase-1 (see Figs. 19-1 to 19-5) increased by disease induction. On the other hand, the increase in these genes was suppressed in Regnase-1 AA mutant mice (Fig. 8B). Regnase-1 AA mutant mice were suggested to have suppressive action on fibrosis through suppression of expression of fibrosis related factors.

### (Changes in the number of various blood cells in blood)

The blood count was taken by XT-2000iV (manufactured by Sysmex Corporation) using blood obtained from mice two weeks after administration of nephrotoxic serum. In wild-type mice, the number of white blood cells, neutrophils, and monocytes per unit blood volume increased following disease induction, but in Regnase-1 AA mutant mice (AADC), increase in the number of monocytes and the number of neutrophils in blood accompanying the disease was suppressed (Fig. 8C). These results suggest that Regnase-1 AA mutant mice may suppress the increase in inflammatory cells associated with the onset of nephritis.

### (Pathological examination)

Two weeks after the start of anti-glomerular basement membrane antibody administration, wild-type mice or Regnase-1 AA mutant mice were necropsied after being euthanized by exsanguination under deep anesthesia. The kidneys and lungs were sampled, immersed and fixed in 4% paraformaldehyde solution, embedded in paraffin by a conventional method, and sliced at about 3 micrometers to produce hematoxylin-eosin (HE) stained specimens. The HE-stained specimens of the kidney and lung were observed histopathologically under an optical microscope. For the kidney, the total glomerular lesions on the specimen were counted for each of the crescent, sclerosis, the sum of the crescent and sclerosis, and the total glomerular lesion, and their percentage was calculated.

As a result, the kidney of wild-type mice (WT) prominently showed various lesions of crescentic glomerulonephritis such as crescent formation and glomerulosclerosis (Figs. 9A and 9B). On the other hand, such glomerulonephritis lesions were reduced in Regnase-1 AA mutant mice (AA) than in wild-type mice. In the lung, wild type mice showed prominent inflammatory cell infiltration or granulomas in the alveoli or perivascular interstitium (Fig. 9C, Table 3). On the other hand, such lung lesions were reduced in Regnase-1 AA mutant mice than in wild-type mice.

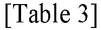

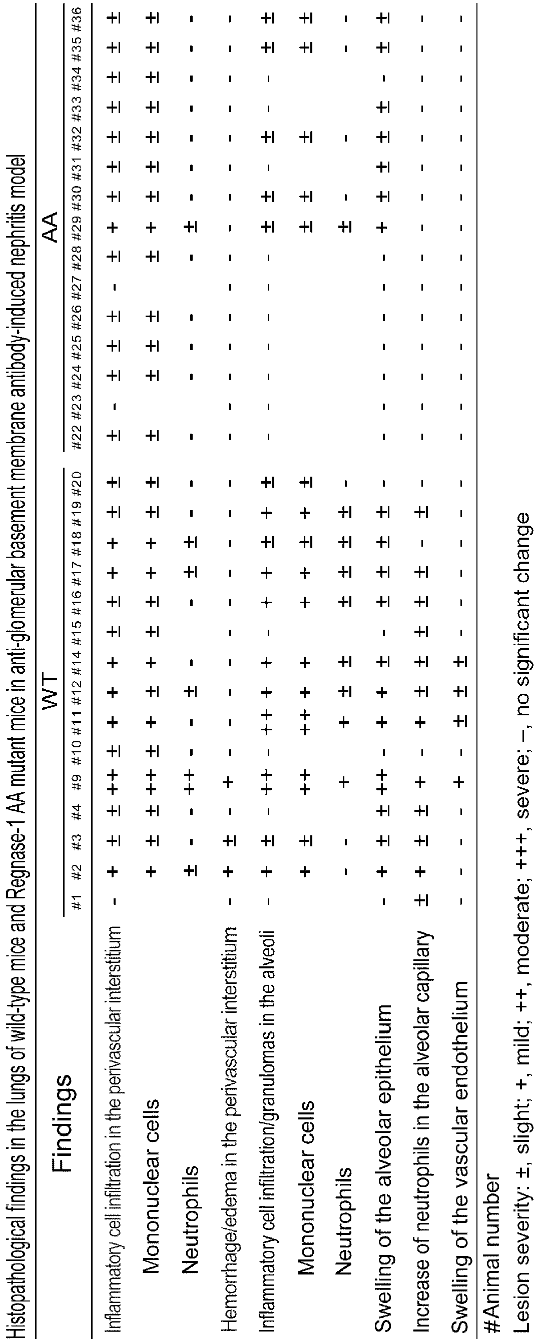

### Example 5. Effect of alleviating pathological condition of Regnase-1 AA mutant mice in bleomycin-induced scleroderma model

The bleomycin-induced scleroderma model was induced in wild-type mice and Regnase-1 AA mutant mice. Bleomycin was administered by a subcutaneous implantation pump, and after four weeks, euthanasia was performed, and the lungs and skin of the administration site were collected. Abbreviations used in each figure are as follows: non-disease-induced wild-type mouse (WTNC); non-disease-induced Regnase-1 AA mutant mouse (AANC); disease-induced wild-type mouse (WTDC); and disease-induced Regnase-1 AA mutant mouse (AADC).

### (Quantification of hydroxyproline in skin)

The collected skin was lyophilized and then weighed. 6 N Hydrochloric acid was added for hydrolysis treatment, and mass spectrometry was carried out to determine the amount of hydroxyproline per skin weight.

As a result, accompanying disease induction, the amount of hydroxyproline, which is an index of tissue fibrosis, was shown to be lower in disease-induced Regnase-1 AA mutant mice (AADC) than in disease-induced wild-type mice (WTDC) (Fig. 10A). Regnase-1 AA mutant mice were suggested to have protective actions against skin fibrosis.

### (Quantification of various gene expressions in the lungs)

Four weeks after the start of bleomycin administration, the mice were euthanized and the lungs were collected. RNA was extracted from the collected lungs using QIAZOL Lysis Reagent (QIAGEN, No. 79306) and RNeasy 96 kit (QIAGEN, No. 74182) according to a conventional method. After obtaining complementary DNA by reverse transcription, expression of the following target genes was measured by Taqman PCR method. Taqman PCR was analyzed with LightCycler 480 II (manufactured by Roche) using QuantiTect Probe RT-PCR Kit (QIAGEN, No. 204445). The Taqman probes used are shown below.
Gapdh (manufactured by Applied biosystems, Mm99999915_gl): endogenous control
Col1a1 (manufactured by Applied biosystems, Mm00801666_gl): organ fibrosis index

As a result, in wild-type mice, the fibrosis index Col1a1 gene increased with disease induction. On the other hand, in Regnase-1 AA mutant mice, gene increase was suppressed (Fig. 10C). Regnase-1 AA mutant mice was suggested to have suppressive actions on fibrosis.

### (Pathological examination of the lungs)

Four weeks after the start of bleomycin administration, wild-type mice or Regnase-1 AA mutant mice were necropsied after euthanasia by exsanguination under deep anesthesia. Lungs were sampled and fixed by immersion in a 4% paraformaldehyde solution, embedded in paraffin by a conventional method, and sliced at approximately 3 micrometers to produce hematoxylin-eosin (HE) stained specimens. The HE-stained specimens of the lung were observed histopathologically under an optical microscope.

As a result, in wild-type mice, alveolar epithelial degeneration and necrosis, inflammatory cell (neutrophil, mononuclear cell/foam cell) infiltration in the alveoli/interstitium, eosinophilic substance/exudate in the alveoli, bronchoalveolar epithelial hyperplasia, alveolar/interstitial fibrosis, and edema/lymphatic dilatation in the perivascular and peribronchial interstitium were prominently observed (Fig. 10B, Table 4). On the other hand, among those lung injury lesions, inflammatory cell infiltration, bronchoalveolar epithelial hyperplasia, alveolar/interstitial fibrosis, and edema/lymphatic dilatation in the perivascular and peribronchial interstitium were especially decreased in Regnase-1 AA mutant mice than in wild-type mice.

**[Table 4]**

| Histopathological findings in the lungs of wild-type mice and Regnase-1 AA mutant mice in bleomycin-induced scleroderma model | | | | | | | | | | | | | | |
|---|---|---|---|---|---|---|---|---|---|---|---|---|---|---|
| Findings | WT | | | | | | | AA | | | | | | |
| | #1 | #2 | #3 | #4 | #5 | #6 | #7 | #8 | #9 | #10 | #11 | #12 | #13 | #14 |
| Alveolar epithelial degeneration and necrosis | ++ | ++ | ++ | ++ | ++ | +++ | ++ | ++ | + | ++ | ++ | ++ | ++ | ++ |

| Inflammatory cell infiltration in the alveoli/interstitium | | | | | | | | | | | | | | |
|---|---|---|---|---|---|---|---|---|---|---|---|---|---|---|
| Neutrophils | ± | ± | + | ± | + | ++ | +++ | ± | ± | + | ± | ± | - | ± |
| Mononuclear cells / foam cells | + | + | ++ | + | ++ | ++ | ++ | + | + | + | + | ++ | + | + |
| Eosinophilic substance/exudate in the alveoli | + | + | + | + | + | + | + | + | + | + | + | + | + | + |
| Bronchoalveolar epithelial hyperplasia | + | + | + | + | ± | ++ | + | + | ± | - | + | ± | - | - |
| Alveolar/interstitial fibrosis | ++ | + | ++ | + | ++ | ++ | ++ | + | + | + | + | ++ | + | + |
| Edema / lymphatic dilatation in the perivascular and peribronchial interstitium | + | ± | + | + | + | ++ | +++ | + | ± | ± | + | + | ± | ++ |

| | | | | | | | | | | | | | | |
|---|---|---|---|---|---|---|---|---|---|---|---|---|---|---|
| #Animal number Lesion severity: ±, slight; +, mild; ++, moderate; +++, severe; -, no significant change | | | | | | | | | | | | | | |

### Example 6. Effect of alleviating pathological condition of Regnase-1 AA mutant mice in experimental autoimmune uveitis model

Complete Freund's adjuvant and IRBP (interphotoreceptor retinoid binding protein) peptide were mixed, and used to immunize C57BL/6 mice (wild-type mice) or Regnase-1 AA mutant mice intradermally. Mice assigned to the non-pathological control group were administered with a mixture of the solvent and complete Freund's adjuvant without adding the peptide. The peptide was administered at 140 nmol or 280 nmol per mouse. The sequence of the peptide used is shown below.
IRBP peptide: LAQGAYRTAVDLESLASQLT (SEQ ID NO: 19)

After the immunization, funduscopic examination was performed approximately three times a week, and the degree of inflammation and retinal structure disorder was scored. Scoring was performed by referring to the literature, and the score was evaluated on a five-step scale of 0 to 4 (Exp Eye Res 87(4), 319-326 (2008)). The results are shown in Figs. 11-1A and 11-1B and Figs. 11-2C and 11-2D.

As a result, in wild-type mice, the inflammatory score and the structural damage score were increased by disease induction, but in Regnase-1 AA mutant mice, the inflammatory score and the structural damage score were hardly increased. Regnase-1 AA mutant mice were suggested to have protective effects against ocular inflammation and retinal structural damage.

### Example 7. Effect of alleviating pathological condition of Regnase-1 AA mutant mice in T cell transfer model of experimental autoimmune uveitis

As in Example 6, complete Freund's adjuvant and IRBP (interphotoreceptor retinoid-binding protein) peptide were mixed and used to immunize C57BL/6 mice (wild-type mice) intradermally.

On day 12 after immunization, mice were euthanized and spleens were collected. After spleen cells were hemolyzed, the cells were cultured for two days in RPMI-1640 medium containing IRBP peptide at 5 µM and ODN1826 (Invitrogen, tlrl-1826) at 0.3 µg/mL. Cells were isolated using Pan T Cell Isolation Kit II, mouse (Miltenyi, 130-095-130), and transferred into wild-type mice and Regnase-1 AA mutant mice by intraperitoneally administering 1 x 10⁶ cells per mouse.

After transfer, funduscopy examination of both eyes was performed from day 11 to day 18, and the degree of inflammation was scored. The score was evaluated on a five-step scale of 0 to 4.

On day 18 after transfer, the animals were necropsied after being euthanized by exsanguination under deep anesthesia. Eyeballs were sampled, fixed by immersion in glutaraldehyde solution or 4% paraformaldehyde solution, embedded in paraffin by a conventional method or AMeX method, sliced at about 3 micrometers, and stained with hematoxylin eosin (HE) to produce specimens. The HE-stained specimens of the eyeball were observed histopathologically under an optical microscope. Regarding structural changes in the retina, each part was scored according to the following criteria, and the sum was calculated. Rod outer segment: score of 1 for cell infiltration; score of 2 for partial loss; score of 3 for moderate loss; and score of 4 for nearly complete loss. Nerve cell layer: score of 1 for cell infiltration; score of 2 for partial loss; score of 3 for moderate loss; and score of 4 for nearly complete loss; and score of 5 for total loss. Retinal structure: score of 1 for less than 10% retinal fold; score of 2 for 10% to 50% retinal fold; and score of 3 for retinal fold more extensive than 50%.

As a result of funduscopic examination, Regnase-1 AA mutant mice (AA) showed an effect of reducing inflammation in the eyes (Fig. 36A). As a result of histopathological analysis, Regnase-1 AA mutant mice (AA) showed a reduction in retinal structural disorder score (Fig. 36B). Therefore, Regnase-1 was suggested to be possibly involved not only in immunization but also in the development of pathological conditions after sensitization is established.

### Example 8.

In this example, Regnase-1 was shown to be phosphorylated by TBK1 and IKKi in the IL-17 receptor signaling pathway.

Next, the inventors investigated how Regnase-1 is post-translationally modified by the IL-17A signaling component. Immunoblotting analysis of Regnase-1 in wild-type and Regnase-1AA/AA MEFs revealed that IL-17A treatment induces Regnase-1 phosphorylation, which was displayed in the form of electrophoretic mobility shift of Regnase-1 (Fig. 12-1A). In wild-type MEF, there was no dramatic loss of Regnase-1 upon IL-17A stimulation (Fig. 12-1A), but Regnase-1 phosphorylation patterns were similar to that seen accompanying IRAK1 and IRAK2-mediated phosphorylation upon LPS or IL-1 stimulation (Nat Immunol 12, 1167-1175 (2011)). IL-17A stimulation results in weak activation of NF-κB (Fig. 12-3I), and this showed that weak IKK activation in the IL-17A signaling pathway results in weak degradation of Regnase-1 upon IL-17A stimulation. In fact, the phosphorylated form of Regnase-1 AA mutant protein gradually accumulated during IL-17A stimulation in Regnase-1AA/AA MEF.

To identify the kinase responsible for Regnase-1 phosphorylation in the IL-17A signaling pathway, the inventors investigated a series of MEFs lacking TBK1, IKKi, TBK1/IKKi, Act1, IKKα/IKKβ, or IRAK1/IRAK2. The inventors discovered that Regnase-1 phosphorylation does not occur in MEFs that lack both TBK1 and IKKi, or Act1, an adapter protein essential for the IL-17 signaling pathway (Fig. 12-1B). MEF lacking either TBK1 or IKKi showed phosphorylation of Regnase-1 in response to IL-17A, indicating that TBK1 and IKKi have Regnase-1 kinase activity independent of each other (Fig. 12-1B). Treatment of MEF with BX795, an inhibitor of both TBK1 and IKKi, inhibited Regnase-1 phosphorylation upon IL-17A stimulation (Fig. 12-1C). To investigate whether TBK1 and IKKi directly phosphorylate Regnase-1, the present inventors prepared recombinant Regnase-1 purified from a MEF cell line stably expressing FLAG-tagged Regnase-1. Phosphorylation of recombinant Regnase-1 by recombinant TBK1 and/or IKKi under *in vitro* conditions was detected as an electrophoretic mobility shift pattern by immunoblotting and ³²P autoradiography (Fig. 12-2D). These results confirmed that both TBK1 and IKKi cause Regnase-1 phosphorylation in response to IL-17A. The phosphorylation pattern of Regnase-1 by IRAK appeared to be similar to that by TBK1 and IKKi, but Regnase-1 phosphorylation occurred in IRAK1/IRAK2 double-deficient MEF (Fig. 12-2E). In contrast, Regnase-1 phosphorylation in response to IL-1β occurred in TBK1/IKKi double-deficient MEF (Fig. 12-3J). These results suggest that TBK1 and IKKi phosphorylate Regnase-1 in an IRAK-independent manner, and IRAK phosphorylates Regnase-1 in a TBK1-and IKKi-independent manner.

### Example 9.

This example showed that by interacting with Regnase-1, Act1 contributes to TBK1/IKKi-mediated Regnase-1 phosphorylation by TBK1/IKKi.

The present inventors investigated whether Regnase-1 interacts with Act1, TBK1, and IKKi. Co-immunoprecipitation of full-length or N-terminal or C-terminal truncated Regnase-1 and Act1 revealed that Regnase-1 binds to Act1 *via* its C-terminal domain (Fig. 12-2F). Next, the present inventors co-expressed Regnase-1 with Act1, TBK1, or IKKi in HEK293 cells. Regnase-1 phosphorylation band shift was seen when co-expressed with Act1, but not when co-expressed with TBK1 or IKKi, but when co-expressed with Act-1 and TBK1 or with Act-1 and IKKi, a stronger band of phosphorylated Regnase-1 was detected (Fig. 12-2G). In contrast, the amount of phosphorylated Regnase-1 decreased when co-expressed with Act-1 mutant lacking the C-terminal part containing the SEFIR domain (Act1 ΔSEFIR) and TBK1 or with Act1 ΔSEFIR and IKKi (Fig. 12-2H). This suggests that Regnase-1 phosphorylation by TBK1 or IKKi requires an interaction between the C-terminal domain of Regnase-1 and Act1. Co-immunoprecipitation analysis showed that Regnase-1 interacts with TBK1, IKKi, and Act-1 SEFIR domains, and that Act1 interacts with TBK1, IKKi, and Regnase-1 (Figs. 12-2G and 12-2H). These results indicate that binding to Regnase-1 *via* the C-terminal domain of Act1 leads to improved accessibility of Regnase-1 to TBK1 and IKKi and simultaneous phosphorylation of Regnase-1 and Act1 by TBK1 or IKKi.

### Example 10.

In this example, phosphorylation of residues in the proline-rich region was shown to dissociate oligomerized Regnase-1.

The present inventors tried to identify Regnase-1 amino acid residues phosphorylated by TBK1 and IKKi. Phosphorylated Regnase-1 (derived from mouse) was prepared by coexpression of Act1 with TBK1 or IKKi. Purified phosphorylated Regnase-1 was digested with protease and analyzed using high-resolution liquid chromatography-mass spectrometry (LC-MS). Five Regnase-1 residues (Ser439, Ser494, Thr505, Ser508, and Ser513) were identified as important phosphorylation sites (Fig. 13-1A, Table 5). One of the five residues (Ser439) corresponded to the phosphorylation target of IKK. The remaining four residues (Ser494, Thr505, Ser508, and Ser513) were located within the proline-rich region of Regnase-1 and did not contain any consensus sequence for phosphorylation. The present inventors investigated whether these residues contribute to the phosphorylation of Regnase-1 in the IL-17 receptor (IL-17R) signaling pathway. Substitution of Ser494 and Ser513 with alanine resulted in loss of phosphorylated Regnase-1 when stimulated by IL-1β or IL-17A in HeLa cells (Fig. 13-1B), indicating that these residues were the central residues that caused the electrophoretic mobility change in phosphorylated Regnase-1, and that they are the phosphorylation sites common to IRAK and TBK1/IKKi.

**[Table 5]**

| Co-expression vectors in HEK293 cells | Regnase-1 alone (control) | Regnase-1 + ACT-1 + TBK1 | Regnase-1 + ACT-1 + IKKi |
|---|---|---|---|
| Phosphorylation sites identified only for the control | Ser18, Ser68, Thr195, Ser299, Ser408, Thr522, Thr555 | | |
| Commonly identified phosphorylation sites | Ser103, Ser114, Ser344, Thr348, Ser354, Ser359, Ser368, Ser374, Ser394, Ser454, Ser482, | Ser103, Ser114, Ser344, Thr348, Ser359, Ser368, Ser374, Ser394, Ser454, Ser482 | Ser103, Ser114, Ser344, Thr348, Ser368, Ser394, Ser454 |
| Phosphorylation sites identified for both TBK1 and IKKi | | Ser28, Ser124, Thr115, Ser288, Ser494, Ser508, Ser513, | |
| Phosphorylation sites identified only for TBK1 | | Thr109, Ser404, Ser454, Ser470, Ser482, Thr498, Thr505, Ser592, | |
| Phosphorylation sites identified only for IKKi | | | Ser21, Ser268, Ser386, Ser439, Ser474 |

Next, the present inventors investigated the role of Regnase-1 phosphorylation in this proline-rich region. A region rich in proline has been reported to be involved in the oligomerization of Regnase-1, and the Regnase-1 truncation mutant lacking this region has lost its RNase activity (Mol Cell 44, 424-436 (2011); and Nucleic Acids Res 41, 3314-3326 (2013)). These findings gave rise to the possibility that phosphorylation of proline-rich segments regulate the self-assembly of Regnase-1. The present inventors produced and purified a GST-tagged Regnase-1 fragment containing a proline-rich region and a C-terminal domain. The present inventors also constructed mutant fragments in which Ser and Thr residues in the proline-rich region were substituted with glutamic acid to mimic phosphorylation. The oligomerization of Regnase-1 fragments (wild-type, S494E/S513E, and S494E/T505E/S508E/S513E) were analyzed using gel filtration chromatography. The wild-type fragment formed some high molecular weight oligomers, but oligomerization was inhibited in the mutant types (Fig. 13-2C). This indicates that introduction of phosphoserine and phosphothreonine residues into the proline-rich region promotes the dissociation of oligomerized Regnase-1. To investigate the molecular assembly of phosphorylated Regnase-1, the present inventors used native PAGE analysis of Regnase-1 purified from MEFs that stably express N-terminal FLAG-tagged Regnase-1. This experiment demonstrated that IL-1β or IL-17A stimulation induces the appearance of Regnase-1 monomer (Fig. 13-3D). Also, *in vitro* phosphorylation of Regnase-1 by recombinant TBK1 and IKKi induced conversion to a monomeric form (Fig. 13-3E). These results support the view that phosphorylation in the proline-rich region of Regnase-1 affects the interactions required for Regnase-1 self-assembly and promotes the conversion from oligomerized forms to monomeric forms.

### Example 11.

In this example, Regnase-1 phosphorylation was shown to change its own intracellular localization from the ER to the cytosol.

Regnase-1 protein is found in the rough ER membrane fraction (Cell 161, 1058-1073 (2015)). The present inventors isolated intracellular compartments such as ER membranes, microsomes, and soluble cytoplasmic fractions from cell homogenates of MEF stimulated with IL-1β or IL-17A, and analyzed their protein distribution using Western blotting. All phosphorylated Regnase-1 proteins were present in the cytoplasm (Fig. 14-1A), while non-phosphorylated Regnase-1 remained localized to ribosome-containing organelles (Fig. 14-1A). This cytosolic distribution of Regnase-1 protein is not observed in TNF-α-stimulated MEF or IL-17A-stimulated Act-1-deficient MEF where Regnase-1 is not phosphorylated (Fig. 14-1B), and accordingly, Regnase-1 phosphorylation and subsequent dissociation of Regnase-1 oligomers were shown to cause the transfer of Regnase-1 from the ER to the cytoplasm. Interestingly, after IL-17A stimulation, the amount of non-phosphorylated Regnase-1 in the ER fraction increased over time in Regnase-1AA/AA MEF than in wild-type cells (Fig. 14-1C). This finding suggests that non-phosphorylated Regnase-1 protein attached to the ER upon IL-17A stimulation mediates regulation of mRNA levels targeting Regnase-1.

The change in the intracellular distribution of phosphorylated Regnase-1 also increases the possibility that Regnase-1 binds to Act1 and TBK1/IKKi in the ER. The present inventors investigated whether the interaction among Regnase-1, Act1, and TBK1/IKKi occurs in the ER by co-immunoprecipitation of intracellular fractions isolated from IL-17A stimulated or unstimulated cells. Regnase-1 that interacted with phosphorylated TBK1 and phosphorylated IKKi was found in the microsomes but not in the soluble cytoplasmic fraction (Fig. 14-2D). Western blot analysis of intracellular fractions isolated from wild-type MEF and Act1-deficient MEF revealed that Act1 is responsible for the phosphorylation of both TBK1 and IKKi in the ER in response to IL-17A and that Act1 promotes kinase-mediated phosphorylation of Regnase-1 (Fig. 14-2E). These results suggest that Act1 acts as a signaling factor for promoting phosphorylation of TBK1, IKKi, and Regnase-1 on the ER membrane after IL-17A stimulation.

### Example 12.

In this example, IL-17-mediated Regnase-1 phosphorylation was shown to result in loss of RNase activity.

Next, the present inventors investigated whether dissociation of phosphorylated Regnase-1 from ER affects IL-6 mRNA level upon cell stimulation. While different from the case of IL-1β and TNF-α stimulation, although IL-17A induces Regnase-1 phosphorylation, this cytokine does not induce IL-6 mRNA sufficiently due to its weak NF-κB activation. TNF-α induces IL-6 mRNA expression with strong NF-κB activation, but cannot induce Regnase-1 phosphorylation. The present inventors measured the level of IL-6 mRNA in MEF which was pretreated with TNF and then stimulated with IL-17A alone. In wild-type MEF, IL-6A mRNA induction was strongly enhanced upon IL-17A stimulation, and this caused Regnase-1 phosphorylation (Fig. 15-1A). In Regnase-1 AA mutant cells and TBK1/IKKi double-deficient cells, IL-6 mRNA induction was suppressed upon IL-17A stimulation, and in particular, it was significantly inhibited in double-deficient MEFs (Fig. 15-1B). TBK1/IKKi double-deficient cells did not show Regnase-1 phosphorylation and maintained subcellular localization in ribosome-containing organelles in response to IL-17A (data not shown).

The present inventors evaluated the influence of Regnase-1 phosphorylation on the ability to degrade mRNA using a Tet-off induction system. Regnase-1 was readily phosphorylated when co-expressed with Act1 and IKKi. IL-6 mRNA and 3'UTR were overexpressed in Tet-off HEK293 cells in the presence of either Regnase-1 or phosphorylated Regnase-1, and then the IL-6 mRNA level was assessed by Northern blotting. IL-6 mRNA degradation by Regnase-1 was blocked by co-expression with Act1 and IKKi (Fig. 15-2C). This strongly indicates that phosphorylated Regnase-1 lacks the ability to degrade its target mRNA.

Next, the present inventors investigated the mechanism of target mRNA suppression in Regnase-1AA/AA cells. As described above, suppression of target mRNA is more enhanced at later stages of IL-17A stimulation. The present inventors used immunoblot analysis of phosphorylated and non-phosphorylated Regnase-1 during the recovery phase after the completion of IL-17A stimulation. Non-phosphorylated Regnase-1 protein gradually appeared during the recovery phase in wild-type MEF, and this was observed in the presence of protein synthesis inhibitors (Fig. 15-2D). The increase in the amount of non-phosphorylated Regnase-1 protein was enhanced much more strongly in Regnase-1AA/AA MEF than in wild type cells. In contrast, non-phosphorylated Regnase-1 did not appear in the presence of okadaic acid, an inhibitor of protein phosphatase-1 and 2A (Fig. 15-2D), indicating that the appearance of non-phosphorylated Regnase-1 is mediated by a phosphatase. In addition, during the recovery phase after treatment of Regnase-1AA/AA MEF with TNF-α and IL-17A, IL-6 mRNA stability was also enhanced in the presence of okadaic acid (Fig. 15-2E). These results suggest that the suppression of Regnase-1 target mRNA observed in Regnase-1AA/AA cells upon IL-17A stimulation requires conversion from phosphorylated Regnase-1 to a non-phosphorylated form.

### Example 13.

In this example, Regnase-1 C-terminal truncation mutation (Regnase-1ΔCTD) and Regnase-1 S513A mutation (Regnase-1 S513A) was shown to inhibit IL-17-mediated phosphorylation and abolish *in vitro* and *in vivo* IL-17 mediated inflammatory responses.

Phosphorylated Regnase-1 is released from the ER by conversion from a constitutively active oligomer to an inactive monomer. This finding suggests the possibility that Regnase-1 mutant may continue to maintain RNase function when it is not phosphorylated. To investigate this, the present inventors searched for Regnase-1 mutants that are resistant to IL-17-mediated phosphorylation by expressing various Regnase-1 mutants that are stable in MEF. The present inventors have found that Regnase-1 mutant lacking the C-terminal domain essential for interaction with Act-1 is not phosphorylated by IL-17A stimulation (Fig. 16-1A). Next, the present inventors tried producing genetically mutated mice expressing C-terminal truncated Regnase-1 by introducing a frameshift and immature stop codon into the proline-rich domain of Regnase-1 (Fig. 17-1A). The inventors succeeded in obtaining mutant mice with a 1bp deletion at codon 517, which led to a frameshift mutation (referred to as Regnase-1ΔCTD, Fig. 17-1B). To examine the effect of this mutation on protein expression levels and any post-translational modifications, MEFs from Regnase-1ΔCTD/ΔCTD mutant mice were stimulated with TNF-α, IL-17A, and IL-1β. These stimuli induced an NF-κB-dependent increase in Regnase-1ΔCTD protein, and no mobility-shifted band indicating phosphorylation was observed (Fig. 16-1B). Myc-Act-1, HA-TBK-1, and HA-IKKi were co-expressed with FLAG-tagged Regnase-1 ΔCTD in HEK293 cells and co-immunoprecipitated to investigate the possibility of Regnase-1ΔCTD protein phosphorylation upon IL-17A stimulation. Regnase-1ΔCTD protein showed significantly reduced phosphorylation compared to the wild type (Fig. 16-1C) and did not co-immunoprecipitate with Act-1 (Fig. 16-1D). These results demonstrate that Regnase-1ΔCTD lacks the binding to Act-1 essential for Regnase-1 phosphorylation and that it remains non-phosphorylated upon IL-17A stimulation.

The present inventors produced mutant mice in which Ser513 has been substituted with alanine (Figs. 17-1C and 17-1D). To examine the effect of this mutation on protein expression levels and any post-translational modifications, MEFs derived from Regnase-1 S513A mutant mice were stimulated with TNF-α, IL-17A, and IL-1β. These stimuli induced an NF-κB-dependent increase in Regnase-1 S513A protein, and no mobility-shifted band indicating phosphorylation was observed (Fig. 16-4L). Next, the protein stability of Regnase-1 mutant proteins was examined. Regnase-1 AA mutant mouse-derived MEF and Regnase-1 S513A mutant mouse-derived MEF were stimulated with TNF-α, IL-1β, LPS, and IL-17A in the presence of the transcription inhibitor cycloheximide. As a result, after IL-1β, LPS, and IL-17A stimulation, Regnase-1 protein level decreased significantly in WT MEFs, whereas Regnase-1 protein levels were not decreased in MEFs from Regnase-1 AA mutant mice and MEFs from Regnase-1 S513A mutant mice (Figs. 16-4M and 16-5N). Therefore, the Regnase-1 S513A mutation was resistant to phosphorylation and degradation induced after IL-17A stimulation, resulting in enhanced stability of the Regnase-1 protein.

Next, the inventors investigated the binding pattern of Regnase-1ΔCTD protein in ribosome-containing organelles. The present inventors isolated cytoplasmic and microsomal fractions from cell homogenates of Regnase-1ΔCTD/ΔCTD MEF stimulated with IL-17A, and analyzed the protein distribution of Regnase-1, ribosomal protein L7a, GAPDH, and phospho-TBK1 by Western blot analysis. Wild-type Regnase-1 was phosphorylated by IL-17A and was no longer localized in the microsome with its translocation to the cytoplasm, but the Regnase-1ΔCTD mutant continues to bind to the microsome after IL-17A stimulation. This was accompanied by an increase in Regnase-1ΔCTD protein in microsomes (Fig. 16-2E). Reganase-1 also binds to ribosomes with translation activity which are assembled on polysomes, and promotes degradation of mRNA in ribosomes for translation (Cell 161, 1058-1073 (2015)). The present inventors confirmed binding of Regnase-1 to polysomes having translation activity in wild-type and Regnase-1ΔCTD/ΔCTD MEFs stimulated with TNF-α and IL-17A (Fig. 16-2F). In wild-type MEFs, the localization of Regnase-1 to polysomes was reduced by cytokine stimulation, but the localization of Regnase-1 in polysomes having translation activity was greater in mutant MEFs stimulated with these cytokines than in those that were not stimulated (Fig. 16-2G). The opposite pattern of protein localization between wild-type and mutant Regnase-1 implies a role for Regnase-1ΔCTD mutation in reducing target mRNA expression even during inflammatory stimulation. To verify this, the present inventors examined mRNA induction of Regnase-1 targeting genes in wild-type MEF, Regnase-1ΔCTD/ΔCTD MEF, and Regnase-1 S513A MEF, stimulated with pro-inflammatory cytokines. While stimulation with TNF-α+IL-17A induced a time-dependent increase in Regnase-1 target mRNAs such as IL-6, LCN-2, and GM-CSF in wild-type MEF, such were not found in mutant MEFs (Fig. 16-3H and Fig. 17-4E). In mutant MEFs, sequential stimulation with TNF-α and IL-17A induced reduced mRNA production of IL-17-related genes such as IL-6, CXCL-1, CXCL-2, CCL-20, LCN-2, and GM-CSF (Figs. 17-2C and 17-4F). Furthermore, the protein production level of IL-6, CXCL-1, and CXCL-2 after stimulation with IL-17A and either IL-6 or TNF-α for 24 hours was significantly lower in the MEF of Regnase-1ΔCTD/ΔCTD than in cells derived from the wild type (Fig. 17-3D). These indicate that Regnase-1ΔCTD protein maintains its subcellular localization in ribosome-containing organelles following IL-17A stimulation, and that it acts as a negative regulator inhibiting the enhancement of IL-17A-mediated mRNA stability and the subsequent inflammatory cytokine production.

To verify whether impaired IL-17-mediated release of Regnase-1 from the ER is important for suppression of TH17 cell-mediated autoimmune disorders, the present inventors induced EAE in Regnase-1ΔCTD/ΔCTD mice. In these mice, EAE severity was strongly attenuated than in wild-type mice (Fig. 16-3I). Flow cytometric analysis of mice 28 days after immunization showed a significant decrease in infiltration of CD4+ T cells and macrophages into the neuronal tissue of Regnase-1ΔCTD/ΔCTD mice (Figs. 16-3J and K). On the other hand, the numbers of TH1 and TH17 cells in spleen and lymph node cells were similar between wild-type and mutant mice, except for the number of spleen-derived TH1 cells, which increased in mutant mice than in wild-type mice (Fig. 18). These results suggest that the Regnase-1ΔCTD mutation also causes suppression of EAE pathogenesis through sustained inhibition of STAT3 activation in endothelial cells, and interrupts TH17 cell-mediated inflammation required for TH17 cells to infiltrate neural organs.

### Example 14. Search for Regnase-1 target gene by luciferase assay

### (Luciferase assay)

pGL3-target gene 3'UTR plasmid or control pGL3-empty plasmid expressing firefly-luciferase, wild-type Regnase-1 expression plasmid, mutant Regnase-1 (D141N) expression plasmid or control empty plasmid were transfected into HEK293. At the same time, the renilla-luciferase expression plasmid was transfected as an internal control. After culturing for 24 hours, the luciferase activity in the cell lysate was measured using a Dual-luciferase reporter assay system (Promega Corporation).

### (Expression plasmid)

Wild-type mouse Regnase-1 and mutant mouse Regnase-1 (D141N) in which the 141st amino acid has been replaced from D to N were inserted into pCXND3 plasmid (Chugai Pharmaceutical Co., Ltd.) to prepare wild-type Regnase-1 expression plasmid and mutant Regnase-1 (D141N) expression plasmid. A FLAG tag was inserted into the pCXND3 plasmid to prepare an empty plasmid for control.

The pGL3-target gene 3'UTR plasmid was prepared by inserting the 3'UTR sequence of the target gene mRNA into the XbaI cleavage site of the pGL3 plasmid (Promega Inc.). Target genes were selected by RNA immunoprecipitation assay using RAW264.7 and immortalized mouse keratinocytes.

As a result, in cells transfected with pGL3 plasmid inserted with Fabp5, Ackr3, Ctgf, Adamts1, Atf2, Cd80, Cyr61, Ddr1, Duox1, Dusp6, Csf3, Hbegf, Id3, 1119, Map3k8, Il1a, mcoln3, Mitf, Orc1, Pdgfb, Ptgs1, Sesn1, Ptger4, Shq1, Sulf1, Tnfrsf9, zc3h12c, Rarb, and Tmem9 3'UTR, luciferase activity was markedly reduced by transfection of Regnase-1 compared to when empty plasmid was transfected, and dose dependence was observed (Figs. 19-1 to 19-5). On the other hand, when mutant Regnase-1 (D141N) expression plasmid was transfected, there was no great change in luciferase activity. These indicated that the above-mentioned genes may be novel target genes controlled by Regnase-1.

### Example 15. Detection of phosphorylation of Regnase-1 by IKKβ or TBK1

### (Preparation of human Regnase-1)

As a target protein, a full-length sequence of human Regnase-1 (UniProt ID: Q5D1E8) (SEQ ID NO: 2) was used, and a construct having a GST tag on the N-terminal side and a biotin ligase BirA recognition sequence on the C-terminal side was prepared. Regnase-1 mentioned above was expressed in mammalian Expi293 cells, purified with glutathione Sepharose, and after cleaving the GST tag by Turbo3C protease (Accelagen), it was isolated by gel filtration chromatography. As necessary, 7 units of lambda phosphatase (SIGMA, P9614) was added to 1 µg of Regnase-1, and after dephosphorylation by incubating at 4°C for two hours, Regnase-1 was isolated by gel filtration chromatography.

### (Phosphorylation reaction)

Using Kinase assay buffer III (manufactured by SignalChem) supplemented with 2 mM DTT, 100 µM sodium vanadate, and 2 mM manganese chloride, a phosphorylation reaction was carried out by reacting biotinylated human Regnase-1 prepared above with a kinase (IKKβ (manufactured by SignalChem) or TBK1 (manufactured by SignalChem)) in the presence of 20 µM ATP at room temperature for one hour.

### (Detection of phosphorylated human Regnase-1)

Phosphorylated human Regnase-1 was detected by Western blotting using the above-discussed antibody. Western blotting was performed by transferring the protein in the SDS-PAGE gel to a PVDF membrane (manufactured by Bio-Rad). The anti-Regnase-1 antibody or anti-phosphorylated Regnase-1 antibody was used as the primary antibody, and the anti-rabbit IgG horseradish peroxidase linked antibody (manufactured by Cell Signaling Technology) was used as the secondary antibody. The peroxidase reaction was performed by Super signal Westpura Extended Duration Substrate (manufactured by Thermo), and chemiluminescence was detected by ImageQuant LAS 4000 mini (manufactured by GE Healthcare). As a result, the anti-phosphorylated Regnase-1 antibody specifically detected phosphorylated human Regnase-1 produced by the phosphorylation reaction by the kinase, and the anti-Regnase-1 antibody specifically detected human Regnase-1 (Fig. 20).

From the results of Example 15, it was confirmed that human Regnase-1 is phosphorylated by IKKβ and TBK1. Furthermore, the phosphorylation site in human Regnase-1 was considered to be a site corresponding to the phosphorylation site in mouse Regnase-1. By using the methods of these Examples, a substance that inhibits Regnase-1phosphorylation can be identified.

Although not intended to be bound by a specific theory, the present inventors consider the following (1) to (5) based on the results of this study.
(1) Regnase-1 undergoes two-step phosphorylation in response to IL-1 or LPS that activates the MyD88-dependent pathway. The initial phosphorylation is mediated by the IRAK family of protein kinases, followed by IKK-mediated phosphorylation, which results in protease-dependent degradation. Regnase-1 protein levels reduced by IKK-dependent degradation appear to attenuate its function as a "brake" of mRNA expression, and this induces expression of the target mRNA of Regnase-1. However, the present results of the present inventors demonstrated that IKK-independent phosphorylation of Regnase-1 contributes to termination of RNase activity. Regnase-1 exists in an oligomeric form in ribosome-containing organelles, and phosphorylation caused by cell stimulation prevents Regnase-1 self-assembly and releases it from ER and polysomes with translation activity. In the experiments of the present inventors, Regnase-1 phosphorylation was maintained for a longer period than degradation, contributing to the stabilization of Regnase-1 target mRNA in response to stimulation by cytokines or TLR ligands.
(2) The present inventors introduced a Regnase-1 AA mutant in which two serine residues phosphorylated by IKK are mutated to alanine residues. IL-17A stimulation induced rapid phosphorylation of Regnase-1 in both wild-type MEF and Regnase-1 AA mutant MEF. In the case of Regnase-1 AA mutant cells, non-phosphorylated Regnase-1 appeared in the later stage of IL-17A stimulation. This may be responsible for the suppression of a series of mRNAs regulated by Regnase-1 protein and the reduced severity of EAE observed in Regnase-1 AA mutant mice. This accumulation of non-phosphorylated Regnase-1 protein in Regnase-1 AA mutant cells occurred even in the presence of protein synthesis inhibitors, suggesting that phosphorylated Regnase-1 was converted to the non-phosphorylated form. The inventors speculated that the appearance of non-phosphorylated Regnase-1 protein may be due to dephosphorylation by phosphatase. This reasoning was confirmed by treating Regnase-1 AA mutant cells with okadaic acid, a phosphatase inhibitor, which resulted in a dramatic increase in the half-life of IL-6 mRNA. Regnase-1 dephosphorylation results in restoration of mRNA degradation activity through attachment to the ER. Therefore, Regnase-1 may inhibit the expression of its target mRNA in response to weak stimuli through reversible dephosphorylation and rapid relocation to the ER.
(3) The present inventors also produced Regnase-1ΔCTD mutant mice expressing a Regnase-1 protein lacking the C-terminal domain necessary for interaction with Act1. Because TH17 cell-mediated inflammation was attenuated in non-hematopoietic cells, these mice also showed reduced EAE severity compared to the wild type, as did Regnase-1 AA mutant mice. In contrast, previous reports have shown that Regnase-1-deficient heterozygotes are more sensitive to EAE because of increased inflammation of non-hematopoietic cells (Immunity, 2015 Sep 15; 43(3): 475-487), which demonstrates an important role for Regnase-1 in the suppression of inflammation in non-hematopoietic cells during EAE pathogenesis. However, the mechanism of inhibition of EAE pathogenesis in Regnase-1ΔCTD/ΔCTD mice may be different from that in Regnase-1AA/AA mice. Because of its lack of degradation induced by IKK-mediated phosphorylation, Regnase-1 AA mutant protein is more abundant than the wild-type protein, and this has a beneficial effect on EAE. Regnase-1ΔCTD mutant shows lower levels of protein expression at steady state, increased expression when stimulated by several inflammatory cytokines, and promotes the accumulation of non-phosphorylated Regnase-1 in the ER, and accelerated target mRNA degradation. Since Regnase-1 mRNA contains its own binding site in the 3'UTR, low levels of Regnase-1ΔCTD at steady state may arise from self-regulation of its own mRNA. This provides important insights regarding regulatory roles of the phosphorylation of Regnase-1, not only in transmitting signals that lead to proteolysis, but also in reducing the target mRNA degradation activity to avoid intracellular accumulation of non-phosphorylated/active proteins.
(4) IL-17 yields a signal in the cytoplasm through the interaction between an IL-17 receptor (IL-17R) and Act1 which is an adapter protein essential in the IL-17 signaling pathway. The present inventors have identified an association between Regnase-1 in the ER and Act1, TBK1, and IKKi. Regnase-1 associates with Act-1 *via* the C-terminal domain. Act1 binding to Regnase-1 strongly promotes Regnase-1 phosphorylation by TBK1 and IKKi, which enhances the transfer of Regnase-1 from the ER to the cytoplasm and blocks its mRNA degradation ability. These findings suggest that Regnase-1 phosphorylation is directly correlated with Act1 activation following IL-17R association and that Regnase-1 is involved in the regulation of IL-17-induced mRNA expression. In fact, a group of genes that are up-regulated in response to IL-17, such as IL-6, IL-8, CXCL1, and CXCL2, correspond to Regnase-1 target mRNA.
(5) Regnase-1 is an RNA-binding RNase that destabilizes a specific mRNA in a steady state and is rapidly inactivated when stimulated with IL-17, and stabilizes a specific mRNA upon response to IL-17. In the studies shown herein by the present inventors, IL-17-induced phosphorylation of Regnase-1 was severely impaired in Regnase-1ΔCTD mutants. Co-stimulation with TNF-α and IL-17 dramatically reduced production of IL-6, CXCL1, CXCL2, CCL-20, Lipocalin-2, and GM-CSF in Regnase-1ΔCTD mutant cells to the same extent as TBK1/IKKi double knockout cells. These results strongly suggest that the interaction among Regnase-1, Act1, and TBK1/IKKi affects the expression of these inflammatory genes induced by IL-17 stimulation, and that Regnase-1 plays a central role in regulating the inflammatory response to IL-17. Blocking Regnase-1 phosphorylation may provide a new strategy for the treatment of TH17 cell-related diseases.

### Example 16. Effect of phosphorylation of Regnase-1 at position 513 on IL-6 production

To confirm that IL-6 mRNA level is regulated by Regnase-1 phosphorylation and its dissociation from ER upon stimulation, a mouse Regnase-1 mutant resistant to phosphorylation by TBK1/IKKi was prepared. Two MEF cell lines expressing Regnase-1 mutants were established: one contained substitution of Ser513 with Ala (Regnase-1 S513A) and the other lacked the C-terminal domain (Regnase-1ΔCTD). Neither mutant showed Regnase-1 phosphorylation and maintained intracellular localization in the ER in response to IL-1β or IL-17A (Fig. 21A). IL-6 mRNA production upon co-stimulation with TNF-α and IL-17A was significantly reduced in these mutant MEFs than in the wild type cells (Fig. 21B). This indicates that IL-6 production is strongly suppressed by inhibiting phosphorylation at position 513 of Regnase-1.

### Example 17. Effect of alleviating pathological condition of Regnase-1 S513A mutant mice in imiquimod-induced psoriasis model

To the right pinna and the shaved dorsocervical skin of C57BL/6 mice (wild-type mice; WT) or Regnase-1 S513A mutant mice (S513A), 62.5 mg of imiquimod cream (Beselna cream 5%; Mochida Pharmaceutical Co., Ltd.) was applied every other day on Days 0, 2, and 4. Evaluation of the pinna lesion was performed by measuring the thickness of the right pinna using a digital thickness gauge (OZAKI MFG. Co., Ltd.) over time from before applying imiquimod. In addition, the dorsocervical skin was evaluated by macroscopically evaluating the extent of erythema, thickening, and scaling individually in four stages, and the combined total score (12 points) was presented.

As a result, a marked thickening of the pinna was observed in the wild-type mouse, accompanying application of imiquimod (Fig. 22A). In addition, psoriasis-like skin lesions such as erythema, thickening, and scaling were induced in the dorsocervical skin to which imiquimod was applied (Fig. 22B). On the other hand, in Regnase-1 S513A mutant mice, thickening of the pinna and psoriasis-like skin lesions (erythema, thickening, and scaling) in the back of the neck were reduced compared to wild-type mice (Figs. 22A and 22B).

### S513A mouse evaluation (quantification of various gene expressions)

Mice were euthanized and the pinnas which were the application site were collected. RNA was extracted according to a conventional method using QIAZOL Lysis Reagent (QIAGEN, No. 79306) and RNeasy 96 kit (QIAGEN, No. 74182). After obtaining complementary DNA by reverse transcription, expression of the following target genes was measured by Taqman PCR method. Taqman PCR was analyzed with LightCycler 480 II (manufactured by Roche) using QuantiTect Probe RT-PCR Kit (QIAGEN, No. 204445). The Taqman probes used are shown below.
B2m (manufactured by Applied biosystems, Mm00437762_m1): endogenous control
116 (manufactured by Applied biosystems, Mm00446190_m1): inflammatory cytokine
Il1a (manufactured by Applied biosystems, Mm00439620_m1): inflammatory cytokine
Cxcl2 (manufactured by Applied biosystems, Mm00436450_m1): leukocyte migration factor
Hbegf (manufactured by Applied biosystems, Mm00439306_m1): cell growth factor
Sprr2i (manufactured by Applied biosystems, Mm00726832_s1): keratinocyte marker
Keratin 6A (manufactured by Applied biosystems, Mm00833464_g1): keratinocyte marker

As a result, since the induction of keratinocyte markers, the Sprr2i gene and the Keratin6A gene, associated with pathogenesis was suppressed in S513A mice, increase in keratinocytes which is characteristic of psoriasis model pathology was found to be suppressed.

In wild-type mice, the genes targeted by Regnase-1 (116, Il1a, Cxcl2, and Hbegf) increased with the initiation of pathological conditions (Fig. 37). On the other hand, in the S513A mutant mouse, increase of these genes induced by the initiation of pathological conditions was suppressed. Regnase-1 AA mutant mice were suggested to have inhibitory effects on inflammatory cytokine production and epidermal cell proliferation activity associated with the pathological condition.

### Example 18. Effect of alleviating pathological condition of Regnase-1 S513A mutant mice in experimental autoimmune encephalomyelitis model

### (EAE model)

Normal EAE was induced by immunization using myelin oligodendrocyte glycoprotein (MOG) (35-55) peptide (AnaSpec). An emulsion of MOG (35-55) peptide (300 ng/mouse) and complete Freund's adjuvant (CFA, InvivoGen) were mixed at a ratio of 1:1 and injected subcutaneously into mice. Following intraperitoneal injection of pertussis toxin (100 ng/mouse) on Day 0 and Day 2, the mice were monitored every two days, and they were evaluated by clinical scoring during 7 to 28 days after immunization. Clinical scores were determined using a previously defined scale (Immunity 14, 471-481 (2001)).

### (Statistical analysis)

Unless otherwise stated, statistical analysis of differences between the two groups was performed by Student's t-test (two-sided). P < 0.05 was considered statistically significant.

In Regnase-1 S513A mutant mice, increase in clinical score associated with the pathological condition was abated, and the pathological condition was alleviated compared to the wild-type mice (Fig. 38).

### Example 19. In vitro selection of target-binding polypeptides (panning)

### 19-1 Randomized double-stranded DNA library encoding a polypeptide library

A DNA library was constructed according to a method described in a patent document (WO2013/100132). The library was prepared so that a triplet of random regions appears in repeats of nine or ten times.

### 19-2 Cyclic polypeptide library

Using a cell-free translation solution and the mRNA-puromycin linker ligation product prepared from the aforementioned double-stranded DNA library, the cyclic polypeptide library was translationally synthesized according to the method described in WO2013/100132. Eighteen natural amino acids except methionine and cysteine were randomly assigned to the random region of the cyclic polypeptide constituting the library.

### 19-3 Performing panning

Using the above-mentioned cyclic polypeptide library, panning was performed according to the method described in WO2013/100132. Biotinylated human Regnase-1 was used as a target molecule for panning. The sequences enriched by repeating multiple rounds of panning were identified as the sequences of the cyclic polypeptide that binds to the target molecule Regnase-1. Among them, PP1 to PP6 were synthesized by the method shown in the examples described later. Furthermore, PP7 to PP25 were synthesized similarly.

### Example 20. Synthesis of cyclic polypeptides

The following abbreviations were used in the examples.
AA: Ammonium acetate
DBU: 1,8-diazabicyclo[5.4.0]-7-undecene
DCM: dichloromethane
DCE: 1,2-dichloroethane
DMF: N,N-dimethylformamide
DMSO: dimethyl sulfoxide
DIC: N,N'-diisopropylcarbodiimide
DIPEA: N,N-diisopropylethylamine
FA: formic acid
MTBE: methyl-tert-butyl ether
NMP: N-methyl-2-pyrrolidone
TFA: trifluoroacetic acid
TFE: 2,2,2-trifluoroethanol
TIPS: triisopropylsilane
HOAt: 1-hydroxy-7-azabenzotriazole
HATU: O-(7-aza-1H-benzotriazol-1-yl)-N,N,N',N'-tetramethyluronium hexafluorophosphate

Peptide synthesis solvents (purchased from Watanabe Chemical Industries and Wako Pure Chemical) were used as the reaction solvents for peptide synthesis and solid phase synthesis. Examples include, DCM, DMF, NMP, 2% DBU in DMF, and TFA. In addition, for reactions without addition of water as solvent, dehydrated solvent, super-dehydrated solvent, and anhydrous solvent (purchased from Kanto Chemical, Wako Pure Chemical, etc.) were used.

LC/MS analysis conditions are as shown in Table 6.

**[Table 6]**

| Analysis conditions | Apparatus | Column (I.D.xlength)(mm) | Mobile phase | Gradient (A/B) | Flow rate (mL/min) | Column temp.(°C) | Wavelength |
|---|---|---|---|---|---|---|---|
| SQDAA05 | Acquity UPLC/SQD | Ascentis Express C18 (2.1x50) | A)10mM AcONH₄,H₂O B)MeOH | 95/5=> 0/100(1.0 min) 0/100(0.4 min) | 1 | 35 | 210-400nm PDA total |
| SQDAA50 | Acquity UPLC/SQD | Ascentis Express C18 (2.1x50) | A)10mM AcONH₄,H₂O B)MeOH | 50/50=> 0/100(0.7 min) 01100(0.7 min) | 1 | 35 | 210-400nm PDA total |
| SQDFA05 | Acquity UPLC/SQD | Ascentis Express C18 (2.1x50) | A)0.1% FA,H₂O) B)0.1% FA.MeCN | 95/5=> 0/100(1.0 min) 0/100(0.4 min) | 1 | 35 | 210-400nm PDA total |
| SQDFA50 | Acquity UPLC/SQD | Ascentis Express C18 (2.1x50) | A)0.1% FA,H₂O B)0.1% FA,MeCN | 50/50=> 0/100(0.7 min) 0/100(0.7min) | 1 | 35 | 210-400nm PDA total |

### Chemical synthesis of peptide compounds

Peptides were elongated by the following basic route according to a peptide synthesis method based on the Fmoc method described in WO2013/100132. Specifically, a five-step process was performed: 1) peptide elongation reaction by Fmoc method from the N-terminus of Asp which has its side chain carboxylic acid supported on a 2-chlorotrityl resin; 2) peptide cleavage from the 2-chlorotrityl resin; 3) amide cyclization by condensation of the Asp side-chain carboxylic acid generated from the 2-chlorotrityl resin by the cleavage and the amino group of the peptide chain N-terminus (triangle unit); 4) deprotection of the protecting group of the side chain functional group included in the peptide chain; and 5) purification of the compound by preparative HPLC. In this example, unless stated otherwise, peptide compounds were synthesized based on this basic route (Fig. 23).

### Fmoc-amino acids used for peptide synthesis by a peptide synthesizer

In the peptide synthesis described herein, the following Fmoc-amino acids were used for synthesis on a peptide synthesizer. Fmoc-Ala-OH, Fmoc-Arg (Pbf)-OH, Fmoc-Asn(Trt)-OH, Fmoc-Asp(OPis)-OH, Fmoc-D-MeAla-OH, Fmoc-Gln(Trt)-OH, Fmoc-Gly-OH, Fmoc-Glu(OtBu)-OH, Fmoc-His(Trt)-OH, Fmoc-Ile-OH, Fmoc-Leu-OH, Fmoc-Lys(Boc)-OH, Fmoc-MeAla-OH, Fmoc-Pro-OH, Fmoc-Phe-OH, Fmoc-Trp-OH, Fmoc-Tyr(tBu)-OH, Fmoc-Val-OH, Fmoc-β-MeAla-OH (also referred to as Fmoc-bMeAla-OH in some cases), Fmoc-Asp(OMpe)-OH, Fmoc-MePhe-OH, and such were purchased from Tokyo Chemical Industry Co., Watanabe Chemical Industries, Chempep, Inc., Chem-Impex, Inc., or Amatek Scientific Co.

Fmoc-Ser(THP)-OH and Fmoc-Thr(THP)-OH were synthesized by the following method.

### Synthesis of (2S)-2-((((9H-fluoren-9-yl)methoxy)carbonyl)amino)-3-((tetrahydro-2H-pyran-2-yl)oxy)propanoic acid (Compound 1, Fmoc-Ser(THP)-OH)

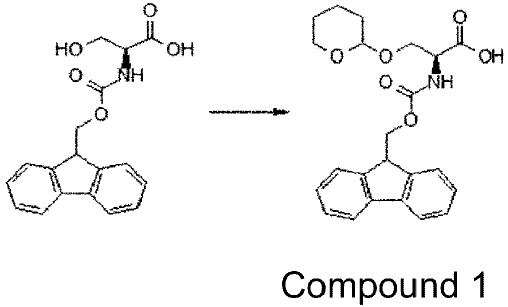

Toluene (10 mL) was added to a mixture of (S)-2-((((9H-fluoren-9-yl)methoxy)carbonyl)amino)-3-hydroxypropanoic acid (Fmoc-Ser-OH, purchased from Watanabe Chemical Industries, 1.0 g, 3.06 mmol) and pyridinium p-toluenesulfonic acid (PPTS, 0.038 g, 0.153 mmol), and the contained water was azeotropically removed with toluene under reduced pressure. Super-dehydrated tetrahydrofuran (THF, 6.1 mL) and 3,4-dihydro-2H-pyran (1.9 mL, 21.3 mmol) were added to the resulting residue, and the mixture was stirred under nitrogen atmosphere at 50°C for four hours. After confirming disappearance of the starting materials by LCMS (SQDFA05), the mixture was cooled to 25°C, and ethyl acetate (6 mL) was added. Subsequently, a saturated aqueous sodium chloride solution (6 mL) was added to wash the organic layer, and the aqueous layer was extracted with ethyl acetate (6 mL). All the organic layers obtained were mixed, and this was further washed twice with a saturated aqueous sodium chloride solution (6 mL). The organic layer was dried over sodium sulfate, and the solvent was removed under reduced pressure.

The obtained residue was dissolved in tetrahydrofuran (THF, 12.2 mL), and then 1.0 M phosphate buffer (12.2 mL) adjusted to pH 8.0 was added. This mixture was stirred at 50°C for three hours. After cooling to 25°C, ethyl acetate (12.2 mL) was added, and the organic layer and the aqueous layer were separated. After extraction was performed by adding ethyl acetate (12.2 mL) to the aqueous layer, all the organic layers obtained were mixed and washed twice with a saturated aqueous sodium chloride solution (12.2 mL). The organic layer was dried over sodium sulfate, the solvent was removed under reduced pressure, and further drying was carried out at 25°C for 30 minutes under reduced pressure using a pump.

The obtained residue was dissolved in dichloromethane (7 mL) and then heptane (16.6 mL) was added. Under controlled vacuum (~ 100 hPa), only dichloromethane was removed and the resulting mixture was filtered to obtain a solid. This washing procedure with heptane was repeated twice. The obtained solid was dried under reduced pressure using a pump at 25°C for two hours to obtain 1.40 g of residue.

To the obtained residue, t-butyl methyl ether (TBME, 25 mL) and a 0.05 M aqueous phosphoric acid solution at pH 2.1 (70 mL) were added, and after stirring the mixture at 25°C for five minutes, the organic layer and the aqueous layer were separated. After extraction by adding t-butyl methyl ether (TBME, 25 mL) to the aqueous layer, all the obtained organic layers were mixed and washed twice with a saturated aqueous sodium chloride solution (25 mL). The organic layer was dried over sodium sulfate, and the solvent was removed under reduced pressure. The residue was dried under reduced pressure using a pump at 25°C for two hours to obtain (2S)-2-((((9H-fluoren-9-yl)methoxy)carbonyl)amino)-3-((tetrahydro-2H-pyran-2-yl)oxy)propanoic acid (Compound 1, Fmoc-Ser (THP)-OH, 1.22 g, 30 mol% of t-butylmethyl ether (TBME) remained). The obtained Fmoc-Ser (THP)-OH was stored in a freezer at -25°C. LCMS (ESI) m/z = 410.2 (M-H)
Retention time: 0.81 minutes (analysis condition SQDFA05)

### Synthesis of (2S,3R)-2-((((9H-fluoren-9-yl)methoxy)carbonyl)amino)-3-((tetrahydro-2H-pyran-2-yl)oxy)butanoic acid (Compound 2, Fmoc-Thr(THP)-OH)

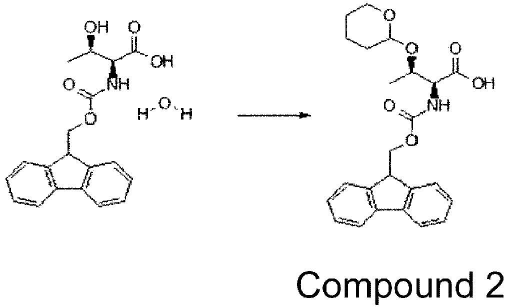

Toluene (50 mL) was added to a mixture of (2S,3R)-2-((((9H-fluoren-9-yl)methoxy)carbonyl)amino)-3-hydroxybutanoic acid monohydrate (Fmoc-Thr-OH monohydrate, purchased from Tokyo Chemical Industry, 5.0 g, 13.9 mmol) and pyridinium p-toluenesulfonate (PPTS, 0.175 g, 0.70 mmol), and the contained water was azeotropically removed with toluene under reduced pressure. Super-dehydrated tetrahydrofuran (THF, 28 mL) and 3,4-dihydro-2H-pyran (8.8 mL, 97 mmol) were added to the obtained residue, and the mixture was stirred at 50°C for four hours under nitrogen atmosphere. After confirming disappearance of the starting materials by LCMS (SQDFA05), the mixture was cooled to 25°C, and ethyl acetate (30 mL) was added. Subsequently, a saturated aqueous sodium chloride solution (30 mL) was added to wash the organic layer, and the aqueous layer was extracted with ethyl acetate (30 mL). All the organic layers obtained were mixed, and this was further washed twice with a saturated aqueous sodium chloride solution (30 mL). The organic layer was dried over sodium sulfate, and the solvent was removed under reduced pressure to obtain 9.3 g of a crude product.

Of the obtained crude product, 4.65 g was dissolved in tetrahydrofuran (THF, 30 mL), and then 1.0 M phosphate buffer (30 mL) adjusted to pH 8.0 was added. This mixture was stirred at 50°C for four hours. After cooling to 25°C, ethyl acetate (30 mL) was added, and the organic layer and the aqueous layer were separated. After extraction was performed by adding ethyl acetate (30 mL) to the aqueous layer, all the obtained organic layers were mixed and washed twice with a saturated aqueous sodium chloride solution (30 mL). The organic layer was dried over sodium sulfate, the solvent was removed under reduced pressure, and this was further dried at 25°C for 30 minutes under reduced pressure with a pump.

The obtained residue was dissolved in diethyl ether (50 mL) and then heptane (50 mL) was added. Under controlled vacuum (~ 100 hPa), only diethyl ether was distilled off and the obtained mixture was filtered to obtain a solid. This washing procedure with heptane was repeated twice. The obtained solid was dried under reduced pressure using a pump at 25°C for two hours to obtain a sodium salt of Fmoc-Thr(THP)-OH (2.80 g, 6.26 mmol).

To the total amount of Fmoc-Thr(THP)-OH sodium salt obtained, ethyl acetate (50 mL) and 0.05 M aqueous phosphoric acid solution (140 mL) at pH 2.1 were added, the mixture was stirred at 25°C for five minutes, and then the organic and aqueous layers were separated. After extraction by adding ethyl acetate (50 mL) to the aqueous layer, all the obtained organic layers were mixed and washed twice with a saturated aqueous sodium chloride solution (50 mL). The organic layer was dried over sodium sulfate, and the solvent was removed under reduced pressure. The residue was dried under reduced pressure using a pump at 25°C for two hours, and then the obtained solid was dissolved in t-butyl methyl ether (TBME, 50 mL), and the solvent was distilled off under reduced pressure. Furthermore, by drying under reduced pressure using a pump at 25°C for one hour, (2S,3R)-2-((((9H-fluoren-9-yl)methoxy)carbonyl)amino)-3-((tetrahydro-2H-pyran-2-yl) oxy)butanoic acid (Compound 2, Fmoc-Thr(THP)-OH, 2.70 g, 30 mol% *t*-butyl methyl ether (TBME) remained) was obtained as a diastereomer derived from an asymmetric carbon on THP protection. The obtained Fmoc-Thr(THP)-OH was stored in a freezer at -25°C.
LCMS (ESI) m/z = 424.2 (M-H)
Retention time: 0.84 minutes, 0.85 minutes (analysis condition SQDFA05)

### Solid-phase peptide synthesis using an automated synthesizer

Peptide synthesis was performed by the Fmoc method using a peptide synthesizer (Multipep RS; manufactured by Intavis). Detailed procedure for the operation was in accordance with the manual attached to the synthesizer.

2-Chlorotrityl resin (100 mg per column) to which the side chain carboxylic acid moiety of aspartic acid protected with Fmoc at its N-terminus was bound, an NMP solution of various Fmoc-amino acids (0.6 mol/L) and 1-hydroxy-7-azabenzotriazole (HOAt), and an N,N-dimethylformamide (DMF) solution (10% v/v) of diisopropylcarbodiimide (DIC) were set in a synthesizer. Fmoc-Thr(THP)-OH and Fmoc-Ser(THP)-OH were made to coexist with oxyma in the NMP solution, and molecular sieves 4A1/8 (Wako Pure Chemical Industries) or molecular sieves 4A1/16 (Wako Pure Chemical Industries) were additionally supplied and set in the synthesizer.

The synthesis was performed using a DMF solution (2% v/v) of diazabicycloundecene (DBU) as the Fmoc deprotection solution. After washing the resin with DMF, Fmoc deprotection followed by Fmoc amino acid condensation reaction was taken as one cycle, and this cycle was repeated to elongate the peptide on the resin surface. After completion of the peptide elongation, the N-terminal Fmoc group of the resin was removed on a peptide synthesizer, and then the resin was washed with DMF.

### Cleavage of the elongated peptide from the resin

After adding DCM to the linear peptide supported on the solid phase obtained by the above-mentioned method and re-swelling the resin, 2,2,2-trifluoroethanol (TFE) / DCM (1/1, v/v, 2 mL) was added to the resin, and shaken at room temperature for two hours. Subsequently, the solution in the tube was filtered through a synthesis column to remove the resin, and furthermore, the remaining resin was washed twice with 2,2,2-trifluoroethanol (TFE) / DCM (1/1, v/v, 1 mL). All of the obtained cleavage solutions were mixed and concentrated under reduced pressure.

### Cyclization of the cleaved peptide

The residue obtained by concentration under reduced pressure after cleavage was dissolved in DMF/DCM (1/1, v/v, 8 mL). 0.5 M O-(7-aza-1H-benzotriazol-1-yl)-N,N,N,N-tetramethyluronium hexafluorophosphate (HATU) / DMF solution (1.5 equivalents relative to the number of moles on the resin used) and DIPEA (1.8 equivalents relative to the number of moles on the resin used) were added and stirred at room temperature for two hours. Thereafter, the solvent was removed under reduced pressure.

### Deprotection of the side chain functional group protecting groups in the cyclic polypeptides, and purification

After addition of a TFA-TIPS-H₂O mixed solution (95/2.5/2.5, v/v/v, 1.5 mL) to dissolve the residue, the mixture was stirred at room temperature for one hour. After removing the solvent under reduced pressure, DMSO was added, insoluble material was removed by filtration, and then purification was performed by preparative-HPLC.

Table 7 summarizes the structural information and analysis data of the synthetic peptides.

### Deprotection of the side chain functional group protecting groups in the cyclic polypeptides, and purification (Part 2)

After addition of a TFA-TIPS-H₂O mixed solution (95/2.5/2.5, v/v/v, 1.5 mL) at 0°C to dissolve the residue, the mixture was stirred at room temperature for 30 minutes. MTBE (10 mL) was added at 0°C, and the resulting precipitate was collected by centrifugation. The recovered crude solid was washed three times with MTBE (15 mL). DMSO was added to the obtained solid to dissolve it, and insoluble matters were removed by filtration, and then purification was performed by preparative-HPLC.

Tables 8A and 8B summarize the structural information and analysis data of the synthetic peptides.

Explanations on Tables 7, 8A, and 8B are as follows. PP1 to PP25 listed in the table each represent a peptide in which an amide bond is formed between the amino group of the "Core 01" amino acid (MeAla (N-methyl-L-alanine) or D-MeAla (N-methyl-D-alanine)) and the side chain carboxy group of the C-terminal amino acid (Asp) to form a cyclized peptide. The "cterm" column refers to the functional group that condenses with the main chain carboxy group of the C-terminal amino acid (Asp), and pyrro means pyrrolidine.

**[Table 7]**

| **id** | **Structure** | **core 01** | **core 02** | **core 03** | **core 04** | **core 05** | **core 06** | **core 07** | **core 08** | **core 09** | **core 10** | **core 11** | **core 12** | **cterm** | **Exact mass** | **Observed (m/z) Analysis conditions SQDAA50** |
|---|---|---|---|---|---|---|---|---|---|---|---|---|---|---|---|---|
| PP1 | 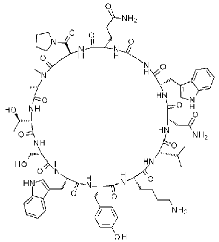 | MeAla | Thr | Ser | Trp | Tyr | Lys | Val | Asn | Trp | Gly | Gln | Asp | pyrro | 1502.7 | 1504.0 (M+H) |
| | | | | | | | | | | | | | | | | 1501.8 (M-H) |
| PP2 | 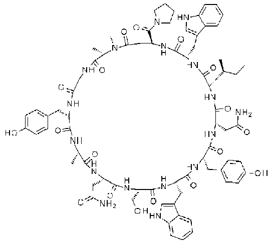 | MeAla | Gly | Tyr | Ala | Asn | Ser | Trp | Tyr | Asn | Ile | Trp | Asp | pyrro | 1507.7 | 1509.1 (M+H) |
| | | | | | | | | | | | | | | | | 1506.8 (M-H) |
| PP3 | 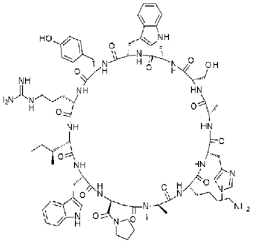 | D-MeAla | Lys | His | Ala | Ser | Ala | Trp | Tyr | Arg | Ile | Trp | Asp | pyrro | 1551.8 | 1553.1 (M+H) |
| | | | | | | | | | | | | | | | | 1551.0 (M-H) |
| PP4 | 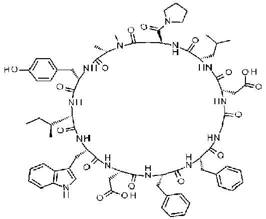 | MeAla | Tyr | Ile | Trp | Asp | Phe | Phe | Gly | Asp | Leu | Asp | | pyrro | 1409.7 | 1410.9 (M+H) |
| | | | | | | | | | | | | | | | | 1408.9 (M-H) |
| PP5 | 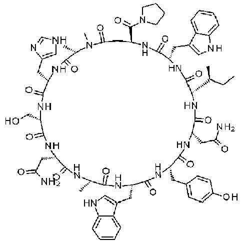 | MeAla | His | Ser | Asn | Ala | Trp | Tyr | Asn | Ile | Trp | Asp | | pyrro | 1424.7 | 1425.9 (M+H) |
| | | | | | | | | | | | | | | | | 1423.8 (M-H) |
| PP6 | 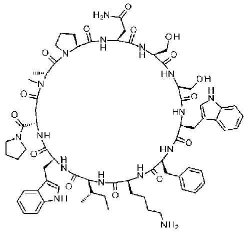 | D-MeAla | Pro | Asn | Ser | Ser | Trp | Phe | Lys | Ile | Trp | Asp | | pyrro | 1398.7 | 1399.9 (M+H) |
| | | | | | | | | | | | | | | | | 1398.1 (M-H) |

**[Table 8A]**

| **id** | **Structure** | **core 01** | **core 02** | **core 03** | **core 04** | **core 05** | **core 06** | **core 07** | **core 08** | **core 09** | **core 10** | **core 11** | **core 12** | **core 13** | **cterm** | **Exact mass** | **Observed (m/z) Analysis conditions SQDAA50** |
|---|---|---|---|---|---|---|---|---|---|---|---|---|---|---|---|---|---|
| PP7 | 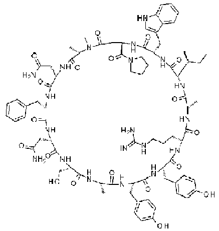 | MeAla | Asn | Phe | Asn | Ser | Ala | Tyr | Tyr | Arg | Ala | Ile | Trp | Asp | pyrro | 1638.8 | 1640.3 (M+H) |
| | | | | | | | | | | | | | | | | | 1638.1 (M-H) |
| PP8 | 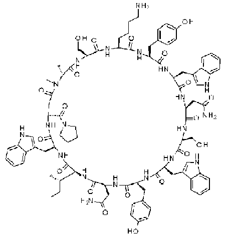 | D-MeAla | Ser | Lys | Tyr | Trp | Asn | Ser | Trp | Tyr | Asn | Ile | Trp | Asp | pyrro | 1780.8 | 1782.3 (M+H) |
| | | | | | | | | | | | | | | | | | 1780.6 (M-H) |
| PP9 | 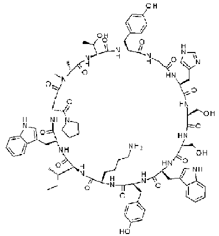 | D-MeAla | Thr | Tyr | Gly | His | Ser | Ser | Trp | Tyr | Lys | Ile | Trp | Asp | pyrro | 1661.8 | 1663.2 (M+H) |
| | | | | | | | | | | | | | | | | | 1661.4 (M-H) |
| PP10 | 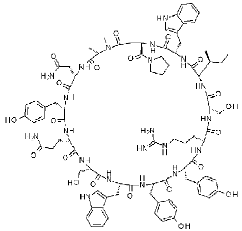 | MeAla | Asn | Tyr | Gln | Ser | Trp | Tyr | Tyr | Arg | Ser | Ile | Trp | Asp | pyrro | 1799.8 | 1802.4 (M+H) |
| | | | | | | | | | | | | | | | | | 1799.6 (M-H) |
| PP11 | 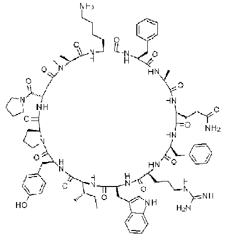 | MeAla | Lys | Phe | Ala | Gln | Phe | Arg | Trp | Ile | Tyr | Pro | Asp | | pyrro | 1589.9 | 1591.4 (M+H) |
| | | | | | | | | | | | | | | | | | 1589.7 (M-H) |
| PP12 | 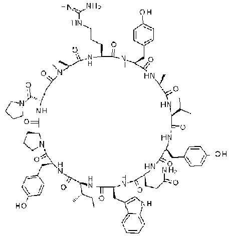 | MeAla | Arg | Tyr | Ala | Val | Tyr | Gln | Trp | Ile | Tyr | Pro | Asp | | pyrro | 1592.8 | 1594.2 (M+H) |
| | | | | | | | | | | | | | | | | | 1592.1 (M-H) |
| PP13 | 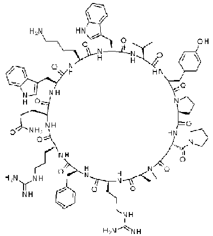 | MeAla | Arg | Phe | Arg | Gln | Trp | Lys | Trp | Val | Tyr | Pro | Asp | | pyrro | 1699.9 | 1701.4 (M+H) |
| | | | | | | | | | | | | | | | | | 1699.5 (M-H) |
| PP14 | 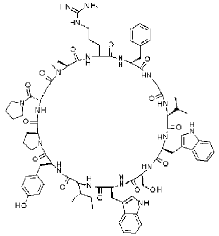 | MeAla | Arg | Phe | Gly | Val | Trp | Ser | Trp | Ile | Tyr | Pro | Asp | | pyrro | 1544.8 | 1546.3 (M+H) |
| | | | | | | | | | | | | | | | | | 1544.1 (M-H) |
| PP15 | 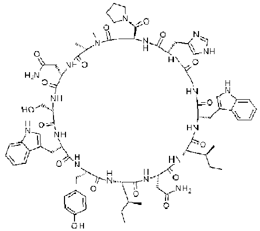 | MeAla | Asn | Ser | Trp | Tyr | Ile | Asn | lie | Trp | Gly | His | Asp | | pyrro | 1523.7 | 1525.2 (M+H) |
| | | | | | | | | | | | | | | | | | 1523.3 (M-H) |

**[Table 8B]**

| | | | | | | | | | | | | | | | | | |
|---|---|---|---|---|---|---|---|---|---|---|---|---|---|---|---|---|---|
| PP16 | 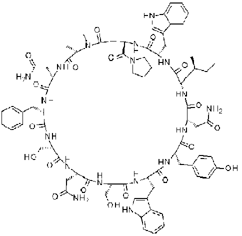 | MeAla | Asn | Phe | Ser | Asn | Ser | Trp | Tyr | Asn | Ile | Trp | Asp | | pyrro | 1564.7 | 1566.2 (M+H) |
| | | | | | | | | | | | | | | | | | 1564.1 (M-H) |
| PP17 | 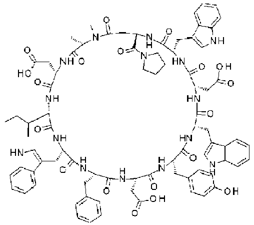 | MeAla | Asp | Ile | Trp | Phe | Asp | Tyr | Trp | Asp | Trp | Asp | | | pyrro | 1579.7 | 1581.1 (M+H) |
| | | | | | | | | | | | | | | | | | 1579.3 (M-H) |
| PP1B | 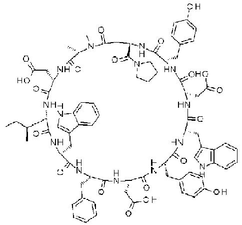 | MeAla | Asp | Ile | Trp | Phe | Asp | Tyr | Trp | Asp | Tyr | Asp | | | pyrro | 1556.7 | 1558.1 (M+H) |
| | | | | | | | | | | | | | | | | | 1556.1 (M-H) |
| PP19 | 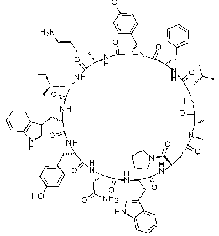 | MeAla | Val | Phe | Tyr | Lys | Ile | Trp | Tyr | Asn | Trp | Asp | | | pyrro | 1552.8 | 1554.3 (M+H) |
| | | | | | | | | | | | | | | | | | 1552.3 (M-H) |
| PP20 | 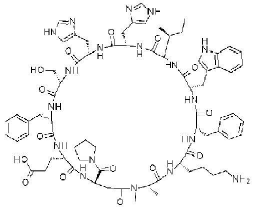 | MeAla | Lys | Phe | Trp | Ile | His | His | Ser | Phe | Glu | Asp | | | pyrro | 1464.7 | 1466.2 (M+H) |
| | | | | | | | | | | | | | | | | | 1464.1 (M-H) |
| PP21 | 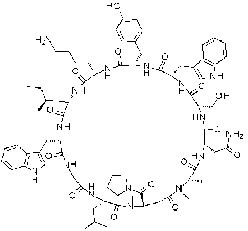 | MeAla | Asn | Ser | Trp | Tyr | Lys | Ile | Trp | Gly | Leu | Asp | | | pyrro | 1400.7 | 1402.2 (M+H) |
| | | | | | | | | | | | | | | | | | 1400.2 (M-H) |
| PP22 | 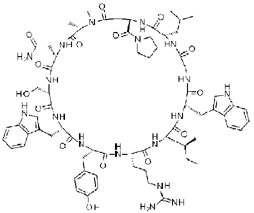 | MeAla | Asn | Ser | Trp | Tyr | Arg | Ile | Trp | Gly | Leu | Asp | | | pyrro | 1428.7 | 1430.2 (M+H) |
| | | | | | | | | | | | | | | | | | 1428.1 (M-H) |
| PP23 | 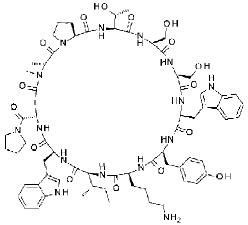 | D-MeAla | Pro | Thr | Ser | Ser | Trp | Tyr | Lys | Ile | Trp | Asp | | | pyrro | 1401.7 | 1403.2 (M+H) |
| | | | | | | | | | | | | | | | | | 1401.2 (M-H) |
| PP24 | 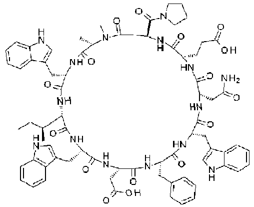 | MeAla | Trp | lie | Trp | Asp | Phe | Trp | Asn | Glu | Asp | | | | pyrro | 1429.6 | 1431.1 (M+H) |
| | | | | | | | | | | | | | | | | | 1429.1 (M-H) |
| PP25 | 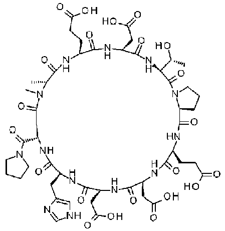 | D-MeAla | Glu | Asp | Thr | Pro | Glu | Asp | Asp | His | Asp | | | | pyrro | 1191.5 | 1192.9 (M+H) |
| | | | | | | | | | | | | | | | | | 1190.8 (M-H) |

### Chemical synthesis of a compound in which the N-terminus of GG-TFPI-tag is linked to the C-terminus (cterm) of a cyclic polypeptide (cyclized product + GG-TFPI-tag)

Using the Fmoc-Asp(O-Trt(2-Cl)-resin)-bMeAla-OAllyl (Compound RS3) resin, a linear peptide in which the C-terminus (cterm) is protected by an allyl group was synthesized by solid-phase peptide synthesis using an automated synthesizer, which is a method already described in this Example. Subsequently, the peptide cleavage from the solid phase was conducted and the obtained peptide was cyclized by the method already described in this Example. The C-terminal (cterm) allyl group of the obtained cyclized product (cyclized product A) was deprotected and converted to cyclized product B, and then cyclized product B was bound by a condensation reaction to the N-terminus of the GG-TFPI-tag supported on the solid phase synthesized by solid-phase peptide synthesis using an automated synthesizer, which is the method already described in this Example, using a Fmoc-Ile-O-Trt (2-Cl)-resin (Compound RS4). Thereafter, cleavage from the solid phase was performed by the method described in the Examples, and all the protecting groups (PG) of the obtained compound were deprotected in a mixed solution of TFA-TIPS-H₂O (95/2.5/2.5, v/v/v). Subsequently, purification by preparative-HPLC gave a compound in which the GG-TFPI-tag is bound to the C terminus (cterm) of the cyclic polypeptide of interest (cyclized product + GG-TFPI-tag) (Fig. 39).

### Method for preparing a resin used for chemical synthesis of a compound in which the N-terminus of GG-TFPI-tag is bound to the C-terminus (cterm) of a cyclic polypeptide (cyclized product + GG-TFPI-tag)

Fmoc-Asp(O-Trt(2-Cl)-resin)-bMeAla-OAllyl (Compound RS3) was synthesized according to the scheme described in Fig. 40.

### Synthesis of 2-phenylpropan-2-yl (S)-3-((((9H-fluoren-9-yl)methoxy)carbonyl)amino)-4-((3-(allyloxy)-3-oxopropyl)(methyl)amino)-4-oxobutanoate (Compound RS1, Fmoc-Asp(OPis)-bMeAla-OAllyl)

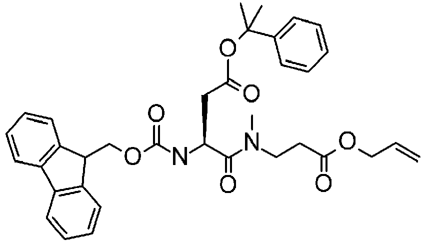

To a solution of commercially available Fmoc-β-MeAla-OH (which may also be referred to as Fmoc-bMeAla-OH, CAS# 172965-84-3) (19.52 g, 60.0 mmol), 1-hydroxybenzotriazole (HOAt) (12.25 g, 90.0 mmol), and 1-ethyl-3-(3-dimethylaminopropyl)carbodiimide hydrochloride (EDCI·HCl) (17.25 g, 90.0 mmol) in dichloromethane (DCM) (120 mL), allyl alcohol (12.24 mL, 180 mmol) and N,N-diisopropylethylamine (DIPEA) (15.72 mL, 90 mmol) were added at 0°C, and the mixture was stirred at room temperature for one hour. Water was added to the reaction solution, and then this was extracted with dichloromethane (DCM) and washed with saturated aqueous ammonium chloride solution and 50% saturated sodium chloride solution. The obtained organic layer was dried over anhydrous sodium sulfate, filtered, and then concentrated under reduced pressure to give allyl 3-((((9H-fluoren-9-yl)methoxy)carbonyl)(methyl)amino)propanoate (Fmoc-bMeAla-OAllyl) (20.9 g) as a crude product.

To a solution of the crude product allyl 3-((((9H-fluoren-9-yl)methoxy)carbonyl)(methyl)amino)propanoate (Fmoc-bMeAla-OAllyl) (10.96 g) in dimethylformamide (60 mL), 1,8-diazabicyclo[5.4.0]-7-undecene (DBU) (4.52 mL, 30 mmol) was added at room temperature, and the mixture was stirred at room temperature for 30 minutes. Pyridine hydrochloride (4.16 g, 36.0 mmol) was added to the reaction solution at room temperature, the mixture was stirred for 30 minutes, and then 1-hydroxybenzotriazole (HOAt) (6.13 g, 45.0 mmol), commercially available Fmoc-Asp(OPis)-OH (CAS# 200336-86-3) (16.34 g, 34.5 mmol), and 1-ethyl-3-(3-dimethylaminopropyl)carbodiimide hydrochloride (EDCI·HCl) (8.63 g, 45.0 mmol) were added at room temperature and stirred for one hour. After adding t-butyl methyl ether to the reaction solution, this was washed twice with a saturated aqueous ammonium chloride solution, twice with a 5% aqueous sodium carbonate solution, with water, and with saturated sodium chloride solution. The obtained organic layer was dried over anhydrous sodium sulfate, filtered, and then concentrated under reduced pressure, and the resulting crude product was purified twice by normal phase column chromatography (hexane/ethyl acetate), to obtain 2-phenylpropan-2-yl (S)-3-((((9H-fluoren-9-yl)methoxy)carbonyl)amino)-4-((3-(allyloxy)-3-oxopropyl)(methyl)amino)-4-oxobutanoate (Compound RS1, Fmoc-Asp (OPis)-bMeAla-OAllyl) (14.499 g, 77% for two steps).
LCMS (ESI) m/z = 621 (M+Na)⁺
Retention time: 1.03 minutes (analysis condition SQDFA05)

### Synthesis of (S)-3-((((9H-fluoren-9-yl)methoxy)carbonyl)amino)-4-((3-(allyloxy)-3-oxopropyl)(methyl)amino)-4-oxobutanoic acid (Compound RS2, Fmoc-Asp-bMeAla-OAllyl)

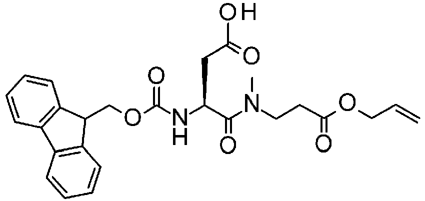

To a solution of 2-phenylpropan-2-yl (S)-3-((((9H-fluoren-9-yl)methoxy)carbonyl)amino)-4-((3-(allyloxy)-3-oxopropyl)(methyl)amino)-4-oxobutanoate (Compound RS1, Fmoc-Asp(OPis)-bMeAla-OAllyl) (14.499 g, 24.22 mmol) in dichloromethane (24.22 mL), trifluoroacetic acid (TFA) (11.19 mL, 145 mmol) was added dropwise at 0°C and stirred for 30 minutes. An 8 M aqueous sodium hydroxide solution (18.16 mL) was added dropwise to the reaction solution, then a saturated aqueous sodium dihydrogen phosphate solution and water were added, and the mixture was extracted twice with ethyl acetate. The obtained organic layer was washed twice with a solution prepared with a saturated aqueous sodium dihydrogen phosphate solution / water = 1/2, dried over anhydrous sodium sulfate, and filtered to obtain (S)-3-((((9H-fluoren-9-yl)methoxy)carbonyl)amino)-4-((3-(allyloxy)-3-oxopropyl)(methyl)amino)-4-oxobutanoic acid (Compound RS2, Fmoc-Asp-bMeAla-OAllyl) (14.2 g) as a crude product.
LCMS (ESI) m/z = 481 (M+H)⁺
Retention time: 0.76 minutes (analysis condition SQDFA05)

### Synthesis of Fmoc-Asp(O-Trt(2-Cl)-resin)-bMeAla-OAllyl (Compound RS3)

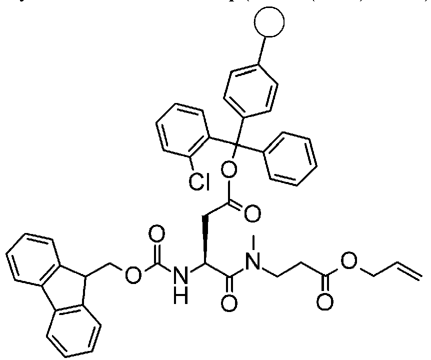

A reaction vessel equipped with a filter was charged with 2-chlorotritylchloride resin (1.60 mmol/g, 100-200 mesh, 1% DVB, purchased from Watanabe Chemical Industries, 30 g, 48.0 mmol) and dichloromethane, and this was shaken at room temperature for 30 minutes. After removing dichloromethane by applying nitrogen pressure, a solution of the above-described (S)-3-((((9H-fluoren-9-yl)methoxy)carbonyl)amino)-4-((3-(allyloxy)-3-oxopropyl)(methyl)amino)-4-oxobutanoic acid (Compound RS2, Fmoc-Asp-bMeAla-OAllyl) (11.6 g) and N,N-diisopropylethylamine (DIPEA) (20.24 mL, 116 mmol) in dichloromethane (241 mL) was added to the reaction vessel, and this was shaken for 40 minutes. After removing the reaction solution by applying nitrogen pressure, a mixture of methanol (27.4 mL) and N,N-diisopropylethylamine (DIPEA) (20.4 mL, 116 mmol) in dichloromethane (241 mL) was added to the reaction vessel and shaken for two hours. After removing the reaction solution by applying nitrogen pressure, dichloromethane was added, this was shaken for five minutes, and procedure of removing dichloromethane by applying nitrogen pressure was repeated five times to wash the resin. The obtained resin was dried overnight under reduced pressure to obtain Fmoc-Asp(O-Trt (2-Cl)-resin)-bMeAla-OAllyl (Compound RS3) (38.0578 g).

The loading amount of the obtained resin was calculated using the method described in the literature (Letters in Peptide Science, 2002, 9, 203). Fmoc-Asp(O-Trt(2-Cl)-resin)-bMeAla-OAllyl (Compound RS3) (5.8 mg) was placed into a reaction vessel, DMF (2 mL) was added, and this was shaken for one hour. DBU (0.04 mL) was added to the reaction solution, this was shaken for 30 minutes, then acetonitrile (10 mL) was added, 1 mL of this was taken out, and this was further diluted with acetonitrile until the solution volume reached 12.5 mL. The absorbance (304 nm) of the obtained solution was measured (measured using Shimadzu, UV-1600PC (cell length 1.0 cm)), and the loading amount of Fmoc-Asp(O-Trt(2-Cl)-resin)-bMeAla-OAllyl (Compound RS3) was calculated to be 0.517 mmol/g.

### Synthesis of Fmoc-Ile-Otrt(2-CD-resin (Compound RS4

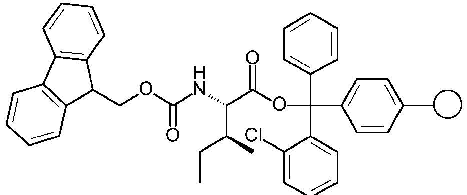

A reaction vessel equipped with a filter was charged with 2-chlorotritylchloride resin (1.60 mmol/g, 100-200 mesh, 1% DVB, purchased from Watanabe Chemical Industries, 10.0 g, 16.0 mmol) and dehydrated dichloromethane, and this was shaken at room temperature for one hour. After removing dichloromethane by applying nitrogen pressure, a solution of commercially available N-(((9H-fluoren-9-yl)methoxy)carbonyl)-N-isoleucine (Fmoc-Ile-OH) (2.83 g, 8.00 mmol) and N,N-diisopropylethylamine (DIPEA) (6.71 mL, 38.4 mmol) in dehydrated dichloromethane (80 mL) was added to the reaction vessel and this was shaken for 30 minutes. After removing the reaction solution by applying nitrogen pressure, a mixture of dehydrated methanol (20 mL) and N,N-diisopropylethylamine (DIPEA) (7.0 mL) in dehydrated dichloromethane (80 mL) was added to the reaction vessel and shaken for two hours. After removing the reaction solution by applying nitrogen pressure, dichloromethane was added, this was shaken for five minutes, and procedure of removing dichloromethane by applying nitrogen pressure was repeated three times to wash the resin. The obtained resin was dried overnight under reduced pressure to obtain Fmoc-Ile-Otrt(2-Cl)-resin (Compound RS4) (12.5113 g).

The loading amount of the obtained resin was calculated using the method described in the literature (Letters in Peptide Science, 2002, 9, 203). Fmoc-Ile-Otrt(2-Cl)-resin (Compound RS4) (10.0 mg) was placed in a reaction vessel, DMF (2 mL) was added, and this was shaken for one hour. DBU (0.04 mL) was added to the reaction solution, this was shaken for 30 minutes, then acetonitrile (10 mL) was added, 1.0 mL was taken out, and this was further diluted with acetonitrile until the solution volume reached 12.5 mL. The absorbance (304 nm) of the obtained solution was measured (measured using Shimadzu, UV-1600PC (cell length: 1.0 cm)), and the loading amount of Fmoc-Ile-Otrt(2-Cl)-resin (Compound RS4) was calculated to be 0.549 mmol/g.

### Deprotection reaction for the C-terminal (cterm) allyl group of cyclized product A

Cyclized product A obtained by using Fmoc-Asp(O-Trt (2-Cl)-resin)-bMeAla-OAllyl (Compound RS3) (600 mg) was dissolved in dichloromethane (6.0 mL), and then phenylsilane (PhSiH₃) (0.096 mL, 0.776 mmol), tetrakis(triphenylphosphine) palladium(O) (Pd(PPh₃)₄) (36 mg, 0.031 mmol) was added, this was stirred at room temperature. After confirming disappearance of the starting material by LC-MS, methanol was added to the reaction solution, and the solvent was removed under reduced pressure. The obtained residue was dissolved in dimethyl sulfoxide and purified by reverse phase column chromatography (10 mM aqueous ammonium acetate solution/methanol) to obtain cyclized product B (Fig. 41).

### Method for synthesizing a cyclized product + GG-TFPI-tag compound

GG-TFPI-tag resin (50 mg) protected by Fmoc group at the N-terminus, which was synthesized by solid phase peptide synthesis on an automated synthesizer by the method already described in the examples using Fmoc-Ile-Otrt(2-Cl)-resin (Compound RS4), was added to a reaction vessel equipped with a filter, then dichloromethane was added, the resin was shaken for 30 minutes, and dichloromethane was removed by filtration. A 2% 1,8-diazabicyclo[5.4.0]-7-undecene (DBU)/dimethylformamide (DMF) solution (0.5 mL) was added to the reaction vessel, and after shaking this for 30 minutes at room temperature, the reaction solution was removed by filtration. Thereafter, dimethylformamide (DMF) (0.5 mL) was added to the reaction vessel, this was shaken for five minutes, and dimethylformamide (DMF) was removed by filtration. This resin washing with dimethylformamide (DMF) was repeated six times. Subsequently, to a mixture of 0.1 M cyclized product B/*N*-methyl-2-pyrrolidone (NMP) solution (250 µL) and 0.75 M 1-hydroxybenzotriazole (HOAt)/*N*-methyl-2-pyrrolidone (NMP) solution (83 µL), 0.75 M N,N-diisopropylcarbodiimide (DIC)/dimethylformamide (DMF) solution (40 µL) was added, and the solution was stirred by pipetting several times. After the solution was added to the reaction vessel, the reaction vessel was shaken overnight at 40°C, and then the reaction solution was removed by filtration. Thereafter, dimethylformamide (DMF) (0.5 mL) was added to the reaction vessel, this was shaken for five minutes, and dimethylformamide (DMF) was removed by filtration. This resin washing with dimethylformamide (DMF) was repeated six times. Dichloromethane (DCM) (0.5 mL) was added to the reaction vessel and after shaking several times, dichloromethane (DCM) was removed by filtration. A trifluoroethanol (TFE) / dichloromethane (DCM) = 1/1 solution (0.5 mL) was added to the reaction vessel, and this was shaken at room temperature for two hours for cleavage from the solid phase. After recovering the cleavage solution by filtration, a trifluoroethanol (TFE) / dichloromethane (DCM) = 1/1 solution (0.5 mL) was added to the reaction vessel, and the resin was washed twice. The obtained solution was collected and concentrated under reduced pressure to obtain a protecting group (PG)-bearing cyclized product + GG-TFPI-tag compound. At 0°C, a mixed TFA-TIPS-H₂O solution (95:2.5:2.5, v/v/v) (1.0 mL) was added to the obtained protecting group (PG)-bearing cyclized product + GG-TFPI-tag compound, and then this was stirred at 0°C. The disappearance of the raw materials was confirmed by LC-MS. At 0°C, t-butyl methyl ether was added to the reaction solution, filtration was performed, the obtained solid was dissolved in dimethyl sulfoxide, and this solution was purified by preparative HPLC to synthesize the target substance, which is the cyclized product + GG-TFPI-tag compound (Fig. 42).

### Structural information of the cyclized product + GG-TFPI-tag compound

Structural information of the cyclized product + GG-TFPI-tag compound is shown in Fig. 43. Furthermore, the sequence information and analysis information of the cyclized product + GG-TFPI-tag compound are shown in Table 9 and Table 10, respectively. In Table 9, Core 01 to 13 are amino acids constituting the cyclic portion, and H 01 to 17 are defined as amino acids that represent the linear portion. Asp2 represents the cyclization site, indicating that the carboxylic acid site of the aspartic acid (Asp) side chain forms a ring with the N-terminus of Core 01 by an amide bond. In addition, the C-terminus of Asp2 and the N-terminus of the linear portion H 01 are connected by an amide bond, and defined as H 01, H 02, and so on, starting from the position closest to Asp2. Furthermore, "cterm" represents the functional group of the main chain carboxylic acid of H 17, and "none" shows that the C-terminus of H 17 is a carboxylic acid.

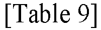

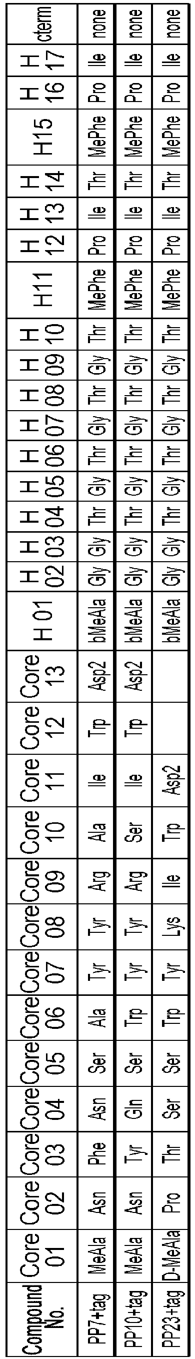

**[Table 10]**

| Compound No. | **Exact MS** | **LCMS(ESI) m/z** | | Retention time | Analysis condition |
|---|---|---|---|---|---|
| PP7+tag | 3203.6 | 1600.7 | (M-2H)²⁻ | 0.48 | SQDAA50 |
| PP10+tag | 3364.6 | 1683.3 | (M+2H)²⁺ | 0.44 | SQDAA50 |
| PP23+tag | 2966.5 | 1484.3 | (M+2H)²⁺ | 0.49 | SQDAA50 |

### PP7 + tag (SEQID NO : 57)

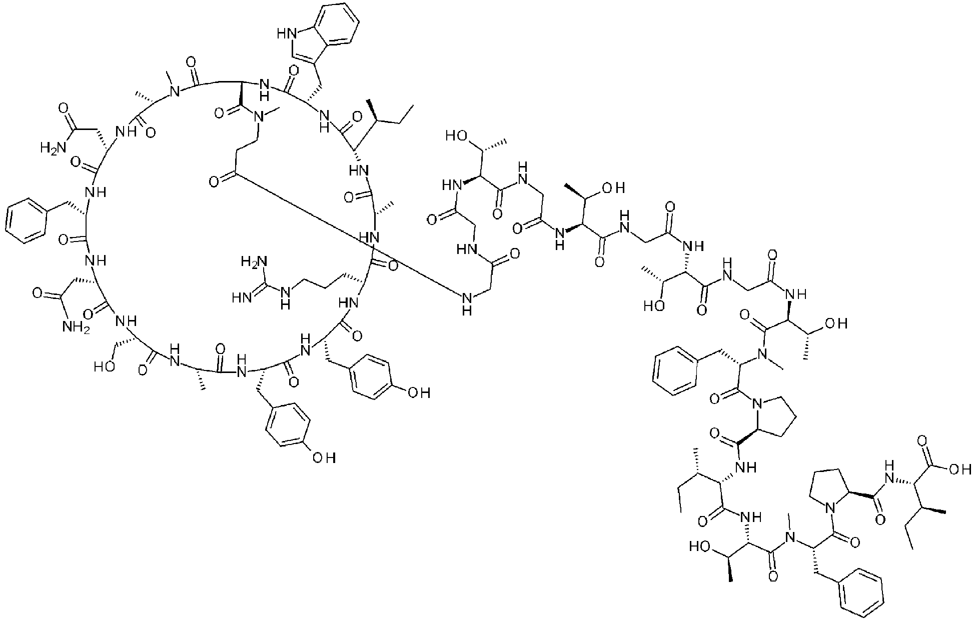

### PP1O + tag (SEQ ID NO: 5 8)

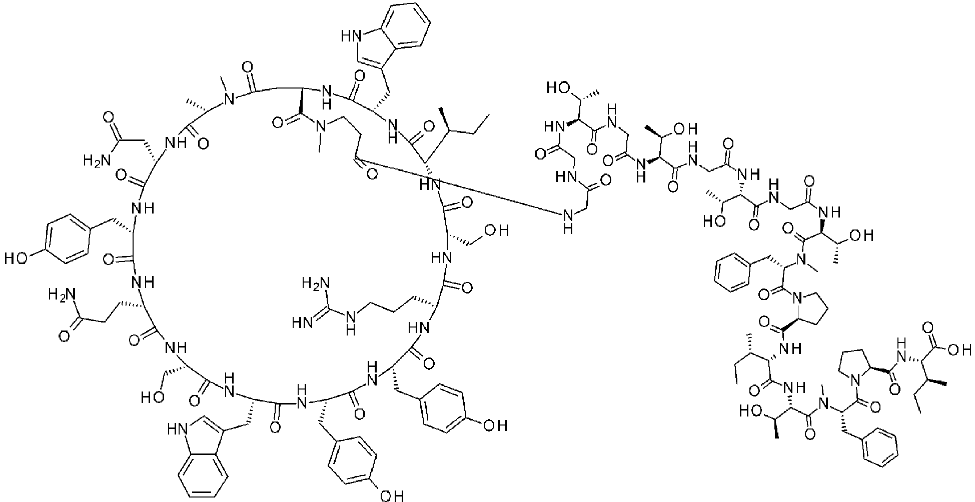

### PP23 + tag (SEQ ID NO : 59)

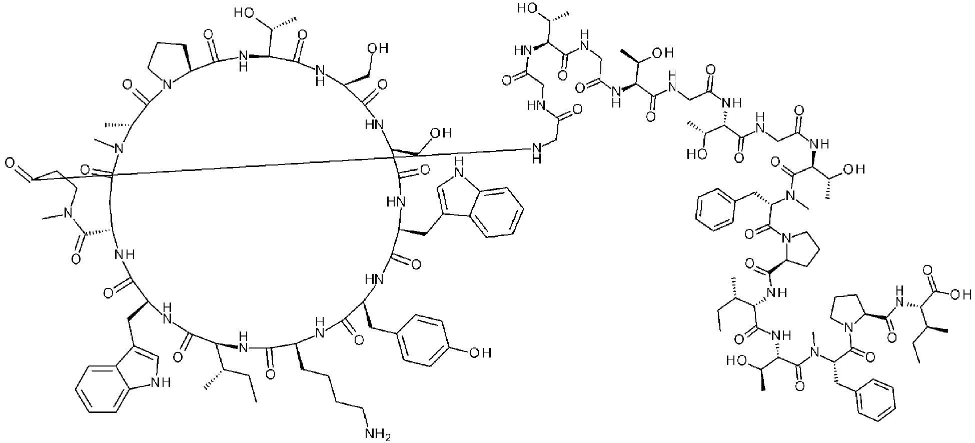

### Example 21. Detection of Regnase-1 phosphorylation by IKKβ or TBK1 (AlphaScreen)

### (Preparation of human Regnase-1)

The full-length sequence of human Regnase-1 (UniProt ID: Q5D1E8) was used to prepare a construct having a FLAG tag on the N-terminal side and a recognition sequence for biotin ligase BirA on the C-terminal side (FL_Reg1) as a target protein. The above-mentioned Regnase-1 was expressed in mammalian Expi293 cells, purified with FLAG M2 agarose, and isolated by gel filtration chromatography. When biotinylation was required, methods from nonpatent documents (BMC biotechnology, 2008,8,41; and Protein Science, 1999,8,921-929) were used.

Among the full-length human Regnase-1 sequence, the 1st to 546th amino acid sequence was prepared and made into a construct having a FLAG tag on the N-terminal side and a biotin ligase BirA recognition sequence on the C-terminal side (herein, a Regnase-1 lacking the C-terminal domain of Regnase-1 (amino acids 547 to 599 in the case of SEQ ID NO: 2) is referred to as "ΔCTD_Reg1". "ΔCTD_Reg1" is different from "Regnase-1ΔCTD", which is a C-terminal domain deletion variant resulting from a frameshift mutation caused by a 1bp deletion at codon 517, as prepared in Example 13). In addition, the sequence from amino acids 301 to 599 of the full-length human Regnase-1 sequence was designated as C-terminal Regnase-1, and a construct having a FLAG tag on the N-terminal side and a recognition sequence for biotin ligase BirA on the C-terminal side was prepared (Reg1_301-599). The above-mentioned Regnase-1 was expressed in mammalian Expi293 cells, purified with FLAG M2 agarose, and isolated by gel filtration chromatography. When biotinylation was required, methods of nonpatent documents (BMC biotechnology, 2008,8,41; and Protein Science, 1999,8,921-929) were used.

### (Phosphorylation reaction)

Using Kinase assay buffer III (manufactured by SignalChem) supplemented with 2 mM DTT, 100 µM sodium vanadate, and 2 mM manganese chloride, kinase (IKKβ (manufactured by SignalChem) or TBK1 (manufactured by SignalChem)) and Regnase-1 were reacted in the presence of 10 µM ATP at room temperature for 2 hours to perform a phosphorylation reaction.

### (Detection of phosphorylated Regnase-1)

Phosphorylated Regnase-1 was detected by the AlphaScreen system. After each of the above-mentioned phosphorylated Regnase-1 antibodies was added and reacted at room temperature for one hour, anti-FLAG AlphaLISA Acceptor Beads (manufactured by PerkinElmer) and Protein A AlphaScreen Donor Beads (manufactured by PerkinElmer) were added, and EnVision (manufactured by PerkinElmer) was used to measure the excited luminescence. As a result, phosphorylated Regnase-1 produced by the phosphorylation reaction by kinase was specifically detected, and signals were confirmed to be enhanced in a manner dependent on Regnase-1 and kinase concentrations (Fig. 24).

### (Phosphorylation inhibitory activity of the compounds)

After mixing 2 nM of the above-mentioned Regnase-1 and the compound, phosphorylated Regnase-1 produced by the phosphorylation reaction was detected by the AlphaScreen system. As the results, the inhibition rate was calculated from the signals obtained when adding the compound at each concentration, by defining the signal obtained with Regnase-1 alone to be 100% inhibition rate, and the signal obtained when the same amount of solvent (DMSO) is added in place of the compound as 0% inhibition rate. The 50% inhibition concentration (µM) was calculated from the obtained dose response curve (Table 11).

As a result, these compounds (PP1, PP2, PP3, PP4, PP5, and PP6) all inhibited phosphorylation of full-length Regnase-1 (FL_Reg1) by both kinases, IKKβ and TBK1, in a concentration-dependent manner (Figs. 25-1 and 25-2). Meanwhile, the compounds PP2, PP3, PP4, PP5, and PP6 also inhibited phosphorylation of the CTD deficient variant of Regnase-1 (ΔCTD_Reg1) by both kinases, as in FL-Reg1, but PP1 did not inhibit the phosphorylation of ΔCTD_Reg1 (Figs. 26-1 and 26-2). These results suggested that the compounds PP2, PP3, PP4, PP5, and PP6 may be exhibiting phosphorylation inhibitory activity by acting on amino acids included in the portion other than the C-terminal domain (the domain of positions 1-546 in SEQ ID NO: 2) of human Regnase-1 (SEQ ID NO: 2), while compound PP1 may be exhibiting phosphorylation inhibitory activity by acting on amino acids included in the C-terminal domain (the domain of positions 547-599 in SEQ ID NO: 2) of human Regnase-1 (SEQ ID NO: 2).

**[Table 11]**

| Inhibitory activity on Regnase-1 phosphorylation due to TBK1 or IKKβ (50% inhibition concentrations resulting from each compound are indicated) | | | | |
|---|---|---|---|---|
| **ID** | **FL_Reg 1** | | **ΔCTD_Reg1** | |
| | **TBK1** | **IKKβ** | **TBK1** | **IKKβ** |
| PP1 | 54.5 | 28.1 | >62.1 | >62.1 |
| PP2 | 21.7 | 16.6 | 17.6 | 14.0 |
| PP3 | 3.9 | 4.6 | 5.7 | 8.3 |
| PP4 | 61.9 | 19.5 | 44.9 | 8.3 |
| PP5 | 24.2 | 23.6 | 19.1 | 20.4 |
| PP6 | 13.1 | 17.0 | 14.5 | 20.3 |

### Example 22. Binding of IKKβ or TBK1 to Regnase-1 (AlphaScreen)

### (Preparation of human Regnase-1)

As a target protein, a construct having a FLAG tag on the N-terminal side and a recognition sequence for biotin ligase BirA on the C-terminal side of the full-length sequence of human Regnase-1 (UniProt ID: Q5D1E8) was prepared (FL_Reg1). The above-mentioned Regnase-1 was expressed in mammalian Expi293 cells, purified with FLAG M2 agarose, and isolated by gel filtration chromatography. When biotinylation was required, methods from nonpatent documents (BMC biotechnology, 2008,8,41; and Protein Science, 1999,8,921-929) were used.

### (Inhibition of kinase-Regnase-1 binding by the compounds)

After mixing 2.5 nM of the above-mentioned Regnase-1 and the compound, 2.5 nM of IKKβ or TBK1 was added and reacted at room temperature for two hours. Then, Anti FLAG AlphaLISA Acceptor Beads (manufactured by PerkinElmer) and Glutathione-coated AlphaScreen Donor Beads (manufactured by PerkinElmer) were added, and excited luminescence was measured using EnVision (manufactured by PerkinElmer). As the results, the inhibition rate from the signals obtained when adding the compound at each concentration were calculated, by defining the signal obtained with Regnase-1 alone to be 100% inhibition rate, and the signal obtained when the same amount of solvent (DMSO) is added in place of the compound as 0% inhibition rate. The 50% inhibition concentration (µM) was calculated from the obtained dose response curve (Table 12).

As a result, PP2, PP3, PP4, PP5, and PP6 inhibited the binding of Regnase-1 to both kinases, but PP1 did not (Figs. 27-1 and 27-2).

**[Table 12]**

| Inhibitory activity on binding between Regnase-1 and TBK1 or IKKβ (50% inhibition concentrations resulting from each compound are indicated) | | |
|---|---|---|
| **ID** | **FL_Reg1** | |
| | **TBK1** | **IKKβ** |
| PP1 | >62.1 | >62.1 |
| PP2 | 15.5 | 35.8 |
| PP3 | 3.1 | 8.3 |
| PP4 | 24.4 | 47.7 |
| PP5 | 11.5 | 24.0 |
| PP6 | 19.2 | 42.1 |

With respect to the compounds PP1 to PP25 identified from the peptide library, the activity to inhibit Regnase-1 phosphorylation by TBK1 and the activity to inhibit the binding between TBK1 and Regnase-1 were measured. The results are summarized in Table 13 (50% inhibition concentrations (µM) resulting from each compound are indicated).

As a result, regarding phosphorylation by TBK1, when full-length Regnase-1 (FL_Reg1) was used as a substrate, PP1 to PP25 all showed inhibitory activity. When a CTD deficient Regnase-1 variant (ΔCTD_Reg1; corresponding to amino acids 1 to 546 of human Regnase-1 (SEQ ID NO: 2)) was used as a substrate, inhibitory activity was observed with compounds other than PP1 and PP20. It was suggested that these compounds may act on this region (the region of amino acids 1-546 of human Regnase-1) and exhibit phosphorylation inhibitory activity. In addition, when the C-terminal Regnase-1 (Reg1_301-599; corresponding to amino acids 301-599 of human Regnase-1 (SEQ ID NO: 2)) was used as a substrate, inhibitory activity by compounds other than PP1, PP11, PP16, and PP20 was observed. It was suggested that these compounds may act on this region (region of positions 301-599 of human Regnase-1) and exhibit phosphorylation inhibitory activity. Regarding the binding between TBK1 and full-length Regnase-1 (FL Regl), inhibitory activity was observed with compounds other than PP1 and PP12.

**[Table 13]**

| ID | Phosphorylation inhibition | | | Binding inhibition |
|---|---|---|---|---|
| | FL_Reg1 | ΔCTD_Reg1 | Reg 1_301-599 | FL_Reg1 |
| PP1 | 54.5 | >62.1 | >62.1 | >62.1 |
| PP2 | 21.7 | 17.6 | 22.4 | 15.5 |
| PP3 | 3.9 | 5.7 | 7.1 | 3.1 |
| PP4 | 61.9 | 44.9 | 35.9 | 24.4 |
| PP5 | 24.2 | 19.1 | 40.4 | 11.5 |
| PP6 | 13.1 | 14.5 | 16.8 | 19.2 |
| PP7 | 2.2 | 4.7 | 4.6 | 17.9 |
| PP8 | 2.9 | 5.5 | 6.0 | 6.5 |
| PP9 | 4.2 | 6.6 | 7.9 | 8.3 |
| PP10 | 0.2 | 0.2 | 0.4 | 9.8 |
| PP11 | 29.1 | 55.6 | >62.1 | 60.9 |
| PP12 | 12.7 | 22.5 | 19.6 | >62.1 |
| PP13 | 8.4 | 17.7 | 18.0 | 13.8 |
| PP14 | 3.1 | 5.2 | 6.8 | 7.1 |
| PP15 | 8.5 | 9.5 | 17.4 | 13.7 |
| PP16 | 52.4 | 38.1 | >62.1 | 20.2 |
| PP17 | <0.06 | 0.3 | <0.06 | <0.06 |
| PP18 | 0.6 | 1.8 | 0.9 | <0.06 |
| PP19 | 3.7 | 4.6 | 5.8 | 2.4 |
| PP20 | 38.5 | >62.1 | >62.1 | 47.6 |
| PP21 | 13.5 | 11.7 | 17.0 | 5.6 |
| PP22 | 24.3 | 22.8 | 25.8 | 11.4 |
| PP23 | 18.8 | 29.7 | 30.7 | 9.2 |
| PP24 | 35.7 | 27.3 | 34.8 | 7.7 |
| PP25 | 18.9 | 16.1 | 16.3 | 13.1 |

In addition, regarding the compounds PP1 to PP25 identified from the peptide library, the activity to inhibit phosphorylation of Regnase-1 by IKKβ and the activity to inhibit the binding between IKKβ and Regnase-1 were measured. The results are summarized in Table 14 (50% inhibition concentrations (µM) resulting from each compound are described).

As a result, regarding phosphorylation by IKKβ, when full-length Regnase-1 (FL_Reg1) was used as a substrate, PP1 to PP25 all showed inhibitory activity. When a CTD-deficient Regnase-1 variant (ΔCTD_Reg1; corresponding to amino acids 1 to 546 of human Regnase-1 (SEQ ID NO: 2)) was used as a substrate, inhibitory activity was observed with compounds other than PP1, PP11, PP20, and PP23. It was suggested that these compounds may act on this region (the region of amino acids 1-546 of human Regnase-1) and exhibit phosphorylation inhibitory activity. In addition, when C-terminal Regnase-1 (Reg1_301-599; corresponding to amino acids 301-599 of human Regnase-1 (SEQ ID NO: 2)) was used as a substrate, inhibitory activity by compounds other than PP1, PP11, PP20, and PP23 was observed. It was suggested that these compounds may act on this region (region of positions 301-599 of human Regnase-1) and exhibit phosphorylation inhibitory activity. Regarding the binding between IKKβ and full-length Regnase-1 (FL_Reg1), inhibitory activity was observed with compounds other than PP1, PP11, PP12, PP16, PP20, and PP25.

**[Table 14]**

| ID | Phosphorylation inhibition | | | Binding inhibition |
|---|---|---|---|---|
| | FL_Reg1 | ΔCTD_Reg1 | Reg 1_301-599 | FL_Reg1 |
| PP1 | 28.1 | >62.1 | >62.1 | >62.1 |
| PP2 | 16.6 | 140 | 18.5 | 35.8 |
| PP3 | 4.6 | 8.3 | 6.8 | 8.3 |
| PP4 | 19.5 | 8.3 | 12.8 | 47.7 |
| PP5 | 23.6 | 20.4 | 31.3 | 24.0 |
| PP6 | 17.0 | 20.3 | 17.5 | 42.1 |
| PP7 | 0.8 | 4.5 | 3.0 | 15.1 |
| PP8 | 1.4 | 7.8 | 5.7 | 7.4 |
| PP9 | 2.7 | 64 | 6.2 | 10.6 |
| PP10 | 0.5 | 2.6 | 1.8 | 11.4 |
| PP11 | 22.3 | >62.1 | >62.1 | >62.1 |
| PP12 | 6.8 | 27.0 | 20.7 | >62.1 |
| PP13 | 3.6 | 43.2 | 23.2 | 16.9 |
| PP14 | 1.6 | 9.0 | 6.5 | 20.1 |
| PP15 | 5.6 | 31.0 | 22.0 | 33.7 |
| PP16 | 20.6 | 30.8 | 42.2 | >62.1 |
| PP17 | 0.5 | 0.3 | 0.7 | <0.06 |
| PP18 | 0.4 | 0.2 | 0.5 | <0.06 |
| PP19 | 5.4 | 12.6 | 10.1 | 5.8 |
| PP20 | 24.7 | >62.1 | >62.1 | >62.1 |
| PP21 | 19.6 | 39.3 | 37.2 | 20.7 |
| PP22 | 15.0 | 34.0 | 31.0 | 26.8 |
| PP23 | 25.6 | >62.1 | >62.1 | 41.3 |
| PP24 | 5.3 | 4.0 | 7.2 | 8.8 |
| PP25 | 27.0 | 47.7 | 27.6 | >62.1 |

### Example 23. Detection of RNA degradation activity by Regnase-1 (PP1-6)

An evaluation system for measuring RNA degradation activity (RNase activity) by recombinant Regnase-1 protein was used to evaluate the effects of the compounds on this activity.

### (Preparation of human IL-8 RNA)

A DNA having a T7 promoter upstream of the transcription start site of the human IL-8 gene (NCBI, Refseq NM_000584.3) was obtained by chemical synthesis and then cloned into the pUC57 vector for amplification in *E. coli.* A part of IL-8 gene containing the T7 promoter was amplified by PCR, and a fraction containing the DNA product of interest was collected by agarose gel electrophoresis. The PCR product was purified using Wizard SV Gel and PCR Clean-Up System (manufactured by Promega). According to the protocol of ScriptMAX Thermo T7 transcription Kit (manufactured by TOYOBO), RNA was generated from the PCR product by *in vitro* RNA polymerase reaction. Subsequently, RNA was purified using RNeasy Mini Kit (manufactured by QIAGEN) and used for the assay. The sequences of the primers used for amplification of T7 promoter-fused IL-8 gene are shown:
Fw (5'-3'): taatacgactcactataggg (SEQ ID NO: 17); and
Rv (5'-3'): gaaaatttaactgggtaccc (SEQ ID NO: 18).

### (Preparation of Regnase-1 D141N mutant)

A construct having a FLAG tag on the N-terminal side and a biotin ligase BirA recognition sequence on the C-terminal side was produced by introducing mutations to a full-length human Regnase-1 sequence so that aspartic acid (D) at position 141 becomes asparagine (N). The above-mentioned Regnase-1 was expressed in mammalian Expi293 cells, purified with FLAG M2 agarose and cOmplete His-Tag Purification Resin (Roche), and isolated by gel filtration chromatography. When biotinylation was required, the methods of non-patent literature (BMC biotechnology, 2008, 8, 41, and non-patent literature Protein Science, 1999, 8, 921-929) were used.

### (Cleavage reaction)

In a Tris-HCl (pH 7.5) buffer containing 1 mM DTT, 5 mM MgCl₂, 0.0125 mM ZnCl₂, and 150 mM NaCl, IL8 RNA and a Regnase-1 protein (FLAG_Regnase-1 or FLAG_Regnase-1 (D141N)) were mixed at a final concentration of 100 nM, and RNA cleavage reaction with Regnase-1 was performed by allowing this mixture to react at 37°C for one hour. For compound evaluation, a compound dissolved in DMSO was added to the mixed solution to produce final concentrations of 12.5 µM and 2.5 µM, and they were allowed to react similarly.

### (RNA detection)

After completion of the reaction, the IL8 RNA concentration in the reaction solution was quantified by Taqman PCR method. A standard curve was prepared by serially diluting IL8 RNA of known concentration used for the reaction, and the IL8 RNA concentration in the reaction solution was calculated. Taqman PCR was performed according to QuantiTect Probe RT-PCR Kit (manufactured by QIAGEN) and analyzed with LightCycler 480 II (manufactured by Roche). The Taqman probe used is shown below:
IL8 (manufactured by Applied biosystems, Hs01553824_g1).

### (Results)

The average of the results of duplicate measurements is shown as the RNA concentration. Compared with the D141N mutant having no RNA degrading activity, a decrease in RNA concentration was observed when Regnase-1 was added; therefore, RNA degradation dependent on Regnase-1 was confirmed in this evaluation system. In addition, when Regnase-1 and the compound were allowed to coexist, none of the compounds inhibited the RNA degradation activity by Regnase-1 (Fig. 28). Fig. 29 shows the effects of adding a compound to D141N. None of the compounds were shown to greatly affect RNA detection itself.

### Example 24. Detection of RNA degradation activity by Regnase-1 (PP7-25)

As in the method described in Example 23, effects of the compounds on RNase activity were evaluated.

### (Cleavage reaction)

In a Tris-HCl (pH 7.5) buffer containing 0.75 mM DTT, 3.8 mM MgCl₂, 9.4 µM ZnCl₂, and 150 mM NaCl, IL8 RNA and Regnase-1 protein (FLAG_Regnase-1 or FLAG_Regnase-1 (D226N, D244N)) were mixed at a final concentration of 100 nM, and RNA cleavage reaction with Regnase-1 was performed by allowing this mixture to react at 37°C for one hour. For compound evaluation, a compound dissolved in DMSO was added to the mixed solution to produce final concentrations of 12.5 µM and 2.5 µM, and they were allowed to react similarly.

### (RNA detection)

After completion of the reaction, the IL8 RNA concentration in the reaction solution was quantified by Taqman PCR method. A standard curve was prepared by serially diluting IL8 RNA of known concentration used for the reaction, and the IL8 RNA concentration in the reaction solution was calculated. Taqman PCR was performed according to QuantiTect Probe RT-PCR Kit (manufactured by QIAGEN) and analyzed with LightCycler 480 II (manufactured by Roche). The Taqman probe used is shown below:
IL8 (manufactured by Applied biosystems, Hs01553824_g1).

### (Preparation of Regnase-1_wt and Regnase-1_D226N,D244N)

A construct having a FLAG tag on the N-terminal side and a Sortase recognition sequence (Leu-Pro-Met-Thr-Gly) (SEQ ID NO: 49) and a histidine tag on the C-terminal side was produced with the full-length human Regnase-1 sequence or with the full-length sequence introduced with mutations that change the 226th amino acid from aspartic acid (D) to asparagine (N) and that change the 244th amino acid from aspartic acid (D) to asparagine (N) (Regnase-1_wt and Regnase-1_D226N,D244N). The above Regnase-1 was expressed in mammalian Expi293 cells, purified with FLAG M2 agarose and, if necessary, purified further with cOmplete His-Tag Purification Resin (Roche), and isolated by gel filtration chromatography.

### (Results)

The average of the results of duplicate measurements is shown as the RNA concentration. Compared with the D226N,D244N mutant having no RNA degrading activity, a decrease in RNA concentration was observed when Regnase-1 was added; therefore, RNA degradation dependent on Regnase-1 was confirmed in this evaluation system. In addition, when Regnase-1 and a compound were allowed to coexist, none of the compounds inhibited the RNA degradation activity by Regnase-1 (Fig. 30). Fig. 31 shows the effect of adding a compound to D226N,D244N. None of the compounds were shown to greatly affect RNA detection itself.

### Example 25. Binding of Regnase-1 and peptide compounds (PP1 to PP25) (Affinity mass analysis)

To a 96 PP plate (Greiner bio-one, 651201) coated with lipidure (Lipidure-CM5206) (NOF CORPORATION, 71S42000001), 2 µL of Tris-Buffer (25 mM Tris-NaOH pH 8.3, 300 mM NaCl, 1 mM DTT, 1 mM EDTA, 1% DMSO) was dispensed, and then 1 mM DMSO sample was added at 20 nL each with a LABCYTE ECHO (registered trademark) acoustic dispensing system. Furthermore, 18 µL of protein prepared at 1 µM FL_Reg1 in advance using Tris-Buffer was added (final compound concentration: 1 µM), and the mixture was incubated in the dark at 23°C for one hour. Then, to a 96-well, 400 µL, Hydrophilic PVDF 0.45 µm, Long drip (Harvard, 74-5556), 100 µL of P-2, Fine (Bio-Rad, 1504114) which had been swollen in advance was added, and conditioning was performed by centrifuging several times at 2000 x G for two minutes using the buffer. Next, 20 µL of the sample after incubation was added to the conditioned 96-well, 400 µL, Hydrophilic PVDF 0.45 µm, Long drip, and SEC treatment was performed by centrifugation at 2000 x G for two minutes. To the liquid from SEC, 32 µL of acetonitrile (LC/MS grade, FUJIFILM Wako Pure Chemical Industries, Ltd.) supplemented with 0.1% formic acid (LC/MS grade, 067-04531, FUJIFILM Wako Pure Chemical Industries, Ltd.) was added, and this was centrifuged at 1800 x G for five minutes, and the supernatant was used as an analysis sample for LC/MS measurement. The results are shown in Table 15. Binding to Regnase-1 was detected for all of the peptide compounds, PP1 to PP25.

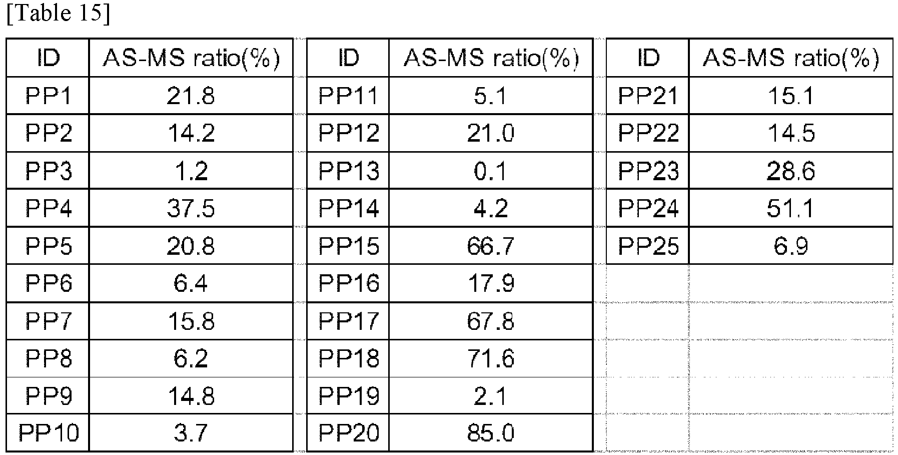

### Example 26. Evaluation of competitiveness by surface plasmon resonance

Evaluation of competitiveness between Regnase-1-binding molecules was analyzed by a method that uses Biacore T200 (GE healthcare). The binding of Regnase-1 was detected by adding a 3 µM Regnase-1 solution or a mixed solution of 3 µM Regnase-1 and 10 µM peptide compound as an analyte to ligand PP7+tag, PP10+tag, or PP23+tag captured *via* anti-TFPI-tag peptide antibody on Sensor chip Protein A (GE healthcare), and the competitiveness was evaluated by comparing the detection results. A 10 mM Glycine (pH 1.5) solution was used for regeneration of the sensor chip surface. The running buffer used was 10 mM Hepes-NaOH, 150 mM NaCl (Nacalai tesque), 5 mM DTT (Wako), 10 mM MgCl₂ (Wako), 0.05% Tween20 (Junsei-Kagaku), and 1% DMSO (Sigma-aldrich). All measurements were performed at 20°C, and the running buffer was used for analyte preparation.

The obtained binding response was processed by solvent correction and double-referencing using the Biacore T200 Evaluation Software (GE healthcare). The processed response was normalized by dividing by the amount of ligand immobilized. The "normalized response when adding a mixture of Regnase-1 and peptide compound" was divided by "the normalized response when adding Regnase-1 solution", and the thus calculated value was used as the index for competitiveness of each peptide compound. The competitiveness indices obtained as a result of the analyses are shown in Table 16. Peptide compounds having a competitiveness index of 0.8 or less were judged to compete with PP7+tag, PP10+tag, or PP23+tag. From the results, peptide compounds that compete with PP7+tag, peptide compounds that compete with PP10+tag, peptide compounds that compete with PP23+tag, and peptide compounds that compete with PP7+tag and PP23+tag but do not compete with PP10+tag were found.

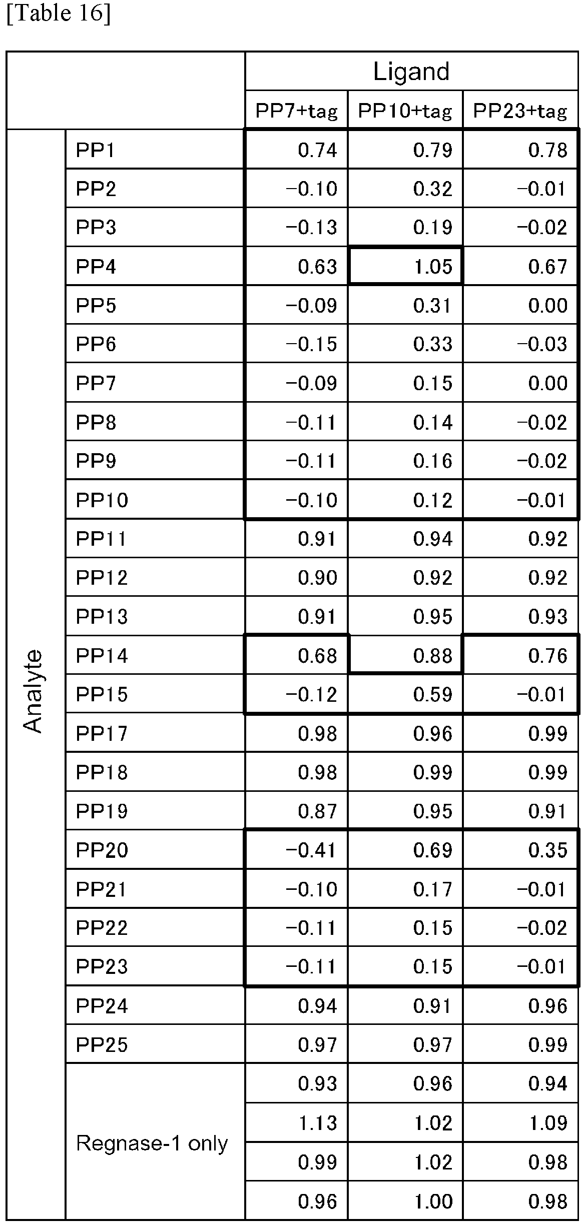

### Example 27. Production of anti-Regnase-1 antibodies

### (Preparation of peptide antigens)

The peptide CLDSGIGSLESQMSELWGVRGG (SEQ ID NO: 50), which consists of a sequence containing serine 438 and serine 442 of the full-length human Regnase-1 sequence and a cysteine residue attached at the end, and the peptide AFPPREYWSEPYPLPPPTC-NH₂ (SEQ ID NO: 51), which consists of a sequence containing serine 516 of the full-length human Regnase-1 sequence and a cysteine residue attached at the end, were synthesized. DMSO was added as necessary to the peptides to increase their solubility. KLH was conjugated to the cysteine residues using Imject Maleimide Activated mcKLH (Thermo Fisher scientific), and this was dialyzed against D-PBS.

### (Anti-non-phosphorylated Regnase-1 peptide antibodies)

The peptide CLDSGIGSLESQMSELWGVRGG, which consists of a sequence containing serine 438 and serine 442 of the full-length human Regnase-1 sequence and a cysteine residue attached at the end, and the peptide AFPPREYWSEPYPLPPPTC-NH₂, which consists of a sequence containing serine 516 of the full-length human Regnase-1 sequence and a cysteine residue attached at the end, were conjugated to KLH at the cysteine residue, and these proteins were mixed. Alternatively, the peptides CLDSGIGSLESQMSELWGVRGG and AFPPREYWSEPYPLPPPPPTC-NH2 were each conjugated to biotin and mixed with the streptavidin protein. Either protein mixture was administered to rabbits for immunization. The immunized rabbit cells were lysed and subjected to RT and PCR to amplify the antibody gene, and then the antibody gene was incorporated into a plasmid. The plasmid containing the antibody gene was introduced into *E. coli,* and the cells were cultured. The plasmid was purified from the cultured *E. coli.* The plasmid carrying the antibody gene was introduced into HEK293 cells, and the antibody was expressed in the culture supernatant. The antibody in the culture supernatant was purified by Protein A. The plasmid was sequenced, and the antibody sequence was determined with Sequencher Ver5 and Genetyx Verl4. Table 17 shows the names of the obtained anti-Regnase-1 antibodies and the heavy-chain and light-chain amino acid sequences.

**[Table 17]**

| Name of antibody | Heavy chain amino acid sequence | Light chain amino acid sequence |
|---|---|---|
| REA0023 | SEQ ID NO: **20** | SEQ ID NO: **21** |
| REA0027 | SEQ ID NO: **22** | SEQ ID NO: **23** |
| REB0007 | SEQ ID NO: **24** | SEQ ID NO: **25** |
| REB0014 | SEQ ID NO: **26** | SEQ ID NO: **27** |
| REB0022 | SEQ ID NO: **28** | SEQ ID NO: **29** |

### (Confirmation of binding of anti-non-phosphorylated Regnase-1 peptide antibody to peptide and full length human Regnase-1)

Biotin-conjugated FL_Reg1, biotin-conjugated peptide CLDSGIGSLESQMSELWGVRGG, biotin-conjugated peptide AFPPREYWSEPYPLPPPTC-NH₂, and biotinylated BSA (Sigma, A8549) were each bound to streptavidin-immobilized beads.

These beads were mixed with each anti-Regnase-1 antibody, and they were allowed to react. Thereafter, Anti-Rabbit IgG1 Alexa488 (molecular probes, A21244) was mixed and reacted, and analysis was performed with iQue screener PLUS (intellicyt). Data was analyzed with FlowJo ver10.

### (Results)

The binding of the anti-non-phosphorylated Regnase-1 peptide antibodies to biotinylated BSA, Peptide 1 (CLDSGIGSLESQMSELWGVRGG), Peptide 2 (AFPPREYWSEPYPLPPPTC-NH2), or FL_Reg1 are shown in histograms (Fig. 32). Binding to biotinylated BSA (dashed line), and binding to Peptide 1, Peptide 2, or FL_Reg1 (solid line) are overlaid. REA0023 and REA0027 bound more strongly to Peptide 1 and FL_Reg1 than to biotinylated BSA. REB0007, REB0014, and REB0022 bound more strongly to Peptide 2 and FL_Reg1 than to biotinylated BSA.

### Example 28. Evaluation of inhibitory activity against Regnase-1 phosphorylation of anti-Regnase-1 antibodies

### (Method)

To a solution (ATP buffer) containing Kinase Assay Buffer III (manufactured by SignalChem) supplemented with 2 mM DTT, 100 µM sodium vanadate, 2 mM manganese chloride, and 15 µM ATP, dephosphorylated Regnase-1 was mixed at 150 nM. Four microliters of this solution and 4 µL of HEPES Buffered Saline containing 50 µg/mL or 15 µg/mL of anti-Regnase-1 antibody were mixed, and this was allowed to react for one hour. This reaction solution was mixed with 4 µL of ATP buffer containing 150 nM of each kinase (IKKβ or TBK1), and further reacted for one hour. After the reaction, 4 µL of 4x Laemmli Sample buffer (manufactured by Bio-Rad) was added and this mixture was incubated at 95°C for five minutes for protein denaturation treatment. This sample was ice-cooled for three minutes, and 10 µL of it was added to SuperSep Phos-tag 7.5% 17 wells (manufactured by Wako) placed in Easy Separator (manufactured by Wako). As a marker, 10 µL of WIDE-VIEW Prestained Protein Size Marker III (manufactured by Wako) was added, and the EasySeparator was filled with Tris-Glycine SDS buffer (manufactured by Takara). After electrophoresis at 175 V for 70 minutes, this was washed with 10% Tris-Glycine SDS buffer containing 5 mM EDTA and 20% MeOH for 10 minutes. After two further washings, washing was performed with 10% Tris-Glycine SDS buffer containing 20% MeOH for ten minutes. The washed gel was placed in the Trans-Blot Turbo Transfer Pack (manufactured by Bio-Rad), and transferred using the BioRad Transfer Turbo (manufactured by Bio-Rad). The transferred PVDF membrane was washed with Tris Buffered Saline with Tween (registered trademark) 20 (TBS-T) (manufactured by Takara) and blocked by immersion in PVDF Blocking Reagent for Can Get Signal (registered trademark) (manufactured by TOYOBO) for 30 minutes. Thereafter, it was immersed in Can Get Signal_Solution. 1 (manufactured by TOYOBO) containing a 4000-fold diluted antibody against phosphorylated Regnase-1, and this was allowed to react overnight at 4°C. On the next day, the membrane was washed for 15 minutes with TBS-T three times, immersed in Can Get Signal_Solution.2 (manufactured by TOYOBO) containing 2000-fold diluted Anti-rabbit IgG, HRP-linked Antibody (manufactured by Cell Signaling Technology), and this was allowed to react at room temperature for one hour. After three 15-minute washes with TBS-T, Super Signal West Dura Extended Duration Substrate (manufactured by Thermo) was added to perform a peroxidase reaction. Chemiluminescence was detected using ImageQuant LAS4000 mini (manufactured by GE Healthcare).

### (Results)

A band for phosphorylated Regnase-1 was detected due to treatment with each kinase (Fig. 33A). Band densities were measured with Image Quant-TL (manufactured by GE Healthcare). The relative value for the band density of the sample to which each compound was added was calculated, considering the band density of the kinase-added sample of the Control group, to which no compound was added, as 1. When REA0023 and REA0027 were added at final concentrations of 16.7 µg/mL and 5.0 µg/mL, the density of the phosphorylated Regnase-1 band in IKKβ treatment was decreased (Fig. 33B). Similarly, when REB0007, REB0014, and REB0022 were added at final concentrations of 16.7 µg/mL and 5.0 µg/mL, the density of the phosphorylated Regnase-1 band in TBK1 treatment was decreased (Fig. 33C). These results indicate that the anti-Regnase-1 antibodies suppress the phosphorylation of Regnase-1 by kinase (IKKβ or TBK1).

### Example 29. Detection of RNA degradation activity by Regnase-1

As in the method described in Example 23, the effect of anti-Regnase-1 antibodies on the RNase activity were evaluated.

### (Cleavage reaction)

IL8 RNA and a Regnase-1 protein (FLAG_Regnase-1 or FLAG Regnase-1(D226N,D244N)) were mixed to a final concentration of 100 nM using a Tris-HCl (pH 7.5) buffer containing 0.75 mM DTT, 3.8 mM MgCl₂, 9.4 µM ZnCl₂, and 150 mM NaCl. By allowing the mixture to react at 37°C for one hour, RNA cleavage reaction by Regnase-1 was performed. For antibody evaluation, an antibody dissolved in HBS (HEPES-buffered saline) was added to the mixture to a final concentration of 30 µg/mL and 9.0 µg/mL, and these were allowed to react similarly.

### (RNA detection)

After completion of the reaction, the IL8 RNA concentration in the reaction solution was quantified by Taqman PCR method. Taqman PCR was performed according to QuantiTect Probe RT-PCR Kit (manufactured by QIAGEN) and analyzed with LightCycler 480 II (manufactured by Roche). The Taqman probe used is shown below.
IL8 (manufactured by Applied biosystems, Hs01553824_g1)

### (Results)

The RNA concentration is the average of the results of duplicate measurements. As shown in Fig. 34, the addition of Regnase-1 resulted in a decrease in the RNA concentration as compared to the D226N,D244N mutant, which had no RNA degradation activity, confirming Regnase-1-dependent RNA degradation in this evaluation system. In addition, when Regnase-1 and each anti-Regnase-1 antibody were allowed to coexist, none of the antibodies inhibited the RNA degradation activity by Regnase-1. Fig. 35 shows the effect of adding each compound to D226N,D244N. None of the antibodies were shown to significantly affect RNA detection itself.

Although the foregoing invention has been described in detail with examples and illustrations to help clear understanding, the descriptions and illustrations herein should not be construed as limiting the scope of the present invention. The disclosures of all patent and scientific documents cited herein are hereby expressly incorporated herein by reference in their entirety.

### [Industrial Applicability]

The present invention found that, for example, inhibiting phosphorylation of a Ser residue in Regnase-1 is effective in treating and/or preventing diseases. The invention also found that, for example, inhibiting the binding of Regnase-1 with at least one factor selected from the group consisting of TBK1, IKKi, Act-1, IKK, and IRAK is effective in treating and/or preventing diseases. The present invention is useful in the field of treatment and/or prevention of diseases associated with Regnase-1.

## Claims

1. A composition for treating and/or preventing a disease associated with Regnase-1, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.

2. A composition for treating and/or preventing a disease associated with Regnase-1, comprising a Regnase-1-binding molecule that inhibits binding between Regnase-1 and at least one binding molecule selected from the group consisting of TBK1, IKKi, Act-1, IKK, and IRAK.

3. The composition of claim 1 or 2, wherein the disease associated with Regnase-1 is at least one disease selected from the group consisting of an inflammatory disease, autoimmune disease, allergic disease, fibrotic disease, and RNA virus infection.

4. The composition of any one of claims 1 to 3, wherein the disease associated with Regnase-1 is a disease the formation, exacerbation, and/or continuation of which are associated with at least one factor selected from the group consisting of IL-17, IL-1, IL-36, and a TLR ligand.

5. The composition of any one of claims 1 to 4, wherein the disease associated with Regnase-1 is a disease the formation, exacerbation, and/or continuation of which are associated with at least one factor selected from the group consisting of IL6, IL1a, CXCL1, CXCL2, HBEGF, CTGF, DDR1, and PDGFB.

6. A composition for suppressing fibrosis of a cell, tissue, or organ, or epithelial hyperplasia, comprising a Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.

7. A composition for suppressing expression of at least one mRNA selected from the group consisting of IL6, ILla, CXCL1, CXCL2, HBEGF, CTGF, DDR1, and PDGFB, comprising a Regnase-1-binding molecule that inhibits phosphorylation of at least one Ser residue selected from the group consisting of positions corresponding respectively to positions 513, 494, 439, and 435 of SEQ ID NO: 1 in Regnase-1.

8. The composition of any one of claims 1 and 3 to 7, wherein the positions corresponding respectively to positions 513, 494, 439, and 435 of SEQ ID NO: 1 are (i) positions 513, 494, 439, and 435 of SEQ ID NO: 1; or (ii) positions 516, 497, 442, and 438 of SEQ ID NO: 2.

9. A method of identifying a substance that inhibits phosphorylation of Regnase-1, wherein phosphorylation of at least one Ser residue selected from the group consisting of positions corresponding respectively to positions 513, 494, 439, and 435 of SEQ ID NO: 1 in Regnase-1 is used as an index.

10. The method of claim 9, comprising the steps of (a) and (b) below:
(a) mixing a kinase capable of phosphorylating Regnase-1 with Regnase-1 in the presence of a test substance and detecting phosphorylation of Regnase-1 by the kinase;
(b) identifying a substance that inhibits the phosphorylation of Regnase-1 by the kinase as compared to the absence of the test substance.

11. The method of claim 10, wherein the kinase is at least one kinase selected from the group consisting of TBK1, IKKi, IKK, and IRAK.

12. A composition for identifying a substance that inhibits phosphorylation of Regnase-1, wherein the composition comprises a predetermined amount of a kinase and Regnase-1.

13. A method for identifying a substance that inhibits binding between Regnase-1 and at least one binding molecule selected from the group consisting of TBK1, IKKi, Act-1, IKK, and IRAK, comprising the steps of (c) and (d) below:
(c) mixing at least one binding molecule selected from the group consisting of TBK1, IKKi, Act-1, IKK, and IRAK with Regnase-1 in the presence of a test substance and measuring the binding activity between the binding molecule and Regnase-1;
(d) identifying a substance capable of reducing the binding activity between the binding molecule and Regnase-1 as compared to the absence of the test substance.

14. An antibody that specifically recognizes Regnase-1 with a phosphorylated amino acid residue at at least one position selected from the group consisting of positions corresponding respectively to positions 513, 494, 439, and 435 of SEQ ID NO: 1.

15. A Regnase-1-binding molecule that inhibits phosphorylation of a Ser residue at at least one position selected from the group consisting of positions corresponding respectively to positions 513, 494, 439, and 435 of SEQ ID NO: 1 in Regnase-1.

16. The Regnase-1-binding molecule of claim 15, wherein the molecule competes for binding to Regnase-1 with at least one compound selected from PP1 to PP25 below:

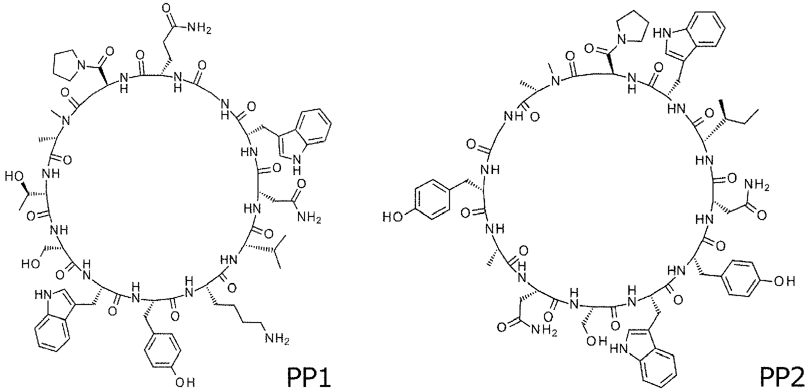

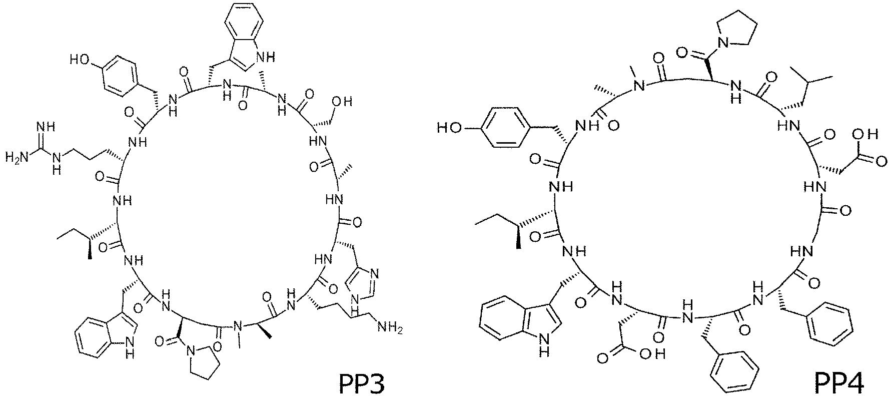

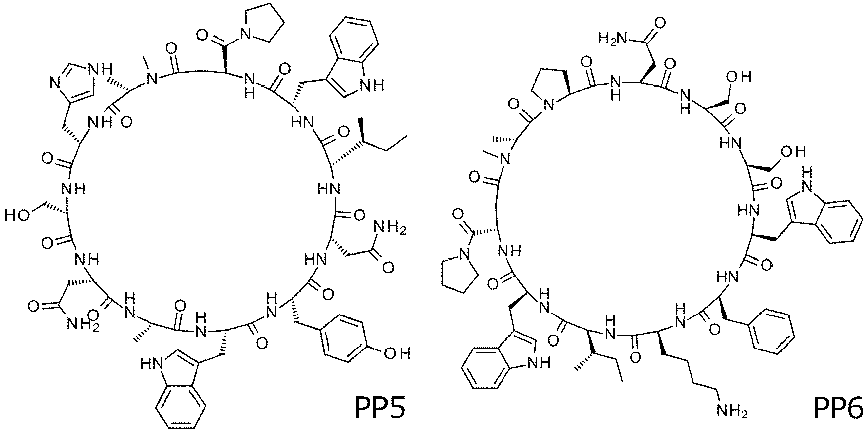

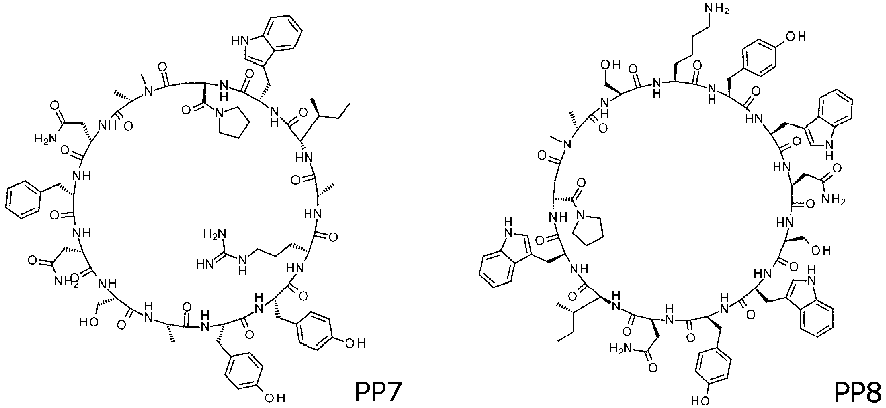

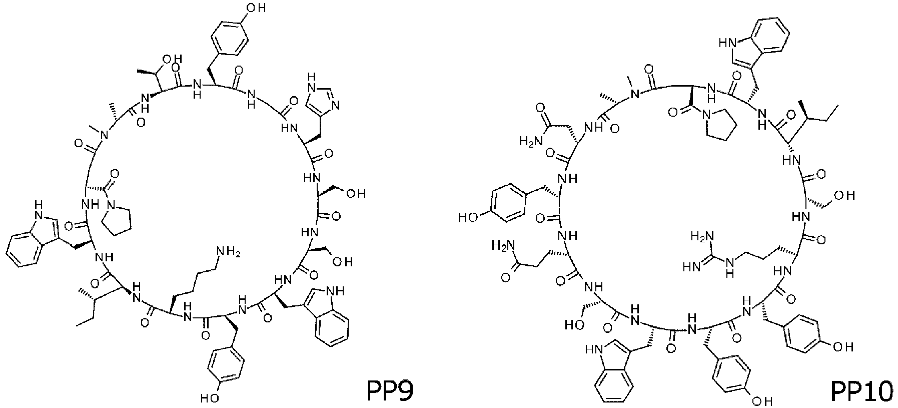

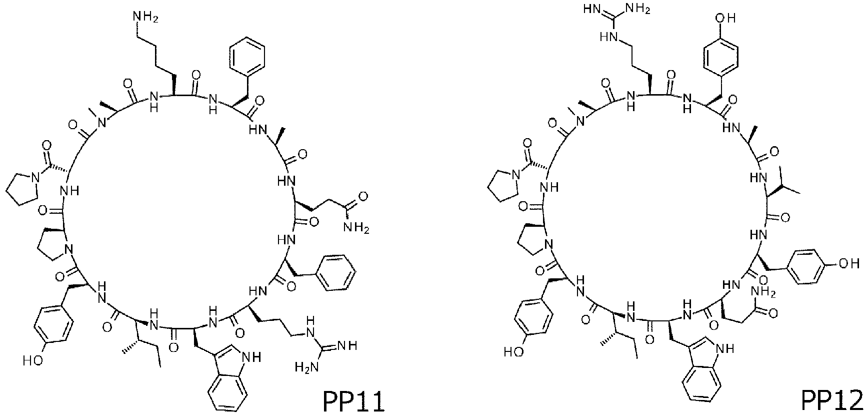

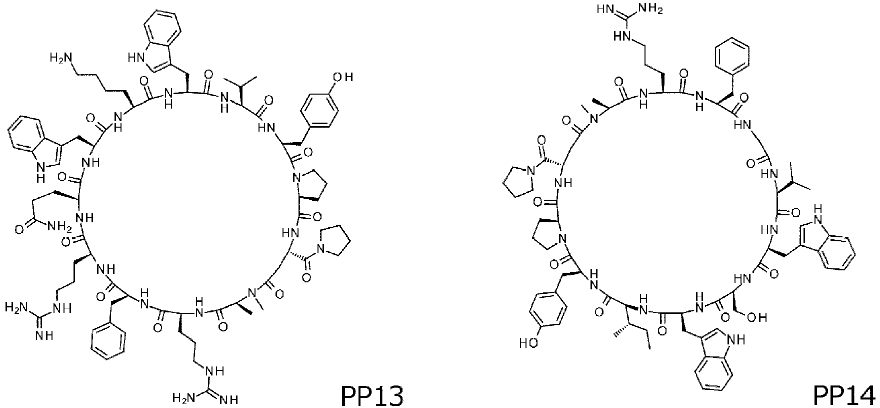

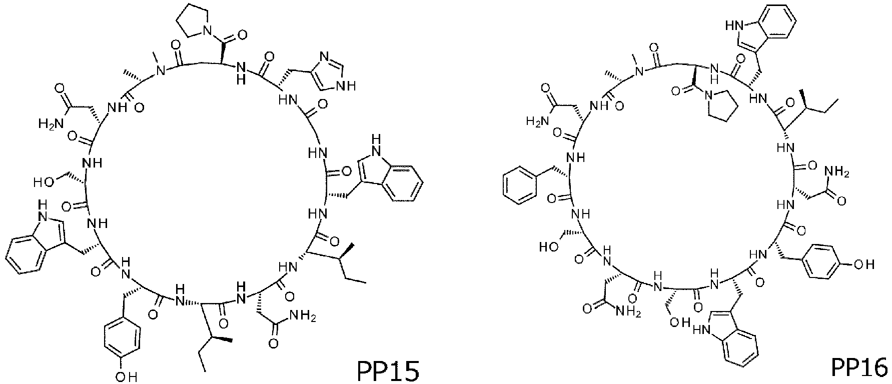

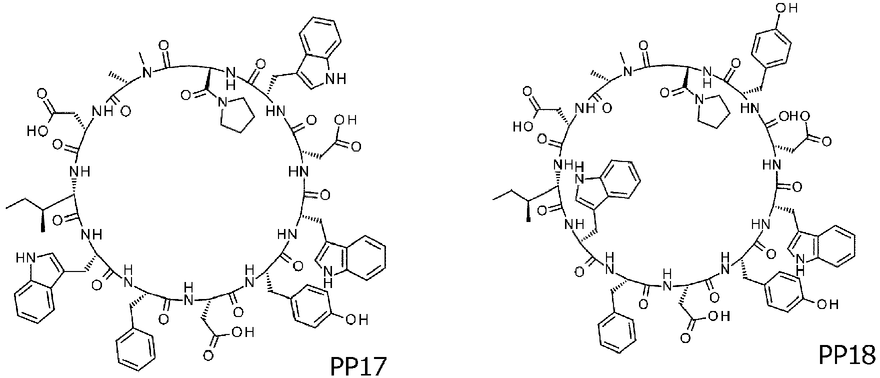

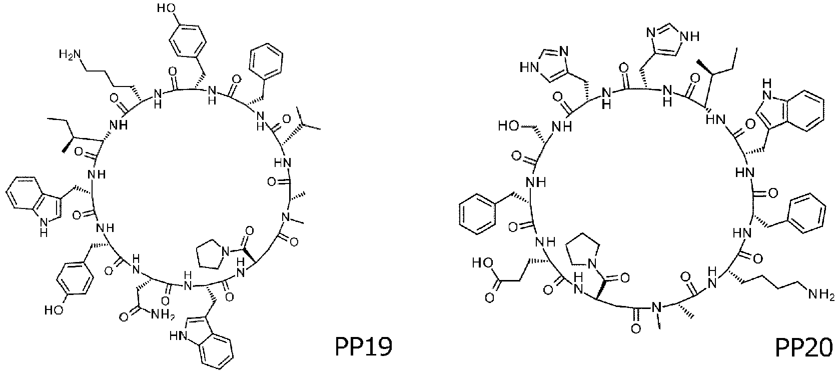

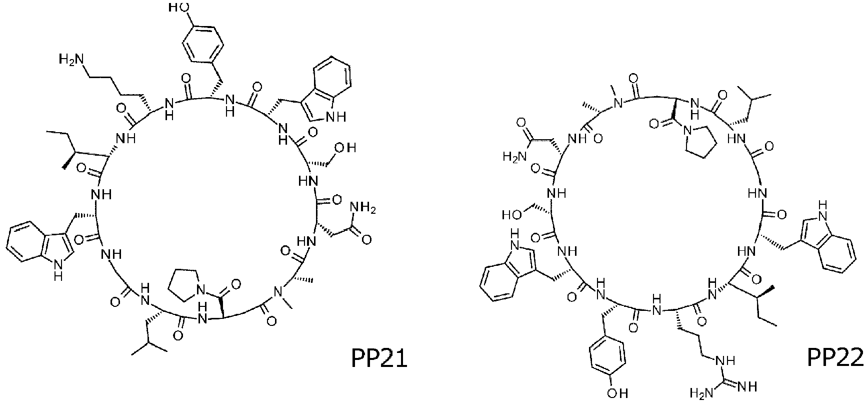

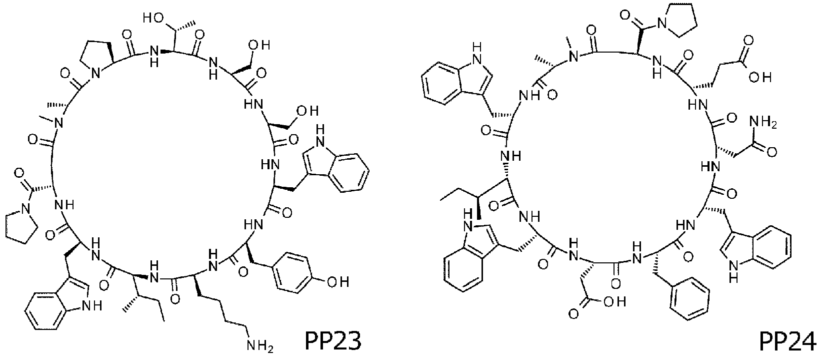

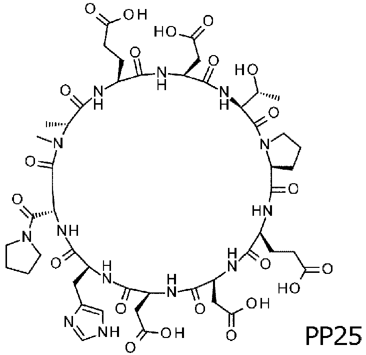

17. The Regnase-1-binding molecule of claim 15 or 16, wherein the molecule inhibits phosphorylation of a Ser residue in (i) and (ii) below:
(i) a Ser residue at either or both positions corresponding respectively to positions 513 and 494 of SEQ ID NO: 1 in Regnase-1; and
(ii) a Ser residue at either or both positions corresponding respectively to positions 439 and 435 of SEQ ID NO: 1 in Regnase-1.


SEQUENCE LISTING

<110>  OSAKA UNIVERSITY
       CHUGAI SEIYAKU KABUSHIKI KAISHA

<120>  Methods of treating and/or preventing Regnase-1 associated
       diseases

<130>  C1X1727Y3P

<150>  JP 2018-108400
<151>  2018-06-06

<150>  JP 2018-167326
<151>  2018-09-06

<150>  JP 2018-229845
<151>  2018-12-07

<150>  JP 2019-089270
<151>  2019-05-09

<160>  62

<170>  PatentIn version 3.5

<210>  1
<211>  596
<212>  PRT
<213>  Mus musculus

<400>  1
   Met Ser Asp Pro Cys Gly Thr Lys Pro Val Gln Glu Ser Asn Pro Thr
   1               5                   10                  15
   Met Ser Leu Trp Ser Leu Glu Asp Arg His Ser Ser Gln Gly Arg Pro
               20                  25                  30
   Gln Pro Asp Gln Asp Pro Val Ala Lys Glu Ala Pro Thr Ser Glu Leu
           35                  40                  45
   Gln Met Lys Val Asp Phe Phe Arg Lys Leu Gly Tyr Ser Ser Ser Glu
       50                  55                  60
   Ile His Ser Val Leu Gln Lys Leu Gly Val Gln Ala Asp Thr Asn Thr
   65                  70                  75                  80
   Val Leu Gly Glu Leu Val Lys His Gly Ser Ala Thr Glu Arg Glu Cys
                   85                  90                  95
   Gln Ala Leu Thr Ala Pro Ser Pro Gln Pro Pro Leu Val Pro Arg Gly
               100                 105                 110
   Gly Ser Thr Pro Lys Pro Ser Thr Leu Glu Pro Ser Leu Pro Glu Glu
           115                 120                 125
   Asp Arg Glu Gly Ser Asp Leu Arg Pro Val Val Ile Asp Gly Ser Asn
       130                 135                 140
   Val Ala Met Ser His Gly Asn Lys Glu Val Phe Ser Cys Arg Gly Ile
   145                 150                 155                 160
   Leu Leu Ala Val Asn Trp Phe Leu Glu Arg Gly His Thr Asp Ile Thr
                   165                 170                 175
   Val Phe Val Pro Ser Trp Arg Lys Glu Gln Pro Arg Pro Asp Val Pro
               180                 185                 190
   Ile Thr Asp Gln His Ile Leu Arg Glu Leu Glu Lys Lys Lys Ile Leu
           195                 200                 205
   Val Phe Thr Pro Ser Arg Arg Val Gly Gly Lys Arg Val Val Cys Tyr
       210                 215                 220
   Asp Asp Arg Phe Ile Val Lys Leu Ala Phe Glu Ser Asp Gly Val Val
   225                 230                 235                 240
   Val Ser Asn Asp Thr Tyr Arg Asp Leu Gln Gly Glu Arg Gln Glu Trp
                   245                 250                 255
   Lys Arg Phe Ile Glu Glu Arg Leu Leu Met Tyr Ser Phe Val Asn Asp
               260                 265                 270
   Lys Phe Met Pro Pro Asp Asp Pro Leu Gly Arg His Gly Pro Ser Leu
           275                 280                 285
   Asp Asn Phe Leu Arg Lys Lys Pro Leu Pro Ser Glu His Arg Lys Gln
       290                 295                 300
   Pro Cys Pro Tyr Gly Lys Lys Cys Thr Tyr Gly Ile Lys Cys Arg Phe
   305                 310                 315                 320
   Phe His Pro Glu Arg Pro Ser Arg Pro Gln Arg Ser Val Ala Asp Glu
                   325                 330                 335
   Leu Arg Ala Asn Ala Leu Leu Ser Pro Pro Arg Thr Pro Val Lys Asp
               340                 345                 350
   Lys Ser Ser Gln Arg Pro Ser Pro Ala Ser Gln Ser Ser Ser Val Ser
           355                 360                 365
   Leu Glu Ala Glu Pro Gly Ser Leu Asp Gly Lys Lys Leu Gly Ala Arg
   370                 375                 380
   Ser Ser Pro Gly Pro His Arg Glu Gly Ser Pro Gln Thr Cys Ala Pro
   385                 390                 395                 400
   Ala Gly Arg Ser Leu Pro Val Ser Gly Gly Ser Phe Gly Pro Thr Glu
                   405                 410                 415
   Trp Leu Ala His Thr Gln Asp Ser Leu Pro Tyr Thr Ser Gln Glu Cys
               420                 425                 430
   Leu Asp Ser Gly Ile Gly Ser Leu Glu Ser Gln Met Ser Glu Leu Trp
           435                 440                 445
   Gly Val Arg Gly Gly Ser Pro Gly Glu Ser Gly Pro Thr Arg Gly Pro
       450                 455                 460
   Tyr Ala Gly Tyr His Ser Tyr Gly Ser Lys Val Pro Ala Ala Pro Ser
   465                 470                 475                 480
   Phe Ser Pro Phe Arg Pro Ala Met Gly Ala Gly His Phe Ser Val Pro
                   485                 490                 495
   Thr Asp Tyr Val Pro Pro Pro Pro Thr Tyr Pro Ser Arg Glu Tyr Trp
               500                 505                 510
   Ser Glu Pro Tyr Pro Leu Pro Pro Pro Thr Pro Val Leu Gln Glu Pro
           515                 520                 525
   Gln Arg Pro Ser Pro Gly Ala Gly Gly Gly Pro Trp Gly Arg Val Gly
       530                 535                 540
   Asp Leu Ala Lys Glu Arg Ala Gly Val Tyr Thr Lys Leu Cys Gly Val
   545                 550                 555                 560
   Phe Pro Pro His Leu Val Glu Ala Val Met Arg Arg Phe Pro Gln Leu
                   565                 570                 575
   Leu Asp Pro Gln Gln Leu Ala Ala Glu Ile Leu Ser Tyr Lys Ser Gln
               580                 585                 590
   His Leu Ser Glu
           595

<210>  2
<211>  599
<212>  PRT
<213>  Homo sapiens

<400>  2

Met Ser Gly Pro Cys Gly Glu Lys Pro Val Leu Glu Ala Ser Pro Thr
1               5                   10                  15

Met Ser Leu Trp Glu Phe Glu Asp Ser His Ser Arg Gln Gly Thr Pro
            20                  25                  30

Arg Pro Gly Gln Glu Leu Ala Ala Glu Glu Ala Ser Ala Leu Glu Leu
        35                  40                  45

Gln Met Lys Val Asp Phe Phe Arg Lys Leu Gly Tyr Ser Ser Thr Glu
    50                  55                  60

Ile His Ser Val Leu Gln Lys Leu Gly Val Gln Ala Asp Thr Asn Thr
65                  70                  75                  80

Val Leu Gly Glu Leu Val Lys His Gly Thr Ala Thr Glu Arg Glu Arg
                85                  90                  95

Gln Thr Ser Pro Asp Pro Cys Pro Gln Leu Pro Leu Val Pro Arg Gly
            100                 105                 110

Gly Gly Thr Pro Lys Ala Pro Asn Leu Glu Pro Pro Leu Pro Glu Glu
        115                 120                 125

Glu Lys Glu Gly Ser Asp Leu Arg Pro Val Val Ile Asp Gly Ser Asn
    130                 135                 140

Val Ala Met Ser His Gly Asn Lys Glu Val Phe Ser Cys Arg Gly Ile
145                 150                 155                 160

Leu Leu Ala Val Asn Trp Phe Leu Glu Arg Gly His Thr Asp Ile Thr
                165                 170                 175

Val Phe Val Pro Ser Trp Arg Lys Glu Gln Pro Arg Pro Asp Val Pro
            180                 185                 190

Ile Thr Asp Gln His Ile Leu Arg Glu Leu Glu Lys Lys Lys Ile Leu
        195                 200                 205

Val Phe Thr Pro Ser Arg Arg Val Gly Gly Lys Arg Val Val Cys Tyr
    210                 215                 220

Asp Asp Arg Phe Ile Val Lys Leu Ala Tyr Glu Ser Asp Gly Ile Val
225                 230                 235                 240

Val Ser Asn Asp Thr Tyr Arg Asp Leu Gln Gly Glu Arg Gln Glu Trp
                245                 250                 255

Lys Arg Phe Ile Glu Glu Arg Leu Leu Met Tyr Ser Phe Val Asn Asp
            260                 265                 270

Lys Phe Met Pro Pro Asp Asp Pro Leu Gly Arg His Gly Pro Ser Leu
        275                 280                 285

Asp Asn Phe Leu Arg Lys Lys Pro Leu Thr Leu Glu His Arg Lys Gln
    290                 295                 300

Pro Cys Pro Tyr Gly Arg Lys Cys Thr Tyr Gly Ile Lys Cys Arg Phe
305                 310                 315                 320

Phe His Pro Glu Arg Pro Ser Cys Pro Gln Arg Ser Val Ala Asp Glu
                325                 330                 335

Leu Arg Ala Asn Ala Leu Leu Ser Pro Pro Arg Ala Pro Ser Lys Asp
            340                 345                 350

Lys Asn Gly Arg Arg Pro Ser Pro Ser Ser Gln Ser Ser Ser Leu Leu
        355                 360                 365

Thr Glu Ser Glu Gln Cys Ser Leu Asp Gly Lys Lys Leu Gly Ala Gln
    370                 375                 380

Ala Ser Pro Gly Ser Arg Gln Glu Gly Leu Thr Gln Thr Tyr Ala Pro
385                 390                 395                 400

Ser Gly Arg Ser Leu Ala Pro Ser Gly Gly Ser Gly Ser Ser Phe Gly
                405                 410                 415

Pro Thr Asp Trp Leu Pro Gln Thr Leu Asp Ser Leu Pro Tyr Val Ser
            420                 425                 430

Gln Asp Cys Leu Asp Ser Gly Ile Gly Ser Leu Glu Ser Gln Met Ser
        435                 440                 445

Glu Leu Trp Gly Val Arg Gly Gly Gly Pro Gly Glu Pro Gly Pro Pro
    450                 455                 460

Arg Ala Pro Tyr Thr Gly Tyr Ser Pro Tyr Gly Ser Glu Leu Pro Ala
465                 470                 475                 480

Thr Ala Ala Phe Ser Ala Phe Gly Arg Ala Met Gly Ala Gly His Phe
                485                 490                 495

Ser Val Pro Ala Asp Tyr Pro Pro Ala Pro Pro Ala Phe Pro Pro Arg
                500                 505                 510

Glu Tyr Trp Ser Glu Pro Tyr Pro Leu Pro Pro Pro Thr Ser Val Leu
        515                 520                 525

Gln Glu Pro Pro Val Gln Ser Pro Gly Ala Gly Arg Ser Pro Trp Gly
    530                 535                 540

Arg Ala Gly Ser Leu Ala Lys Glu Gln Ala Ser Val Tyr Thr Lys Leu
545                 550                 555                 560

Cys Gly Val Phe Pro Pro His Leu Val Glu Ala Val Met Gly Arg Phe
                565                 570                 575

Pro Gln Leu Leu Asp Pro Gln Gln Leu Ala Ala Glu Ile Leu Ser Tyr
            580                 585                 590

Lys Ser Gln His Pro Ser Glu
        595

<210>  3
<211>  5
<212>  PRT
<213>  Homo sapiens

<400>  3

Tyr Trp Ser Glu Pro
1               5

<210>  4
<211>  5
<212>  PRT
<213>  Homo sapiens

<400>  4

His Phe Ser Val Pro
1               5

<210>  5
<211>  6
<212>  PRT
<213>  Homo sapiens

<400>  5

Asp Ser Gly Ile Gly Ser
1               5

<210>  6

<211>  15
<212>  PRT
<213>  Mus musculus

<220>
<221>  MOD_RES
<222>  (3)..(3)
<223>  PHOSPHORYLATION

<220>
<221>  MOD_RES
<222>  (7)..(7)
<223>  PHOSPHORYLATION

<400>  6

Leu Asp Ser Gly Ile Gly Ser Leu Glu Ser Gln Met Ser Glu Cys
1               5                   10                  15

<210>  7
<211>  13
<212>  PRT
<213>  Mus musculus

<220>
<221>  MOD_RES
<222>  (10)..(10)
<223>  PHOSPHORYLATION

<400>  7

Cys Thr Tyr Pro Ser Arg Glu Tyr Trp Ser Glu Pro Tyr
1               5                   10

<210>  8
<211>  14
<212>  PRT
<213>  Mus musculus

<400>  8

Leu Asp Ser Gly Ile Gly Ser Leu Glu Ser Gln Met Ser Glu
1               5                   10

<210>  9
<211>  8
<212>  PRT
<213>  Mus musculus

<400>  9

Arg Glu Tyr Trp Ser Glu Pro Tyr
1               5

<210>  10
<211>  13
<212>  PRT

<213>  Human immunodeficiency virus 1

<400>  10

Gly Arg Lys Lys Arg Arg Gln Arg Arg Arg Pro Pro Gln
1               5                   10


<210>  11
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  11

Xaa Thr Ser Trp Tyr Lys Val Asn Trp Gly Gln Asp
1               5                   10


<210>  12
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  12

Xaa Gly Tyr Ala Asn Ser Trp Tyr Asn Ile Trp Asp
1               5                   10


<210>  13
<211>  12
<212>  PRT
<213>  Artificial Sequence

<220>
<223>  An artificially synthesized sequence


<220>
<221>  MISC_FEATURE
<222>  (1)..(1)
<223>  Xaa = D-MeAla

<400>  13

Xaa Lys His Ala Ser Ala Trp Tyr Arg Ile Trp Asp
1               5                   10

<210>  14
<211>  11
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  14

Xaa Tyr Ile Trp Asp Phe Phe Gly Asp Leu Asp
1               5                   10

<210>  15
<211>  11
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  15

Xaa His Ser Asn Ala Trp Tyr Asn Ile Trp Asp
1               5                   10

<210>  16
<211>  11
<212>  PRT
<213>  Artificial Sequence

<220>
<223>  An artificially synthesized sequence

<220>
<221>  MISC_FEATURE
<222>  (1)..(1)
<223>  Xaa = D-MeAla

<400>  16

Xaa Pro Asn Ser Ser Trp Phe Lys Ile Trp Asp
1               5                   10

<210>  17
<211>  20
<212>  DNA
<213>  Artificial Sequence

<220>
<223>  An artificially synthesized primer sequence

<400>  17
taatacgact cactataggg                                                   20


<210>  18
<211>  20
<212>  DNA
<213>  Artificial Sequence

<220>
<223>  An artificially synthesized primer sequence

<400>  18
gaaaatttaa ctgggtaccc                                                   20


<210>  19
<211>  20
<212>  PRT
<213>  Artificial Sequence

<220>
<223>  An artificially synthesized sequence

<400>  19

Leu Ala Gln Gly Ala Tyr Arg Thr Ala Val Asp Leu Glu Ser Leu Ala
1               5                   10                  15


Ser Gln Leu Thr
            20


<210>  20
<211>  436
<212>  PRT
<213>  Artificial Sequence

<220>
<223>  An artificially synthesized sequence

<400>  20

Gln Ser Leu Glu Glu Ser Gly Gly Asp Leu Val Lys Pro Gly Ala Ser
1               5                   10                  15


Leu Thr Leu Thr Cys Thr Asp Ala Gly Phe Ser Phe Ser Gly Val Tyr
            20                  25                  30


Trp Met His Trp Val Arg Gln Ala Pro Gly Lys Gly Leu Glu Trp Ile

        35                  40                  45

Ala Cys Ile Asn Ala Gly Ser Ser Gly Ser Thr Tyr Tyr Ala Ser Trp
    50                  55                  60

Ala Lys Gly Arg Phe Thr Ile Ser Lys Pro Ser Ser Thr Thr Val Thr
65                  70                  75                  80

Leu Gln Met Thr Ser Leu Thr Val Ala Asp Thr Ala Thr Tyr Phe Cys
                85                  90                  95

Ala Arg Ser Phe Thr Leu Trp Gly Pro Gly Thr Leu Val Thr Val Ser
            100                 105                 110

Ser Gly Gln Pro Lys Ala Pro Ser Val Phe Pro Leu Ala Pro Cys Cys
        115                 120                 125

Gly Asp Thr Pro Ser Ser Thr Val Thr Leu Gly Cys Leu Val Lys Gly
    130                 135                 140

Tyr Leu Pro Glu Pro Val Thr Val Thr Trp Asn Ser Gly Thr Leu Thr
145                 150                 155                 160

Asn Gly Val Arg Thr Phe Pro Ser Val Arg Gln Ser Ser Gly Leu Tyr
                165                 170                 175

Ser Leu Ser Ser Val Val Ser Val Thr Ser Ser Ser Gln Pro Val Thr
            180                 185                 190

Cys Asn Val Ala His Pro Ala Thr Asn Thr Lys Val Asp Lys Thr Val
        195                 200                 205

Ala Pro Ser Thr Cys Ser Lys Pro Thr Cys Pro Pro Pro Glu Leu Leu
    210                 215                 220

Gly Gly Pro Ser Val Phe Ile Phe Pro Pro Lys Pro Lys Asp Thr Leu
225                 230                 235                 240

Met Ile Ser Arg Thr Pro Glu Val Thr Cys Val Val Val Asp Val Ser
                245                 250                 255

Gln Asp Asp Pro Glu Val Gln Phe Thr Trp Tyr Ile Asn Asn Glu Gln
            260                 265                 270

Val Arg Thr Ala Arg Pro Pro Leu Arg Glu Gln Gln Phe Asn Ser Thr
        275                 280                 285

Ile Arg Val Val Ser Thr Leu Pro Ile Thr His Gln Asp Trp Leu Arg
    290                 295                 300

Gly Lys Glu Phe Lys Cys Lys Val His Asn Lys Ala Leu Pro Ala Pro
305                 310                 315                 320

Ile Glu Lys Thr Ile Ser Lys Ala Arg Gly Gln Pro Leu Glu Pro Lys
                325                 330                 335

Val Tyr Thr Met Gly Pro Pro Arg Glu Glu Leu Ser Ser Arg Ser Val
            340                 345                 350

Ser Leu Thr Cys Met Ile Asn Gly Phe Tyr Pro Ser Asp Ile Ser Val
        355                 360                 365

Glu Trp Glu Lys Asn Gly Lys Ala Glu Asp Asn Tyr Lys Thr Thr Pro
    370                 375                 380

Ala Val Leu Asp Ser Asp Gly Ser Tyr Phe Leu Tyr Asn Lys Leu Ser
385                 390                 395                 400

Val Pro Thr Ser Glu Trp Gln Arg Gly Asp Val Phe Thr Cys Ser Val
                405                 410                 415

Met His Glu Ala Leu His Asn His Tyr Thr Gln Lys Ser Ile Ser Arg
            420                 425                 430

Ser Pro Gly Lys
        435

<210>  21
<211>  216
<212>  PRT
<213>  Artificial Sequence

<220>
<223>  An artificially synthesized sequence

<400>  21

Ala Val Val Thr Gln Thr Pro Ser Ser Val Ser Ala Ala Val Gly Gly
1               5                   10                  15

Thr Val Thr Ile Lys Cys Gln Ser Ser Gln Ser Val Gly Asn Asn Asn
            20                  25                  30

Tyr Leu Ala Trp Phe Gln Gln Lys Pro Gly Gln Pro Pro Lys Leu Leu
        35                  40                  45

Ile Tyr Ser Ala Ser Thr Leu Ala Ser Gly Val Pro Ser Arg Phe Ser

50                  55                  60

Gly Ser Gly Ser Gly Thr Gln Phe Thr Leu Thr Ile Ser Gly Val Gln
65                  70                  75                  80

Cys Asp Asp Ala Ala Thr Tyr Tyr Cys Leu Gly Gly Tyr Asp Gly Asp
                85                  90                  95

Gly Glu Ala Phe Tyr Val Phe Gly Gly Gly Thr Glu Val Val Val Lys
            100                 105                 110

Gly Asp Pro Val Ala Pro Thr Val Leu Ile Phe Pro Pro Ala Ala Asp
        115                 120                 125

Gln Val Ala Thr Gly Thr Val Thr Ile Val Cys Val Ala Asn Lys Tyr
    130                 135                 140

Phe Pro Asp Val Thr Val Thr Trp Glu Val Asp Gly Thr Thr Gln Thr
145                 150                 155                 160

Thr Gly Ile Glu Asn Ser Lys Thr Pro Gln Asn Ser Ala Asp Cys Thr
                165                 170                 175

Tyr Asn Leu Ser Ser Thr Leu Thr Leu Thr Ser Thr Gln Tyr Asn Ser
            180                 185                 190

His Lys Glu Tyr Thr Cys Lys Val Thr Gln Gly Thr Thr Ser Val Val
        195                 200                 205

Gln Ser Phe Asn Arg Gly Asp Cys
    210                 215

<210>  22
<211>  436
<212>  PRT
<213>  Artificial Sequence

<220>
<223>  An artificially synthesized sequence

<400>  22

Gln Ser Leu Glu Glu Ser Gly Gly Asp Leu Val Lys Pro Gly Ala Ser
1               5                   10                  15

Leu Thr Leu Thr Cys Thr Ala Ser Gly Phe Gly Phe Ser Ser Ser Tyr
            20                  25                  30

Trp Met His Trp Val Arg Gln Ala Pro Gly Lys Gly Leu Glu Trp Ile
        35                  40                  45

Ala Cys Ile Asn Ala Gly Ser Ser Thr Lys Thr Tyr Tyr Ala Ser Trp
    50                  55                  60

Ala Lys Gly Arg Phe Thr Ile Ser Lys Pro Ser Ser Thr Thr Val Thr
65                  70                  75                  80

Leu Gln Met Thr Ser Leu Thr Val Ala Asp Thr Ala Thr Tyr Phe Cys
                85                  90                  95

Ala Trp Ser Phe Asn Leu Trp Gly Pro Gly Thr Leu Val Thr Val Ser
            100                 105                 110

Ser Gly Gln Pro Lys Ala Pro Ser Val Phe Pro Leu Ala Pro Cys Cys
        115                 120                 125

Gly Asp Thr Pro Ser Ser Thr Val Thr Leu Gly Cys Leu Val Lys Gly
    130                 135                 140

Tyr Leu Pro Glu Pro Val Thr Val Thr Trp Asn Ser Gly Thr Leu Thr
145                 150                 155                 160

Asn Gly Val Arg Thr Phe Pro Ser Val Arg Gln Ser Ser Gly Leu Tyr
                165                 170                 175

Ser Leu Ser Ser Val Val Ser Val Thr Ser Ser Ser Gln Pro Val Thr
            180                 185                 190

Cys Asn Val Ala His Pro Ala Thr Asn Thr Lys Val Asp Lys Thr Val
        195                 200                 205

Ala Pro Ser Thr Cys Ser Lys Pro Thr Cys Pro Pro Pro Glu Leu Leu
    210                 215                 220

Gly Gly Pro Ser Val Phe Ile Phe Pro Pro Lys Pro Lys Asp Thr Leu
225                 230                 235                 240

Met Ile Ser Arg Thr Pro Glu Val Thr Cys Val Val Val Asp Val Ser
                245                 250                 255

Gln Asp Asp Pro Glu Val Gln Phe Thr Trp Tyr Ile Asn Asn Glu Gln
            260                 265                 270

Val Arg Thr Ala Arg Pro Pro Leu Arg Glu Gln Gln Phe Asn Ser Thr
        275                 280                 285

Ile Arg Val Val Ser Thr Leu Pro Ile Thr His Gln Asp Trp Leu Arg

290                 295                 300

Gly Lys Glu Phe Lys Cys Lys Val His Asn Lys Ala Leu Pro Ala Pro
305                 310                 315                 320

Ile Glu Lys Thr Ile Ser Lys Ala Arg Gly Gln Pro Leu Glu Pro Lys
                325                 330                 335

Val Tyr Thr Met Gly Pro Pro Arg Glu Glu Leu Ser Ser Arg Ser Val
            340                 345                 350

Ser Leu Thr Cys Met Ile Asn Gly Phe Tyr Pro Ser Asp Ile Ser Val
        355                 360                 365

Glu Trp Glu Lys Asn Gly Lys Ala Glu Asp Asn Tyr Lys Thr Thr Pro
    370                 375                 380

Ala Val Leu Asp Ser Asp Gly Ser Tyr Phe Leu Tyr Asn Lys Leu Ser
385                 390                 395                 400

Val Pro Thr Ser Glu Trp Gln Arg Gly Asp Val Phe Thr Cys Ser Val
                405                 410                 415

Met His Glu Ala Leu His Asn His Tyr Thr Gln Lys Ser Ile Ser Arg
            420                 425                 430

Ser Pro Gly Lys
        435

<210>  23
<211>  216
<212>  PRT
<213>  Artificial Sequence

<220>
<223>  An artificially synthesized sequence

<400>  23

Ala Val Val Thr Gln Thr Pro Ser Ser Val Ser Ala Ala Val Gly Gly
1               5                   10                  15

Thr Val Thr Ile Lys Cys Gln Ser Ser Gln Ser Val Gly Asn Asn Asn
            20                  25                  30

Trp Leu Ala Trp Phe Gln Gln Lys Pro Gly Gln Pro Pro Lys Leu Leu
        35                  40                  45

Ile Tyr Ser Ala Ser Thr Leu Ala Ser Gly Val Pro Ser Arg Phe Ser
    50                  55                  60

Gly Ser Gly Ser Gly Thr Gln Phe Thr Leu Thr Ile Ser Gly Val Gln
65                  70                  75                  80

Cys Asp Asp Ala Ala Thr Tyr Tyr Cys Leu Gly Gly Tyr Asp Gly Asp
                85                  90                  95

Gly Glu Ala Phe Tyr Val Phe Gly Gly Gly Thr Glu Val Val Val Lys
            100                 105                 110

Gly Asp Pro Val Ala Pro Thr Val Leu Ile Phe Pro Pro Ala Ala Asp
        115                 120                 125

Gln Val Ala Thr Gly Thr Val Thr Ile Val Cys Val Ala Asn Lys Tyr
    130                 135                 140

Phe Pro Asp Val Thr Val Thr Trp Glu Val Asp Gly Thr Thr Gln Thr
145                 150                 155                 160

Thr Gly Ile Glu Asn Ser Lys Thr Pro Gln Asn Ser Ala Asp Cys Thr
                165                 170                 175

Tyr Asn Leu Ser Ser Thr Leu Thr Leu Thr Ser Thr Gln Tyr Asn Ser
            180                 185                 190

His Lys Glu Tyr Thr Cys Lys Val Thr Gln Gly Thr Thr Ser Val Val
        195                 200                 205

Gln Ser Phe Asn Arg Gly Asp Cys
    210                 215

<210>  24
<211>  441
<212>  PRT
<213>  Artificial Sequence

<220>
<223>  An artificially synthesized sequence

<400>  24

Gln Glu Gln Leu Glu Glu Ser Gly Gly Asp Leu Val Lys Pro Glu Gly
1               5                   10                  15

Ser Leu Thr Leu Thr Cys Thr Ala Ser Gly Phe Ser Phe Asn Ser Arg
            20                  25                  30

Tyr Trp Ile Cys Trp Val Arg Gln Ala Pro Gly Lys Gly Leu Glu Trp
        35                  40                  45

Ile Gly Cys Ile Val Ser Gly Asp Ser Asn Thr Tyr Tyr Ala Ser Trp
    50                  55                  60

Ala Lys Gly Arg Phe Thr Ile Ser Lys Thr Ser Ser Thr Thr Val Thr
65                  70                  75                  80

Leu Gln Met Thr Ser Leu Thr Ala Ala Asp Thr Ala Thr Tyr Phe Cys
                85                  90                  95

Ala Arg Trp Tyr Thr Ser Ser Arg Pro Gly Leu Trp Gly Pro Gly Thr
            100                 105                 110

Leu Val Thr Val Ser Ser Gly Gln Pro Lys Ala Pro Ser Val Phe Pro
        115                 120                 125

Leu Ala Pro Cys Cys Gly Asp Thr Pro Ser Ser Thr Val Thr Leu Gly
    130                 135                 140

Cys Leu Val Lys Gly Tyr Leu Pro Glu Pro Val Thr Val Thr Trp Asn
145                 150                 155                 160

Ser Gly Thr Leu Thr Asn Gly Val Arg Thr Phe Pro Ser Val Arg Gln
                165                 170                 175

Ser Ser Gly Leu Tyr Ser Leu Ser Ser Val Val Ser Val Thr Ser Ser
            180                 185                 190

Ser Gln Pro Val Thr Cys Asn Val Ala His Pro Ala Thr Asn Thr Lys
        195                 200                 205

Val Asp Lys Thr Val Ala Pro Ser Thr Cys Ser Lys Pro Thr Cys Pro
    210                 215                 220

Pro Pro Glu Leu Leu Gly Gly Pro Ser Val Phe Ile Phe Pro Pro Lys
225                 230                 235                 240

Pro Lys Asp Thr Leu Met Ile Ser Arg Thr Pro Glu Val Thr Cys Val
                245                 250                 255

Val Val Asp Val Ser Gln Asp Asp Pro Glu Val Gln Phe Thr Trp Tyr
            260                 265                 270

Ile Asn Asn Glu Gln Val Arg Thr Ala Arg Pro Pro Leu Arg Glu Gln
        275                 280                 285

Gln Phe Asn Ser Thr Ile Arg Val Val Ser Thr Leu Pro Ile Thr His
    290                 295                 300

Gln Asp Trp Leu Arg Gly Lys Glu Phe Lys Cys Lys Val His Asn Lys
305                 310                 315                 320

Ala Leu Pro Ala Pro Ile Glu Lys Thr Ile Ser Lys Ala Arg Gly Gln
                325                 330                 335

Pro Leu Glu Pro Lys Val Tyr Thr Met Gly Pro Pro Arg Glu Glu Leu
            340                 345                 350

Ser Ser Arg Ser Val Ser Leu Thr Cys Met Ile Asn Gly Phe Tyr Pro
        355                 360                 365

Ser Asp Ile Ser Val Glu Trp Glu Lys Asn Gly Lys Ala Glu Asp Asn
    370                 375                 380

Tyr Lys Thr Thr Pro Ala Val Leu Asp Ser Asp Gly Ser Tyr Phe Leu
385                 390                 395                 400

Tyr Asn Lys Leu Ser Val Pro Thr Ser Glu Trp Gln Arg Gly Asp Val
                405                 410                 415

Phe Thr Cys Ser Val Met His Glu Ala Leu His Asn His Tyr Thr Gln
            420                 425                 430

Lys Ser Ile Ser Arg Ser Pro Gly Lys
        435                 440

<210>  25
<211>  211
<212>  PRT
<213>  Artificial Sequence

<220>
<223>  An artificially synthesized sequence

<400>  25

Asp Val Val Met Thr Gln Thr Pro Ala Ser Val Ser Glu Pro Val Gly
1               5                   10                  15

Gly Thr Val Thr Ile Lys Cys Gln Ala Ser Gln Ser Ile Ser Ser Tyr
            20                  25                  30

Leu Ser Trp Tyr Gln Gln Lys Pro Gly Gln Pro Pro Lys Leu Leu Ile
        35                  40                  45

Tyr Ser Ala Thr Asn Leu Ala Ser Gly Val Ser Ser Arg Phe Lys Gly
    50                  55                  60

Ser Arg Phe Gly Thr Glu Phe Thr Leu Thr Ile Ser Asp Leu Glu Cys
65                  70                  75                  80

Asp Asp Ala Ala Thr Tyr Tyr Cys Gln Cys Ser Tyr Tyr Val Ser Gly
                85                  90                  95

Phe Ala Phe Gly Gly Gly Thr Glu Val Val Val Lys Gly Asp Pro Val
            100                 105                 110

Ala Pro Thr Val Leu Ile Phe Pro Pro Ala Ala Asp Gln Val Ala Thr
        115                 120                 125

Gly Thr Val Thr Ile Val Cys Val Ala Asn Lys Tyr Phe Pro Asp Val
    130                 135                 140

Thr Val Thr Trp Glu Val Asp Gly Thr Thr Gln Thr Thr Gly Ile Glu
145                 150                 155                 160

Asn Ser Lys Thr Pro Gln Asn Ser Ala Asp Cys Thr Tyr Asn Leu Ser
                165                 170                 175

Ser Thr Leu Thr Leu Thr Ser Thr Gln Tyr Asn Ser His Lys Glu Tyr
            180                 185                 190

Thr Cys Lys Val Thr Gln Gly Thr Thr Ser Val Val Gln Ser Phe Asn
        195                 200                 205

Arg Gly Asp
    210

<210>  26
<211>  436
<212>  PRT
<213>  Artificial Sequence

<220>
<223>  An artificially synthesized sequence

<400>  26

Gln Ser Leu Glu Glu Ser Gly Gly Gly Leu Val Lys Pro Gly Gly Thr
1               5                   10                  15

Leu Thr Leu Thr Cys Lys Ala Ser Gly Ile Asp Phe Ser Ser Tyr Tyr
            20                  25                  30

Tyr Met Cys Trp Val Arg Gln Ala Pro Gly Lys Gly Leu Glu Trp Ile
        35                  40                  45

Ala Cys Ile Tyr Ser Gly Asp Gly Ser Ser Tyr Tyr Val Ser Trp Ala
    50                  55                  60

Asn Gly Arg Phe Thr Ile Ser Lys Thr Ser Ser Ala Thr Val Thr Leu
65                  70                  75                  80

Gln Met Thr Ser Leu Thr Ala Ala Asp Thr Ala Thr Tyr Phe Cys Ala
                85                  90                  95

Arg Asp Leu Gly Asn Leu Trp Gly Pro Gly Thr Leu Val Thr Val Ser
            100                 105                 110

Ser Gly Gln Pro Lys Ala Pro Ser Val Phe Pro Leu Ala Pro Cys Cys
        115                 120                 125

Gly Asp Thr Pro Ser Ser Thr Val Thr Leu Gly Cys Leu Val Lys Gly
    130                 135                 140

Tyr Leu Pro Glu Pro Val Thr Val Thr Trp Asn Ser Gly Thr Leu Thr
145                 150                 155                 160

Asn Gly Val Arg Thr Phe Pro Ser Val Arg Gln Ser Ser Gly Leu Tyr
                165                 170                 175

Ser Leu Ser Ser Val Val Ser Val Thr Ser Ser Ser Gln Pro Val Thr
            180                 185                 190

Cys Asn Val Ala His Pro Ala Thr Asn Thr Lys Val Asp Lys Thr Val
        195                 200                 205

Ala Pro Ser Thr Cys Ser Lys Pro Thr Cys Pro Pro Pro Glu Leu Leu
    210                 215                 220

Gly Gly Pro Ser Val Phe Ile Phe Pro Pro Lys Pro Lys Asp Thr Leu
225                 230                 235                 240

Met Ile Ser Arg Thr Pro Glu Val Thr Cys Val Val Val Asp Val Ser
                245                 250                 255

Gln Asp Asp Pro Glu Val Gln Phe Thr Trp Tyr Ile Asn Asn Glu Gln
            260                 265                 270

Val Arg Thr Ala Arg Pro Pro Leu Arg Glu Gln Gln Phe Asn Ser Thr
        275                 280                 285

Ile Arg Val Val Ser Thr Leu Pro Ile Thr His Gln Asp Trp Leu Arg
    290                 295                 300

Gly Lys Glu Phe Lys Cys Lys Val His Asn Lys Ala Leu Pro Ala Pro
305                 310                 315                 320

Ile Glu Lys Thr Ile Ser Lys Ala Arg Gly Gln Pro Leu Glu Pro Lys
                325                 330                 335

Val Tyr Thr Met Gly Pro Pro Arg Glu Glu Leu Ser Ser Arg Ser Val
            340                 345                 350

Ser Leu Thr Cys Met Ile Asn Gly Phe Tyr Pro Ser Asp Ile Ser Val
        355                 360                 365

Glu Trp Glu Lys Asn Gly Lys Ala Glu Asp Asn Tyr Lys Thr Thr Pro
    370                 375                 380

Ala Val Leu Asp Ser Asp Gly Ser Tyr Phe Leu Tyr Asn Lys Leu Ser
385                 390                 395                 400

Val Pro Thr Ser Glu Trp Gln Arg Gly Asp Val Phe Thr Cys Ser Val
                405                 410                 415

Met His Glu Ala Leu His Asn His Tyr Thr Gln Lys Ser Ile Ser Arg
            420                 425                 430

Ser Pro Gly Lys
        435

<210>  27
<211>  216
<212>  PRT
<213>  Artificial Sequence

<220>
<223>  An artificially synthesized sequence

<400>  27

Gln Val Leu Thr Gln Thr Pro Ala Ser Val Ser Ala Ala Val Gly Gly
1               5                   10                  15

Thr Val Thr Ile Asn Cys Gln Ala Ser Gln Ser Val Tyr Lys Asn Ser
            20                  25                  30

Gly Leu Ser Trp Phe Gln Gln Lys Pro Gly Gln Ser Pro Lys Arg Leu
        35                  40                  45

Ile Tyr Ser Ala Ser Thr Leu Asp Ser Gly Val Pro Ser Arg Phe Lys
    50                  55                  60

Gly Ser Gly Ser Gly Thr Gln Phe Thr Leu Thr Ile Ser Asp Val Gln
65                  70                  75                  80

Cys Asp Asp Ala Ala Thr Tyr Tyr Cys Leu Gly Ile Tyr Val Cys Ser
                85                  90                  95

Ser Val Asp Cys Gly Ala Phe Gly Gly Gly Thr Glu Val Val Val Lys
            100                 105                 110

Gly Asp Pro Val Ala Pro Thr Val Leu Ile Phe Pro Pro Ala Ala Asp
        115                 120                 125

Gln Val Ala Thr Gly Thr Val Thr Ile Val Cys Val Ala Asn Lys Tyr
    130                 135                 140

Phe Pro Asp Val Thr Val Thr Trp Glu Val Asp Gly Thr Thr Gln Thr
145                 150                 155                 160

Thr Gly Ile Glu Asn Ser Lys Thr Pro Gln Asn Ser Ala Asp Cys Thr
                165                 170                 175

Tyr Asn Leu Ser Ser Thr Leu Thr Leu Thr Ser Thr Gln Tyr Asn Ser
            180                 185                 190

His Lys Glu Tyr Thr Cys Lys Val Thr Gln Gly Thr Thr Ser Val Val
        195                 200                 205

Gln Ser Phe Asn Arg Gly Asp Cys
    210                 215

<210>  28
<211>  441
<212>  PRT
<213>  Artificial Sequence

<220>
<223>  An artificially synthesized sequence

<400>  28

Gln Glu Gln Leu Glu Glu Ser Gly Gly Asp Leu Val Lys Pro Glu Gly
1               5                   10                  15

Ser Leu Thr Leu Thr Cys Thr Ala Ser Gly Gly Ser Phe Ser Ser Arg
            20                  25                  30

Tyr Trp Met Cys Trp Val Arg Gln Ala Pro Gly Lys Gly Leu Glu Trp
        35                  40                  45

Ile Ala Cys Ile Val Ile Gly Asp Arg Asn Asn Tyr Tyr Ala Ser Trp

50                  55                  60

Ala Lys Gly Arg Phe Thr Ile Ser Lys Thr Ser Ser Thr Thr Val Thr
65                  70                  75                  80

Leu Gln Met Thr Ser Leu Thr Ala Ala Asp Thr Ala Thr Tyr Phe Cys
                85                  90                  95

Ala Arg Trp His Ser Ser Thr Ala Pro Ser Leu Trp Gly Pro Gly Thr
            100                 105                 110

Leu Val Thr Val Ser Ser Gly Gln Pro Lys Ala Pro Ser Val Phe Pro
        115                 120                 125

Leu Ala Pro Cys Cys Gly Asp Thr Pro Ser Ser Thr Val Thr Leu Gly
    130                 135                 140

Cys Leu Val Lys Gly Tyr Leu Pro Glu Pro Val Thr Val Thr Trp Asn
145                 150                 155                 160

Ser Gly Thr Leu Thr Asn Gly Val Arg Thr Phe Pro Ser Val Arg Gln
                165                 170                 175

Ser Ser Gly Leu Tyr Ser Leu Ser Ser Val Val Ser Val Thr Ser Ser
            180                 185                 190

Ser Gln Pro Val Thr Cys Asn Val Ala His Pro Ala Thr Asn Thr Lys
        195                 200                 205

Val Asp Lys Thr Val Ala Pro Ser Thr Cys Ser Lys Pro Thr Cys Pro
    210                 215                 220

Pro Pro Glu Leu Leu Gly Gly Pro Ser Val Phe Ile Phe Pro Pro Lys
225                 230                 235                 240

Pro Lys Asp Thr Leu Met Ile Ser Arg Thr Pro Glu Val Thr Cys Val
                245                 250                 255

Val Val Asp Val Ser Gln Asp Asp Pro Glu Val Gln Phe Thr Trp Tyr
            260                 265                 270

Ile Asn Asn Glu Gln Val Arg Thr Ala Arg Pro Pro Leu Arg Glu Gln
        275                 280                 285

Gln Phe Asn Ser Thr Ile Arg Val Val Ser Thr Leu Pro Ile Thr His
    290                 295                 300

Gln Asp Trp Leu Arg Gly Lys Glu Phe Lys Cys Lys Val His Asn Lys
305                 310                 315                 320

Ala Leu Pro Ala Pro Ile Glu Lys Thr Ile Ser Lys Ala Arg Gly Gln
                325                 330                 335

Pro Leu Glu Pro Lys Val Tyr Thr Met Gly Pro Pro Arg Glu Glu Leu
            340                 345                 350

Ser Ser Arg Ser Val Ser Leu Thr Cys Met Ile Asn Gly Phe Tyr Pro
        355                 360                 365

Ser Asp Ile Ser Val Glu Trp Glu Lys Asn Gly Lys Ala Glu Asp Asn
    370                 375                 380

Tyr Lys Thr Thr Pro Ala Val Leu Asp Ser Asp Gly Ser Tyr Phe Leu
385                 390                 395                 400

Tyr Asn Lys Leu Ser Val Pro Thr Ser Glu Trp Gln Arg Gly Asp Val
                405                 410                 415

Phe Thr Cys Ser Val Met His Glu Ala Leu His Asn His Tyr Thr Gln
            420                 425                 430

Lys Ser Ile Ser Arg Ser Pro Gly Lys
        435                 440

<210>  29
<211>  212
<212>  PRT
<213>  Artificial Sequence

<220>
<223>  An artificially synthesized sequence

<400>  29

Asp Val Val Met Thr Gln Thr Pro Ala Ser Val Ser Glu Pro Val Gly
1               5                   10                  15

Gly Thr Val Thr Ile Asn Cys Gln Ala Ser Gln Ser Ile Ser Ser Tyr
            20                  25                  30

Leu Ser Trp Tyr Gln Gln Lys Pro Gly Gln Pro Pro Lys Leu Leu Ile
        35                  40                  45

Tyr Lys Ala Ser Thr Leu Ala Ser Gly Val Ser Ser Arg Phe Lys Gly
    50                  55                  60

Ser Gly Ser Gly Thr Glu Phe Thr Leu Thr Ile Ser Asp Leu Glu Cys

65                  70                  75                  80

Ala Asp Ala Ala Thr Tyr Tyr Cys Gln Cys Ser Tyr Tyr Val Ser Gly
                85                  90                  95

Phe Val Phe Gly Gly Gly Thr Glu Val Val Val Lys Gly Asp Pro Val
            100                 105                 110

Ala Pro Thr Val Leu Ile Phe Pro Pro Ala Ala Asp Gln Val Ala Thr
        115                 120                 125

Gly Thr Val Thr Ile Val Cys Val Ala Asn Lys Tyr Phe Pro Asp Val
    130                 135                 140

Thr Val Thr Trp Glu Val Asp Gly Thr Thr Gln Thr Thr Gly Ile Glu
145                 150                 155                 160

Asn Ser Lys Thr Pro Gln Asn Ser Ala Asp Cys Thr Tyr Asn Leu Ser
                165                 170                 175

Ser Thr Leu Thr Leu Thr Ser Thr Gln Tyr Asn Ser His Lys Glu Tyr
            180                 185                 190

Thr Cys Lys Val Thr Gln Gly Thr Thr Ser Val Val Gln Ser Phe Asn
        195                 200                 205

Arg Gly Asp Cys
    210

<210>  30
<211>  13
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  30

Xaa Asn Phe Asn Ser Ala Tyr Tyr Arg Ala Ile Trp Asp
1               5                   10

<210>  31
<211>  13
<212>  PRT
<213>  Artificial Sequence

<220>
<223>  An artificially synthesized sequence


<220>
<221>  MISC_FEATURE
<222>  (1)..(1)
<223>  Xaa = D-MeAla

<400>  31

Xaa Ser Lys Tyr Trp Asn Ser Trp Tyr Asn Ile Trp Asp
1               5                   10


<210>  32
<211>  13
<212>  PRT
<213>  Artificial Sequence

<220>
<223>  An artificially synthesized sequence


<220>
<221>  MISC_FEATURE
<222>  (1)..(1)
<223>  Xaa = D-MeAla

<400>  32

Xaa Thr Tyr Gly His Ser Ser Trp Tyr Lys Ile Trp Asp
1               5                   10


<210>  33
<211>  13
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  33

Xaa Asn Tyr Gln Ser Trp Tyr Tyr Arg Ser Ile Trp Asp
1               5                   10


<210>  34
<211>  12
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  34

Xaa Lys Phe Ala Gln Phe Arg Trp Ile Tyr Pro Asp
1               5                   10


<210>  35
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  35

Xaa Arg Tyr Ala Val Tyr Gln Trp Ile Tyr Pro Asp
1               5                   10


<210>  36
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  36

Xaa Arg Phe Arg Gln Trp Lys Trp Val Tyr Pro Asp
1               5                   10


<210>  37
<211>  12
<212>  PRT
<213>  Artificial Sequence

<220>
<223>  An artificially synthesized sequence


<220>

<221>  MISC_FEATURE
<222>  (1)..(1)
<223>  Xaa = MeAla

<400>  37

Xaa Arg Phe Gly Val Trp Ser Trp Ile Tyr Pro Asp
1               5                   10


<210>  38
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  38

Xaa Asn Ser Trp Tyr Ile Asn Ile Trp Gly His Asp
1               5                   10


<210>  39
<211>  12
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  39

Xaa Asn Phe Ser Asn Ser Trp Tyr Asn Ile Trp Asp
1               5                   10


<210>  40
<211>  11
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  40

Xaa Asp Ile Trp Phe Asp Tyr Trp Asp Trp Asp
1               5                   10


<210>  41
<211>  11
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  41

Xaa Asp Ile Trp Phe Asp Tyr Trp Asp Tyr Asp
1               5                   10


<210>  42
<211>  11
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  42

Xaa Val Phe Tyr Lys Ile Trp Tyr Asn Trp Asp
1               5                   10


<210>  43
<211>  11
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  43

Xaa Lys Phe Trp Ile His His Ser Phe Glu Asp
1               5                   10

<210>  44
<211>  11
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  44

Xaa Asn Ser Trp Tyr Lys Ile Trp Gly Leu Asp
1               5                   10

<210>  45
<211>  11
<212>  PRT
<213>  Artificial Sequence

<220>
<223>  An artificially synthesized sequence

<220>
<221>  MISC_FEATURE
<222>  (1)..(1)
<223>  Xaa = MeAla

<400>  45

Xaa Asn Ser Trp Tyr Arg Ile Trp Gly Leu Asp
1               5                   10

<210>  46
<211>  11
<212>  PRT
<213>  Artificial Sequence

<220>
<223>  An artificially synthesized sequence

<220>
<221>  MISC_FEATURE
<222>  (1)..(1)
<223>  Xaa = D-MeAla

<400>  46

Xaa Pro Thr Ser Ser Trp Tyr Lys Ile Trp Asp
1               5                   10

<210>  47
<211>  10
<212>  PRT
<213>  Artificial Sequence

<220>
<223>  An artificially synthesized sequence


<220>
<221>  MISC_FEATURE
<222>  (1)..(1)
<223>  Xaa = MeAla

<400>  47

Xaa Trp Ile Trp Asp Phe Trp Asn Glu Asp
1               5                   10


<210>  48
<211>  10
<212>  PRT
<213>  Artificial Sequence

<220>
<223>  An artificially synthesized sequence


<220>
<221>  MISC_FEATURE
<222>  (1)..(1)
<223>  Xaa = D-MeAla

<400>  48

Xaa Glu Asp Thr Pro Glu Asp Asp His Asp
1               5                   10


<210>  49
<211>  5
<212>  PRT
<213>  Artificial Sequence

<220>
<223>  An artificially synthesized sequence

<400>  49

Leu Pro Met Thr Gly
1               5


<210>  50
<211>  22
<212>  PRT
<213>  Artificial Sequence

<220>
<223>  An artificially synthesized sequence

<400>  50

Cys Leu Asp Ser Gly Ile Gly Ser Leu Glu Ser Gln Met Ser Glu Leu
1               5                   10                  15

Trp Gly Val Arg Gly Gly
            20

<210>  51
<211>  19
<212>  PRT
<213>  Artificial Sequence

<220>
<223>  An artificially synthesized sequence

<220>
<221>  MOD_RES
<222>  (19)..(19)
<223>  AMIDATION

<400>  51

Ala Phe Pro Pro Arg Glu Tyr Trp Ser Glu Pro Tyr Pro Leu Pro Pro
1               5                   10                  15

Pro Thr Cys

<210>  52
<211>  20
<212>  DNA
<213>  Artificial Sequence

<220>
<223>  An artificially synthesized sequence

<400>  52
gtgggtgggg gtaatgggta                                                   20

<210>  53
<211>  18
<212>  DNA
<213>  Artificial Sequence

<220>
<223>  An artificially synthesized sequence

<400>  53
cctacccatc cagagtac                                                     18

<210>  54
<211>  103
<212>  DNA
<213>  Artificial Sequence

<220>
<223>  An artificially synthesized sequence

<400>  54
ccaccgacta tgtgcccccg ccacccacct acccatccag agagtattgg gctgagccgt      60

atccattacc cccacccact cctgtccttc aggagcccca gag                        103


<210>  55
<211>  22
<212>  PRT
<213>  Artificial Sequence

<220>
<223>  An artificially synthesized sequence

<400>  55

Phe Asn Asn Phe Thr Val Ser Phe Trp Leu Arg Val Pro Lys Val Ser
1               5                   10                  15


Ala Ser His Leu Glu Gly
            20


<210>  56
<211>  14
<212>  PRT
<213>  Artificial Sequence

<220>
<223>  An artificially synthesized sequence


<220>
<221>  MISC_FEATURE
<222>  (8)..(8)
<223>  Xaa = MePhe

<220>
<221>  MISC_FEATURE
<222>  (12)..(12)
<223>  Xaa = MePhe

<400>  56

Thr Gly Thr Gly Thr Gly Thr Xaa Pro Ile Thr Xaa Pro Ile
1               5                   10


<210>  57
<211>  30
<212>  PRT
<213>  Artificial Sequence

<220>
<223>  An artificially synthesized sequence


<220>
<221>  MISC_FEATURE

<222>  (1)..(1)
<223>  Xaa = MeAla

<220>
<221>  MISC_FEATURE
<222>  (14)..(14)
<223>  Xaa = bMeAla

<220>
<221>  MISC_FEATURE
<222>  (24)..(24)
<223>  Xaa = MePhe

<220>
<221>  MISC_FEATURE
<222>  (28)..(28)
<223>  Xaa = MePhe

<400>  57

Xaa Asn Phe Asn Ser Ala Tyr Tyr Arg Ala Ile Trp Asp Xaa Gly Gly
1               5                   10                  15

Thr Gly Thr Gly Thr Gly Thr Xaa Pro Ile Thr Xaa Pro Ile
            20                  25                  30

<210>  58
<211>  30
<212>  PRT
<213>  Artificial Sequence

<220>
<223>  An artificially synthesized sequence

<220>
<221>  MISC_FEATURE
<222>  (1)..(1)
<223>  Xaa = MeAla

<220>
<221>  MISC_FEATURE
<222>  (14)..(14)
<223>  Xaa = bMeAla

<220>
<221>  MISC_FEATURE
<222>  (24)..(24)
<223>  Xaa = MePhe

<220>
<221>  MISC_FEATURE
<222>  (28)..(28)
<223>  Xaa = MePhe

<400>  58

Xaa Asn Tyr Gln Ser Trp Tyr Tyr Arg Ser Ile Trp Asp Xaa Gly Gly
1               5                   10                  15

Thr Gly Thr Gly Thr Gly Thr Xaa Pro Ile Thr Xaa Pro Ile
            20                  25                  30

<210>  59
<211>  28
<212>  PRT
<213>  Artificial Sequence

<220>
<223>  An artificially synthesized sequence

<220>
<221>  MISC_FEATURE
<222>  (1)..(1)
<223>  Xaa = D-MeAla

<220>
<221>  MISC_FEATURE
<222>  (12)..(12)
<223>  Xaa = bMeAla

<220>
<221>  MISC_FEATURE
<222>  (22)..(22)
<223>  Xaa = MePhe

<220>
<221>  MISC_FEATURE
<222>  (26)..(26)
<223>  Xaa = MePhe

<400>  59

Xaa Pro Thr Ser Ser Trp Tyr Lys Ile Trp Asp Xaa Gly Gly Thr Gly
1               5                   10                  15

Thr Gly Thr Gly Thr Xaa Pro Ile Thr Xaa Pro Ile
            20                  25

<210>  60
<211>  16
<212>  PRT
<213>  Artificial Sequence

<220>
<223>  An artificially synthesized sequence

<220>
<221>  MISC_FEATURE
<222>  (10)..(10)
<223>  Xaa = MePhe

<220>
<221>  MISC_FEATURE
<222>  (14)..(14)
<223>  Xaa = MePhe

<400>  60

Gly Gly Thr Gly Thr Gly Thr Gly Thr Xaa Pro Ile Thr Xaa Pro Ile
1               5                   10                  15

<210>  61
<211>  4
<212>  PRT
<213>  Artificial Sequence

<220>
<223>  An artificially synthesized sequence

<220>
<221>  MISC_FEATURE
<222>  (2)..(2)
<223>  Xaa = MePhe

<400>  61

Thr Xaa Pro Ile
1

<210>  62
<211>  4
<212>  PRT
<213>  Artificial Sequence

<220>
<223>  An artificially synthesized sequence

<400>  62

Gly Gly Gly Ser
1